(19) Europäisches Patentamt
European Patent Office
Office européen des brevets

(11) EP 4 289 835 A1

(12) **EUROPEAN PATENT APPLICATION**
published in accordance with Art. 153(4) EPC

(43) Date of publication:
**13.12.2023 Bulletin 2023/50**

(21) Application number: **22749072.9**

(22) Date of filing: **28.01.2022**

(51) International Patent Classification (IPC):
***C07D 401/14*** *(2006.01)* ***C07D 403/12*** *(2006.01)*
***A61K 31/506*** *(2006.01)* ***A61P 35/00*** *(2006.01)*

(52) Cooperative Patent Classification (CPC):
**A61K 31/506; A61P 35/00; C07D 401/14; C07D 403/12**

(86) International application number:
**PCT/CN2022/074491**

(87) International publication number:
**WO 2022/166793 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.02.2021 CN 202110161786**
**30.04.2021 CN 202110483256**
**10.09.2021 CN 202111062178**
**19.11.2021 CN 202111398260**
**17.01.2022 CN 202210048365**

(71) Applicant: **Shanghai Qilu Pharmaceutical Research and Development Centre Ltd.**
**Shanghai 201203 (CN)**

(72) Inventors:
- **YAN, Xiaoxia**
  **Shanghai 201203 (CN)**
- **SUN, Daqing**
  **Shanghai 201203 (CN)**

(74) Representative: **Hoffmann Eitle**
**Patent- und Rechtsanwälte PartmbB**
**Arabellastraße 30**
**81925 München (DE)**

(54) **CDK INHIBITOR**

(57) A compound serving as a selective CDK inhibitor, a pharmaceutical composition containing same, a useful intermediate for preparing the compound, and a use of the compound for preparing a drug for the treatment of cell proliferative diseases, such as cancer. The compound has the structure shown in formula (I-A).

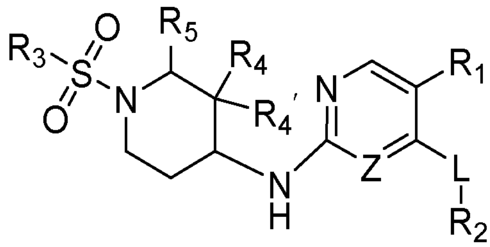

(I-A)

EP 4 289 835 A1

## Description

The present application claims the priorities of Chinese patent application 202110161786.5 filed on February 5, 2021, Chinese patent application 202110483256.2 filed on April 30, 2021, Chinese patent application 202111062178.5 filed on September 10, 2021, Chinese patent application 202111398260.5 filed on November 19, 2021 and Chinese patent application 202210048365.6 filed on January 17, 2022. The contents of the above Chinese patent applications are incorporated herein by reference in its entirety.

TECHNICAL FIELD

The present disclosure belongs to the field of pharmaceutical chemistry, and particularly relates to a novel compound with CDK2/4/6 inhibitory activity, a pharmaceutical composition containing the compound, a useful intermediate for preparing the compound, and a method for treating cell proliferative diseases (such as cancer) using the compound of the present disclosure.

BACKGROUND

The cell cycle is the basic process of cell life activities, which controls the growth, proliferation, and differentiation of cells. Cyclin-dependent kinases (CDKs) are a class of important cellular enzymes that cooperate with cyclins and play an important role in the regulation of the cell cycle. Cyclin B/CDK1, cyclin A/CDK2, cyclin E/CDK2, cyclin D/CDK4, cyclin D/CDK6 and other possible heterodimers are important regulatory factors of different phases of the cell cycle (Harper, J. W., Adams, P. D., Cyclin-Dependent Kinases, Chem. Rev. 2001, 101, 2511-2526).

Serving as an important regulatory factor in the cell cycle, CDK2 forms a kinase complex with cyclin E or cyclin A, and plays a decisive role in the process of driving the cell cycle from G1 phase to S phase and maintaining S phase. The main mechanism is that cyclin E and CDK2 work together to phosphorylate the retinoblastoma susceptibility gene (Rb) protein. The phosphorylation of the Rb protein leads to the release of E2F (a transcription factor). The released E2F binds to the upstream of some genes (usually locates in the promoter region or enhancer region), initiating the transcriptional expression of those genes related to the cell cycle, so that the cells enter the S phase at the end of G1. Many studies have shown that the abnormal expression of CDK2 is closely related to the occurrence of cancer, such as ovarian cancer with CCNE1 amplification, KRAS mutant lung cancer, hormone-dependent breast and prostate cancer, etc. (Tadesse S, Anshabo AT, Portman N, Lim E, Tilley W, Caldon CE, Wang S, Targeting CDK2 in cancer: challenges and opportunities for therapy, Drug Discovery Today, 2020, 25, 406-413).

As the important role of cyclin-dependent kinases (CDKs) in cell cycle regulation has been identified, CDK inhibitors have become the current research hotspot of anti-tumor drugs. At present, several CDK inhibitors have been approved for marketing in the world, but most of them act on CDK4/6 targets, mainly targeting breast cancer, such as Palbociclib from Pfizer, Ribociclib from Novartis and abemaciclib from Eli Lilly. Multi-target inhibitors including fadraciclib, Roscovitine, and PF-06873600 that target CDK2 are in different clinical stages. Currently, no CDK2 inhibitor has been approved for marketing. Therefore, It is of great research significance to continue to develop novel CDK inhibitors, especially effective for CDK2 targets.

CONTENT OF THE PRESENT INVENTION

The present disclosure aims to provide a class of novel compounds with CDK2/4/6 inhibitory activity, a pharmaceutical composition containing the compound, a useful intermediate for preparing the compound, and a use of the compound in the manufacture of a medicament for the treatment of cancer.

The present disclosure provides a compound of formula (I-A),

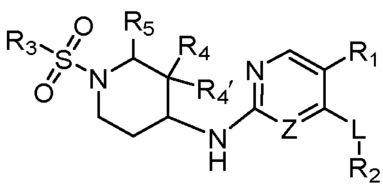

( I-A)

a stereoisomer thereof, a tautomer thereof, a pharmaceutically acceptable salt thereof, a prodrug thereof, a hydrate thereof, a solvate thereof, or an isotope-labeled derivative thereof,

wherein

$R_1$ is selected from halogen, -CN, $-NO_2$ or $C_{1-4}$ haloalkyl;

Z is selected from -CH- or N;

L is selected from a bond, $-NR_a-$, -O-, -S-, $-SO_2-$, -SO-, -CO-, $-CR_aR_b-$ or -CH=, and the $R_a$ and $R_b$ are optionally and independently selected from H, $C_{1-4}$ alkyl, $C_{1-4}$ haloalkyl, $C_{3-6}$ cycloalkyl, 3- to 6-membered heterocycloalkyl, $-SO_2R_c$, $-SOR_c$, $-COR_c$, $-(CH_2)_mNR_{aa}R_{ab}$ or - $(CH_2)_mC(O)NR_{aa}R_{ab}$, wherein the $R_c$ is selected from H, $C_{1-4}$ alkyl, and the $R_{aa}$ and $R_{ab}$ are optionally and independently selected from H, $C_{1-4}$ alkyl, $C_{1-4}$ haloalkyl, $C_{3-6}$ cycloalkyl, 3- to 6-membered heterocycloalkyl, or $R_{aa}$ and $R_{ab}$ together with the N atom to which they are attached form a 4- to 6-membered heterocycloalkyl;

$R_2$ is selected from $C_{1-4}$ alkyl, $C_{3-6}$ cycloalkyl, 3- to 6-membered heterocycloalkyl, aryl or 5- to 6-membered heteroaryl, and the $C_{1-4}$ alkyl, $C_{3-6}$ cycloalkyl, 3- to 6-membered heterocycloalkyl, aryl and 5- to 6-membered heteroaryl are optionally substituted by one or more than one $R_d$; the $R_d$ is optionally and independently selected from H, halogen, $C_{1-4}$ alkyl, $C_{1-4}$ haloalkyl, $C_{1-4}$ alkoxy, $-(CH_2)_mOH$, $-(CH_2)_mNR_eR_f$, $C_{3-6}$ cycloalkyl or 3- to 6-membered heterocycloalkyl; the $C_{3-6}$ cycloalkyl and 3- to 6-membered heterocycloalkyl in $R_d$ are optionally substituted by $C_{1-4}$ alkyl, $C_{1-4}$ haloalkyl, -OH or $-NH_2$;

$R_3$ is selected from $C_{1-4}$ alkyl, $C_{1-4}$ haloalkyl, $-(CH_2)_mNR_eR_f$, $-(CH_2)_mOH$, $-L_1$-aryl, - $L_1$-(5- to 6-membered heteroaryl), $-L_1$-($C_{3-6}$ cycloalkyl) or $-L_1$-(3- to 6-membered heterocycloalkyl), and the aryl, 5- to 6-membered heteroaryl, $C_{3-6}$ cycloalkyl and 3- to 6-membered heterocycloalkyl are optionally substituted by one or more than one $R_g$, and the $R_g$ can be $R_{ga}$ or $R_{gb}$;

$R_{ga}$ is optionally and independently selected from H, halogen, -OH, $C_{1-4}$ alkyl, $C_{1-4}$ haloalkyl, $-(CH_2)_mNR_eR_f$, $R_eR_f$-NC(O)-$C_{1-4}$ alkyl-, -$C_{1-4}$ alkyl-OH, $-S(O)_2$-(5- to 6-membered heteroaryl) or $C_{1-4}$ alkoxy;

$R_{gb}$ is optionally and independently selected from $-L_2$-($C_{3-6}$ cycloalkyl), $-L_2$-(3- to 6-membered heterocycloalkyl), $-L_2$-(3- to 6-membered heterocycloalkenyl), $-L_2$-aryl, $-L_2$-(5- to 6-membered heteroaryl), $-L_2$-(7- to 11-membered spiroheterocyclyl), $-L_2$-(6- to 14-membered fused heterocyclyl), wherein the $C_{3-6}$ cycloalkyl, 3- to 6-membered heterocycloalkyl, 3- to 6-membered heterocycloalkenyl, aryl, 5- to 6-membered heteroaryl, 7- to 11-membered spiroheterocyclyl, $-L_2$-(6- to 14-membered fused heterocyclyl) are optionally substituted by one or more than one $R_{gc}$, and the $R_{gc}$ is optionally and independently selected from $C_{1-4}$ alkyl, halogen, $C_{1-4}$ haloalkyl, $-(CH_2)_mNR_eR_f$, $-(CH_2)_mOH$ or cyano, or any two $R_{gc}$ are connected to form a $C_{1-2}$ alkylene chain;

$L_1$ is a bond or $L_1$ is optionally and independently selected from $C_{1-4}$ alkylene;

$L_2$ is a bond or $L_2$ is optionally and independently selected from $C_{1-4}$ alkylene or NH;

$R_e$ and Rf are optionally and independently selected from H or $C_{1-4}$ alkyl;

m is optionally and independently selected from 0, 1, 2, 3 or 4;

$R_4$, $R_4$' and $R_5$ are optionally and independently selected from H, OH, halogen, $C_{1-4}$ alkyl, $C_{1-4}$ haloalkyl or $C_{1-4}$ alkoxy; and when L is the bond, $R_2$ is not

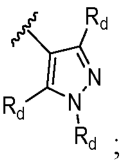

when L is -NH-, $R_2$ is not

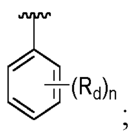

and when the compound of formula (I) is

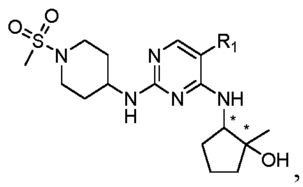

$R_1$ is not halogen.

In some embodiments of the present disclosure, the compound of formula (I-A), the stereoisomer thereof, the tautomer thereof, the pharmaceutically acceptable salt thereof, the prodrug thereof, the hydrate thereof, the solvate thereof or the isotope-labeled derivative thereof, wherein the compound can be represented by formula (I-B),

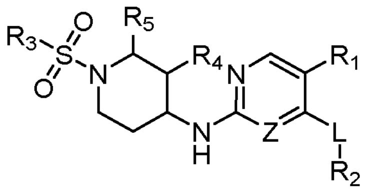

( I-B )

wherein

$R_1$ is selected from halogen, -CN, $-NO_2$ or $C_{1-4}$ haloalkyl;
Z is selected from -CH- or N;
L is selected from a bond, $-NR_a-$, -O-, -S-, $-SO_2-$, -SO-, -CO-, $-CR_aR_b-$ or -CH=, and the $R_a$ and $R_b$ are optionally and independently selected from H, $C_{1-4}$ alkyl, $C_{1-4}$ haloalkyl, $C_{3-6}$ cycloalkyl, 3- to 6-membered heterocycloalkyl, $-SO_2R_c$, $-SOR_c$, $-COR_c$, $-(CH_2)_mNR_{aa}R_{ab}$ or - $(CH_2)_mC(O)NR_{aa}R_{ab}$, wherein the $R_c$ is selected from H, $C_{1-4}$ alkyl, and the $R_{aa}$ and $R_{ab}$ are optionally and independently selected from H, $C_{1-4}$ alkyl, $C_{1-4}$ haloalkyl, $C_{3-6}$ cycloalkyl, 3- to 6-membered heterocycloalkyl, or $R_{aa}$ and $R_{ab}$ together with the N atom to which they are attached form a 4- to 6-membered heterocycloalkyl;
$R_2$ is selected from $C_{1-4}$ alkyl, $C_{3-6}$ cycloalkyl, 3- to 6-membered heterocycloalkyl, aryl or 5- to 6-membered heteroaryl, and the $C_{1-4}$ alkyl, $C_{3-6}$ cycloalkyl, 3- to 6-membered heterocycloalkyl, aryl and 5- to 6-membered heteroaryl are optionally substituted by one or more than one $R_d$; the $R_d$ is optionally and independently selected from H, halogen, $C_{1-4}$ alkyl, $C_{1-4}$ haloalkyl, $C_{1-4}$ alkoxy, $-(CH_2)_mOH$, $-(CH_2)_mNR_eR_f$, $C_{3-6}$ cycloalkyl or 3- to 6-membered heterocycloalkyl; the $C_{3-6}$ cycloalkyl and 3- to 6-membered heterocycloalkyl in $R_d$ are optionally substituted by $C_{1-4}$ alkyl, $C_{1-4}$ haloalkyl, -OH or $-NH_2$;
$R_3$ is selected from $C_{1-4}$ alkyl, $C_{1-4}$ haloalkyl, $-(CH_2)_mNR_eR_f$, $-(CH_2)_mOH$, $-L_1$-aryl, - $L_1$-(5- to 6-membered heteroaryl), $-L_1$-($C_{3-6}$ cycloalkyl) or $-L_1$-(3- to 6-membered heterocycloalkyl), and the aryl, 5- to 6-membered heteroaryl, $C_{3-6}$ cycloalkyl and 3- to 6-membered heterocycloalkyl are optionally substituted by one or more than one $R_g$, and the $R_g$ is $R_{ga}$ or $R_{gb}$;
$R_{ga}$ is optionally and independently selected from H, halogen, -OH, $C_{1-4}$ alkyl, $C_{1-4}$ haloalkyl, $-(CH_2)_mNR_eR_f$, $R_eR_f$-NC(O)-$C_{1-4}$ alkyl-, -$C_{1-4}$ alkyl-OH, $-S(O)_2$-(5- to 6-membered heteroaryl) or $C_{1-4}$ alkoxy;
$R_{gb}$ is optionally and independently selected from $-L_2$-($C_{3-6}$ cycloalkyl), $-L_2$-(3- to 6-membered heterocycloalkyl), $-L_2$-(3- to 6-membered heterocycloalkenyl), $-L_2$-aryl, $-L_2$-(5- to 6-membered heteroaryl), $-L_2$-(7- to 11-membered spiroheterocyclyl), $-L_2$-(6- to 14-membered fused heterocyclyl), wherein the $C_{3-6}$ cycloalkyl, 3- to 6-membered heterocycloalkyl, 3- to 6-membered heterocycloalkenyl, aryl, 5- to 6-membered heteroaryl, 7- to 11-membered spiroheterocyclyl, 6- to 14-membered fused heterocyclyl in $R_{gb}$ are optionally substituted by one or more than one $R_{gc}$;
$R_{gc}$ is optionally and independently selected from $C_{1-4}$ alkyl, halogen, $C_{1-4}$ haloalkyl, $-(CH_2)_mNR_eR_f$, $-(CH_2)_mOH$ or cyano;
or any two $R_{gc}$ are connected to form a $C_{1-2}$ alkylene chain;
$L_1$ is a bond or $L_1$ is optionally and independently selected from $C_{1-4}$ alkylene;
$L_2$ is a bond or $L_2$ is optionally and independently selected from $C_{1-4}$ alkylene or NH;
$R_e$ and Rf are optionally and independently selected from H or $C_{1-4}$ alkyl;
m is optionally and independently selected from 0, 1, 2, 3 or 4;
$R_4$ and $R_5$ are optionally and independently selected from H, OH, halogen, $C_{1-4}$ alkyl, $C_{1-4}$ haloalkyl or $C_{1-4}$ alkoxy.

In some embodiments of the present disclosure, $R_1$ is selected from -CN, $-CF_3$ or - $CHF_2$.
In some embodiments of the present disclosure, $R_1$ is selected from -CN or $-CF_3$.
In some embodiments of the present disclosure, $R_1$ is selected from $-CF_3$.
In some embodiments of the present disclosure, $R_1$ is selected from -CN.
In some embodiments of the present disclosure, Z is selected from N.
In some embodiments of the present disclosure, L is selected from -NH-, $-N(CH_3)-$, - O-, $-CH_2-$, CH=, $-N(CH(CH)_2)-$ (i.e.,

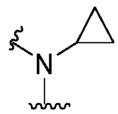

), $-N(SO_2CH_3)-$, $-SO_2-$, $-SO-$, $-N(CH_2CF_3)-$,

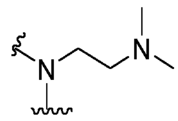 or 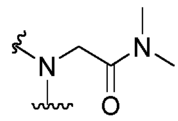 .

In some embodiments of the present disclosure, L is selected from the bond or -S-.

In some embodiments of the present disclosure, L is selected from -NH- or -O-.

In some embodiments of the present disclosure, L is selected from -NH-.

In some embodiments of the present disclosure, L is selected from -O-.

In some embodiments of the present disclosure, $-L_2$-(7- to 11-membered spiroheterocyclyl) in $R_{gb}$ is selected from $-L_2$-(7- to 11-membered azaspirocyclyl), and $-L_2$-(6-to 14-membered fused heterocyclyl) is selected from $-L_2$-(6- to 14-membered fused azacyclyl).

In some embodiments of the present disclosure, $R_2$ is selected from methyl, ethyl, n-propyl, isopropyl, butyl, tert-butyl,

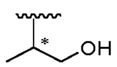 , 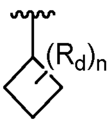 , 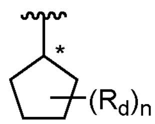

(for example

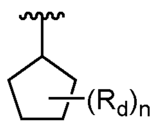 ), 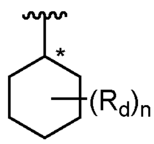

(for example

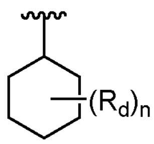

),

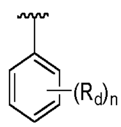 , 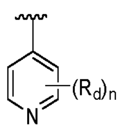 , 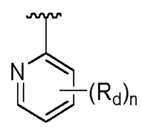 ,

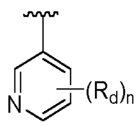 , 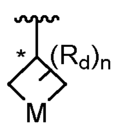

(for example

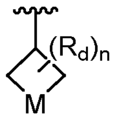

),

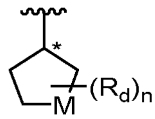

(for example

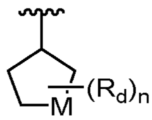

) or

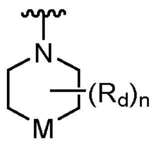

,

wherein M is optionally and independently selected from -O- or $-NR_a-$, $R_a$ and $R_d$ are as defined in any embodiment of the present disclosure, and n is independently 0, 1, 2, 3 or 4.

In some embodiments of the present disclosure, the 3- to 6-membered heterocycloalkyl in $R_d$ is 3- to 6-membered azacycloalkyl, for example

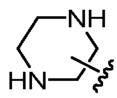

,

for another example

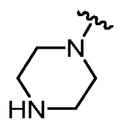

.

In some embodiments of the present disclosure, $R_d$ is selected from H, -OH, -F, $-N(CH_3)CH_3$, $-CH_3$, $-OCH_3$, $-CH_2N(CH_3)CH_3$ or

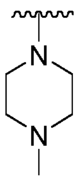

.

In some embodiments of the present disclosure, $R_d$ is selected from -OH, -F, $-CH_3$.

In some embodiments of the present disclosure, the 3- to 6-membered heterocycloalkyl in $R_2$ is selected from

3- to 6-membered oxacycloalkyl, such as tetrahydrofuryl

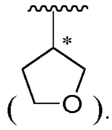.

In some embodiments of the present disclosure, $R_2$ is selected from isopropyl, tert-butyl,

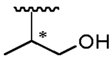, 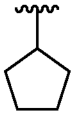, 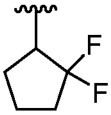, 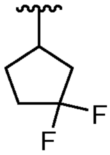, 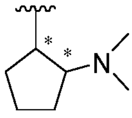, 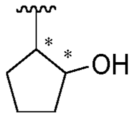, 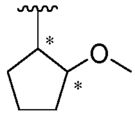, 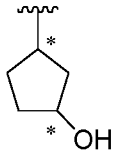,

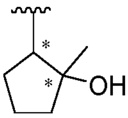, 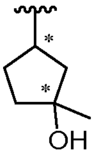, 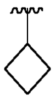, 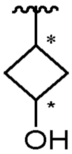, 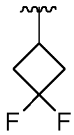, 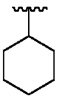, 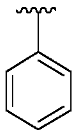, 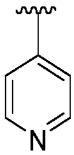, 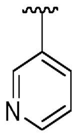, 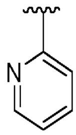, 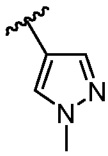,

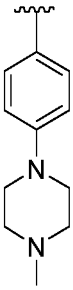, 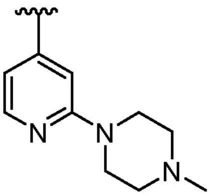, 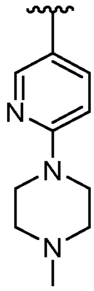, 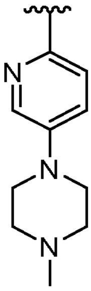, 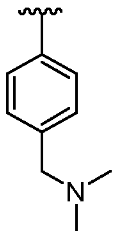, 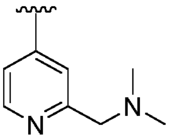, 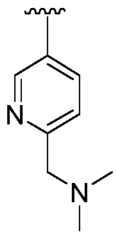, 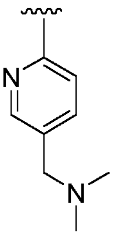,

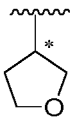, 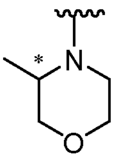, 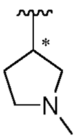, 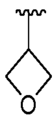, 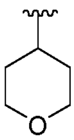, 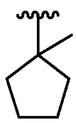, 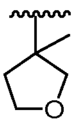.

In some embodiments of the present disclosure, $R_2$ is selected from

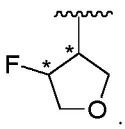.

In some embodiments of the present disclosure, $R_2$ is selected from

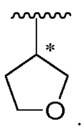.

In some embodiments of the present disclosure, $R_2$ is selected from

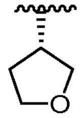.

In some embodiments of the present disclosure, $R_3$ is selected from methyl, isopropyl, $-(CH_2)_3N(CH_3)CH_3$,

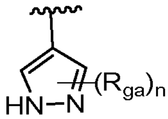, 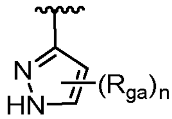, 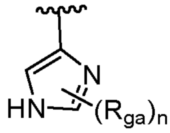, 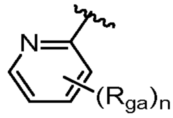,

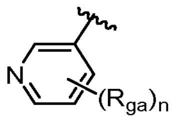, 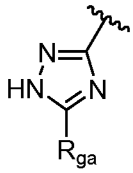, 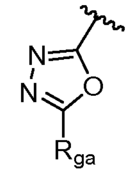, 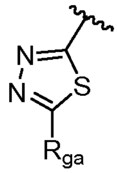, 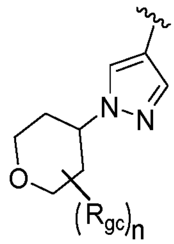, 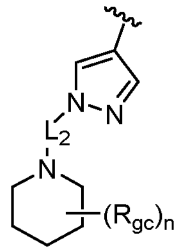,

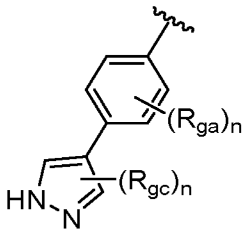, 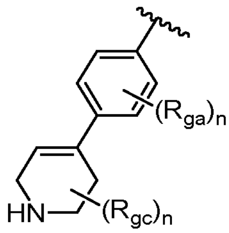, 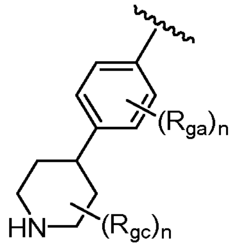, 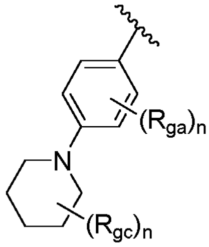,

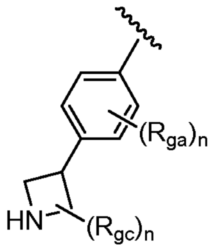, 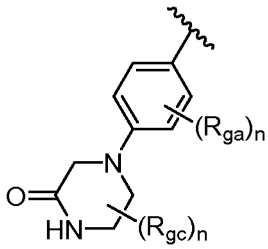, 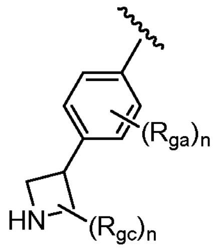, 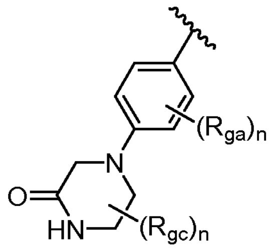,

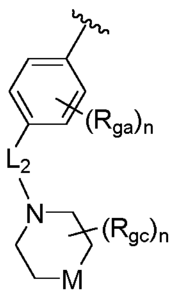, 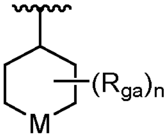, 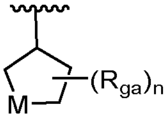, 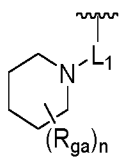, 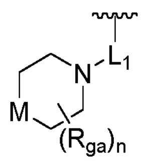, 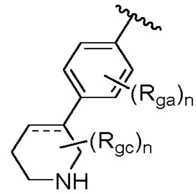,

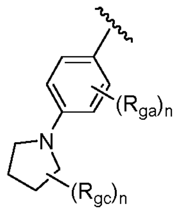, 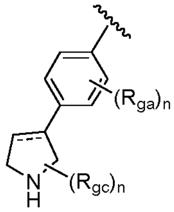, 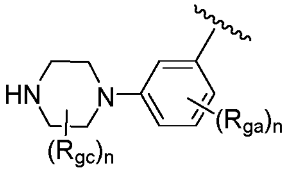, 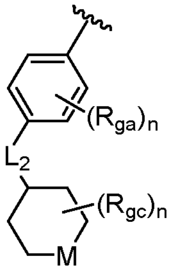, 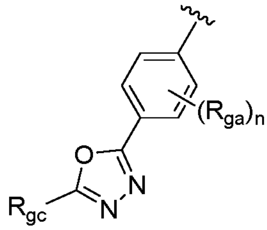,

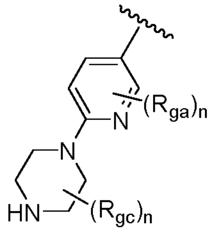, 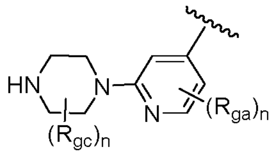, 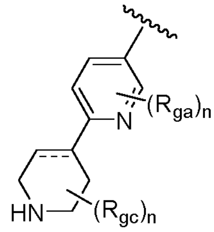, 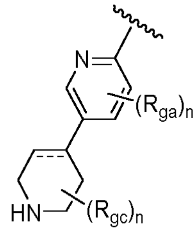, 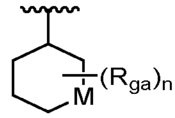,

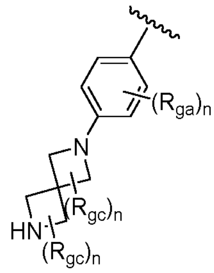, 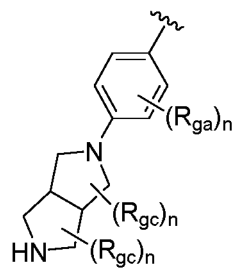, 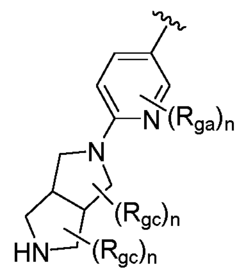,

wherein M, n, $R_{ga}$, $R_{gc}$, $L_1$, $L_2$ are as defined in any embodiment of the present disclosure.

In some embodiments of the present disclosure, $R_3$ is selected from

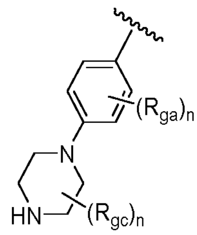,

wherein n, $R_{ga}$, $R_{gc}$ are as defined in any embodiment of the present disclosure.

In some embodiments of the present disclosure, in $R_3$, any two $R_{gc}$ in

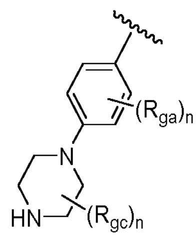

are connected to form the $C_{1-2}$ alkylene chain, such as

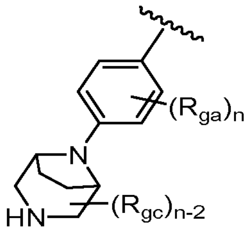

,

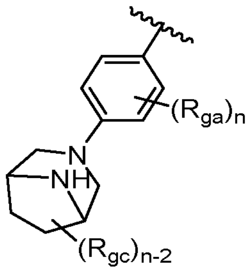

,

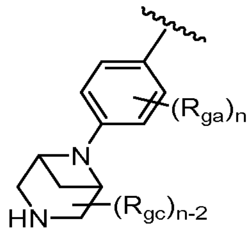

,

wherein n, $R_{ga}$, $R_{gc}$ are as defined in any embodiment of the present disclosure.

In some embodiments of the present disclosure, in $R_3$, any two $R_{gc}$ in

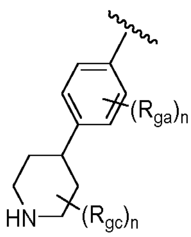

are connected to form the $C_{1-2}$ alkylene chain, such as

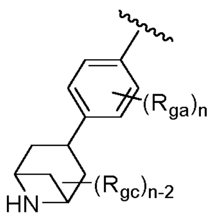

,

wherein n, $R_{ga}$, $R_{gc}$ are as defined in any embodiment of the present disclosure.

In some embodiments of the present disclosure, in $R_3$, any two $R_{gc}$ in

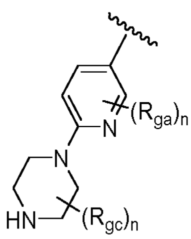

are connected to form the $C_{1-2}$ alkylene chain, such as

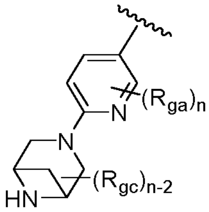 , 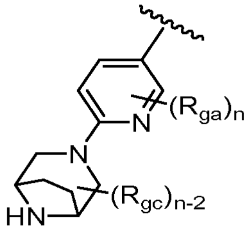 ,

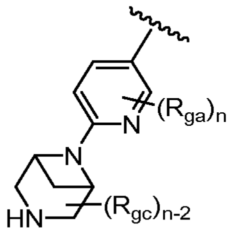 , 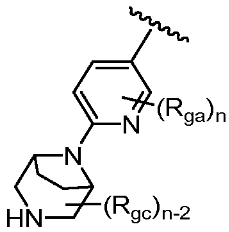 ,

wherein n, $R_{ga}$, $R_{gc}$ are as defined in any embodiment of the present disclosure.

In some embodiments of the present disclosure, $R_eR_fNC(O)$-$C_{1-4}$ alkyl- in $R_{ga}$ is $NH_2COC(CH_3)_2$-.

In some embodiments of the present disclosure, -$S(O)_2$-(5- to 6-membered heteroaryl) in $R_{ga}$ is -$S(O)_2$-(5- to 6-membered azaaryl), such as

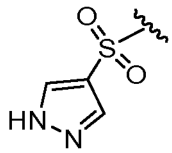 .

In some embodiments of the present disclosure, $R_{ga}$ is selected from H, Br, F, -$CH_3$, - $CH(CH_3)_2$, -$CH_2CH_2N(CH_3)CH_3$, -$NH_2$, -$CH_2CH_2OH$, -$C(CH_3)_2OH$, $NH_2COC(CH_3)_2$-,

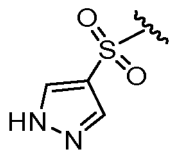 ,

-$OCH_3$.

In some embodiments of the present disclosure, $L_2$ in $R_{gb}$ is the bond or -$(CH_2)_2$-.

In some embodiments of the present disclosure, $R_2$ is selected from

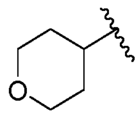 ,

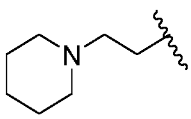 , 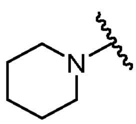 , 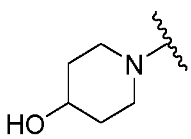 , 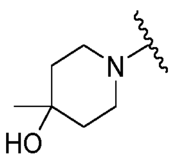 , 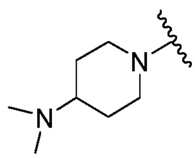 , 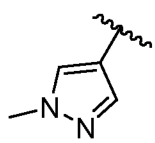 ,

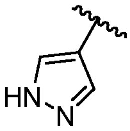, 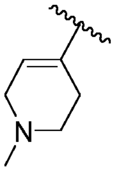, 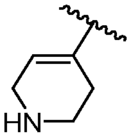, 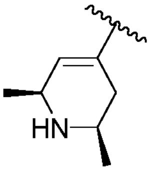, 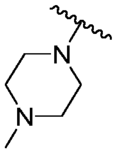, 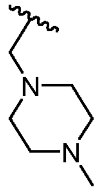, 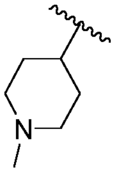, 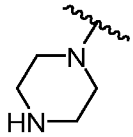,

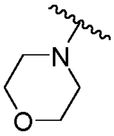, 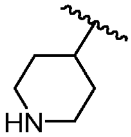, 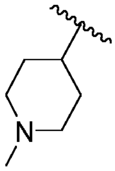, 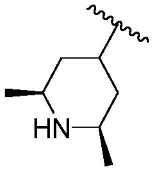, 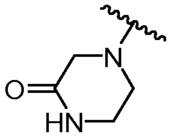, 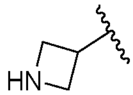, 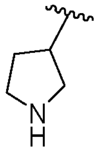, 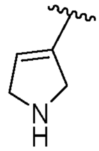,

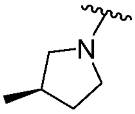, 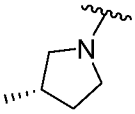, 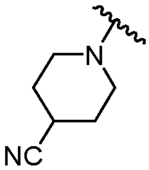, 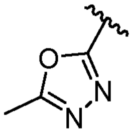, 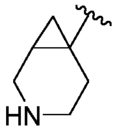, 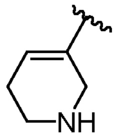, 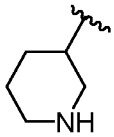,

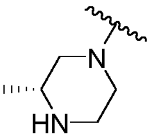, 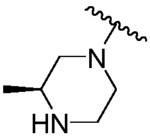, 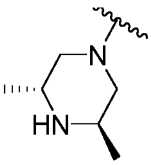, 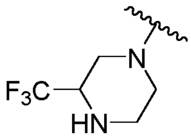, 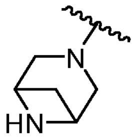, 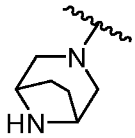,

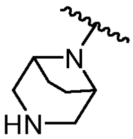, 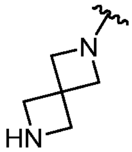, 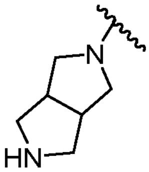, 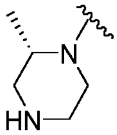, 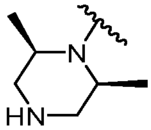, 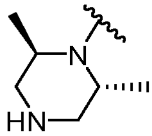.

In some embodiments of the present disclosure, $R_{gb}$ is selected from

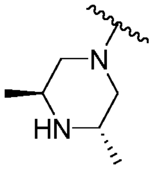,

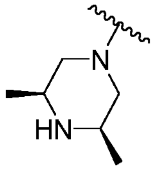.

In some embodiments of the present disclosure, $R_{gb}$ is selected from

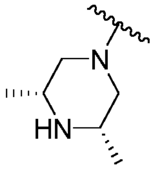,

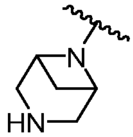.

In some embodiments of the present disclosure, $R_{gc}$ is selected from H, $-CH_3$, F, -OH, $-NH_2$, $-N(CH_3)CH_3$, CN, $-CF_3$;
or any two $R_{gc}$ are connected to form $-CH_2-$ or $-CH_2CH_2-$ chain.

In some embodiments of the present disclosure, any two non-adjacent $R_{gc}$ are connected to form a $C_{1-2}$ alkylene chain.

In some embodiments of the present disclosure, any two non-adjacent $R_{gc}$ are connected to form $-CH_2-$ or $-CH_2CH_2-$ chain.

In some embodiments of the present disclosure, $-L_1$-aryl in $R_3$ is selected from $-L_1$-phenyl.

In some embodiments of the present disclosure, $-L_1$-(5- to 6-membered heteroaryl) in $R_3$ is selected from $-L_1$-(5- to 6-membered azaaryl), such as $-L_1$-pyrazolyl, $-L_1$-pyridyl.

In some embodiments of the present disclosure, $L_1$ in $R_3$ is the bond.

In some embodiments of the present disclosure, $R_3$ is selected from methyl, isopropyl, $-(CH_2)_3N(CH_3)CH_3$,

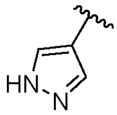, 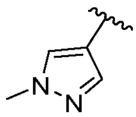, 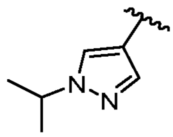, 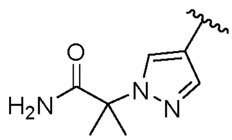,

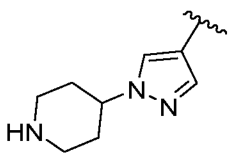, 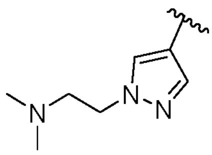, 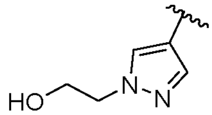, 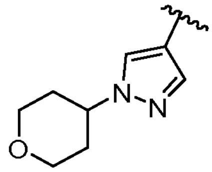,

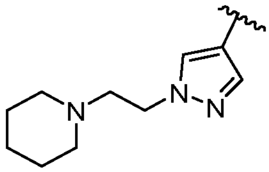, 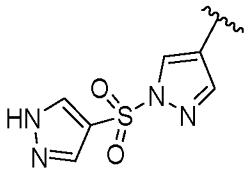, 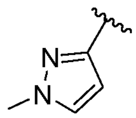, 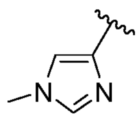, 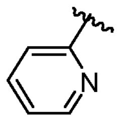, 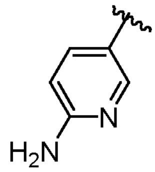,

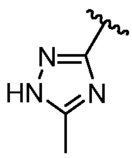, 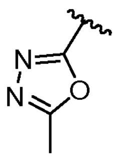, 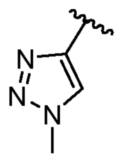, 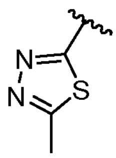, 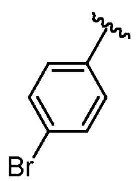, 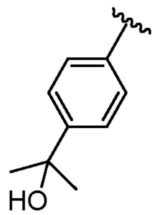, 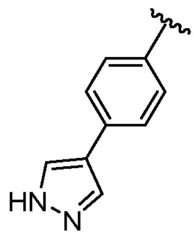,

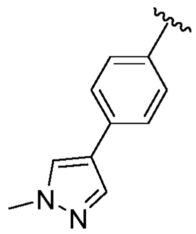 , 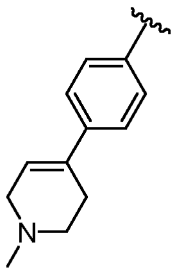 , 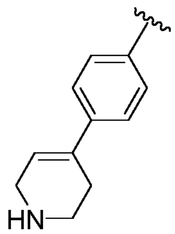 , 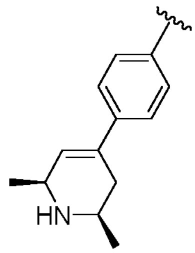 , 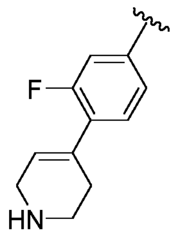 , 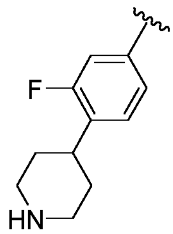 ,

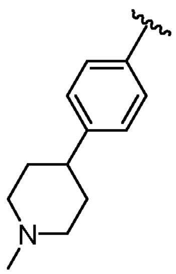 , 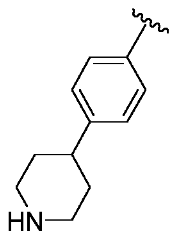 , 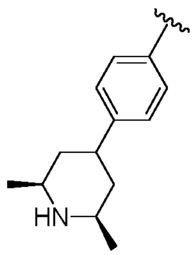 , 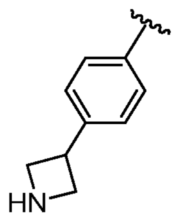 , 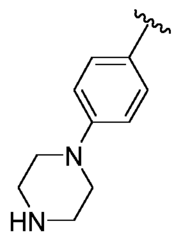 , 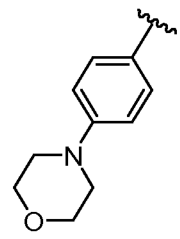 ,

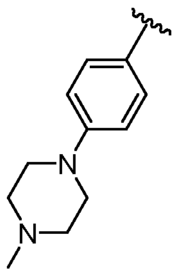 , 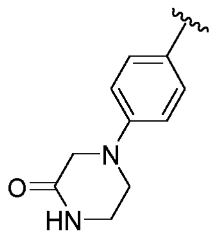 , 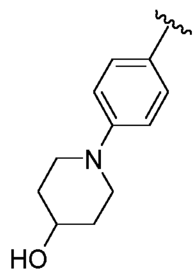 , 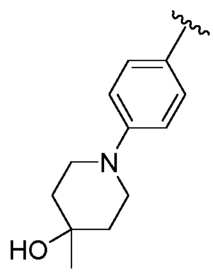 , 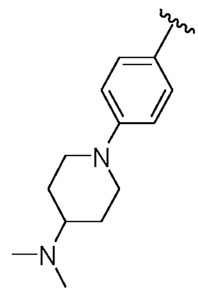 ,

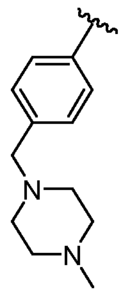 , 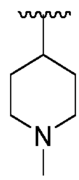 , 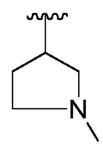 , 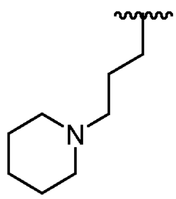 , 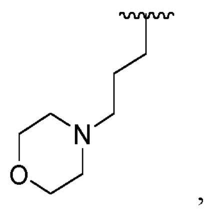 , 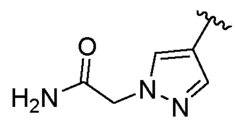 ,

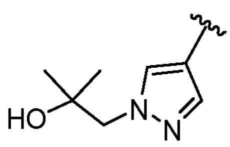 , 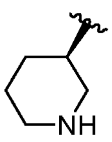 , 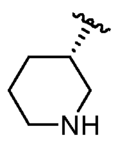 , 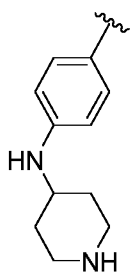 , 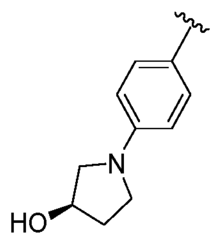 , 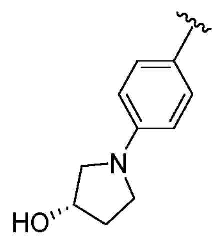 ,

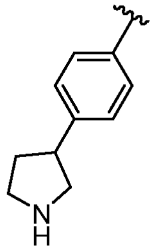 , 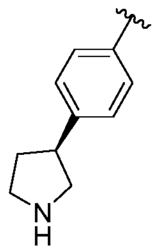 , 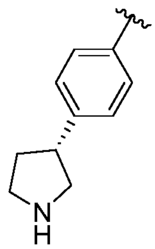 , 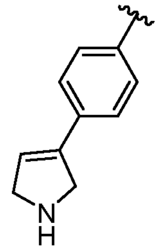 , 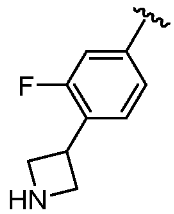 , 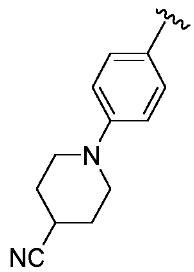 ,

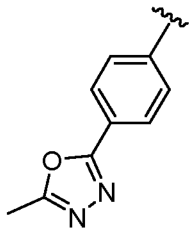 , 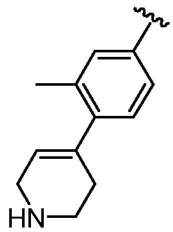 , 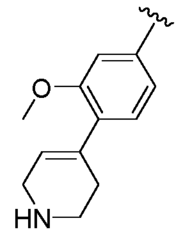 , 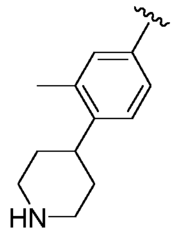 , 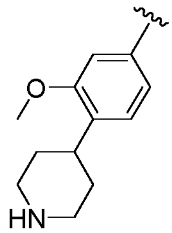 , 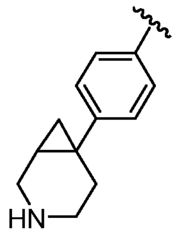 ,

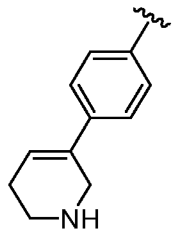 , 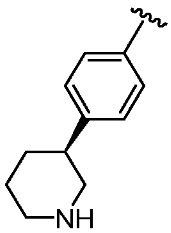 , 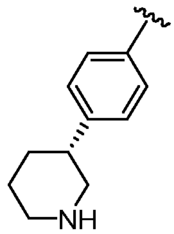 , 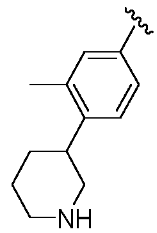 , 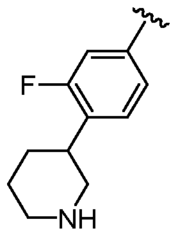 , 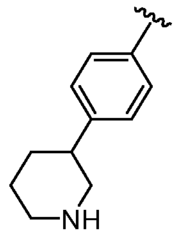 ,

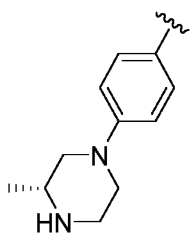 , 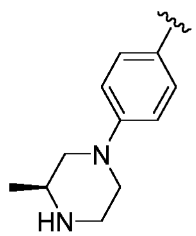 , 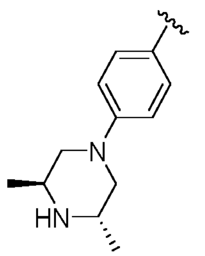 , 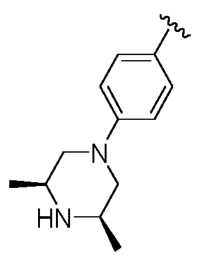 , 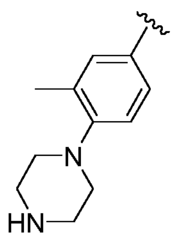 ,

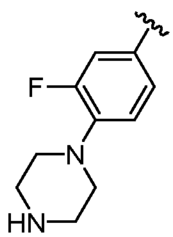 , 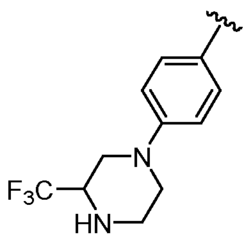 , 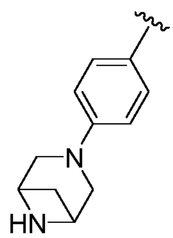 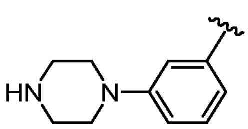 , 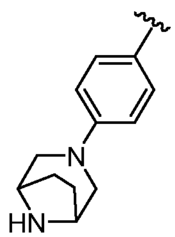 ,

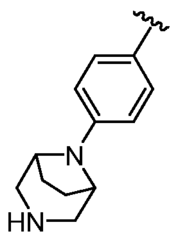 , 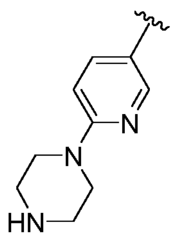 , 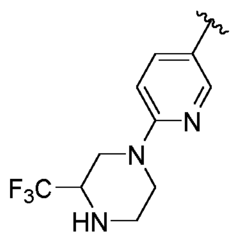 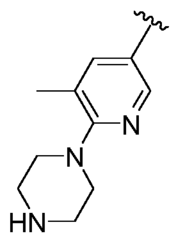 , 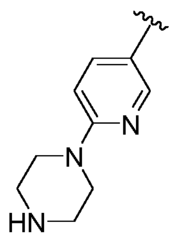 , 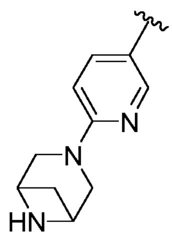 ,

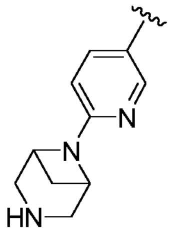, 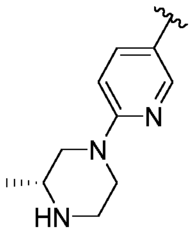, 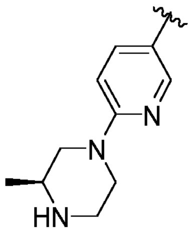, 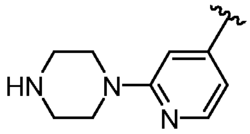, 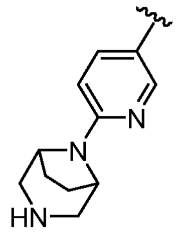,

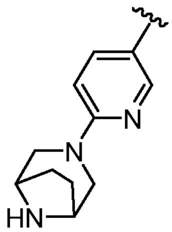, 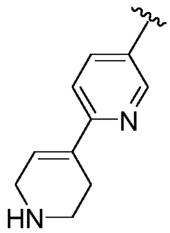, 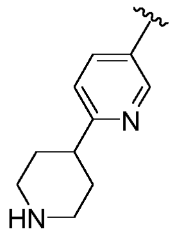, 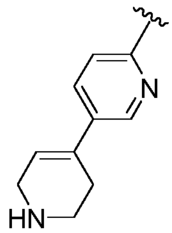, 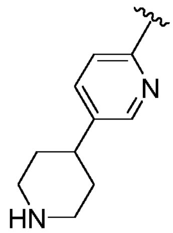, 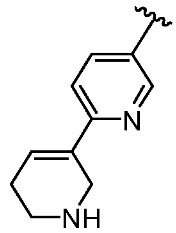,

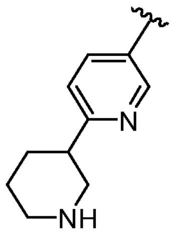, 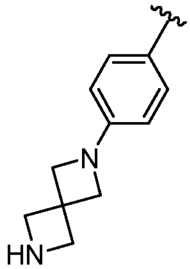, 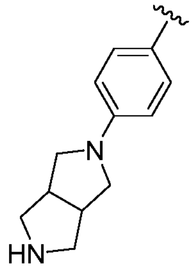, 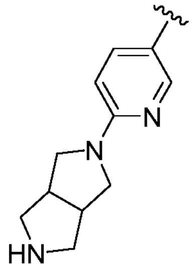, 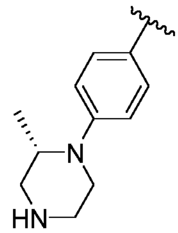,

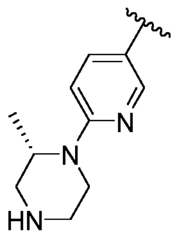, 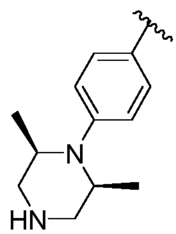, 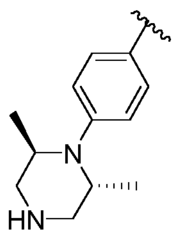.

In some embodiments of the present disclosure, $R_3$ is selected from

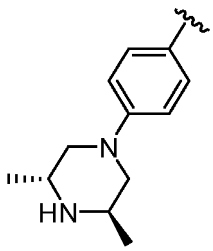.

In some embodiments of the present disclosure, $R_3$ is selected from

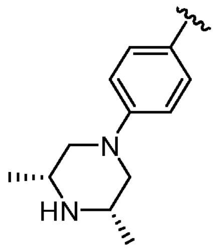

,

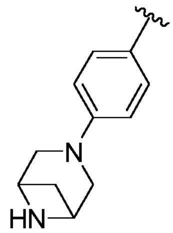

.

In some embodiments of the present disclosure, $R_3$ is selected from

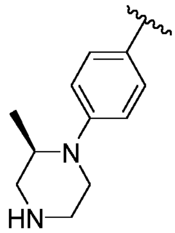

,

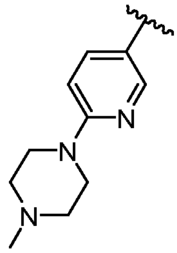

.

In some embodiments of the present disclosure, $R_3$ is selected from

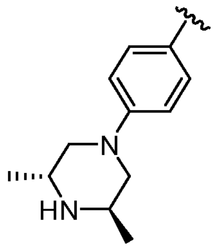

,

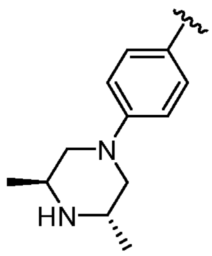,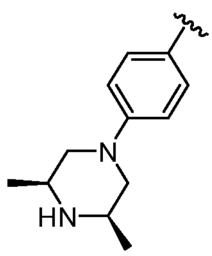.

In some embodiments of the present disclosure, $R_3$ is selected from

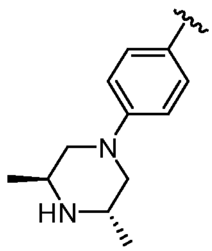.

In some embodiments of the present disclosure, $R_4$ and $R_5$ are optionally and independently selected from H, F, OH, $CH_3$.

In some embodiments of the present disclosure, $R_4$ and $R_5$ are optionally and independently selected from H, F.

In some embodiments of the present disclosure, $R_4$ and $R_5$ are selected from H.

In some embodiments of the present disclosure, for the compounds of formula (I-A) and formula (I-B), the stereoisomer thereof, the tautomer thereof, the pharmaceutically acceptable salt thereof, the prodrug thereof, the hydrate thereof, the solvate thereof or the isotope-labeled derivative thereof, wherein the compound can be represented by formula (II),

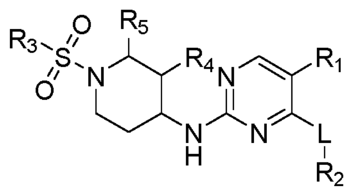

(II)

wherein

$R_1$, $R_2$, $R_3$, $R_4$, $R_5$, L are as defined in any embodiment of the present disclosure.

In some embodiments of the present disclosure, for the compounds of formula (I-A) and formula (I-B), the stereoisomer thereof, the tautomer thereof, the pharmaceutically acceptable salt thereof, the prodrug thereof, the hydrate thereof, the solvate thereof or the isotope-labeled derivative thereof, wherein the compound can be represented by any structure in formula (II-A), formula (II-B) and formula (II-C),

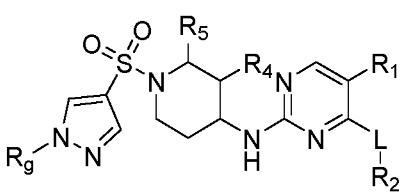

(II-A)

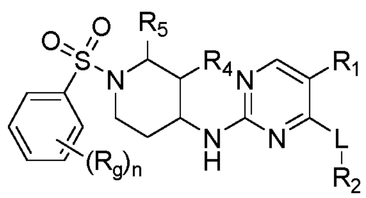

(II-B)

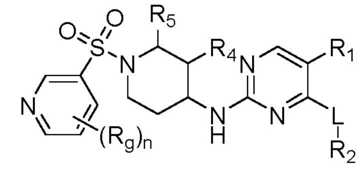

( II-C )

wherein

$R_1$, $R_2$, $R_4$, $R_5$, L, $R_g$, n are as defined in any embodiment of the present disclosure.

In some embodiments of the present disclosure, for the compounds of formula (I-A) and formula (I-B), the stereoisomer thereof, the tautomer thereof, the pharmaceutically acceptable salt thereof, the prodrug thereof, the hydrate thereof, the solvate thereof or the isotope-labeled derivative thereof, wherein the compound can be represented by any one of the following structures,

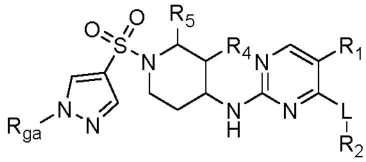

( II-A-1 )

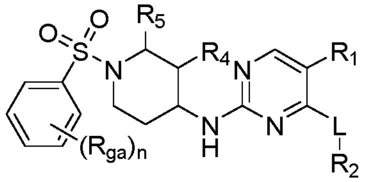

( II-B-1 )

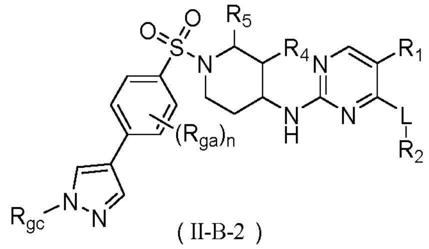

( II-B-2 )

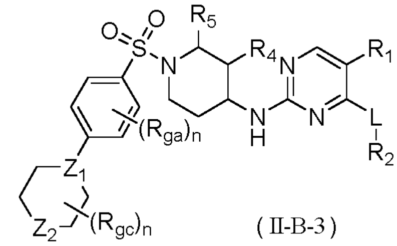

( II-B-3 )

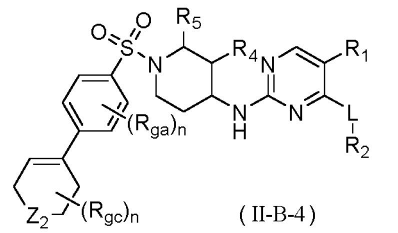

( II-B-4 )

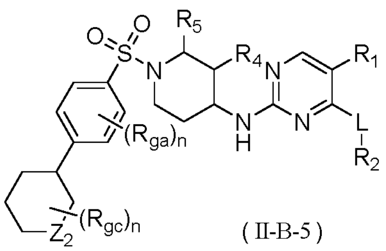

( II-B-5 )

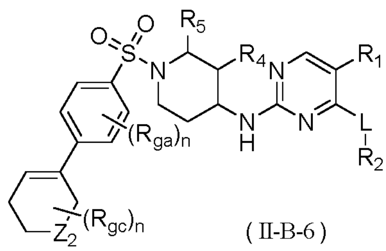

( II-B-6 )

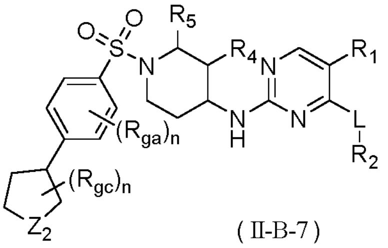

( II-B-7 )

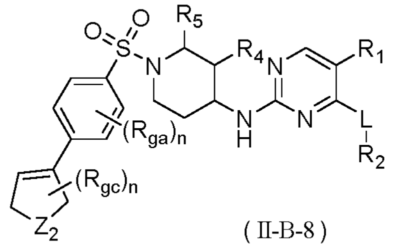

( II-B-8 )

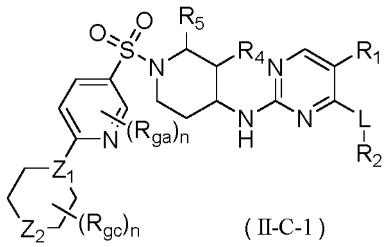

( II-C-1 )

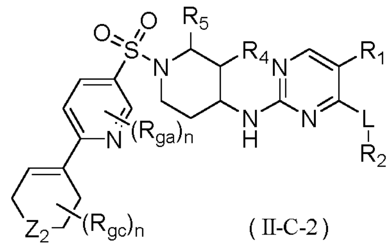

( II-C-2 )

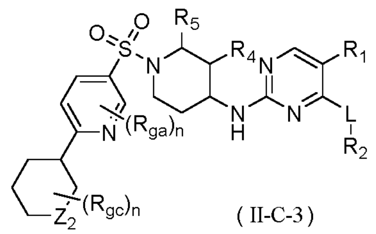

( II-C-3 )

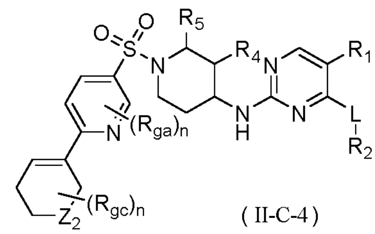

( II-C-4 )

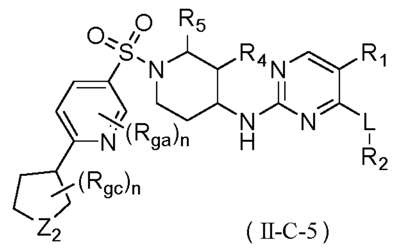

( II-C-5 )

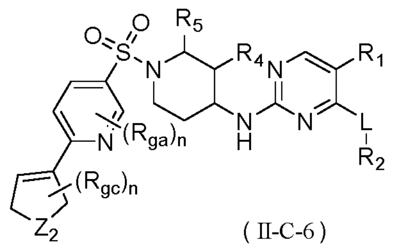

( II-C-6 )

wherein

$Z_1$ is selected from C, CH or N;
$Z_2$ is selected from $CH_2$, NH or O;
$R_1$, $R_2$, $R_4$, $R_5$, L, n, $R_{ga}$, $R_{gc}$ are as defined in any embodiment of the present disclosure.

In some embodiments of the present disclosure, for the compounds of formula (I-A) and formula (I-B), the stereoisomer thereof, the tautomer thereof, the pharmaceutically acceptable salt thereof, the prodrug thereof, the hydrate thereof, the solvate thereof or the isotope-labeled derivative thereof, wherein the compound can be represented by any one of the following structures,

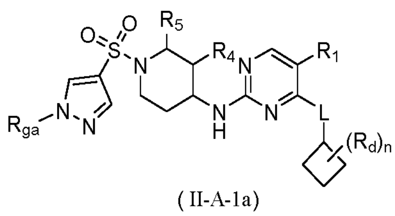

( II-A-1a)

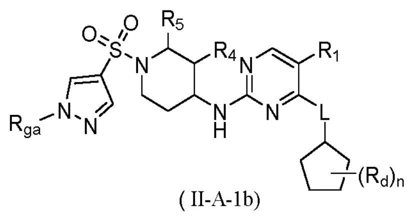

( II-A-1b)

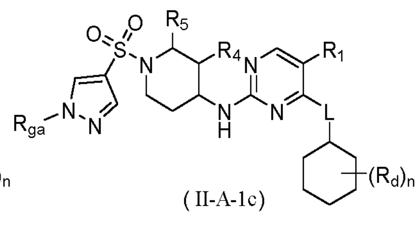

( II-A-1c)

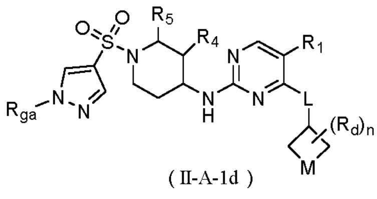
( II-A-1d )

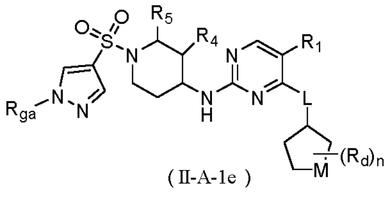
( II-A-1e )

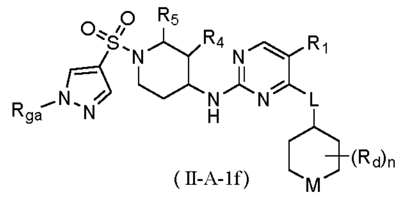
( II-A-1f)

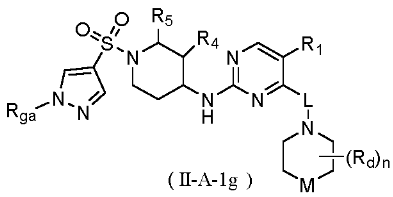
( II-A-1g )

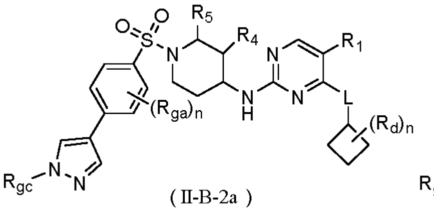
( II-B-2a )

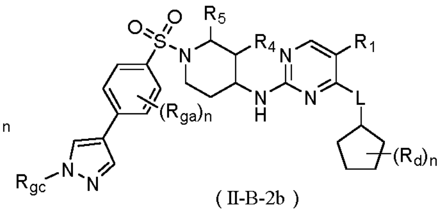
( II-B-2b )

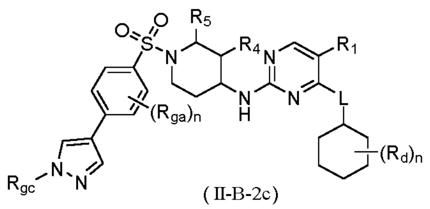
( II-B-2c)

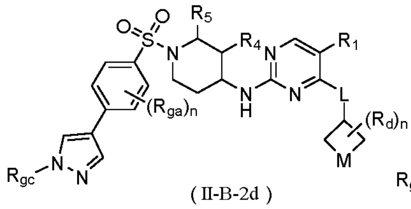
( II-B-2d )

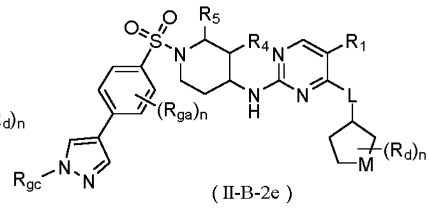
( II-B-2e )

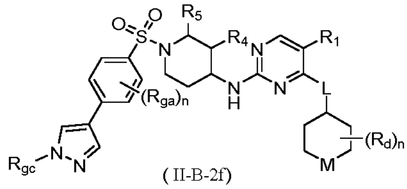
( II-B-2f)

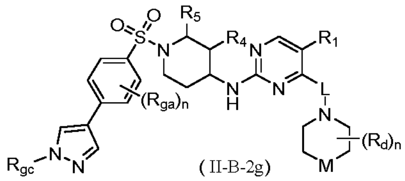
( II-B-2g)

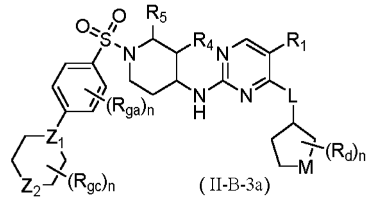
( II-B-3a)

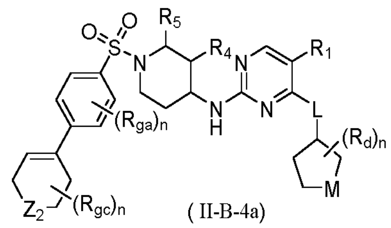
( II-B-4a)

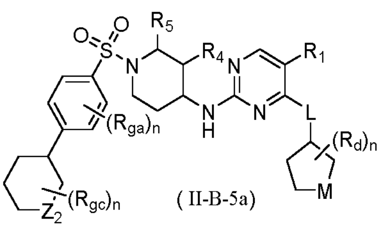
( II-B-5a)

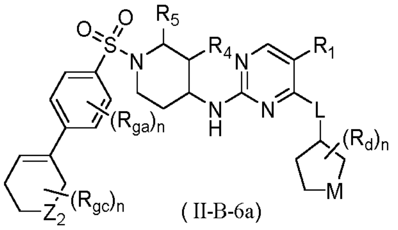
( II-B-6a)

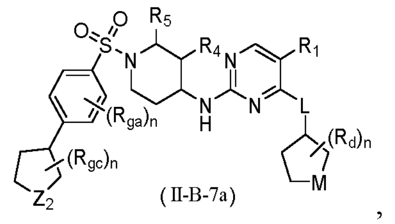
( II-B-7a) ,

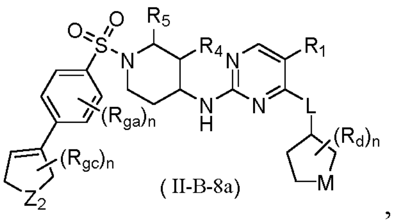
( II-B-8a) ,

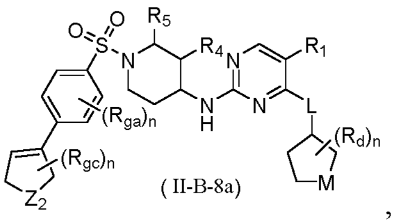
( II-B-8a) ,

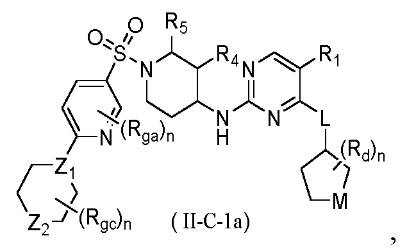
( II-C-1a) ,

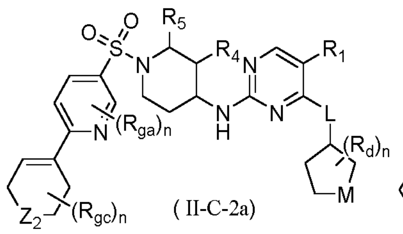
( II-C-2a)

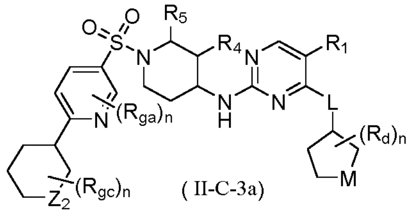
( II-C-3a)

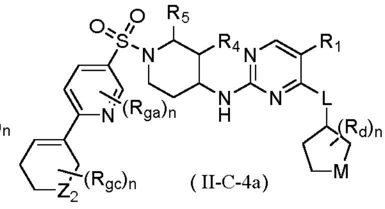
( II-C-4a)

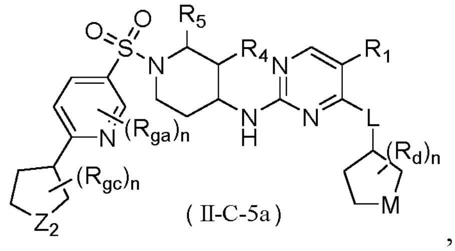
( II-C-5a ) ,

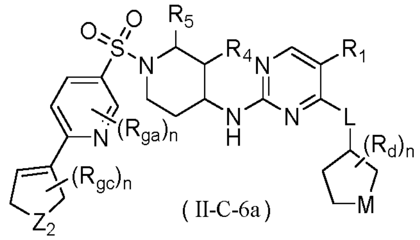
( II-C-6a ) ,

wherein $R_1$, L, $R_d$, M, n, $R_4$, $R_5$, $R_{ga}$, $R_{gc}$, $Z_1$, $Z_2$ are as defined in any embodiment of the present disclosure.

In some embodiments of the present disclosure, for the compounds of formula (I-A) and formula (I-B), the stereoisomer thereof, the tautomer thereof, the pharmaceutically acceptable salt thereof, the prodrug thereof, the hydrate thereof, the solvate thereof or the isotope-labeled derivative thereof, wherein the compound can be represented by any one of the following structures,

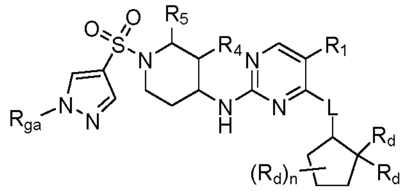
( II-A-1b-1)

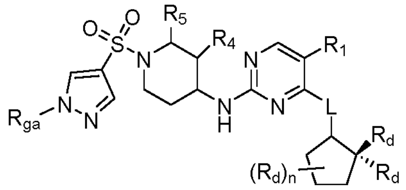
( II-A-1b-2)

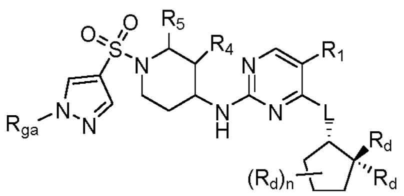
( II-A-1b-3)

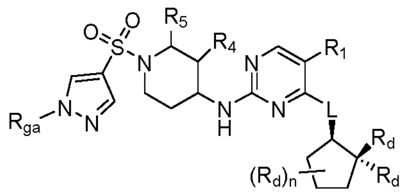
( II-A-1b-4)

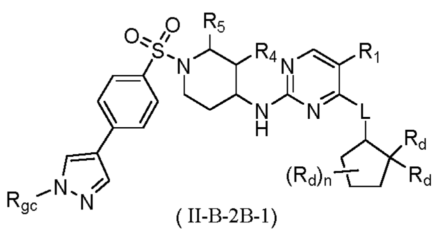
( II-B-2B-1)

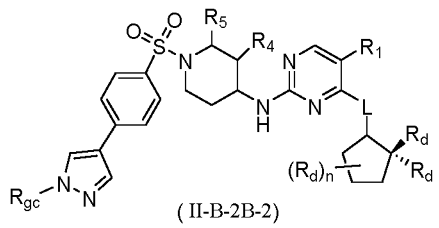
( II-B-2B-2)

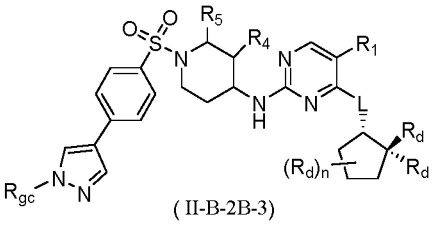
( II-B-2B-3)

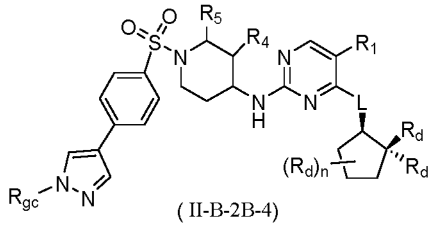
( II-B-2B-4)

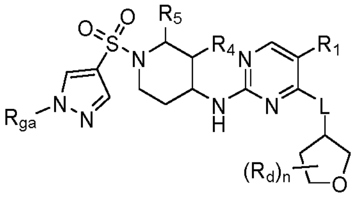
( II-A-1b-5 )

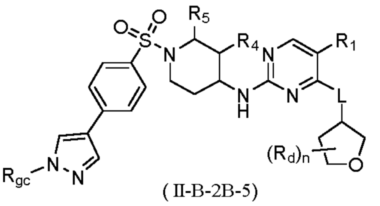
( II-B-2B-5)

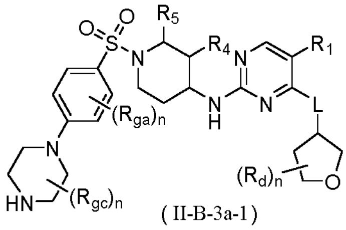
( II-B-3a-1)

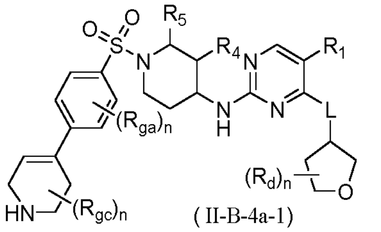
( II-B-4a-1)

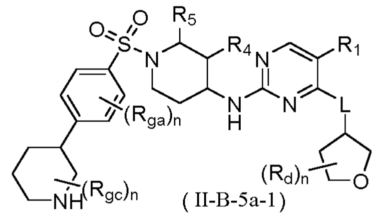
( II-B-5a-1)

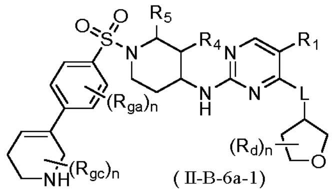
( II-B-6a-1)

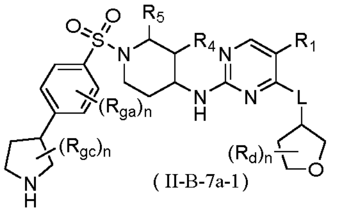
( II-B-7a-1)

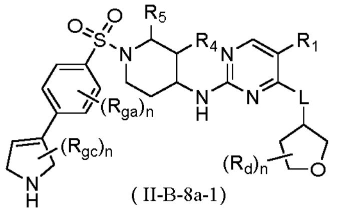
( II-B-8a-1)

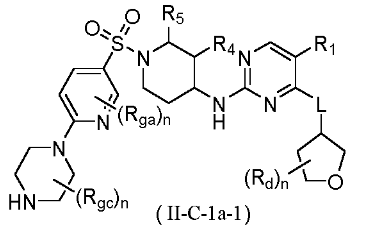
( II-C-1a-1)

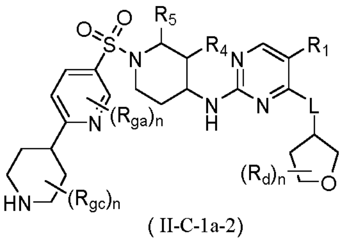
( II-C-1a-2)

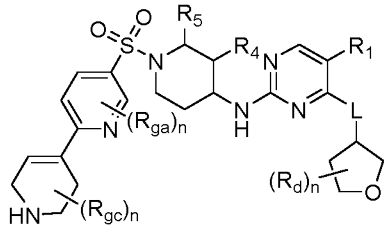
( II-C-2a-1 )

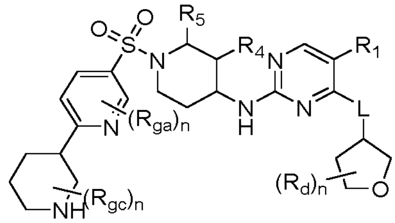
( II-C-3a-1 )

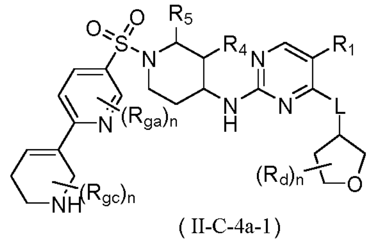
( II-C-4a-1)

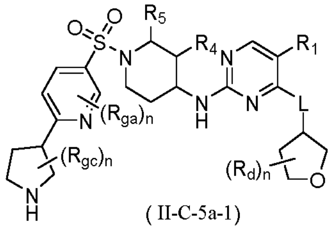
( II-C-5a-1)

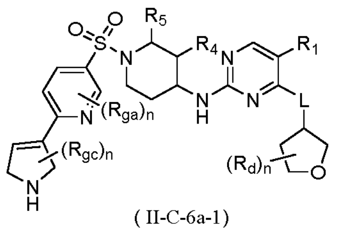
( II-C-6a-1)

$R_1$, L, $R_d$, n, $R_4$, $R_5$, $R_{ga}$, $R_{gc}$ are as defined in any embodiment of the present disclosure.

In some embodiments of the present disclosure, for the compounds of formula (I-A) and formula (I-B), the stereoisomer thereof, the tautomer thereof, the pharmaceutically acceptable salt thereof, the prodrug thereof, the hydrate

thereof, the solvate thereof or the isotope-labeled derivative thereof, wherein the compound can be represented by any one of the following structures,

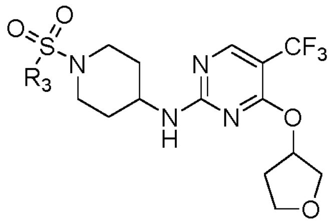

( II-D)

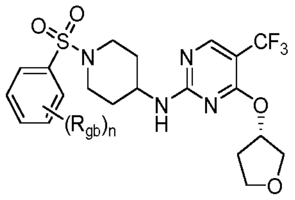

( II-D-1 )

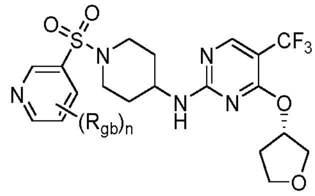

( II-D-2 )

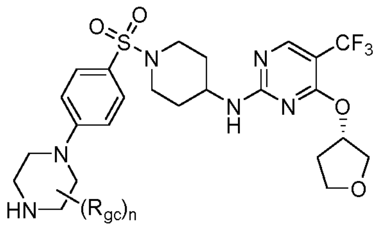

( II-D-3)

wherein $R_3$, $R_{gb}$, $R_{gc}$, n are as defined in any embodiment of the present disclosure.

In some embodiments of the present disclosure, the compound of formula (I-A), the stereoisomer thereof, the tautomer thereof, the pharmaceutically acceptable salt thereof, the prodrug thereof, the hydrate thereof, the solvate thereof or the isotope-labeled derivative thereof, wherein the compound can be represented by formula (I),

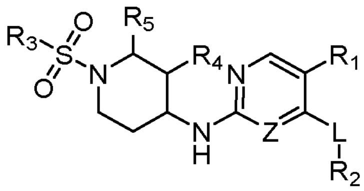

( I)

wherein

$R_1$ is selected from halogen, -CN, $-NO_2$ or $C_{1-4}$ haloalkyl;

Z is selected from -CH- or N;

L is selected from a bond, $-NR_a-$, -O-, -S-, $-SO_2-$, -SO-, -CO-, $-CR_aR_b-$ or -CH=, and the $R_a$ and $R_b$ are optionally and independently selected from H, $C_{1-4}$ alkyl, $C_{1-4}$ haloalkyl, $C_{3-6}$ cycloalkyl, 3- to 6-membered heterocycloalkyl, $-SO_2R_c$, $-SOR_c$, $-COR_c$, $-(CH_2)_mNR_{aa}R_{ab}$ or - $(CH_2)_mC(O)NR_{aa}R_{ab}$, wherein the $R_c$ is selected from H, $C_{1-4}$ alkyl, and the $R_{aa}$ and $R_{ab}$ are optionally and independently selected from H, $C_{1-4}$ alkyl, $C_{1-4}$ haloalkyl, $C_{3-6}$ cycloalkyl, 3- to 6-membered heterocycloalkyl, or $R_{aa}$ and $R_{ab}$ together with the N atom to which they are attached form a 4- to 6-membered heterocycloalkyl;

$R_2$ is selected from $C_{1-4}$ alkyl, $C_{3-6}$ cycloalkyl, 3- to 6-membered heterocycloalkyl, aryl or 5- to 6-membered heteroaryl, and the $C_{1-4}$ alkyl, $C_{3-6}$ cycloalkyl, 3- to 6-membered heterocycloalkyl, aryl and 5- to 6-membered heteroaryl are optionally substituted by one or more than one $R_d$; the $R_d$ is optionally and independently selected from H, halogen, $C_{1-4}$ alkyl, $C_{1-4}$ haloalkyl, $C_{1-4}$ alkoxy, $-(CH_2)_mOH$, $-(CH_2)_mNR_eR_f$, $C_{3-6}$ cycloalkyl or 3- to 6-membered heterocycloalkyl; the $C_{3-6}$ cycloalkyl and 3- to 6-membered heterocycloalkyl in $R_d$ are optionally substituted by $C_{1-4}$ alkyl, $C_{1-4}$ haloalkyl, -OH or $-NH_2$;

$R_3$ is selected from $C_{1-4}$ alkyl, $C_{1-4}$ haloalkyl, $-(CH_2)_mNR_eR_f$, $-(CH_2)_mOH$, $-L_1$-aryl, - $L_1$-(5- to 6-membered heteroaryl), $-L_1$-($C_{3-6}$ cycloalkyl) or $-L_1$-(3- to 6-membered heterocycloalkyl), and the aryl, 5- to 6-membered heteroaryl, $C_{3-6}$ cycloalkyl and 3- to 6-membered heterocycloalkyl are optionally substituted by one or more than one $R_g$, and the $R_g$ can be $R_{ga}$ or $R_{gb}$;

$R_{ga}$ is optionally and independently selected from H, halogen, -OH, $C_{1-4}$ alkyl, $C_{1-4}$ haloalkyl, $-(CH_2)_mNR_eR_f$, $R_eR_f$-

NC(O)-$C_{1-4}$ alkyl-, -$C_{1-4}$ alkyl-OH or -$S(O)_2$-(5- to 6-membered heteroaryl);

$R_{gb}$ is optionally and independently selected from -$L_2$-($C_{3-6}$ cycloalkyl), -$L_2$-(3- to 6-membered heterocycloalkyl), -$L_2$-(3- to 6-membered heterocycloalkenyl), -$L_2$-aryl, -$L_2$-(5- to 6-membered heteroaryl), wherein the $C_{3-6}$ cycloalkyl, 3- to 6-membered heterocycloalkyl, 3-to 6-membered heterocycloalkenyl, aryl, 5- to 6-membered heteroaryl are optionally substituted by one or more than one $R_{gc}$, and the $R_{gc}$ is optionally and independently selected from $C_{1-4}$ alkyl, halogen, $C_{1-4}$ haloalkyl, -$(CH_2)_mNR_eR_f$, or -$(CH_2)_mOH$;

$L_1$ is a bond or $L_1$ is optionally and independently selected from $C_{1-4}$ alkylene;

$L_2$ is a bond or $L_2$ is optionally and independently selected from $C_{1-4}$ alkylene;

$R_e$ and Rf are optionally and independently selected from H or $C_{1-4}$ alkyl;

m is optionally and independently selected from 0, 1, 2, 3 or 4;

$R_4$ and $R_5$ are optionally and independently selected from H, OH, halogen, $C_{1-4}$ alkyl, $C_{1-4}$ haloalkyl or $C_{1-4}$ alkoxy; and when L is the bond, $R_2$ is not

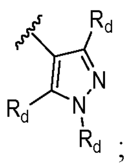
;

when L is -NH-, $R_2$ is not

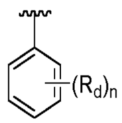
;

and when the compound of formula (I) is

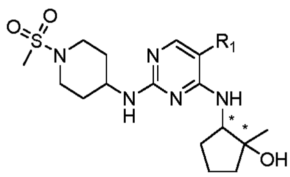
,

$R_1$ is not halogen.

In some embodiments of the present disclosure, $R_1$ is selected from -CN, -$CF_3$ or - $CHF_2$.

In some embodiments of the present disclosure, $R_1$ is selected from -CN or -$CF_3$.

In some embodiments of the present disclosure, $R_1$ is selected from -CN.

In some embodiments of the present disclosure, Z is selected from N.

In some embodiments of the present disclosure, L is selected from -NH-, -N($CH_3$)-, - O-, -$CH_2$-, CH=, -N(CH$(CH)_2$)-, -N($SO_2CH_3$)-, -$SO_2$-, -SO-, -N($CH_2CF_3$)-,

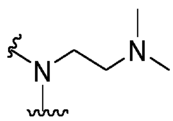

or

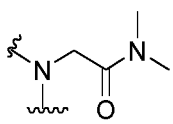
.

In some embodiments of the present disclosure, L is selected from -NH- or -O-.

In some embodiments of the present disclosure, L is selected from -NH-.

In some embodiments of the present disclosure, $R_2$ is selected from methyl, ethyl, n-propyl, isopropyl, butyl, tert-butyl,

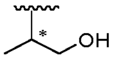, 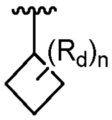, 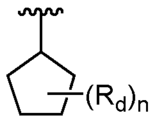, 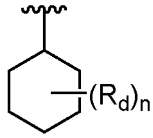, 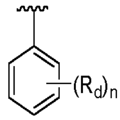,

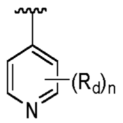, 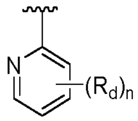, 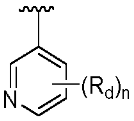, 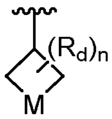, 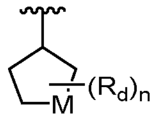, 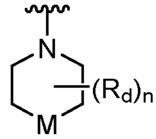,

wherein M is optionally and independently selected from -O- or $-NR_a-$, $R_a$ and $R_d$ are as defined in any embodiment of the present disclosure, and n is independently 0, 1, 2, 3 or 4.

In some embodiments of the present disclosure, $R_d$ is selected from H, -OH, -F, - $N(CH_3)CH_3$, $-CH_3$, $-OCH_3$, $-CH_2N(CH_3)CH_3$ or

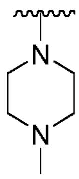.

In some embodiments of the present disclosure, $R_d$ is selected from -OH, -F, $-CH_3$.

In some embodiments of the present disclosure, $R_2$ is selected from isopropyl, tert-butyl,

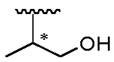, 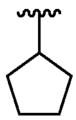, 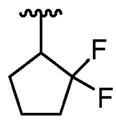, 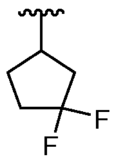, 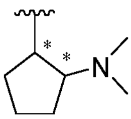, 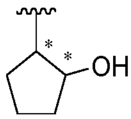, 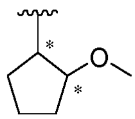, 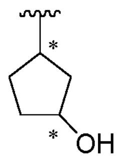,

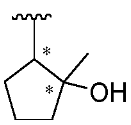, 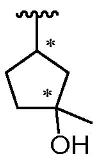, 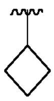, 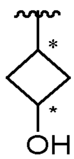, 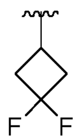, 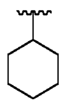, 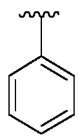, 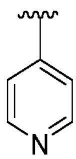, 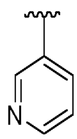, 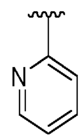, 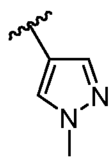,

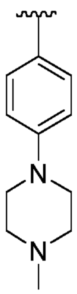, 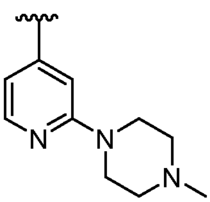, 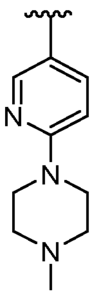, 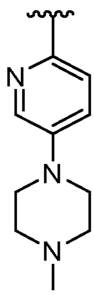, 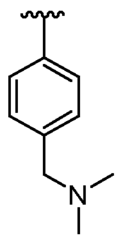, 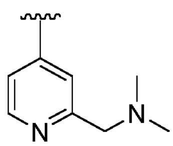, 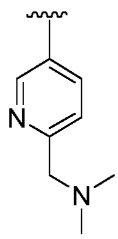, 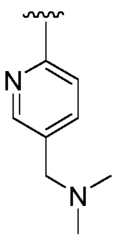,

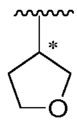, 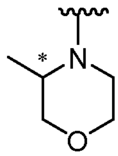, 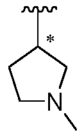, 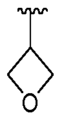 or 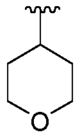.

In some embodiments of the present disclosure, $R_3$ is selected from methyl, isopropyl, $-(CH_2)_3N(CH_3)CH_3$,

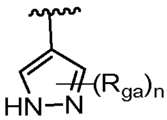, 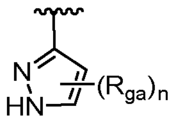, 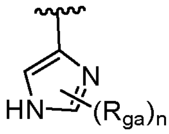, 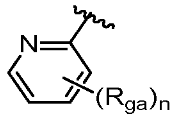, 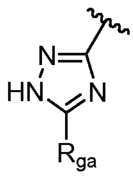,

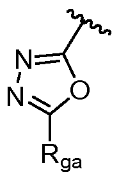, 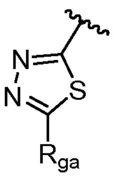, 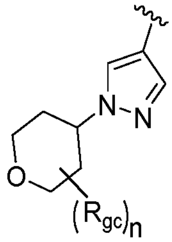, 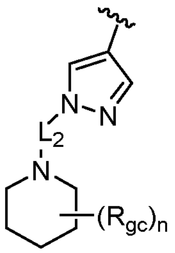, 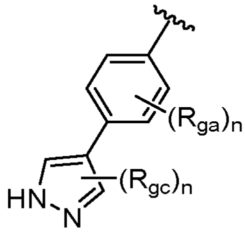, 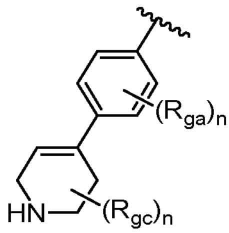,

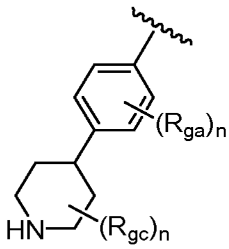, 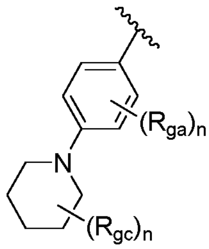, 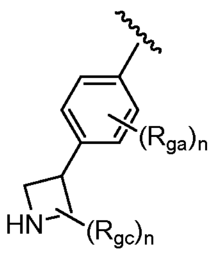, 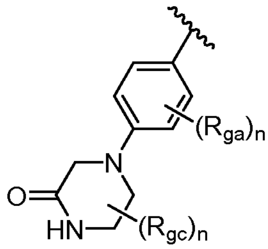,

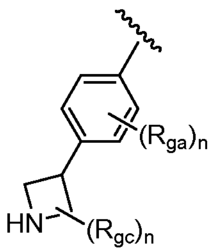, 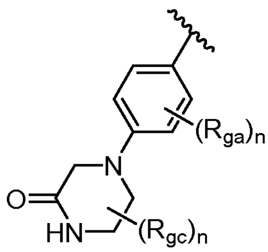, 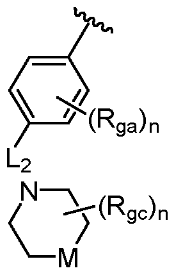, 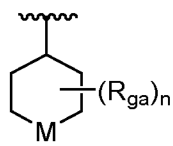, 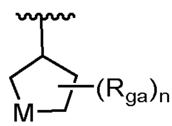,

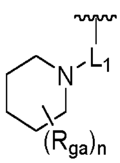, 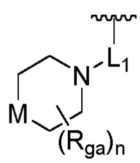,

wherein M, n, $R_{ga}$, $R_{gc}$, $L_1$ are as defined in any embodiment of the present disclosure.

In some embodiments of the present disclosure, $R_{ga}$ is selected from H, Br, F, $-CH_3$, $-CH(CH_3)_2$, $-CH_2CH_2N(CH_3)CH_3$, $-NH_2$, $-CH_2CH_2OH$, $-C(CH_3)_2OH$, $NH_2COC(CH_3)_2-$,

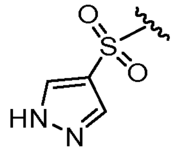.

In some embodiments of the present disclosure, $R_{gb}$ is selected from

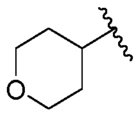,

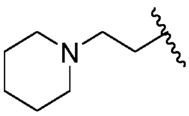, 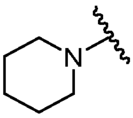, 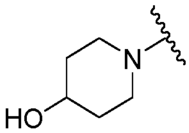, 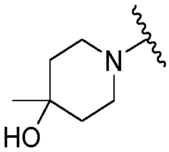, 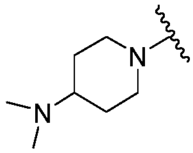, 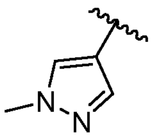,

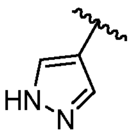, 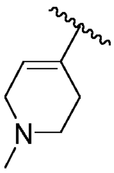, 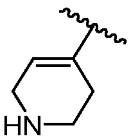, 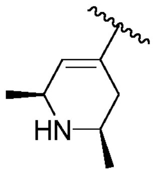, 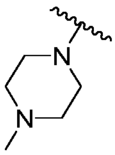, 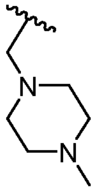, 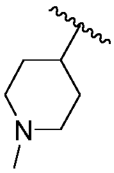, 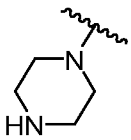,

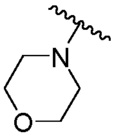, 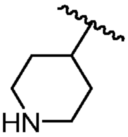, 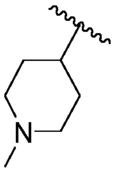, 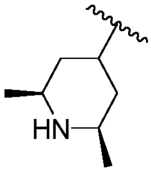, 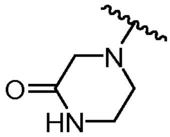, 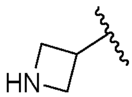.

In some embodiments of the present disclosure, $R_{gc}$ is selected from H, $-CH_3$, F, -OH, $-NH_2$, $-N(CH_3)CH_3$.

In some embodiments of the present disclosure, $R_3$ is selected from methyl, isopropyl, $-(CH_2)_3N(CH_3)CH_3$,

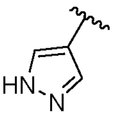, 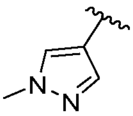, 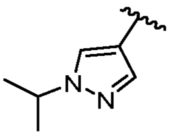, 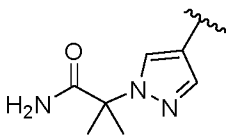, 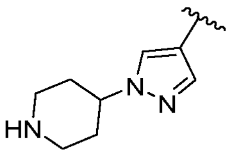,

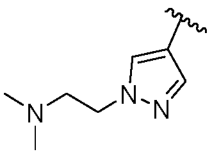, 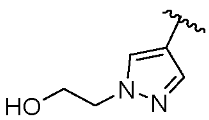, 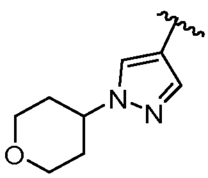, 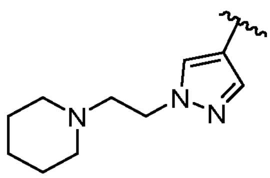,

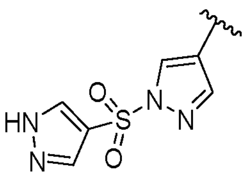, 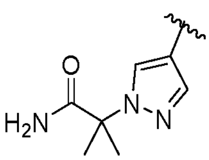, 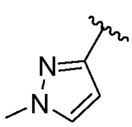, 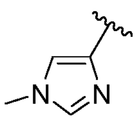, 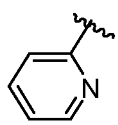, 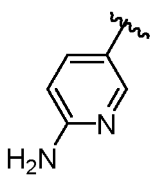,

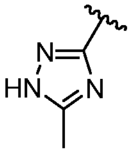, 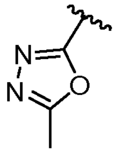, 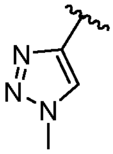, 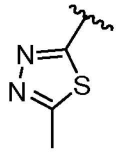, 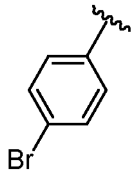, 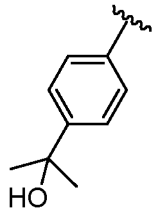, 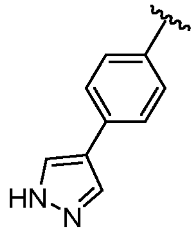,

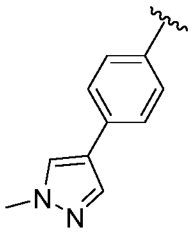, 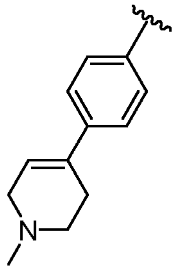, 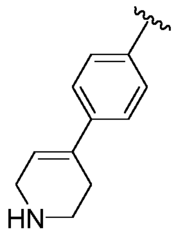, 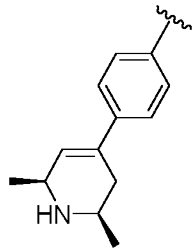, 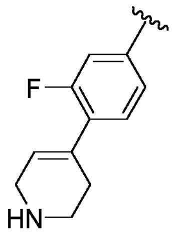, 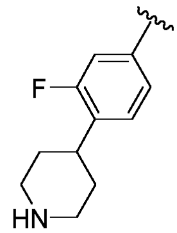,

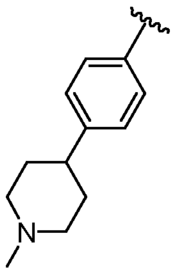, 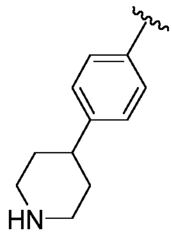, 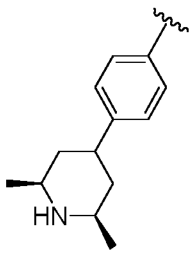, 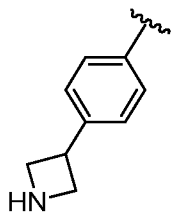, 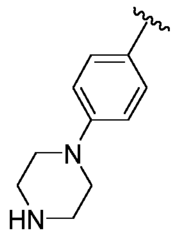, 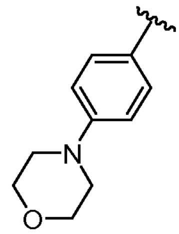,

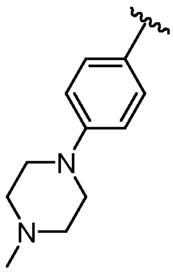, 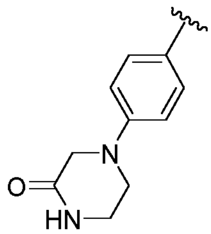, 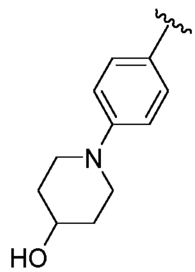, 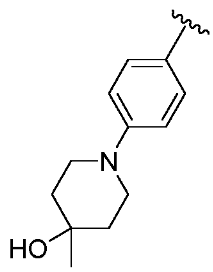, 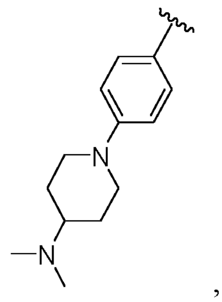,

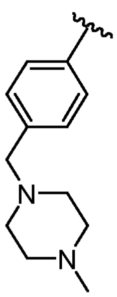, 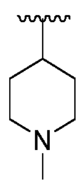, 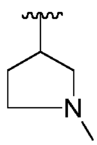, 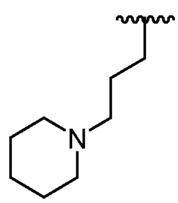, 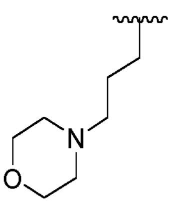.

In some embodiments of the present disclosure, $R_4$ and $R_5$ are optionally and independently selected from H, F, OH, $CH_3$.

In some embodiments of the present disclosure, $R_4$ and $R_5$ are optionally and independently selected from H, F.

In some embodiments of the present disclosure, for the compound, the stereoisomer thereof, the tautomer thereof, the pharmaceutically acceptable salt thereof, the prodrug thereof, the hydrate thereof, the solvate thereof, or the isotope-labeled derivative thereof, wherein the compound can be represented by formula (II),

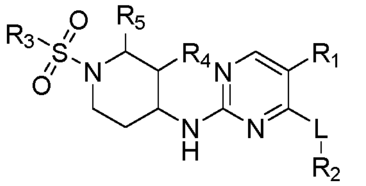

( II )

wherein

$R_1$, $R_2$, $R_3$, $R_4$, $R_5$, L are as defined in any embodiment of the present disclosure.

In some embodiments of the present disclosure, for the compound, the stereoisomer thereof, the tautomer thereof, the pharmaceutically acceptable salt thereof, the prodrug thereof, the hydrate thereof, the solvate thereof, or the isotope-labeled derivative thereof, wherein the compound can be represented by any one of the following structures,

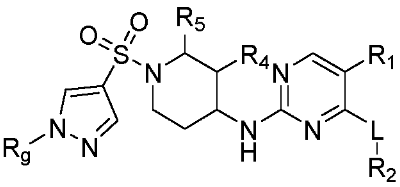

( II-A )

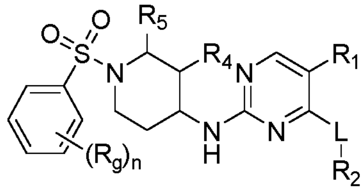

( II-B )

wherein

$R_1$, $R_2$, $R_4$, $R_5$, L, $R_g$, n are as defined in any embodiment of the present disclosure;

In some embodiments of the present disclosure, for the compound, the stereoisomer thereof, the tautomer thereof, the pharmaceutically acceptable salt thereof, the prodrug thereof, the hydrate thereof, the solvate thereof, or the isotope-labeled derivative thereof, wherein the compound can be represented by any one of the following structures,

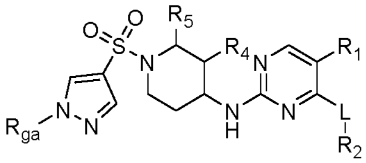

( II-A-1 )

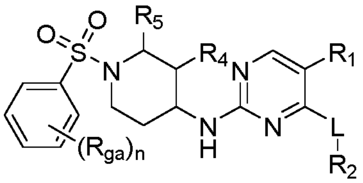

( II-B-1 )

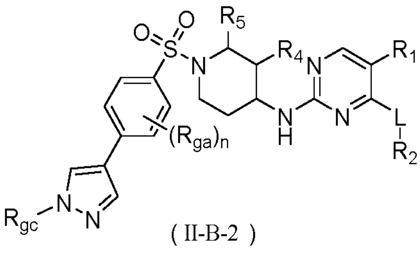

( II-B-2 )

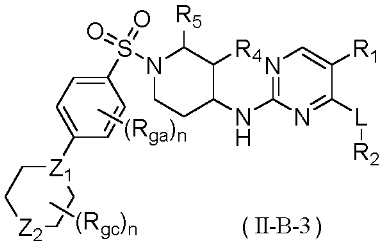

( II-B-3 )

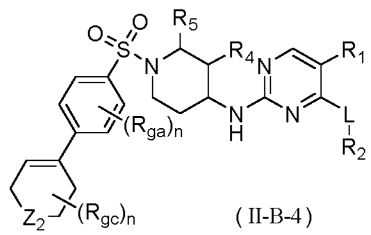

( II-B-4 )

wherein

$Z_1$ is selected from C, CH or N;
$Z_2$ is selected from $CH_2$, NH or O;
$R_1$, $R_2$, $R_4$, $R_5$, L, $R_g$, n, $R_{ga}$, $R_{gc}$ are as defined in any embodiment of the present disclosure.

In some embodiments of the present disclosure, for the compound, the stereoisomer thereof, the tautomer thereof, the pharmaceutically acceptable salt thereof, the prodrug thereof, the hydrate thereof, the solvate thereof, or the isotope-labeled derivative thereof, wherein the compound can be represented by any one of the following structures,

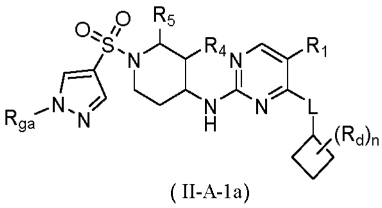
( II-A-1a)

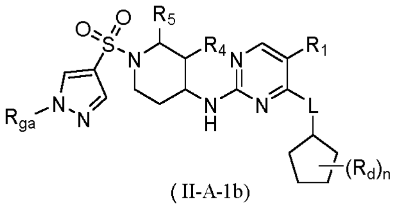
( II-A-1b)

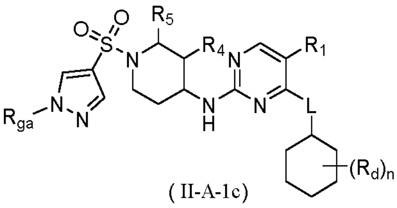
( II-A-1c)

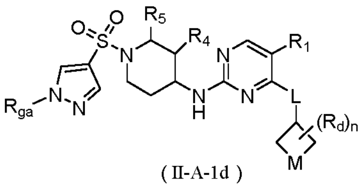
( II-A-1d )

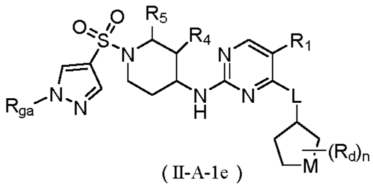
( II-A-1e )

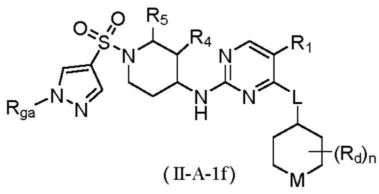
( II-A-1f)

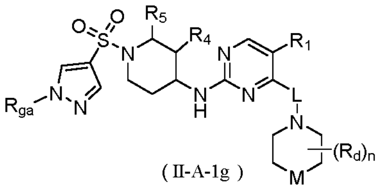
( II-A-1g )

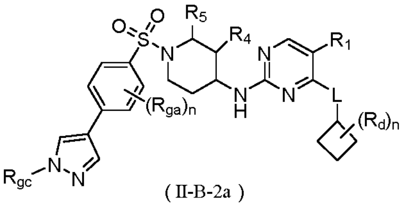
( II-B-2a )

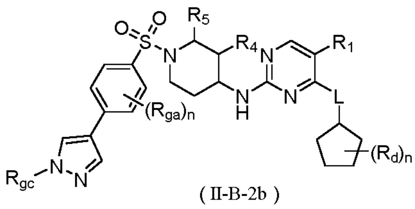
( II-B-2b )

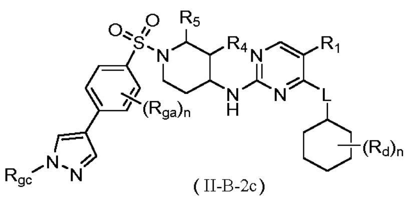
( II-B-2c)

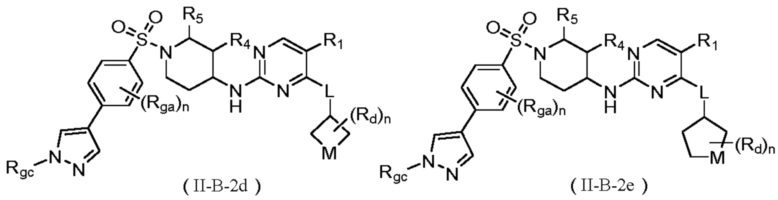
( II-B-2d ) ( II-B-2e )

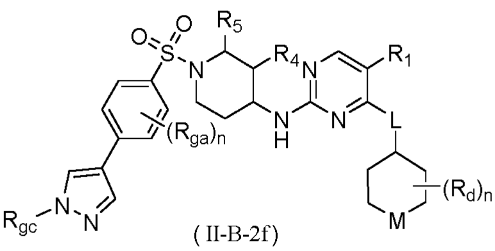
( II-B-2f)

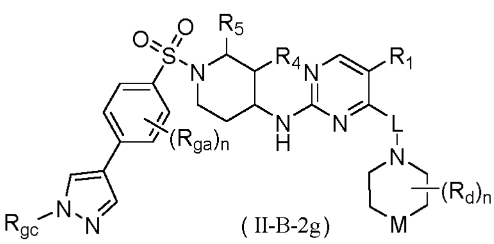
( II-B-2g)

wherein $R_1$, L, $R_d$, $R_g$, M, n, $R_4$, $R_5$, $R_{ga}$, $R_{gc}$ are as defined in any embodiment of the present disclosure.

In some embodiments of the present disclosure, for the compound, the stereoisomer thereof, the tautomer thereof, the pharmaceutically acceptable salt thereof, the prodrug thereof, the hydrate thereof, the solvate thereof, or the isotope-labeled derivative thereof, wherein the compound can be represented by any one of the following structures,

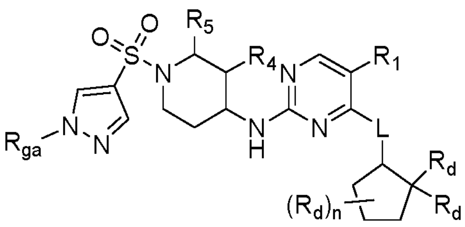

( II-A-1b-1 )

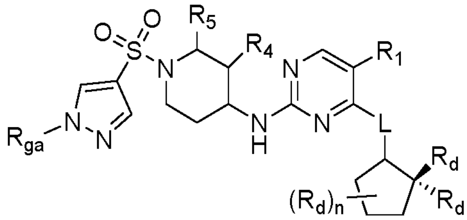

( II-A-1b-2 )

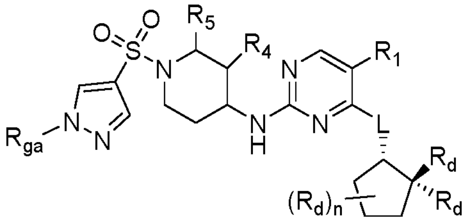

( II-A-1b-3 )

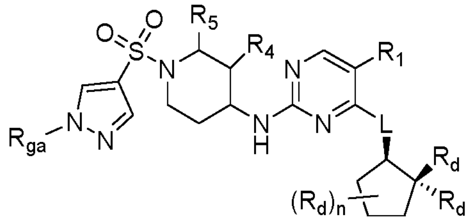

( II-A-1b-4 )

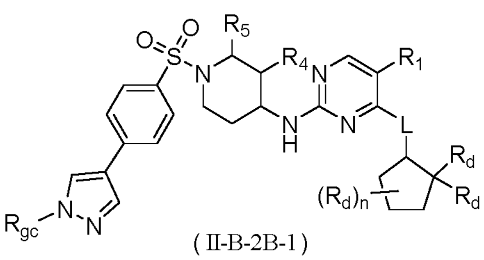

( II-B-2B-1 )

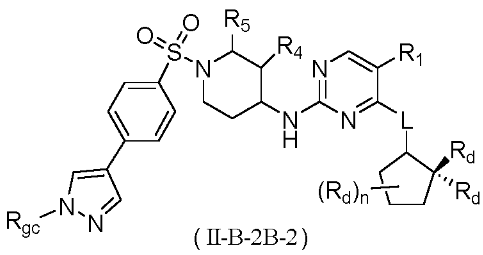

( II-B-2B-2 )

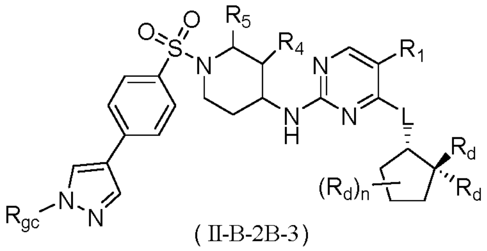

( II-B-2B-3 )

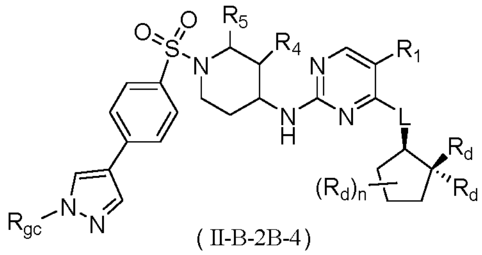

( II-B-2B-4 )

$R_1$, L, $R_d$, $R_g$, n, $R_4$, $R_5$, $R_{ga}$, $R_{gc}$ are as defined in any embodiment of the present disclosure.

The present disclosure further provides the following compound, a stereoisomer thereof, a tautomer thereof, a pharmaceutically acceptable salt thereof, a prodrug thereof, a hydrate thereof, a solvate thereof, or an isotope-labeled derivative thereof, wherein the compound can be selected from any one of the following structures,

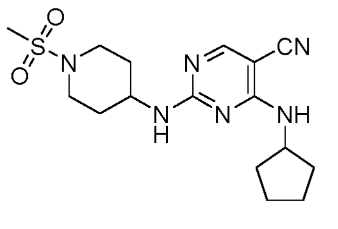

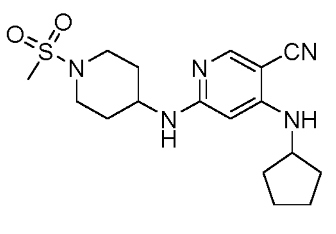

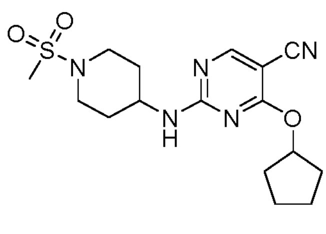

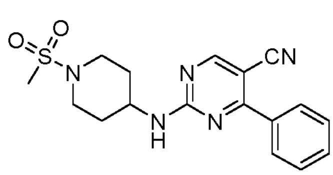
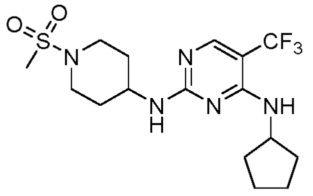
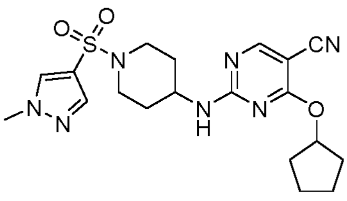

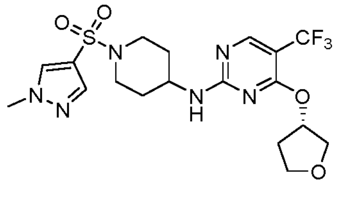
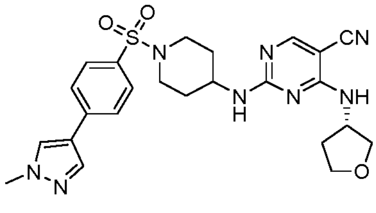
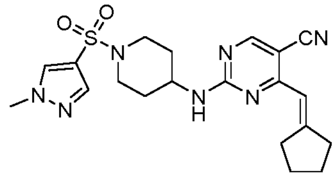

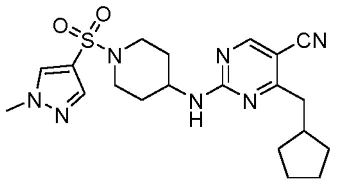
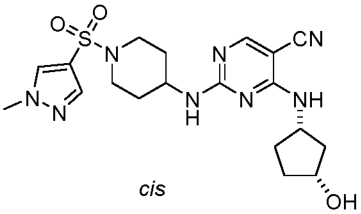

*cis*

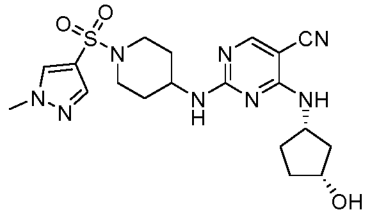

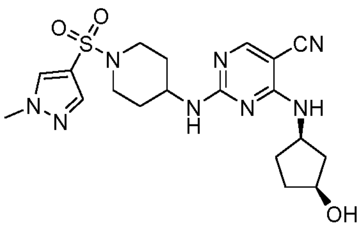
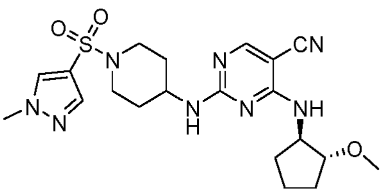
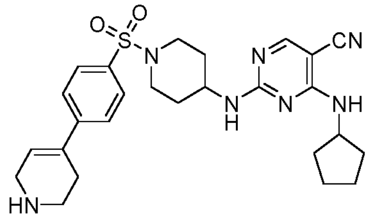

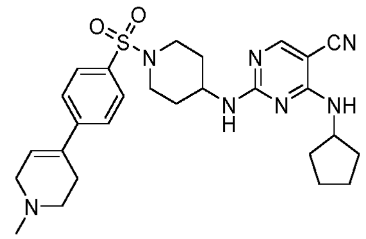
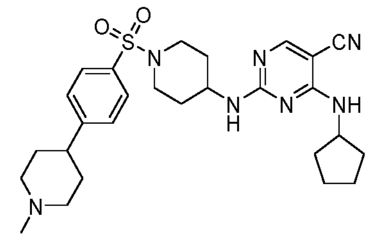
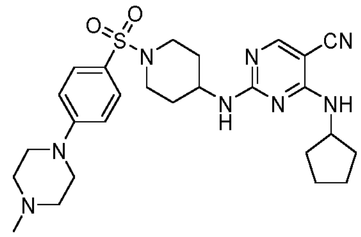

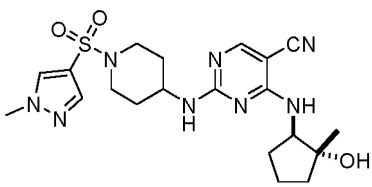
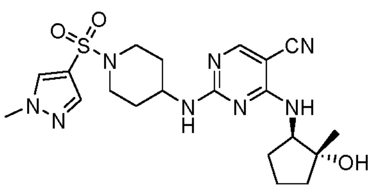
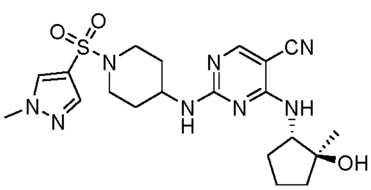

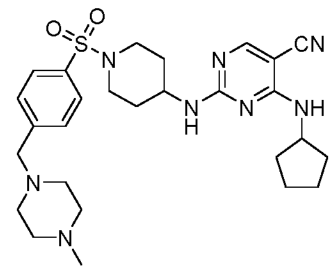
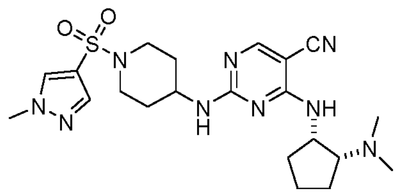

*cis*

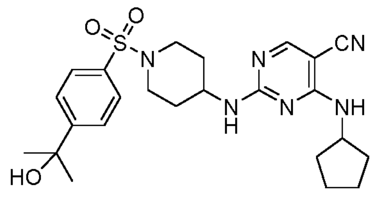

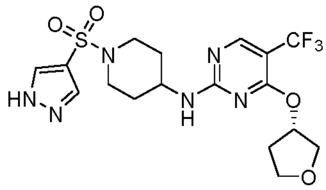
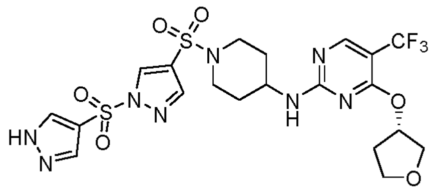
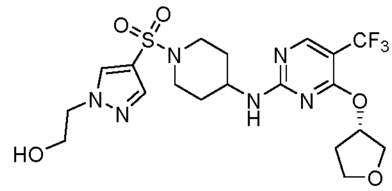

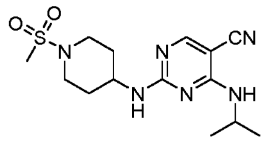
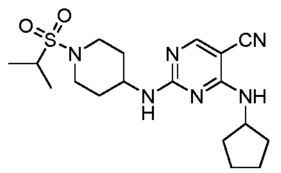
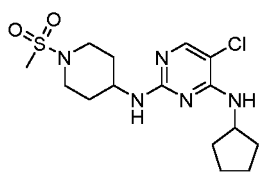
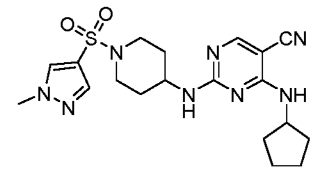

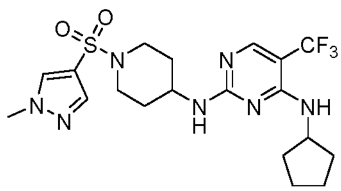
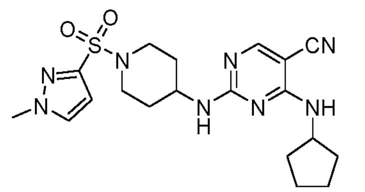
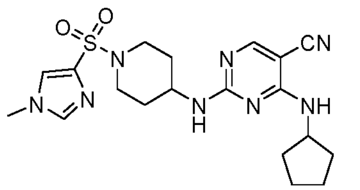

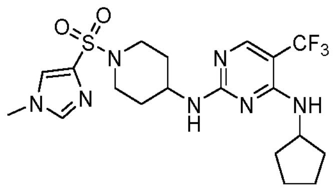
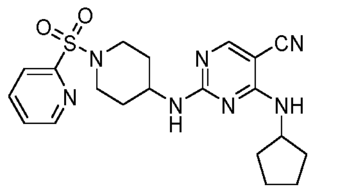
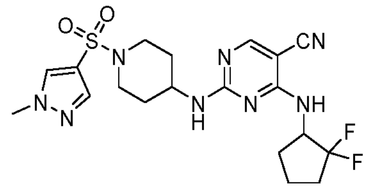

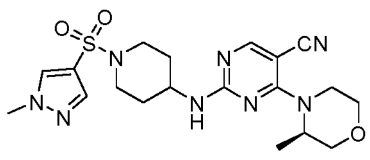
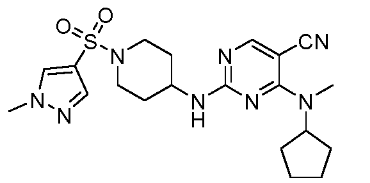
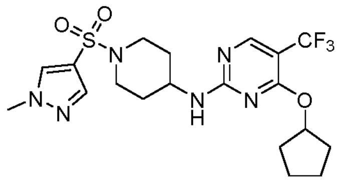

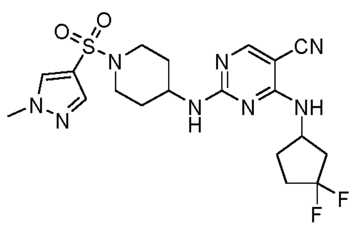
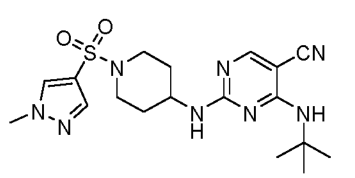
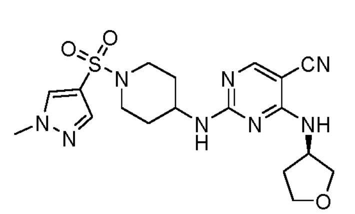

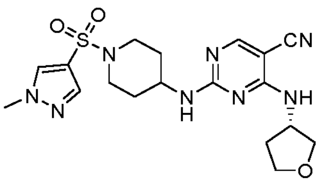
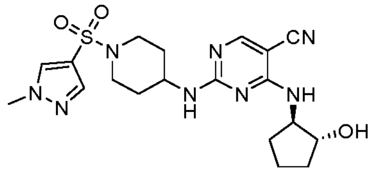
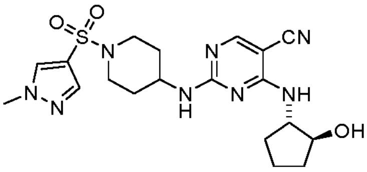

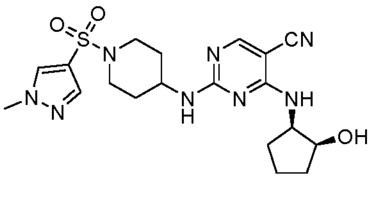
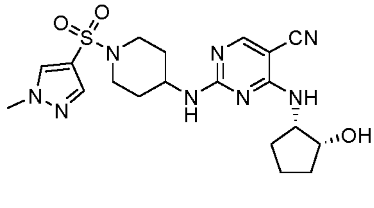
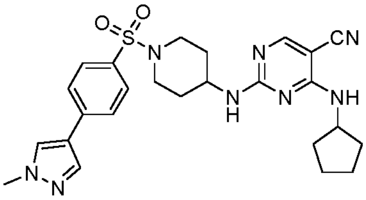

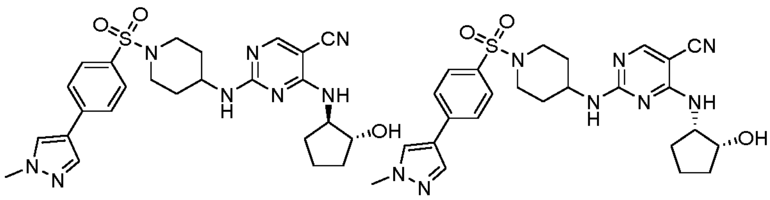
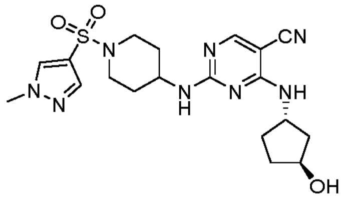

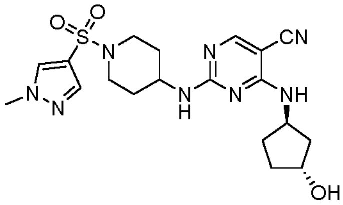
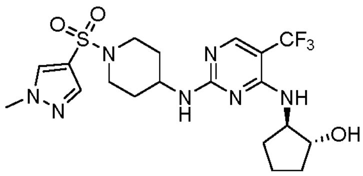
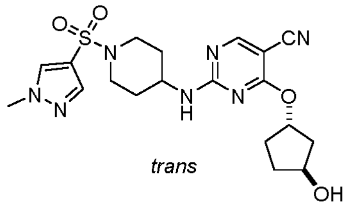

*trans*

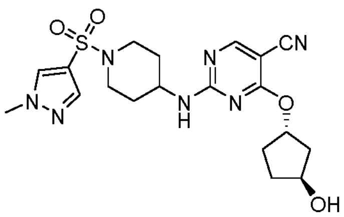
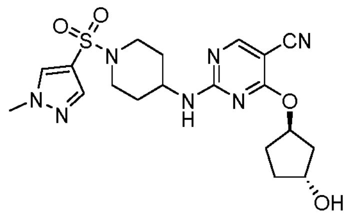
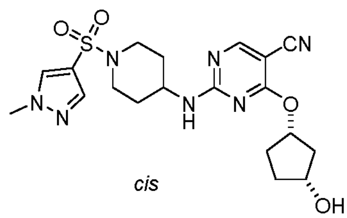

*cis*

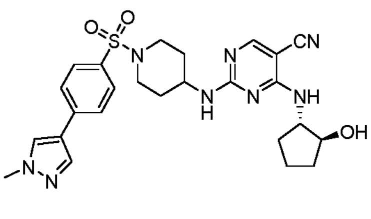
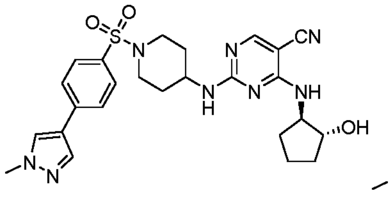
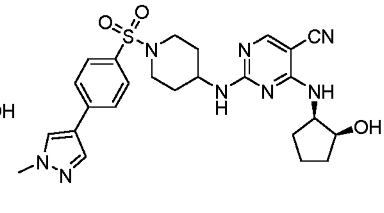

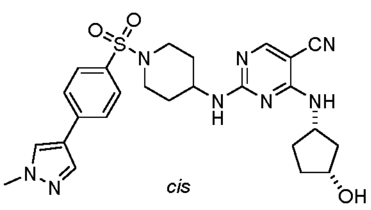

*cis*

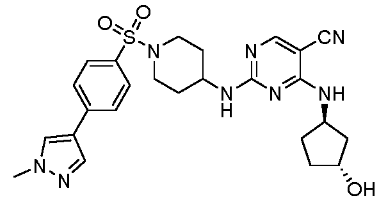
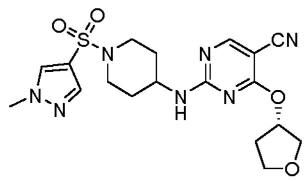

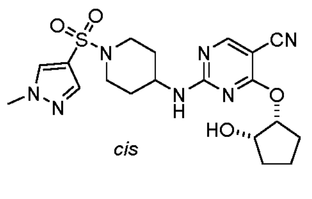

cis

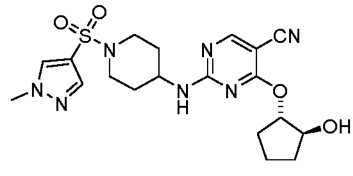

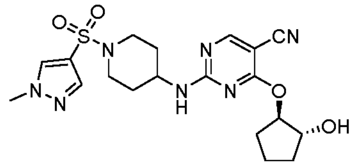

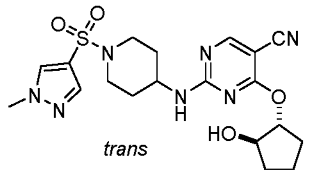

trans

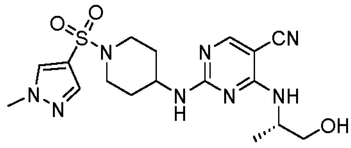

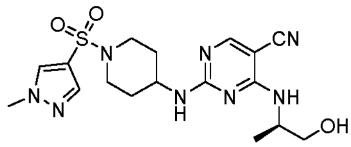

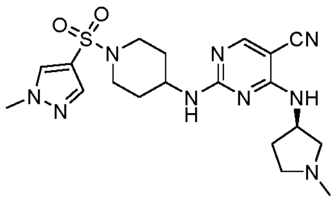

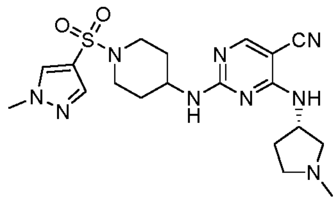

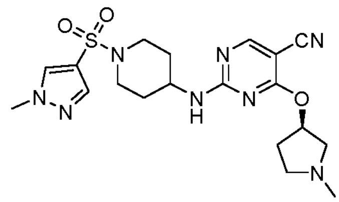

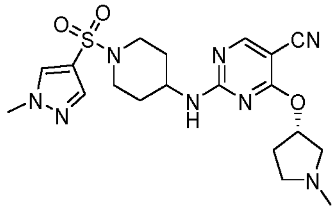

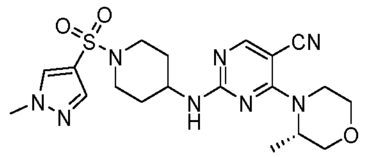

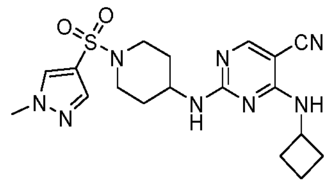

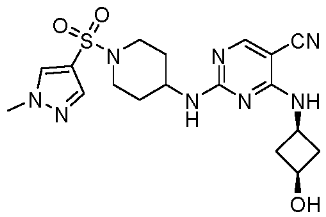

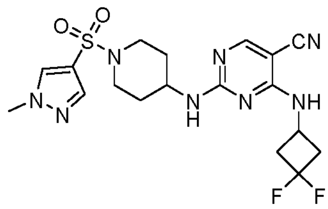

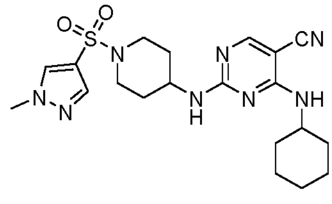

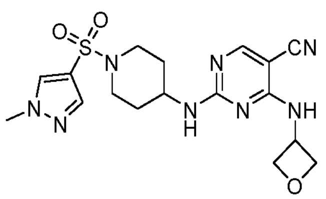

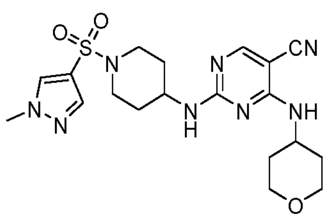

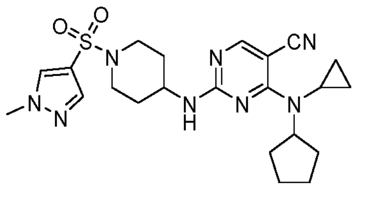

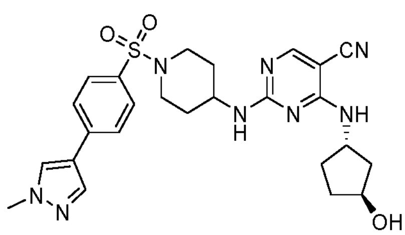

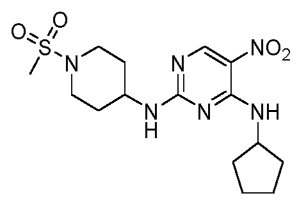

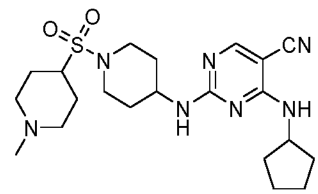

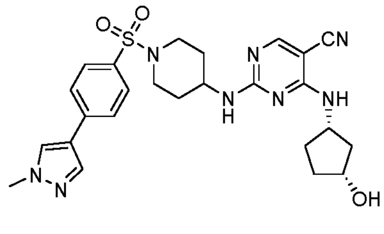 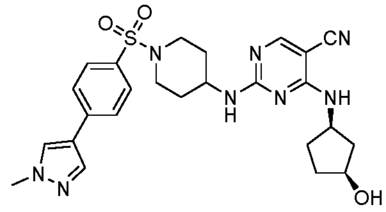 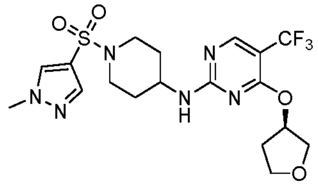

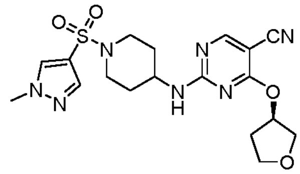 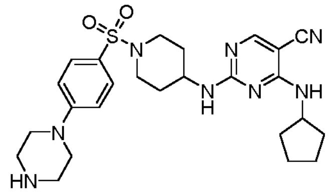 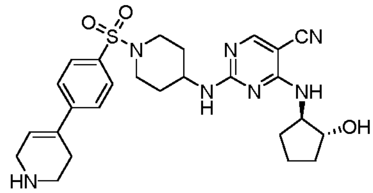

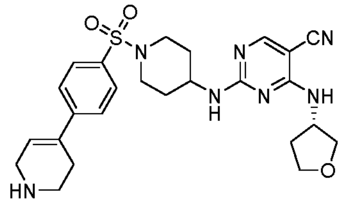 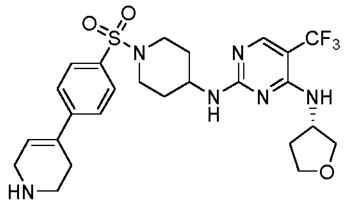 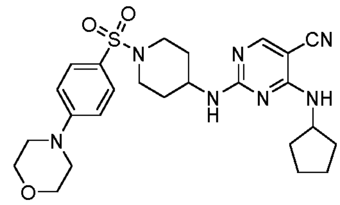

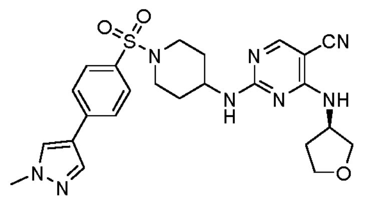 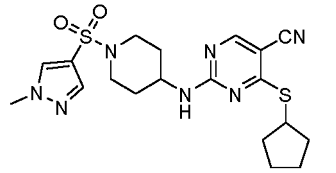 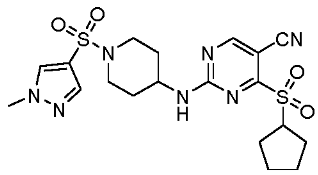

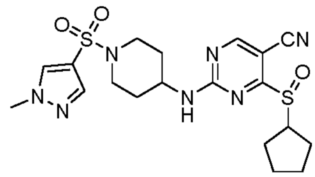 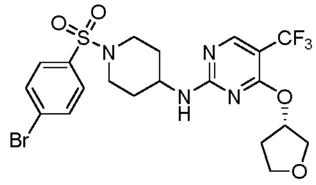 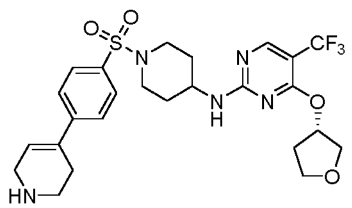

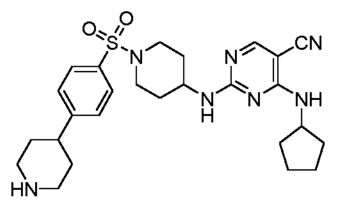 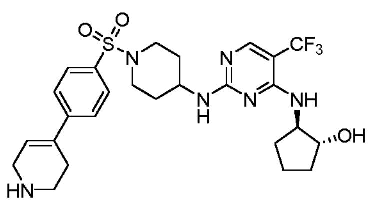 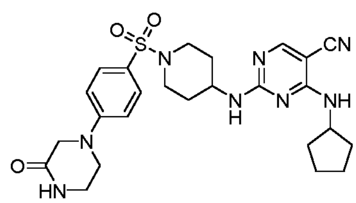

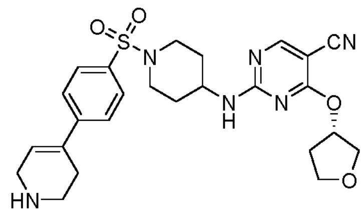 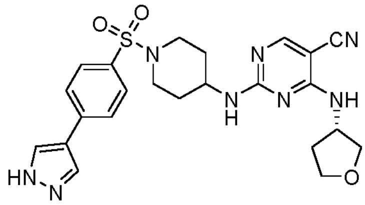 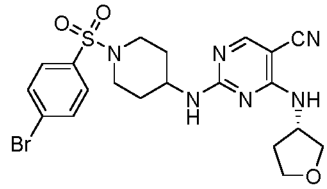

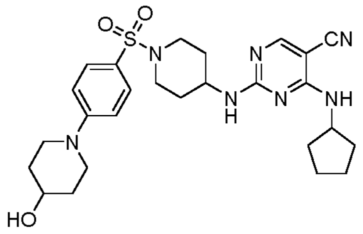
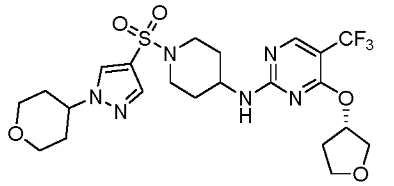
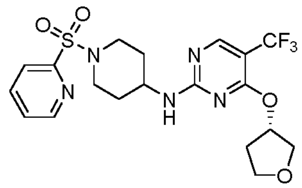

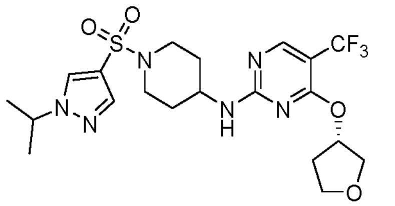
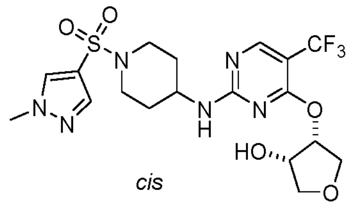

*cis*

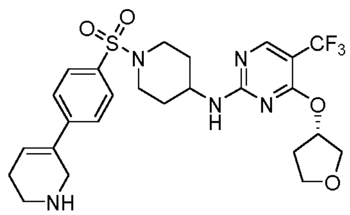
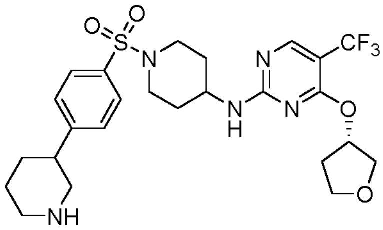
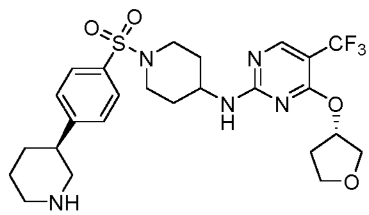

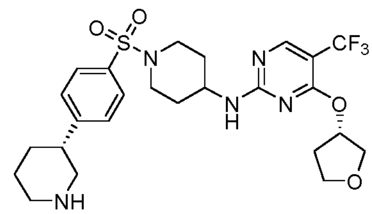
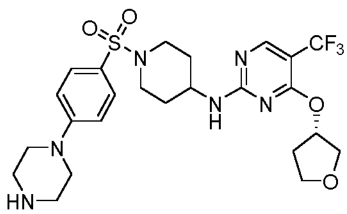
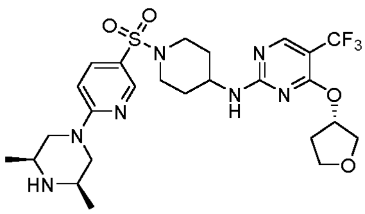

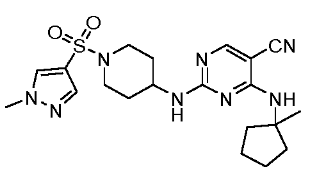
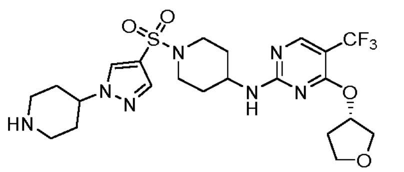
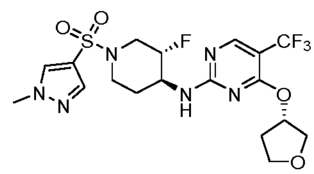

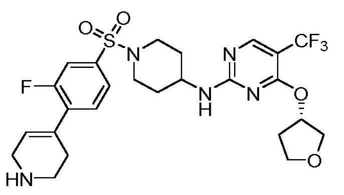
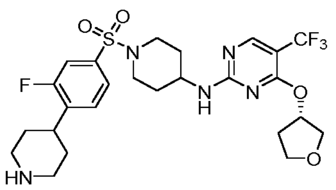
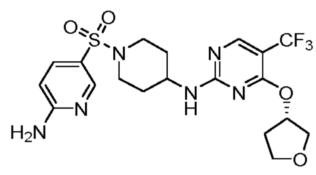

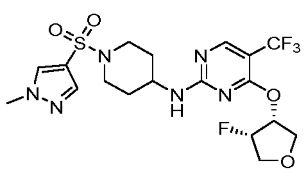
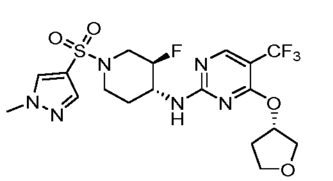
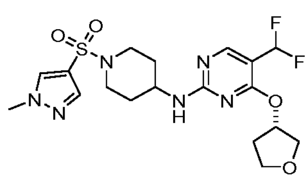

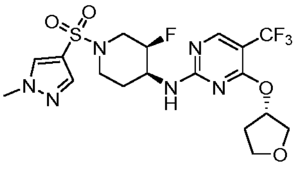

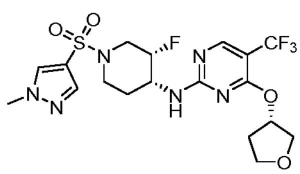

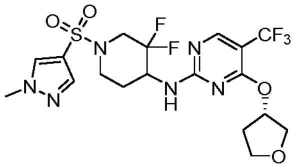

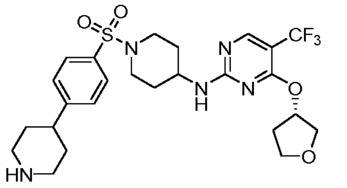

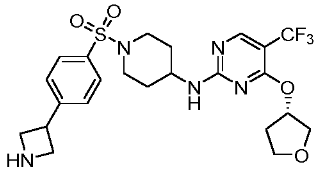

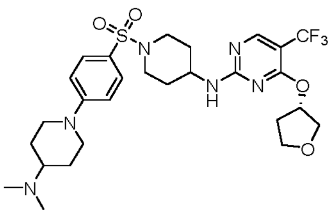

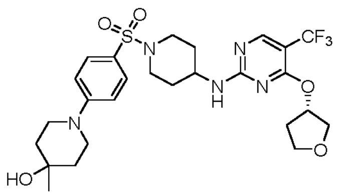

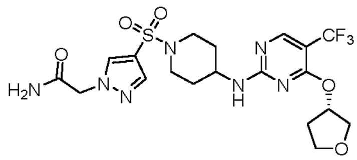

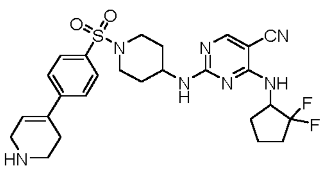

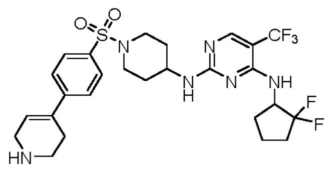

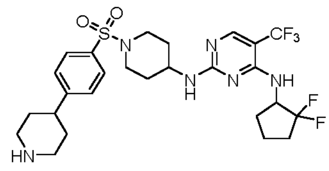

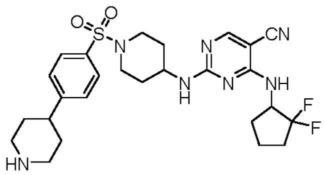

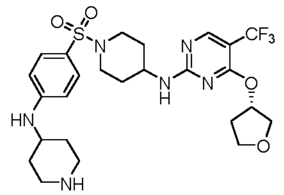

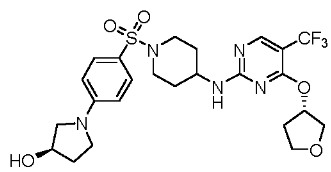

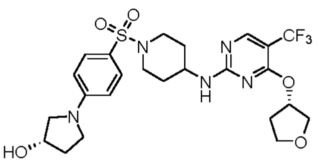

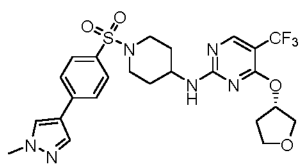

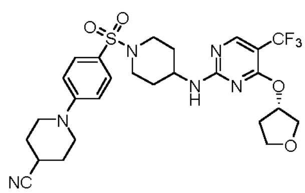

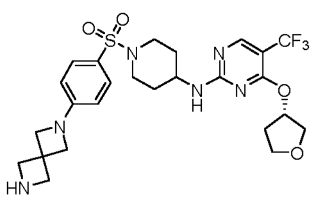

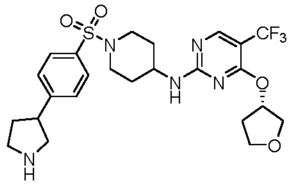

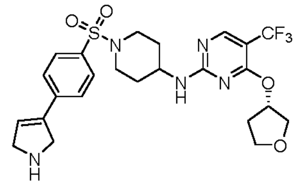

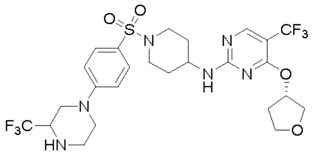
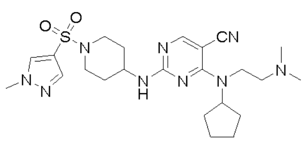
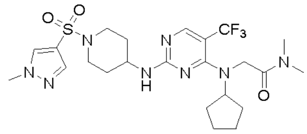

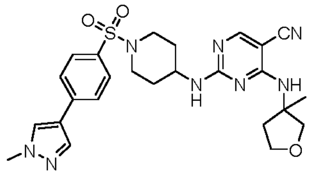
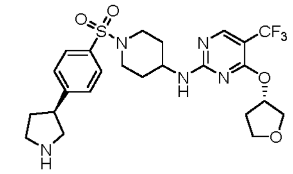
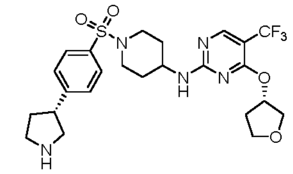

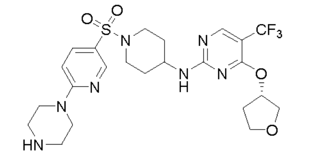
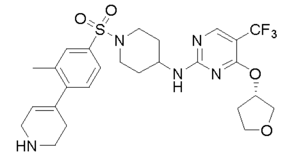
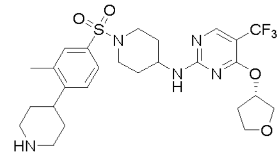

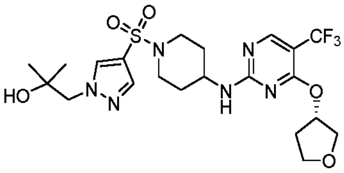
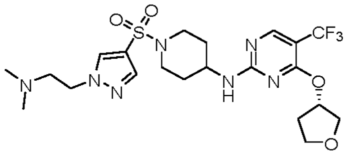
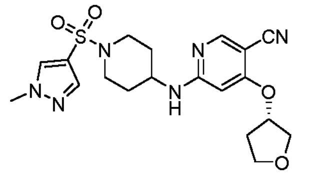

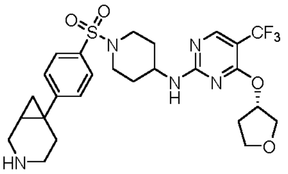
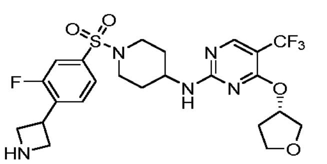
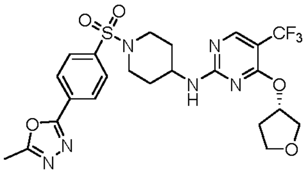

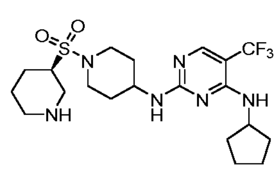
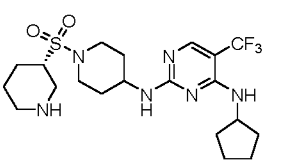
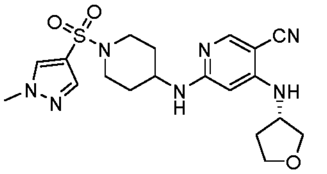

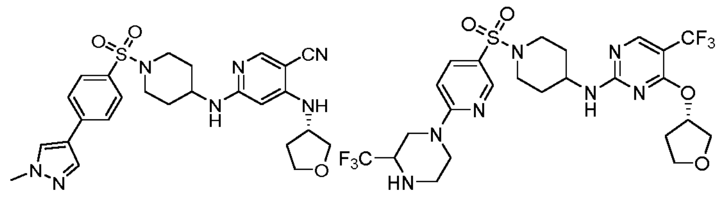
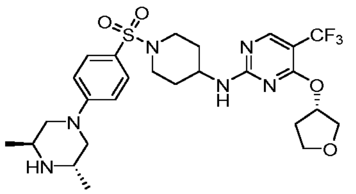

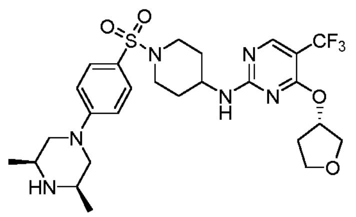
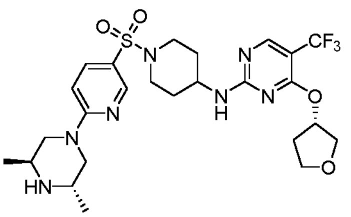
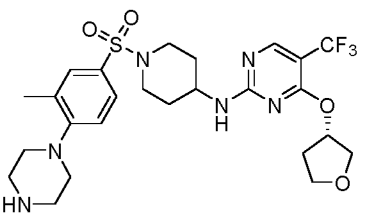

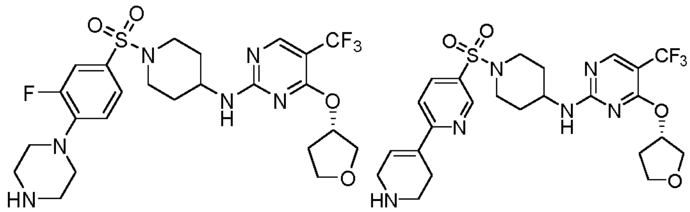
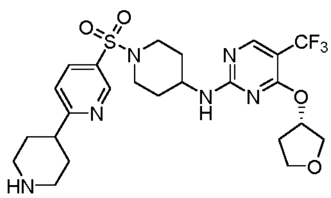

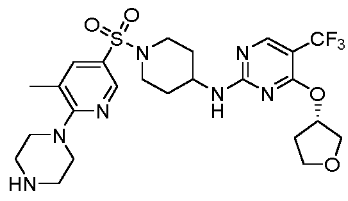
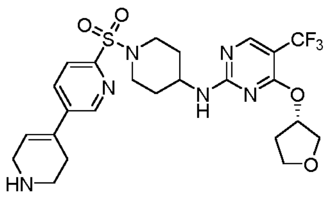
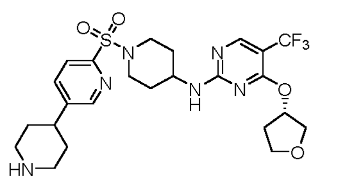

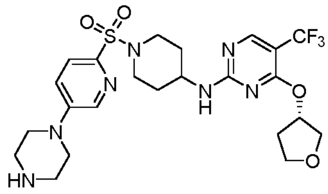
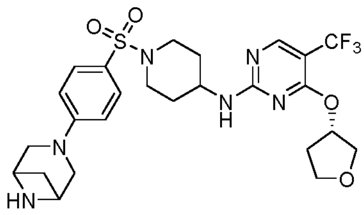
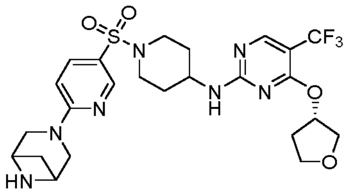

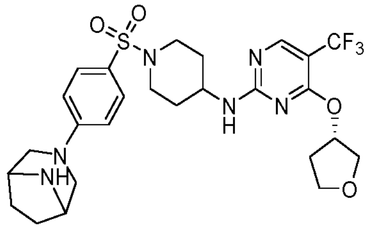
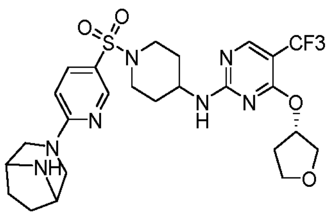
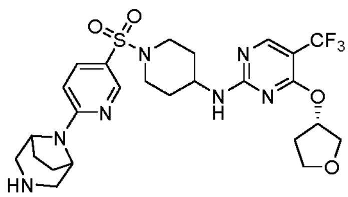

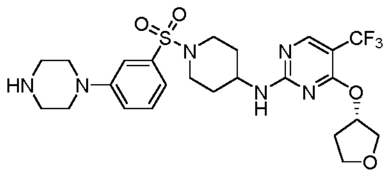
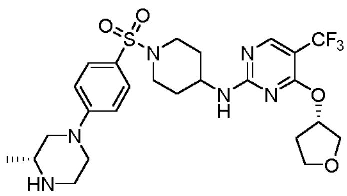
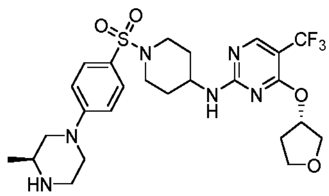

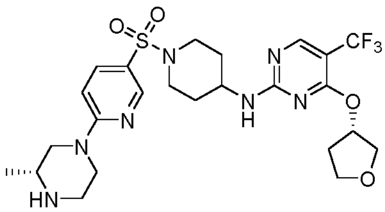
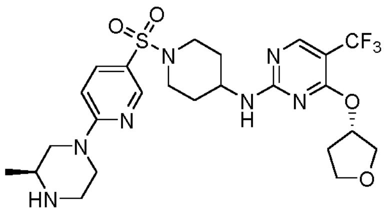
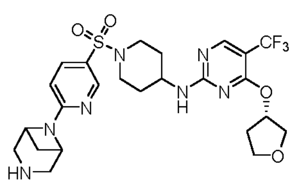

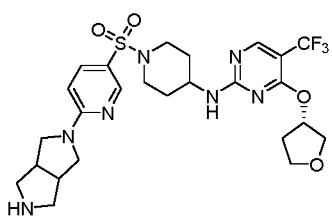
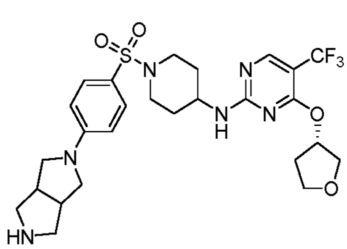
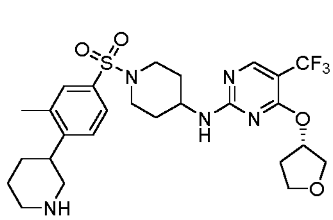

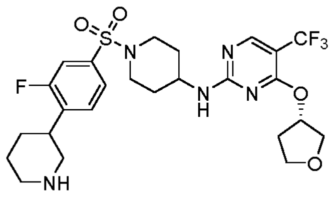
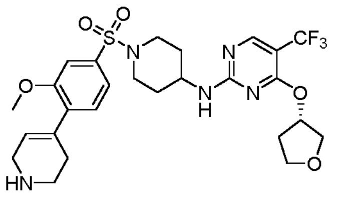
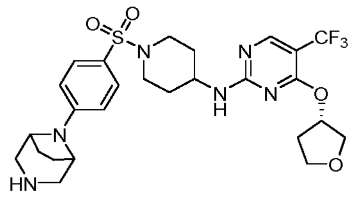

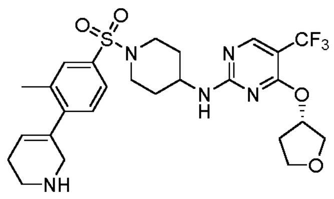
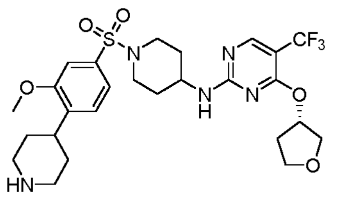
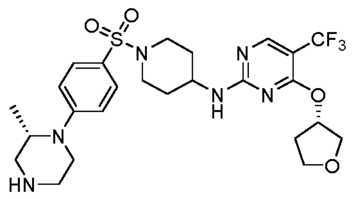

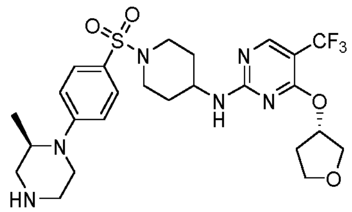
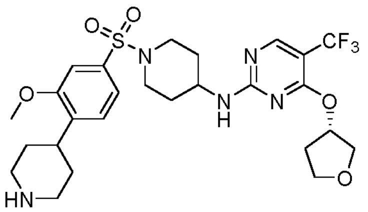
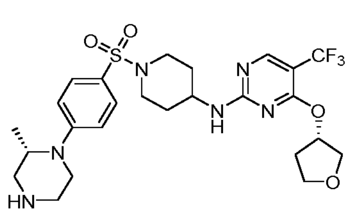

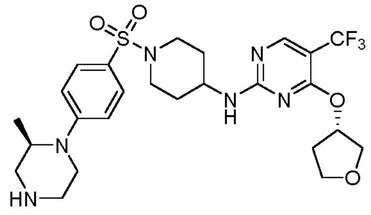
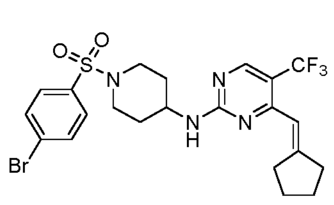
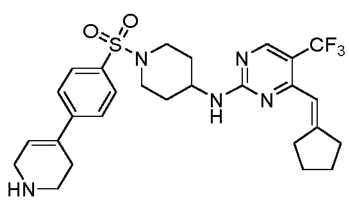

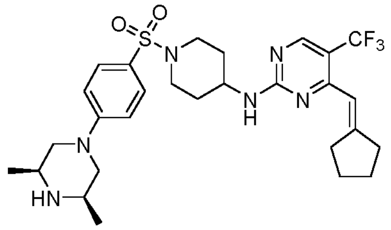
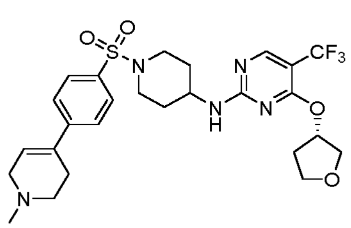
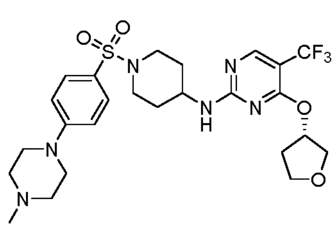

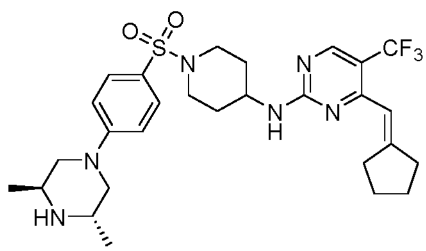
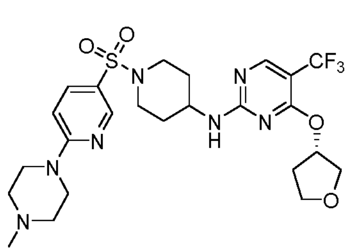

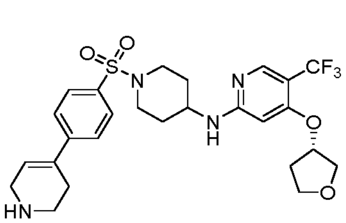
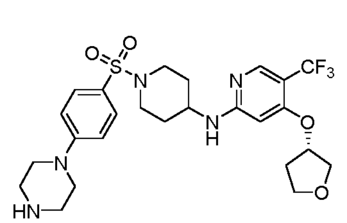
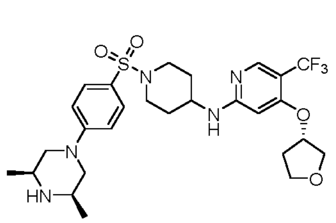

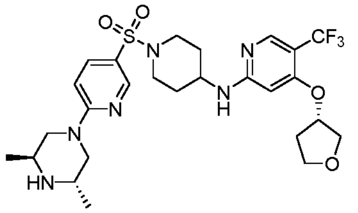
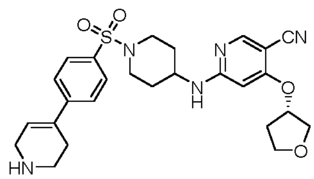
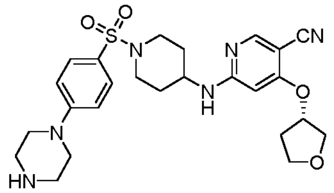

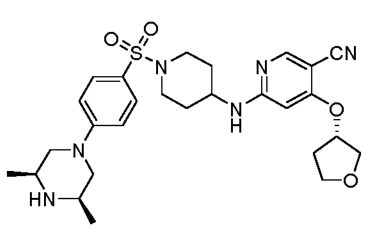
,
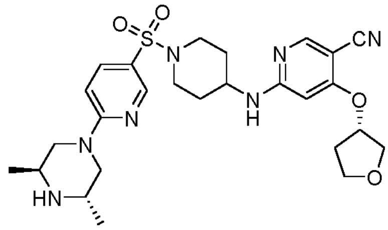
,
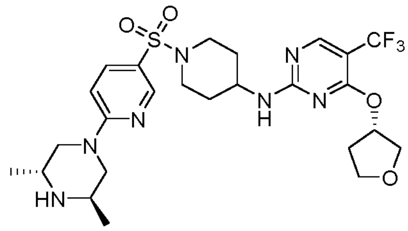
,

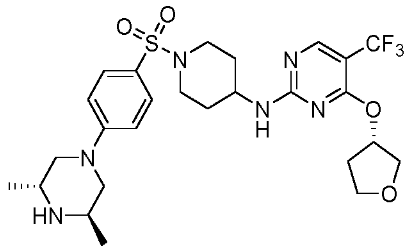
,
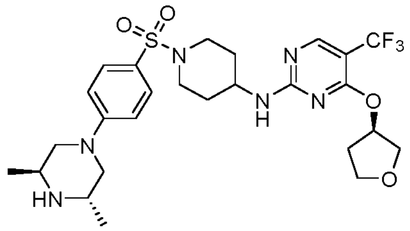
,

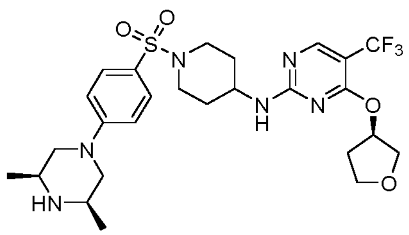
.

The present disclosure also provides a pharmaceutical composition comprising the (preferably a therapeutically effective amount of) compound, the stereoisomer thereof, the tautomer thereof, the pharmaceutically acceptable salt thereof, the prodrug thereof, the hydrate thereof, the solvate thereof, or the isotope-labeled derivative thereof; and a pharmaceutically acceptable carrier, diluent and excipient. The pharmaceutical composition can be formulated for specific routes of administration, such as oral administration, parenteral administration, rectal administration, and the like. Oral administration, for example, a tablet, a capsule (including a sustained release or timed release prescription), a pill, a powder, a granule, an elixir, a tincture, a suspension (including a nano-suspension, a micro-suspension, a spray-dried dispersant), a syrup and an emulsion; sublingual administration; buccal administration; parenteral administration, for example, by subcutaneous, intravenous, intramuscular, or intrasternal injection, or infusion techniques (for example, as a sterile injectable aqueous solution or non-aqueous solution or a suspension); nasal administration, including administration to the nasal mucosa, for example, by inhalation spray; topical administration, for example, in the form of a cream or an ointment; or rectal administration, for example, in the form of a suppository. They can be administered alone, but they are usually administered together with a pharmaceutical carrier selected according to the selected route of administration and standard pharmaceutical practice.

"Pharmaceutically acceptable carrier" refers to a medium generally acceptable in the art for delivering a biologically active agent to animals, particularly mammals, including, for example, an adjuvant, an excipient or a vehicle, such as a diluent, a preservative, a filler, a flow regulator, a disintegrant, a wetting agent, an emulsifier, a suspending agent, a sweetener, a flavor, a fragrance, an antibacterial agent, an antifungal agent, a lubricants agent and a dispersant, according to the mode of administration and the nature of dosage forms. The pharmaceutically acceptable carrier is formulated according to a number of factors that are within the purview of those skilled in the art. The factors include, but are not limited to, the type and nature of the active agent formulated, the subjects to which the composition containing the agent is to be administered, the expected route of administration of the composition, and the target therapeutic

indication. The pharmaceutically acceptable carriers include both aqueous and non-aqueous media and various solid and semisolid dosage forms. In addition to the active agent, such carriers include many different ingredients and additives, and such additional ingredients included in prescriptions for a variety of reasons (e.g., stabilizing the active agent and an adhesive, etc.) are well known to those of ordinary skilled in the art.

It is certain that administration regimens for the compounds of the present disclosure may vary depending on known factors, such as the pharmacodynamic characteristics of a specific drug and its mode and route of administration, the species, age, sex, health, medical condition and weight of the recipient, nature and extent of symptoms, types of coexisting treatments, frequency of treatments, routes of administration, renal and hepatic function of the patient, and desired effects. A therapeutically effective amount of the compound, a pharmaceutical composition or a combination thereof depends on the species, weight, age and individual condition of the subject, the disorder or disease being treated or its severity. A physician, clinician or veterinarian of ordinary skill may readily determine the effective amount of each active ingredient required to prevent, treat or inhibit the progression of a disorder or disease.

Serving as an important regulator in the cell cycle, CDK2 forms a kinase complex with cyclin E or cyclin A, and plays a decisive role in the process of driving the cell cycle from G1 phase to S phase and maintaining S phase. The main mechanism is that cyclin E and CDK2 work together to phosphorylate the retinoblastoma susceptibility gene (Rb) protein. The phosphorylation of the Rb protein leads to the release of E2F (a transcription factor). The released E2F binds to the upstream of some genes (usually locates in the promoter region or enhancer region), initiating the transcriptional expression of those genes related to the cell cycle, so that the cells enter the S phase at the end of G1. Many studies have shown that the abnormal expression of CDK2 is closely related to the occurrence of cancer, such as ovarian cancer with CCNE1 amplification, KRAS mutant lung cancer, hormone-dependent breast and prostate cancer, etc. (Tadesse S, Anshabo AT, Portman N, Lim E, Tilley W, Caldon CE, Wang S, Targeting CDK2 in cancer: challenges and opportunities for therapy, Drug Discovery Today, 2020, 25, 406-413).

The present disclosure also provides a use of the compound, the stereoisomer thereof, the tautomer thereof, the pharmaceutically acceptable salt thereof, the prodrug thereof, the hydrate thereof, the solvate thereof, or the isotope-labeled derivative thereof, or the pharmaceutical composition in the manufacture of a medicament (preferably a medicament for the treatment of CDK-mediated cancer).

The present disclosure also provides a method for the treatment of CDK-mediated cancer, comprising administering to a patient a therapeutically effective amount of the compound, the stereoisomer thereof, the tautomer thereof, the pharmaceutically acceptable salt thereof, the prodrug thereof, the hydrate thereof, the solvate thereof, or the isotope-labeled derivative thereof, or the pharmaceutical composition.

In some embodiments of the present disclosure, in the use, the cancer comprises ovarian cancer, breast cancer, acute myeloid leukemia (AML), chronic lymphocytic leukemia (CLL) or small lymphocytic lymphoma (SLL).

The present disclosure also provides a compound of formula (III), a stereoisomer thereof or a pharmaceutically acceptable salt thereof,

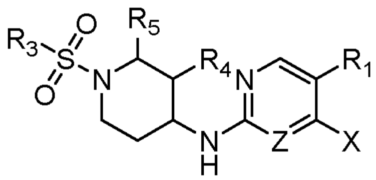

( III )

wherein

X is selected from halogen, OH, $-SO_2Me$, -OMs, OTf, OTs and H; preferably halogen (e.g. Cl);
Z, $R_1$, $R_3$, $R_4$, $R_5$ are as defined in any embodiment of the present disclosure.

In some embodiments of the present disclosure, the compound of formula (III) is selected from

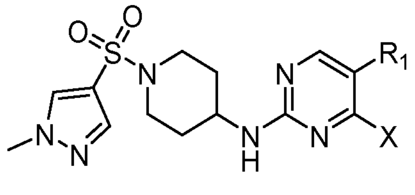

III-A

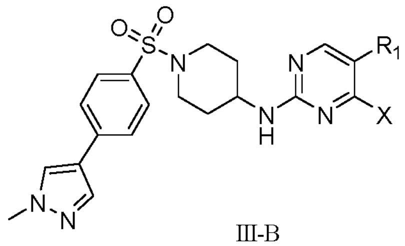

III-B

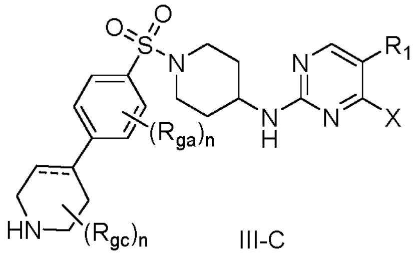

III-C

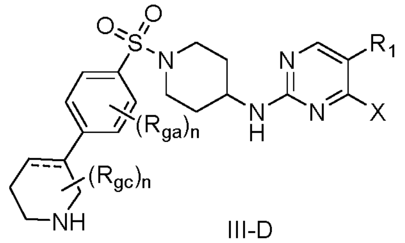

III-D

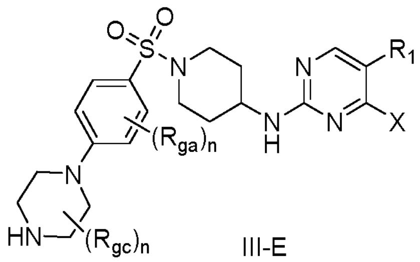

III-E

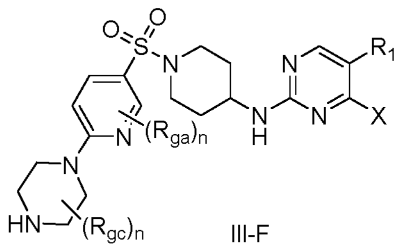

III-F

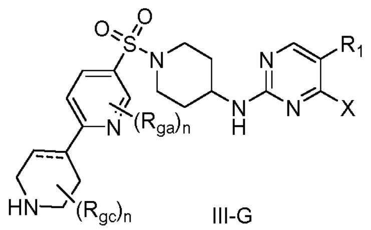

III-G

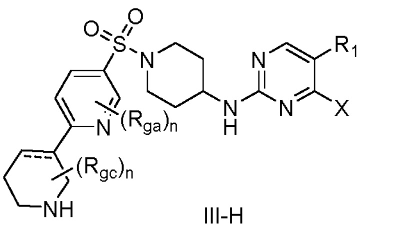

III-H

wherein $R_1$, X, $R_{ga}$, $R_{gc}$ and n are as defined in any embodiment of the present disclosure.

The present disclosure also provides compounds of formula (IV-1) and formula (IV-2), a stereoisomer thereof or a pharmaceutically acceptable salt thereof,

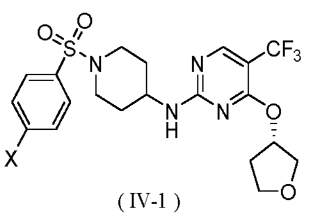

( IV-1 )

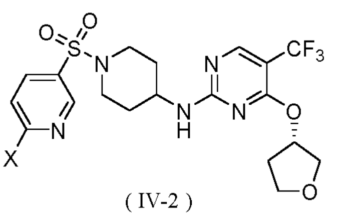

( IV-2 )

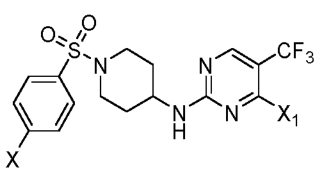

( IV-3 )

wherein X is selected from halogen, OH, $-SO_2Me$, -OMs, OTf, OTs; preferably halogen (e.g. Br).

X is selected from halogen, OH, $-SO_2Me$, -OMs, OTf, OTs; preferably halogen (e.g. Cl).

The above compounds are used to prepare CDK inhibitor compounds of formula (I-A), formula (I-B) and formula (I) in the present disclosure.

**Technical effect:**

The compounds of the present disclosure have better CDK 2/4/6 kinase inhibitory activity, especially excellent

in the inhibitory activity of CDK 2 kinase; the compounds of the present disclosure can selectively inhibit CDK 2/4/6 kinases and especially has good selectivity for CDK 2 kinase. Some compounds have inhibitory selectivity of nearly ten times, even tens of times, or 100 times or more on CDK 1/7/9 kinases compared with CDK 2 kinase.

The compounds of the present disclosure have better inhibitory activity toward cell proliferation, showing better tumor inhibitory activity and good tolerance in drug efficacy experiments in vivo.

**Description and definition**

Unless otherwise specified, the following terms and phrases when used herein have the following meanings. A specific term or phrase should not be considered indefinite or unclear in the absence of a particular definition, but should be understood in the ordinary sense.

The term "pharmaceutically acceptable" refers to those compounds, materials, compositions and/or dosage forms, which are suitable for use in contact with human and animal tissues within the scope of reasonable medical judgment, without excessive toxicity, irritation, allergic reaction or other problems or complications, and is commensurate with a reasonable benefit/risk ratio.

The term "pharmaceutically acceptable salt" refers to a derivative prepared by the compound of the present disclosure with a relatively nontoxic acid or base. These salts may be prepared during the synthesis, separation and purification of the compound, or the free form of the purified compound may be used alone to react with a suitable acid or base. When the compound contains a relatively acidic functional group, the compound reacts with alkali metal, alkaline earth metal hydroxide or organic amine to obtain an alkali addition salt, including cations based on alkali metals and alkaline earth metals and non-toxic ammonium, quaternary ammonium and amine cations. Salts of amino acids are also covered. When the compound contains a relatively basic functional group, the compound reacts with an organic acid or an inorganic acid to obtain an acid addition salt.

The compounds provided by the present disclosure also include the form of prodrugs, which means that the compounds which are rapidly converted in vivo to the parent compound of the above formula are converted into the compounds of the present disclosure in the in vivo or in vitro environment by chemical or biochemical methods, such as hydrolysis in blood.

The compounds of the present disclosure may exist in unsolvated as well as solvated forms, and the solvated form includes a hydrate form. In general, the solvated form is equivalent to the unsolvated form, which is also encompassed within the scope of the present disclosure.

The compounds of the present disclosure have geometric isomers as well as stereoisomers, such as cis-trans isomers, enantiomers, diastereoisomers, and racemic mixtures thereof, and other mixtures, all of which are within the scope of the present disclosure.

The term "enantiomer" refers to stereoisomers that are mirror images of each other.

The term "diastereomer" refers to stereoisomers in which the molecules have two or more chiral centers and the relationship between the molecules is not mirror images.

The term "cis-trans isomer" refers to a configuration in which a double bond or a single bond of a ring-forming carbon atom in a molecule cannot rotate freely.

Unless otherwise specified, the term "tautomer" or "tautomeric form" means that isomers with different functional groups are in dynamic equilibrium at room temperature and are rapidly interconvertible. If tautomers are possible (e.g., in solution), then chemical equilibrium of the tautomers can be achieved. For example, keto-enol isomerization and imine-enamine isomerization.

Unless otherwise specified, the absolute configuration of a stereogenic center is represented by a wedged solid bond 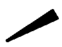 and a wedged dashed bond 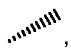, and the relative configuration of a stereogenic center is represented by a straight solid bond 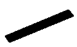 and a straight dashed bond 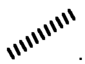. For example,

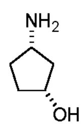

represents that the hydroxyl group and the amino group are located on the same side of cyclopentane, which can be

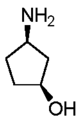 or 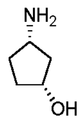.

Stereoisomers of the compounds of the present disclosure may be prepared by chiral synthesis or chiral reagents or other conventional techniques. For example, an enantiomer of a certain compound of the present disclosure may be prepared by an asymmetric catalysis technique or a chiral auxiliary derivatization technique. Alternatively, compounds with a single stereo configuration may be obtained from a mixture by a chiral resolution technique. Alternatively, it can be prepared directly from chiral starting materials. Separation of optically pure compounds in the present disclosure is usually accomplished by preparative chromatography, and a chiral chromatographic column is used to achieve the purpose of separating chiral compounds.

The absolute stereo configuration of a compound may be confirmed by conventional technical means in the art. For example, a single crystal X-ray diffraction method may also confirm the absolute configuration of the compound by the chiral structure of the raw material and the reaction mechanism of asymmetric synthesis. Compounds marked herein as "absolute configuration not determined" are typically split from racemic compounds into single isomers by chiral preparative SFC, which are then characterized and tested.

For example, the cis-compound 11 shown below is split by SFC chiral preparation to obtain compound 12 and compound 13 in a single configuration. Compounds 12 and 13 are enantiomers of each other, but the absolute stereo configurations corresponding to compounds 12 and 13 cannot be determined.

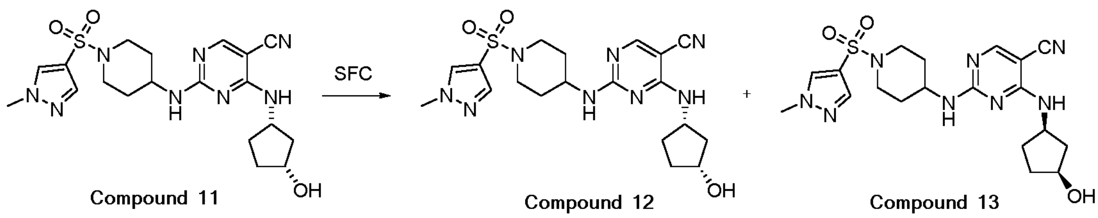

Compound 11 Compound 12 Compound 13

The term "optically pure" or "enriched in enantiomers" refers to the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

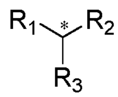

It indicates that the carbon atom is a chiral carbon atom, and the structure represents an optically pure compound in which the stereo configuration of the carbon atom is (*R*) configuration or (*S*) configuration and a mixture thereof, and the ratio of the mixture may be 1:1 or other ratios. For example,

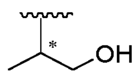

represents that the structure may be

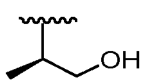, 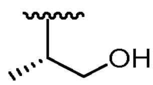,

or a mixture of the two, and when the ratio of the mixture is 1:1, the structure is a racemic compound

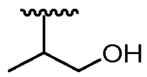;

the present disclosure also comprises isotope-labeled compounds, including isotopes of hydrogen, carbon, nitrogen, oxygen, phosphorus, sulfur, fluorine and chlorine, such as $^2H$, $^3H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$. Compounds of the present disclosure containing the above isotopes and/or other isotopes of other atoms are within the scope of the present disclosure.

The term "pharmaceutically acceptable carrier" refers to a medium generally acceptable in the art for delivering a biologically active agent to animals, particularly mammals, including, for example, an adjuvant, an excipient or a vehicle, such as a diluent, a preservative, a filler, a flow regulator, a disintegrant, a wetting agent, an emulsifier, a suspending agent, a sweetener, a flavor, a fragrance, an antibacterial agent, an antifungal agent, a lubricants agent and a dispersant, according to the mode of administration and the nature of dosage forms. The pharmaceutically acceptable carrier is formulated according to a number of factors that are within the purview of those skilled in the art. The factors include, but are not limited to, the type and nature of the active agent formulated, the subjects to which the composition containing the agent is to be administered, the expected route of administration of the composition, and the target therapeutic indication. The pharmaceutically acceptable carriers include both aqueous and non-aqueous media and various solid and semisolid dosage forms. In addition to the active agent, such carriers include many different ingredients and additives, and such additional ingredients included in prescriptions for a variety of reasons (e.g., stabilizing the active agent and an adhesive, etc.) are well known to those of ordinary skilled in the art.

The term "excipient" generally refers to a carrier, diluent and/or medium required to formulate an effective pharmaceutical composition.

The term "prophylactically or therapeutically effective amount" refers to a sufficient amount of the compound or the pharmaceutically acceptable salt thereof of the present disclosure, which is sufficient to treat a disorder at a reasonable effect/risk ratio suitable for any medical treatment and/or prophylaxis. It should be recognized that the total daily dose of the compound of formula I, or the pharmaceutically acceptable salt thereof and the composition thereof in the present disclosure, is required to be determined by the attending physician within the scope of reliable medical judgment. For any specific patient, the specific therapeutically effective dose level depends on a variety of factors, including a disorder being treated and the severity of the disorder; activity of a specific compound used; a specific composition used; age, weight, general health status, sex and diet of the patient; administration time, administration route and excretion rate of the specific compound used; duration of treatment; drugs used in combination or simultaneously with the specific compounds used; and similar factors known in the medical field.

The term "optionally substituted" means an atom can be substituted by a substituent or not, unless otherwise specified, the type and number of the substituent may be arbitrary as long as being chemically achievable. For example, the term "optionally substituted by one or more than one $R_d$" means that an atom may or may not be substituted by one or more than one $R_d$.

When any variable (such as $R_d$) occurs in the constitution or structure of the compound more than once, the definition of the variable at each occurrence is independent. For example,

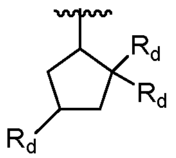

indicates that the cyclopentyl is substituted by 3 $R_d$, and each $R_d$ has an independent option.

When the number of a linking group is 0 or defined as a bond, such as $-O(CH_2)_nCH_3$, n = 0 indicates that the linking group is a single bond, i.e., $-OCH_3$; for example, $R_3$ is $-L_1-(C_{3-6}$ cycloalkyl), $L_1$ is a bond or optionally and independently selected from $C_{1-4}$ alkylene, when $L_1$ is a bond, it indicates that $L_1$ does not exist, i.e., $R_3$ is $-(C_{3-6}$ cycloalkyl).

When the bond of a substituent may be cross-connected to two atoms on a ring, the substituent may be bonded to any atom on the ring. For example, the structure moiety

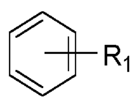

indicates that the substituent $R_1$ may be substituted at any position on a benzene ring.

When a listed substituent does not indicate through which atom it is attached to a compound included in the general formula but not specifically mentioned, such substituent may be bonded through any of its atoms. For example, pyrazole serves as a substituent indicates that any carbon atom on a pyrazole ring is connected to a substituted group; when  or ----- appears in the structure, it indicates that the atom is a bonded atom, for example,

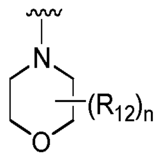 and 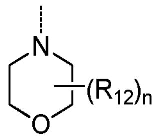

both indicate that the N atom on a morpholine ring is a bonded atom.

Unless otherwise specified, "ring" refers to saturated, partially saturated or unsaturated monocyclic and polycyclic rings, and the "polycyclic rings" include a linked ring, a spiro ring, a fused ring and a bridged ring. Representative "rings" include substituted or unsubstituted cycloalkyl, heterocycloalkyl, cycloalkenyl, heterocycloalkenyl, cycloalkynyl, heterocycloalkynyl, aryl, or heteroaryl. The term "hetero" refers to substituted or unsubstituted heteroatoms and oxidized forms of heteroatoms, the heteroatoms being generally selected from N, O, S, and the oxidized forms generally including NO, SO and $S(O)_2$. Nitrogen atoms may be substituted, namely NR (R is H or other substituents defined herein); the number of atoms on the ring is usually defined as the number of the ring members, for example, "3- to 6-membered heterocycloalkyl" refers to a ring formed by 3 to 6 atoms arranged around it, and each ring optionally contains 1 to 3 heteroatoms, namely N, O, S, NO, SO, $S(O)_2$ or NR, and each ring is optionally substituted by R, and R is a group as defined herein.

Unless otherwise specified, the term "aryl" refers to an unsaturated, usually aromatic, hydrocarbon group, which may be a single ring or multiple rings fused together. $C_{5-10}$ aryl is preferred, $C_{5-8}$ aryl is more preferred, monocyclic $C_{5-6}$ aryl is most preferred; examples of aryl include, but are not limited to, phenyl, naphthyl.

Unless otherwise specified, the term "heteroaryl" refers to a stable monocyclic or polycyclic aromatic hydrocarbon group containing at least one heteroatom (N, O, S, NO, SO, $S(O)_2$ or NR). 5- or 6-membered monocyclic heteroaryl is preferred. Examples of heteroaryl include, but are not limited to, pyrrolyl, pyrazolyl, imidazolyl, pyrazinyl, oxazolyl, isoxazolyl, thiazolyl, furyl, thienyl, pyridyl, pyrimidinyl.

Unless otherwise specified, "cycloalkyl" refers to a saturated monocyclic or polycyclic hydrocarbon group. The cycloalkyl is preferably 3- to 8-membered monocycloalkyl, more preferably 3- to 6-membered monocycloalkyl. Examples of monocycloalkyl include, but are not limited to, cyclopropyl, cyclobutyl, cyclopentyl, cyclohexyl , cycloheptyl, cyclooctyl.

Unless otherwise specified, "heterocycloalkyl" refers to monoheterocycloalkyl and polyheterocycloalkyl containing a certain number of heteroatoms in the ring, and the heteroatoms are generally selected from N, O, S, NO, SO, $S(O)_2$, and NR. The heterocycloalkyl is preferably 3- to 8-membered monoheterocycloalkyl, more preferably 3- to 6-membered monoheterocycloalkyl. Examples of monoheterocycloalkyl include, but are not limited to, oxiranyl, tetrahydropyrrolyl, piperidinyl, piperazinyl, morpholinyl, tetrahydrofuranyl, tetrahydrothiophenyl, tetrahydropyranyl, 1,3-dioxolane, 1,4-dioxane, etc.

Unless otherwise specified, "heterocycloalkenyl" refers to cyclic monoalkenyl containing heteroatoms, including 3- to 10-membered heterocyclylalkenyl, preferably 3- to 6-membered heterocyclylalkenyl, most preferably 5- to 6-membered heterocyclylalkenyl. Examples of heterocycloalkenyl include, but are not limited to,

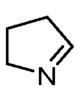, 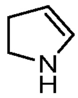, 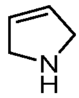, 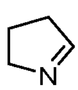,

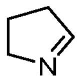, 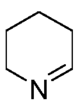, 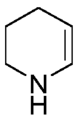, 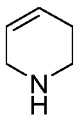, 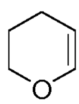, 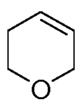,

etc.

Unless otherwise specified, the term "alkyl" is used to refer to a linear or branched saturated hydrocarbyl. $C_{1-6}$ alkyl is preferred, and $C_{1-4}$ alkyl is more preferred. Examples of alkyl include, but are not limited to, methyl, ethyl, n-propyl, isopropyl, butyl, isobutyl, pentyl, isopentyl, neopentyl, n-hexyl, etc.

Unless otherwise specified, "spiroheterocyclyl" refers to a spirocyclyl in which one or more carbon atoms in the spirocyclic skeleton structure are substituted by heteroatoms selected from N, O, and S. Spiroheterocyclyl is preferably 5- to 13-membered spiroheterocyclyl, 6-to 12-membered spiroheterocyclyl, or 7- to 11-membered spiroheterocyclyl. Examples of spiroheterocyclyl include, but are not limited to, 2-oxa-7-azaspiro[5.3]nonan-7-yl, 2-oxa-7-azaspiro[4.4]nonan-7-yl, 2-oxa-6-azaspiro[3.3]heptan-6-yl, 2-oxa-8-azaspiro[4.5]decan-8-yl, 1,4,9-triazaspiro[5.5]undecan-9-yl, 3-oxa-9-azaspiro[5.5]undecan-9-yl, 2,6-diazaspiro[3.3]heptan-2-yl, 2,7-diazaspiro[5.3]nonan-7-yl, 2,7-dioxaspiro[5.3]nonyl, 3,9-diazaspiro[5.5]undecan-3-yl, 1-oxa-4,9-diazaspiro[5.5]undecan-9-yl, 1-oxa-4,8-diazaspiro[5.4]decan-8-yl, 3-azaspiro[5.5]undecan-3-yl, 7-azaspiro[3.5]decan-7-yl, 1-oxa-4,9-diazaspiro[5.5]undecan-4-yl, 6-oxa-2,9-diazaspiro[4.5]decan-9-yl, 9-oxa-2,6-diazaspiro[4.5]decan-6-yl, 3-azaspiro[5.5]undecan-3-yl, 4-oxa-1,9-diazaspiro[5.5]undecan-9-yl.

Unless otherwise specified, the term "fused cyclyl" refers to a polycyclic hydrocarbon group sharing two adjacent carbon atoms, and the fused cyclyl is preferably $C_{8-10}$ fused bicyclyl, more preferably a three-membered ring-fused a five-membered ring, a five-membered ring-fused a five-membered ring, a five-membered ring-fused a six-membered ring, etc., and examples of fused cyclyl include, but are not limited to, bicyclo[3.1.0]hexyl, bicyclo[3.2.0]heptyl, bicyclo[3.3.0]octyl, bicyclo[4.1.0]heptyl, bicyclo[4.2.0]octyl, bicyclo[4.3.0]nonyl, bicyclo[4.4.0]decyl, etc.

Unless otherwise specified, the term "fused heterocyclyl" means that the skeleton carbon atoms in the fused ring are substituted by 1 to 3 heteroatoms selected from N, O, and S. Examples of fused heterocyclyl include, but are limited to, 1,4-diazabicyclo[4.4.0]decan-4-yl, 1,4-diazabicyclo[4.3.0]-nonan-4-yl, 8-oxa-1,4-diazabicyclo[4.4.0]decan-4-yl, 1,4-diazabicyclo[4.4.0]decan-4-yl, 4,7-diazabicyclo[4.3.0]nonan- 4-yl, 3,7-diazabicyclo[4.3.0]nonan-3-yl, 3,7-diazabicyclo[3.3.0]octan-3-yl, 3,7-diazabicyclo[4.4.0]decan-3-yl, 3,6-diazabicyclo[4.3.0]nonan-3-yl, 3,6-diazabicyclo[4.4.0]decan-3-yl, 3,6,9-triazabicyclo[4.4.0]decan-3-yl, 3,7-diazabicyclo[4.2.0]octan-3-yl and 3,7-diazabicyclo[3.3.0]octan-3-yl.

Unless otherwise specified, the term "alkylene" means a divalent hydrocarbon group with the specified number of carbon atoms, including straight chain alkylene and branched chain alkylene, preferably $C_{1-6}$ alkylene, more preferably $C_{1-4}$ alkylene. Examples of alkylene include, but are not limited to, $-CH_2-$, $-CH_2CH_2-$, $-CH_2CH_2CH_2-$, $-CH_2CH_2CH_2CH_2-$, $-CH_2CH(CH_3)-$, $-CH_2CH(CH_3)CH_2-$, $-CH_2CH_2CH(CH_3)-$, $-CH_2CH_2CH(CH_3)CH_2-$ and $-CH_2CH_2CH_2CH(CH_3)-$, etc.

Unless otherwise specified, the term "alkoxy" refers to alkyl connected by an oxygen bridge, that is, a group obtained by substituting hydrogen atoms in hydroxyl with alkyl. $C_{1-6}$ alkoxy is preferred, and $C_{1-4}$ alkoxy is more preferred. Examples of alkoxy include, but are not limited to, methoxy, ethoxy, n-propoxy, isopropoxy, n-butoxy, sec-butoxy, tert-butoxy, n-pentoxy, neopentoxy and n-hexyloxy.

Unless otherwise specified, the term "halogen" refers to fluorine, chlorine, bromine or iodine atom.

Unless otherwise specified, the term "haloalkyl" refers to alkyl in which one or more hydrogen atoms are substituted by halogen atoms. $C_{1-6}$ haloalkyl is preferred, and $C_{1-4}$ haloalkyl is more preferred. Examples of haloalkyl include, but are not limited to, fluoromethyl, difluoromethyl, trifluoromethyl, trichloromethyl, tribromomethyl, 2,2,2-trifluoroethyl, 2,2,2-trichloroethyl, etc.

Unless otherwise specified, the term "$C_{1-4}$ alkyl-OH" refers to a structure in which hydrogen atoms in a $C_{1-4}$ alkyl are optionally substituted by hydroxyl. Examples of "$C_{1-4}$ alkyl-OH" include, but are not limited to, $-CH_2OH$ , $-CH_2CH_2OH$, $-CH(OH)CH_3$, $-CH_2CH_2CH_2OH$, $-CH_2CH(OH)CH_3$,

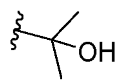
,

$-CH_2CH_2CH_2CH_2OH$, $-CH_2CH(OH)CH_2CH_3$, $-CH_2CH_2CH(OH)CH_3$,

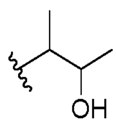 , 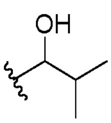 , 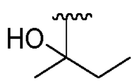 , 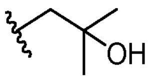 ,

etc.

Unless otherwise specified, "- - - - - -" in the structure means that the bond can be a single bond or a double bond, for example, the structural moiety

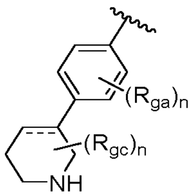

can be

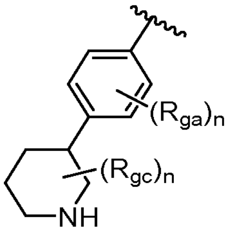

,

or can be

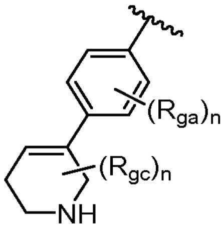

.

Specifically, all combinations of the substituent and/or the variant thereof herein are allowed only when such combinations result in a stable compound.

In the examples of the present disclosure, the nomenclature of the title compound is converted from the compound structure by means of Chemdraw. If there is any inconsistency between the compound name and the compound structure, it may be determined by synthesizing relevant information and reaction routes; if it cannot be confirmed by other methods, the given structural formula of the compound shall prevail.

The preparation methods of some compounds in the present disclosure refer to the preparation methods of the aforementioned similar compounds. Those skilled in the art should know that when using or referring to the preparation methods cited therein, the feed ratio of reactants, reaction solvent, reaction temperature, etc. may be appropriately adjusted according to the different reactants.

The compounds of the present disclosure can be prepared by a variety of synthetic methods known to those skilled in the art, including the specific examples listed below, the examples formed by their combination with other chemical synthesis methods, and equivalent alternatives known to those skilled in the art, preferred embodiments include but are not limited to the examples of the present disclosure.

Abbreviations used in the examples of the present disclosure and their corresponding chemical names are as follows:

| Abbreviation | Description |
| --- | --- |
| HCl | Hydrogen chloride |
| DIPEA/IDEA | *N,N*-Diisopropylethylamine |
| DCM | Dichloromethane |
| LiOH | Lithium hydroxide |
| THF | Tetrahydrofuran |
| $NH_3$. $H_2O$ | Ammonia water |

(continued)

| Abbreviation | Description |
|---|---|
| $Cs_2CO_3$ | Cesium carbonate |
| TFAA | Trifluoroacetic anhydride |
| NaH | Sodium hydride |
| $Pd(PPh_3)_2Cl_2$ | Bis(triphenylphosphine)palladium(II) chloride |
| $PhB(OH)_2$ | Phenylboronic acid |
| MeCN | Acetonitrile |
| DMF | *N,N*-Dimethylformamide |
| Pd/C | Palladium-carbon |
| $PdCl_2$(dppf) | [1,1'-Bis(diphenylphosphino)ferrocene]dichloropalladium(II) |
| $Na_2CO_3$ | Sodium carbonate |
| DMA | *N,N*-Dimethylacetamide |
| $CH_3I$ | Iodomethane |
| $NaBH(OAc)_3$ | Sodium triacetoxyborohydride |
| MeOH | Methanol |
| HCHO | Formaldehyde |
| $Pd_2(dba)_3$ | Tris(dibenzylideneacetone)dipalladium |
| X-Phos | 2-Dicyclohexylphosphino-2',4',6'-triisopropylbiphenyl |
| n-BuLi | n-Butyllithium |
| $PPh_3$ | Triphenylphosphine |
| DIAD | Diisopropyl azodicarboxylate |
| TsOH | p-Toluenesulfonic acid |

DETAILED DESCRIPTION OF THE PREFERRED EMBODIMENT

The structures of the compounds of the present disclosure were determined by nuclear magnetic resonance (NMR) or/and liquid chromatography-mass spectrometry (LC-MS), or ultra-performance liquid chromatography-mass spectrometry (UPLC-MS). NMR chemical shift ($\delta$) was given in units of parts per million (ppm). NMR was determined using a Bruker Neo 400M or Bruker Ascend 400 NMR instrument with deuterated dimethyl sulfoxide (DMSO-$d_6$), deuterated methanol ($CD_3OD$), deuterated chloroform ($CDCl_3$) and heavy water ($D_2O$) as solvents and tetramethylsilane (TMS) as an internal standard.

Liquid chromatography-mass chromatography LC-MS was determined using an Agilent 1260-6125B single quadrupole mass spectrometer, and the column was Welch Biomate column (C18, 2.7 $\mu$m, 4.6 * 50 mm) or waters H-Class SQD2, and the column was Welch Ultimate column (XB-C18, 1.8 $\mu$m, 2.1 * 50 mm) mass spectrometer (ion source is electrospray ionization).

Ultra-performance liquid chromatography-mass spectrometry UPLC-MS was determined using a Waters UPLC H-class SQD mass spectrometer (an ion source was electrospray ionization).

HPLC was determined using Waters e2695-2998 or Waters ARC and Agilent 1260 or Agilent Poroshell HPH high performance liquid chromatography.

Waters 2555-2489 (10 $\mu$m, ODS 250 cm $\times$ 5 cm) or GILSON Trilution LC was used as preparative HPLC, and the column was Welch XB-C18 column (5 $\mu$m, 21.2 * 150 mm).

Chiral HPLC was determined using waters aquity UPC2; the column was Daicel chiralpak AD-H (5 $\mu$m, 4.6 * 250 mm), Daicel chiralpak OD-H (5 $\mu$m, 4.6 * 250 mm), Daicel chiralpak IG-3 (3 $\mu$m, 4.6 * 150 mm), Chiral Technologies Europe AD-3 (3 $\mu$m, 3.0 * 150 mm) and Trefoil TM Technology Trefoil TM AMY1 (2.5 $\mu$m, 3.0 * 150 mm).

Supercritical fluid chromatography (SFC) was determined using waters SFC 80Q, and the column was Daicel Chiralcel OD/OJ/OZ (20 $\times$ 250 mm, 10 $\mu$m) or Daicel Chiralpak IC/IG/IH/AD/AS (20 $\times$ 250 mm, 10 $\mu$m).

GF254 silica gel plate from Yantai Jiangyou Silica Gel Development Co., Ltd. or Rushan Shangbang New Materials Co., Ltd. was used as a thin layer chromatography silica gel plate. The specification used for TLC was 0.15 mm to 0.20 mm. The specification used for preparative TLC was 20 × 20 cm. Column chromatography generally used 200 to 300 mesh silica gel from Yucheng Chemical as a carrier.

The starting materials in the examples of the present disclosure are known and commercially available, or can be synthesized by using or following methods known in the art.

Unless otherwise specified, all reactions in the present disclosure are carried out under continuous magnetic stirring and dry nitrogen or argon atmosphere. The solvent is a dry solvent, and the unit of reaction temperature is Celsius.

**Intermediate 1A**

**4-Chloro-2-((1-((1-methyl-1*H*-pyrazol-4-yl)sulfonyl)piperidin-4-yl)amino)pyrimidine-5-carbonitrile**

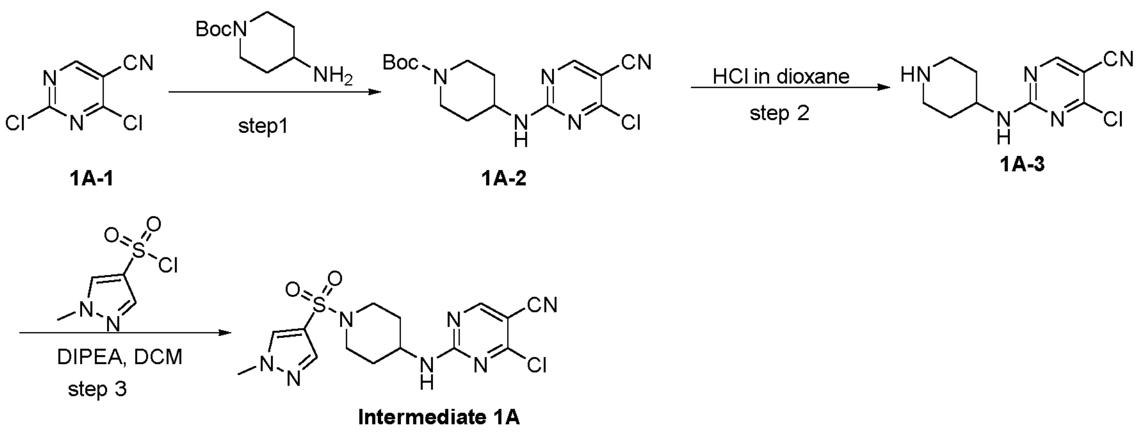


1A-1 1A-2 1A-3

Intermediate 1A

**Step 1:** 2,4-Dichloro-5-cyanopyrimidine (5.0 g, 28.4 mmol) was dissolved in a 1/1 mixed solvent (70 mL) of tert-butanol and 1,2-dichloroethane at room temperature. Subsequently, under cooling in an ice-water bath and nitrogen atmosphere, a tetrahydrofuran solution of zinc chloride (1 mol/L, 33.6 mL, 33.6 mmol) was slowly added dropwise to the above solution, and the reaction mixture was stirred for 1 hour under an ice-water bath. Under cooling in an ice-water bath, a solution of tert-butyl 4-aminopiperazine-1-carboxylate (5.9 g, 28.4 mmol) in a 1/1 mixed solvent of tert-butanol and 1,2-dichloroethane and a solution of triethylamine (3.6 mL, 33.6 mmol) in a 1/1 mixed solvent of tert-butanol and 1,2-dichloroethane were slowly added dropwise in portions to the above reaction mixture. The reaction mixture was heated to room temperature and stirred for 2 hours. Water (50 mL) was added to the reaction mixture to quench the reaction. The reaction mixture was concentrated under reduced pressure. The mixture was extracted with dichloromethane (40 mL × 3 times), and the organic phases were combined. The organic phases were washed with saturated brine (30 mL) first, then dried over anhydrous sodium sulfate, filtered and finally concentrated under reduced pressure. The resulting residue was purified by silica gel column chromatography to obtain 3.8 g of tert-butyl 4-((4-chloro-5-cyanopyrimidin-2-yl)amino)piperidine-1-carboxylate (1A-2).

MS (ESI) m/z: 338.1 $[M+H]^+$.

$^1$H NMR (400 MHz, DMSO-$d_6$) δ 8.84 - 8.77 (m, 1H), 8.76 (s, 0.5H), 8.69 (s, 0.4H), 4.09 - 3.83 (m, 3H), 2.87 (br.s., 2H), 1.80 (d, *J* = 10.9 Hz, 2H), 1.40 (s, 9H), 1.38 - 1.29 (m, 2H).

**Step 2:** Compound **1A-2** (1 g, 2.9 mmol) was dissolved in 1,4-dioxane (3 mL) at room temperature. Subsequently, a hydrogen chloride-dioxane solution (2 mol/L, 3 mL, 6 mmol) was added thereto. The reaction mixture was stirred at room temperature for 2 hours. The reaction mixture was concentrated under reduced pressure to obtain 700 mg of 4-chloro-2-(piperidin-4-ylamino)pyrimidine-5-carbonitrile (**1A-3**).

MS (ESI) m/z: 238.1 $[M+H]^+$.

**Step 3:** Compound **1A-3** (700 mg, 2.9 mmol) was dissolved in dichloromethane (10 mL) at room temperature. Subsequently, *N,N*-diisopropylethylamine (1.08 g, 8.4 mmol) and 1-methyl-1*H*-pyrazole-4-sulfonyl chloride (637.0 mg, 3.5 mmol) were sequentially added to the above reaction mixture. The reaction mixture was stirred at room temperature for 1.5 hours. The reaction mixture was concentrated under reduced pressure, and water (30 mL) was added to the resulting residue to quench the reaction. The mixture was extracted with dichloromethane (30 mL × 3 times), and the organic phases were combined. The organic phases were washed with saturated brine (10 mL × 3 times) first, then dried over anhydrous sodium sulfate, filtered and finally concentrated under reduced pressure. The resulting residue

was purified by silica gel column chromatography to obtain 740.0 mg of compound 4-chloro-2-((1-((1-methyl-1*H*-pyrazol-4-yl)sulfonyl)piperidin-4-yl)amino)pyrimidine-5-carbonitrile (intermediate **1A**).

MS (ESI) m/z: 382.0 $[M+H]^+$.

$^1H$ NMR (400 MHz, DMSO-$d_6$) δ 8.94 - 8.80 (m, 1H), 8.73 (s, 0.5H), 8.69 (s, 0.4H), 8.32 (s, 1H), 7.77 (s, 1H), 3.91 (s, 3H), 3.87 - 3.69 (m, 1H), 3.53 - 3.40 (m, 2H), 2.48 - 2.38 (m, 2H), 2.04 - 1.82 (m, 2H), 1.69 - 1.52 (m, 2H).

**Intermediate 1B**

**4-Chloro-*N*-(1-((1-methyl-1*H*-pyrazol-4-yl)sulfonyl)piperidin-4-yl)-5-(trifluoromethyl)pyrimidin-2-amine**

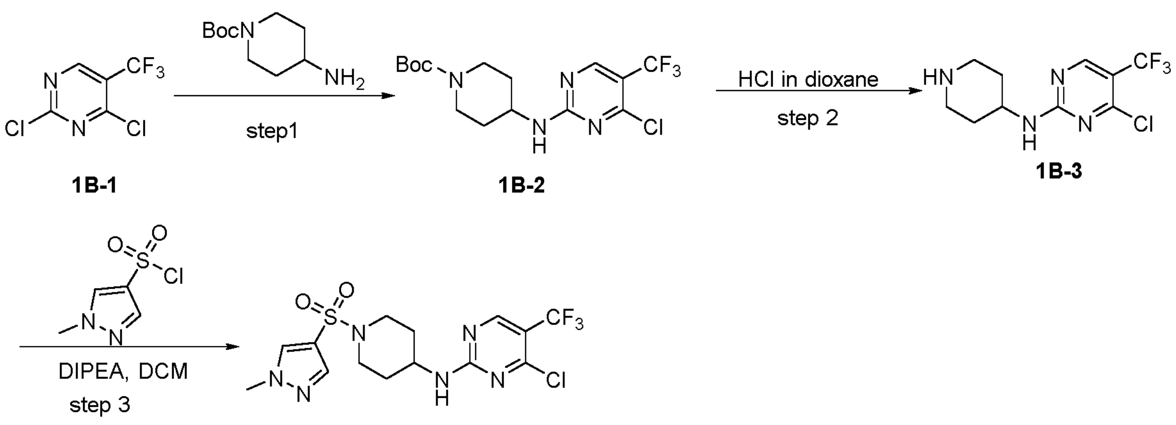


1B-1 1B-2 1B-3

Intermediate 1B

**Step 1:** 2,4-Dichloro-5-trifluoromethylpyrimidine (500 mg, 2.3 mmol) was dissolved in acetonitrile (10 mL) at room temperature. Subsequently, triethylamine (349.0 mg, 3.5 mmol) and 1-tert-butoxycarbonyl-4-amino-piperidine (552.0 mg, 2.8 mmol) were slowly added to the above solution under cooling in an ice-water bath. The reaction mixture was stirred for 1 hour under cooling in an ice-water bath. Water (30 mL) was added to the reaction mixture to quench the reaction. The mixture was concentrated under reduced pressure and extracted with ethyl acetate (8 mL × 3 times), and the organic phases were combined. The organic phases were washed with saturated brine (30 mL) first, then dried over anhydrous sodium sulfate, filtered and finally concentrated under reduced pressure. The resulting residue was purified by silica gel column chromatography to obtain 340 mg of tert-butyl 4-((4-chloro-5-(trifluoromethyl)pyrimidin-2-yl)amino)piperidine-1-carboxylate (**1B-2**).

MS (ESI) m/z: 325.0 [M+H-*t*-Bu]$^+$.

$^1H$ NMR (400MHz, DMSO-$d_6$) δ 8.63 (s, 0.6H), 8.61 - 8.49 (m, 1.4H), 4.05 - 3.82 (m, 3H), 2.87 (br.s., 2H), 1.81 (d, *J* = 11.4 Hz, 2H), 1.50 - 1.21 (m, 11H).

**Step 2:** Compound **1B-2** (130 mg, 0.3 mmol) was dissolved in 1,4-dioxane (1 mL) at room temperature. Subsequently, a hydrogen chloride-dioxane solution (4 mol/L, 2 mL, 8 mmol) was added to the above solution. The reaction mixture was stirred at room temperature for 2 hours. The reaction mixture was concentrated under reduced pressure to obtain 95.0 mg of 4-chloro-*N*-(piperidin-4-yl)-5-(trifluoromethyl)pyrimidin-2-amine **(1B-3).**

MS (ESI) m/z: 281.0 $[M+H]^+$.

**Step 3:** Compound **1B-3** (95.0 mg, 0.3 mmol) was dissolved in dichloromethane (5 mL) at room temperature. Subsequently, *N,N*-diisopropylethylamine (132.0 mg, 1.0 mmol) and 1-methyl-1*H*-pyrazole-4-sulfonyl chloride (72.0 mg, 0.4 mmol) were sequentially added to the above reaction mixture under cooling in an ice-water bath. The reaction mixture was stirred at room temperature for 15 hours. Water (30 mL) was added to the reaction mixture to quench the reaction. The mixture was extracted with dichloromethane (10 mL × 3 times), and the organic phases were combined. The organic phases were washed with saturated brine (10 mL × 3 times) first, then dried over anhydrous sodium sulfate, filtered, and finally concentrated under reduced pressure. The resulting residue was purified by silica gel column chromatography to obtain 120.0 mg of 4-chloro-*N*-(1-((1-methyl-1*H*-pyrazol-4-yl)sulfonyl)piperidin-4-yl)-5-(trifluoromethyl)pyrimidin-2-amine (intermediate **1B**).

MS (ESI) m/z: 425.0 $[M+H]^+$.

$^1H$ NMR (400 MHz, DMSO-$d_6$) δ 8.69 - 8.52 (m, 2H), 8.32 (s, 1H), 7.77 (s, 1H), 3.91 (s, 3H), 3.86 - 3.66 (m, 1H), 3.51 - 3.39 (m, 2H), 2.48 - 2.32 (m, 2H), 1.97 - 1.86 (m, 2H), 1.66 - 1.50 (m, 2H).

**Example 1:**

**4-(Cyclopentylamino)-2-(((1-(methylsulfonyl)piperidin-4-yl)amino)pyrimidine-5-carbonitrile**

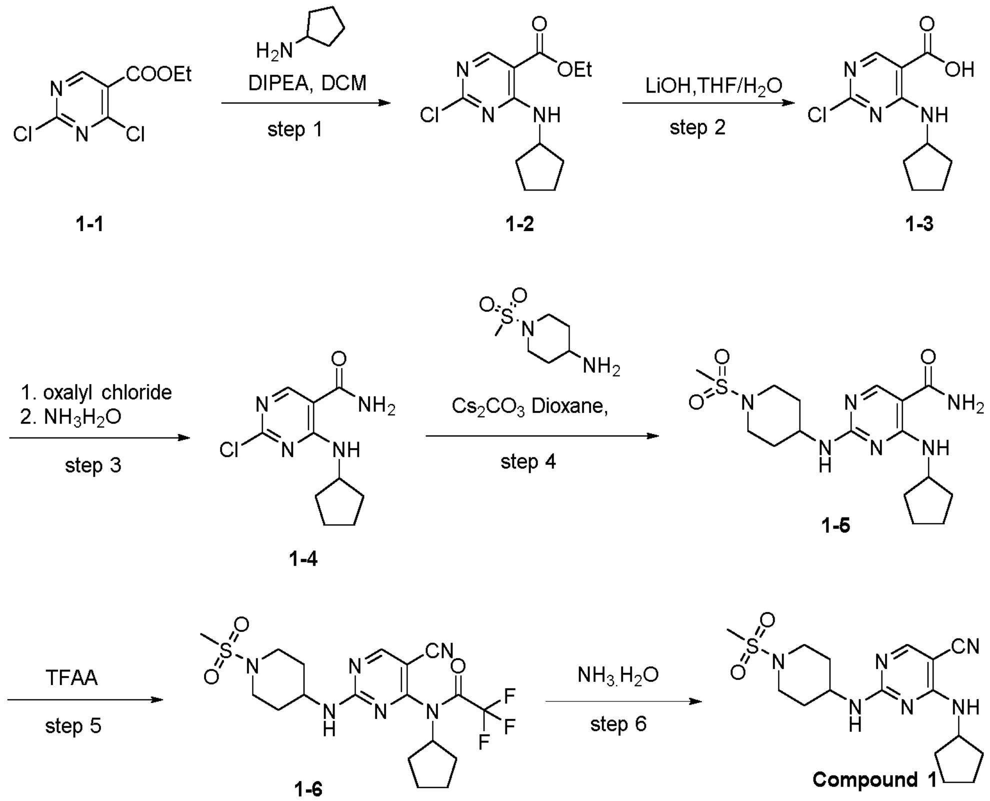


1-1 1-2 1-3 1-4 1-5 1-6 Compound 1

**Step 1:** Compound **1-1** (9.0 g, 40.7 mmol) and diisopropylethylamine (5.3 g, 40.7 mmol) were dissolved in 90 mL of dichloromethane. Cyclopentylamine (3.64 g, 42.8 mmol) was slowly added dropwise to the above solution at 0°C. After the dropwise addition was completed, the reaction mixture was continued to stir at room temperature for 1 hour. Saturated sodium bicarbonate aqueous solution (40 mL) was added to the reaction mixture to quench the reaction. The mixture was extracted with dichloromethane (100 mL × 2 times), and the organic phases were combined. The organic phases were washed with saturated brine first, then dried over anhydrous sodium sulfate, filtered, and finally concentrated under reduced pressure to obtain 12.3 g of 4-(cyclopentylamino)-2-(((1-(methylsulfonyl)piperidin-4-yl)amino)pyrimidine-5-carbonitrile **(1-2)**. This product was used directly in the next reaction step without purification.

MS (ESI) m/z: 270.0 $[M+H]^+$.

**Step 2:** Compound **1-2** (12.3 g, 45.6 mmol) was dissolved in THF/water (2/1, 150 mL) at room temperature. Subsequently, lithium hydroxide monohydrate (3.83 g, 91.3 mmol) was added in portions to the above solution. The reaction mixture was stirred at room temperature for 3 hours and concentrated under reduced pressure to remove tetrahydrofuran, and the solution was neutralized to pH = 4 with 3 mol/L hydrochloric acid aqueous solution. The mixture was extracted with dichloromethane (100 mL × 2 times), and the organic phases were combined. The organic phases were washed with saturated brine first, then dried over anhydrous sodium sulfate, filtered, and finally concentrated under reduced pressure to obtain 7.5 g of 2-chloro-4-(cyclopentylamino)pyrimidine-5-carboxylic acid **(1-3).**

MS (ESI) m/z: 240.1 $[M-H]^-$.

**Step 3:** *N,N*-Dimethylformamide (10 drops) and **1-3** (7.5 g, 31.1 mmol) were dissolved in dichloromethane (100 mL) at room temperature. The reaction mixture was cooled to 0°C, and then oxalyl chloride (5.75 mL, 68.5 mmol) was slowly added dropwise to the above reaction mixture within 20 minutes. The reaction mixture was warmed to room temperature and stirred for 2 hours. At 0°C, the above reaction mixture was added dropwise to ammonia water (100 mL) within 30 minutes. The reaction mixture was filtered and dried to obtain 6.0 g of 2-chloro-4-(cyclopentylamino)pyrimidine-5-carboxamide **(1-4).**

MS (ESI) m/z: 241.0 $[M+H]^+$.

**Step 4: 1-4** (400 mg, 1.8 mmol), 1-(methylsulfonyl)piperidin-4-amine (581 mg, 2.7 mmol) and cesium carbonate

(1.77 g, 5.4 mmol) were dissolved in 1,4-dioxane (10 mL). The reaction mixture was heated to 120°C and reacted for 3 hours, then cooled to room temperature. The reaction mixture was concentrated under reduced pressure, and the residue was extracted with dichloromethane. The organic phases were combined, washed with saturated brine first, then dried over anhydrous sodium sulfate, filtered and finally concentrated under reduced pressure. The resulting residue was purified by silica gel column chromatography to obtain 405 mg of 4-(cyclopentylamino)-2-(((1-(methylsulfonyl)piperidin-4-yl)amino)pyrimidine-5-carboxamide **(1-5).**

MS (ESI) m/z: 383.4 $[M+H]^+$.

**Step 5: 1-5** (200 mg, 0.5 mmol) and triethylamine (636 mg, 6.3 mmol) were dissolved in THF (10 mL). At -65°C, trifluoroacetic anhydride (1.1 g, 5.2 mmol) was added to the reaction mixture. The reaction mixture was continued to stir for 30 minutes at -65°C. Saturated sodium bicarbonate aqueous solution was added to the reaction mixture. The mixture was extracted with dichloromethane (100 mL × 2 times). The organic phases were combined, washed with saturated brine first, then dried over anhydrous sodium sulfate, filtered and finally concentrated under reduced pressure to obtain 100 mg of *N*-(5-cyano-2-(((1-(methylsulfonyl)piperidin-4-yl)amino)pyrimidin-4-yl)-A-cyclopentyl-2,2,2-trifluoroacetamide **(1-6).**

MS (ESI) m/z: 461.2 $[M+H]^+$.

**Step 6: 1-6** (100 mg, 0.22 mmol) was dissolved in THF (5 mL) at room temperature, and ammonia water (5 mL) was added to the solution. After the reaction mixture was reacted at room temperature for 1 hour, water was added to quench the reaction. The mixture was extracted with dichloromethane (100 mL × 2 times), and the organic phases were combined. The organic phases were washed with saturated brine first, then dried over anhydrous sodium sulfate, filtered and finally concentrated under reduced pressure. The resulting residue was purified by silica gel column chromatography to obtain 40 mg of 4-(cyclopentylamino)-2-(((1-(methylsulfonyl)piperidin-4-yl)amino)pyrimidine-5-carbonitrile (compound **1**).

MS (ESI) m/z: 365.4 $[M+H]^+$.

$^1$HNMR (400 MHz, DMSO-$d_6$+TFA) δ 9.06 - 8.82 (m, 2H), 8.57 (br.s., 1H), 4.52 - 4.31 (m, 1H), 4.02 - 3.67 (m, 1H), 3.64 - 3.45 (m, 2H), 3.01 - 2.65 (m, 5H), 2.09 - 1.81 (m, 4H), 1.78 -1.41 (m, 8H).

**Example 2:**

**4-(Cyclopentylamino)-6-((1-(methylsulfonyl)piperidin-4-yl)amino)nicotinamide**

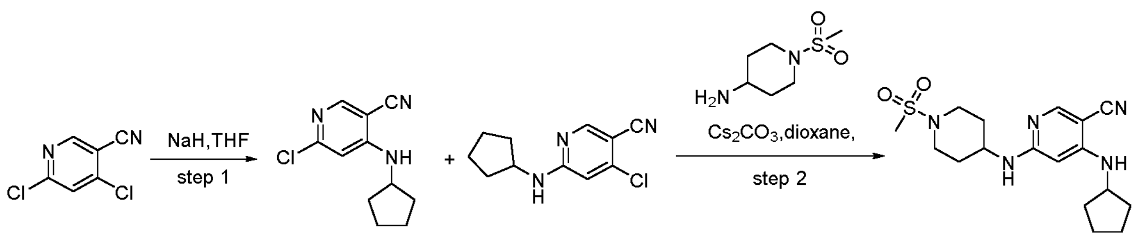


2-1 2-2A 2-2B Compound 2

**Step 1:** Cyclopentylamine (830 mg, 9.8 mmol) was dissolved in THF (10 mL) at room temperature. At 0°C, sodium hydride (60% dispersed in mineral oil, 530 mg, 13.3 mmol) was slowly added to the above solution in portions, and the reaction mixture was continued to stir and react at 0°C for 20 minutes. Subsequently, a solution of **2-1** (1.54 g, 8.9 mmol) in tetrahydrofuran (5 mL) was slowly added dropwise to the above reaction mixture at this temperature. After the dropwise addition was completed, the reaction mixture was stirred at room temperature for 1 hour. The reaction was quenched by adding saturated ammonium chloride aqueous solution. The mixture was extracted with dichloromethane (40 mL × 2 times), and the organic phases were combined. The organic phases were washed with saturated brine first, then dried over anhydrous sodium sulfate, filtered, and finally concentrated under reduced pressure. The resulting residue was purified by silica gel column chromatography to obtain 810 mg of a mixture of **2-2A** and **2-2B.**

**Step 2:** The mixture of **2-2A** and **2-2B** (200 mg, 0.9 mmol), 1-(methylsulfonyl)piperidin-4-amine (400 mg, 2.25 mmol) and cesium carbonate (734 mg, 2.25 mmol) was dissolved in 1,4-dioxane (10 mL). The reaction system was heated to 120°C and continued to stir for 5 hours. The reaction mixture was cooled to room temperature and concentrated under reduced pressure to remove most of the solvent. The residue was extracted with dichloromethane (40 mL × 2 times). The organic phase was combined, washed with saturated brine first, then dried over anhydrous sodium sulfate, filtered and finally concentrated under reduced pressure. The resulting residue was purified by silica gel column chromatography to obtain 25 mg of 4-(cyclopentylamino)-6-((1-(methylsulfonyl)piperidin-4-yl)amino)nicotinamide (compound

2).

MS (ESI) m/z: 364.3 $[M+H]^+$.

$^1$HNMR (400 MHz, $CDCl_3$) δ 8.02 (s, 1H), 5.45 (s, 1H), 4.69 (d, *J* = 5.2 Hz, 1H), 3.92 - 3.82 (m, 1H), 3.82 - 3.69 (m, 3H), 3.01 - 2.90 (m, 2H), 2.81 (s, 3H), 2.19 - 2.10 (m, 2H), 2.10 - 1.98 (m, 2H), 1.84 - 1.72 (m, 2H), 1.71 - 1.51 (m, 7H).

**Example 3:**

**4-(Cyclopentyloxy)-2-((1-(methylsulfonyl)piperidin-4-yl)amino)pyrimidine-5-carbonitrile**

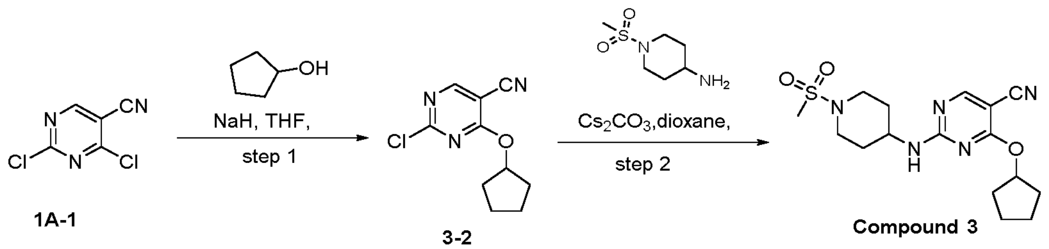


1A-1 3-2 Compound 3

**Step 1:** Cyclopentanol (270 mg, 3.16 mmol) was dissolved in THF (10 mL) at room temperature. The reaction mixture was cooled to 0°C. Sodium hydride (60% dispersed in mineral oil, 130 mg, 3.16 mmol) was slowly added to the above solution in portions, and the reaction mixture was continued to stir and react at room temperature for 15 minutes. Subsequently, **1A-1** (0.5 g, 2.87 mmol) was slowly added dropwise to the above reaction mixture at 0°C. After the dropwise addition was completed, the reaction mixture was continued to stir at room temperature for 1 hour. The reaction was quenched by adding saturated ammonium chloride aqueous solution. The mixture was extracted with ethyl acetate, and the organic phases were combined. The organic phase was washed with saturated brine first, then dried over anhydrous sodium sulfate, filtered, and finally concentrated under reduced pressure to obtain 420 mg of 2-chloro-4-(cyclopentyloxy)pyrimidine-5-carbonitrile **(3-2).** This product was directly used in the next reaction step without purification.

**Step 2: 3-2** (400 mg, 1.79 mmol), 1-(methylsulfonyl)piperidin-4-amine (479 mg, 2.69 mmol) and cesium carbonate (1.24 g, 4.48 mmol) were dissolved in 1,4-dioxane (10 mL). The reaction system was heated to 120°C and continued to stir for 3 hours. The reaction mixture was cooled to room temperature, and water (30 mL) and ethyl acetate (30 mL) were added thereto. The reaction mixture was filtered. The filter cake was washed with water, dried in vacuo and purified by preparative high-performance liquid chromatography to obtain 30 mg of 4-(cyclopentyloxy)-2-((1-(methylsulfonyl)piperidin-4-yl)amino)pyrimidine-5-carbonitrile (compound **3**).

MS (ESI) m/z: 366.2 $[M+H]^+$.

$^1$HNMR (400 MHz, $CDCl_3$+TFA) δ 8.55 (s, 1H), 5.66 - 5.56 (m, 1H), 4.21 - 4.08 (m, 1H), 3.76 - 3.59 (m, 2H), 3.22 - 3.07 (m, 2H), 2.96 (s, 3H), 2.13 - 1.87 (m, 10 H), 1.77 - 1.73 (m, 2H).

**Example 4:**

**2-((1-(Methylsulfonyl)piperidin-4-yl)amino)-4-phenylpyrimidine-5-carbonitrile**

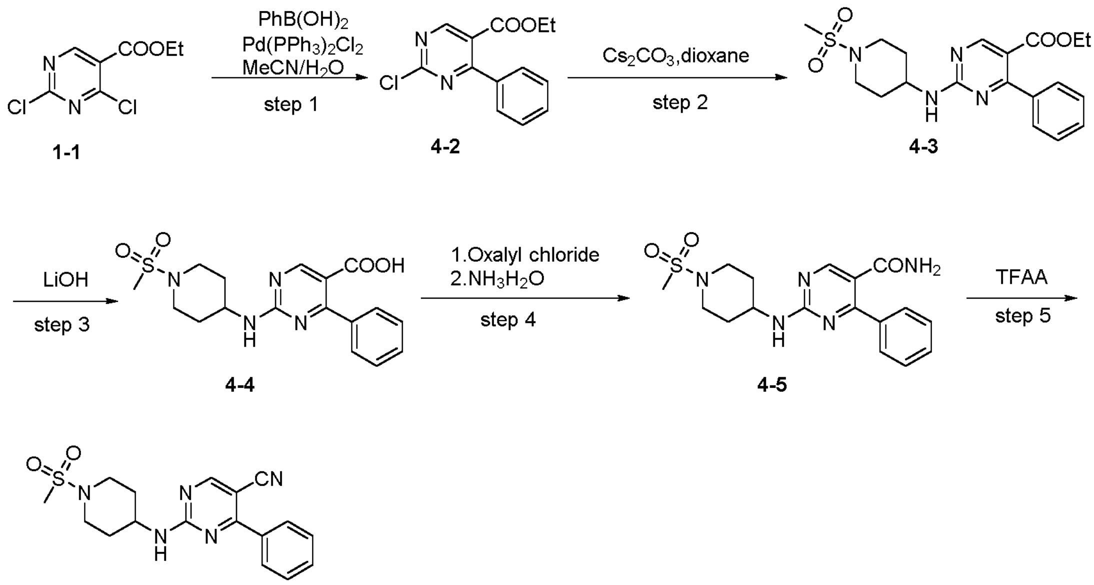


1-1

4-2

4-3

4-4

4-5

Compound 4

**Step 1:** Compound **1-1** (1.0 g, 4.52 mmol), phenylboronic acid (0.55 g, 4.52 mmol), sodium carbonate (1.45 g, 13.57 mmol) and bis(triphenylphosphine)palladium(II) chloride (158 mg, 0.22 mmol) were dissolved in 1,4-dioxane/water (10/1, 10 mL). The reaction system was replaced three times with nitrogen. The reaction mixture was heated to 95°C and continued to stir for 12 hours. After the reaction was completed, the reaction mixture was cooled to room temperature, and water (25 mL) was added to the reaction mixture. The mixture was extracted with ethyl acetate (20 mL × 2 times), and the organic phases were combined. The organic phases were washed with saturated brine first, then dried over anhydrous sodium sulfate, filtered and finally concentrated under reduced pressure. The resulting residue was purified by silica gel column chromatography to obtain 0.65 g of ethyl 2-chloro-4-phenylpyrimidine-5-carboxylate **(4-2).**

MS (ESI) m/z: 263.2 $[M+H]^+$.

**Step 2: 4-2** (650 mg, 2.48 mmol), 1-(methylsulfonyl)piperidin-4-amine (880 mg, 4.95 mmol) and cesium carbonate (2.44 g, 7.45 mmol) were dissolved in 1,4-dioxane (10 mL). The reaction system was heated to 120°C and continued to stir for 3 hours. The reaction mixture was cooled to room temperature and concentrated under reduced pressure to remove most of the solvent. The residue was extracted with dichloromethane. The organic phase was combined, washed with saturated brine first, then dried over anhydrous sodium sulfate, filtered and finally concentrated under reduced pressure. The resulting residue was purified by silica gel column chromatography to obtain 600 mg of ethyl 2-((1-(methylsulfonyl)piperidin-4-yl)amino)-4-phenylpyrimidine-5-carboxylate **(4-3)**.

MS (ESI) m/z: 405.3 $[M+H]^+$.

**Step 3:** Compound **4-3** (600 mg, 2.29 mmol) was dissolved in THF/water (2.5/1, 7.5 mL) at room temperature. Subsequently, lithium hydroxide monohydrate (290 mg, 2.85 mmol) was added to the above solution. The reaction mixture was stirred at room temperature for 3 hours and concentrated under reduced pressure to remove tetrahydrofuran, and the solution was neutralized to pH = 4 with 3 mol/L hydrochloric acid aqueous solution. The mixture was extracted with dichloromethane (100 mL × 2 times), and the organic phases were combined. The organic phases were washed with saturated brine first, then dried over anhydrous sodium sulfate, filtered, and finally concentrated under reduced pressure to obtain 450 mg of 2-((1-(methylsulfonyl)piperidin-4-yl)amino)-4-phenylpyrimidine-5-carboxylic acid **(4-4).**

MS (ESI) m/z: 377.2 $[M+H]^+$.

**Step 4:** *N,N*-Dimethylformamide (1 drop) and **4-4** (200 mg, 0.53 mmol) were dissolved in dichloromethane (5 mL) at room temperature. The reaction mixture was cooled to 0°C, and then oxalyl chloride (203 mg, 1.6 mmol) was added dropwise to the above reaction mixture within 5 minutes. The reaction mixture was warmed to room temperature and stirred for 0.5 hours. At 0°C, the above reaction mixture was added dropwise to ammonia water (10 mL) within 30 minutes. The reaction mixture was filtrated and dried to obtain 155 mg of 2-((1-(methylsulfonyl)piperidin-4-yl)amino)-4-phenylpyrimidine-5-carboxamide **(4-5).**

MS (ESI) m/z: 376.2 $[M+H]^+$.

**Step 5: 4-5** (100 mg, 0.27 mmol) and triethylamine (300 mg, 3.24 mmol) were dissolved in THF (5 mL). The reaction system was cooled to -65°C, and trifluoroacetic anhydride (270 mg, 1.3 mmol) was slowly added dropwise to the solution. The reaction mixture was continued to stir and react at -65°C for 30 minutes. The reaction was quenched by adding saturated sodium bicarbonate aqueous solution to the reaction mixture. The mixture was extracted with dichloromethane (100 mL × 2 times), and the organic phases were combined. The organic phases were washed with

saturated brine first, then dried over anhydrous sodium sulfate, filtered and finally concentrated under reduced pressure. The resulting residue was purified by silica gel column chromatography to obtain 50 mg of 2-((1-(methylsulfonyl)piperidin-4-yl)amino)-4-phenylpyrimidine-5-carbonitrile (compound **4**).

MS (ESI) m/z: 358.3 $[M+H]^+$.

$^1$HNMR (400 MHz, $CDCl_3$) δ 8.63 (s, 0.6 H), 8.56 (s, 0.5H), 8.01 (d, *J* = 6.7 Hz, 1H), 7.96 (d, *J* = 7.0 Hz, 1H), 7.64 - 7.48 (m, 3H), 5.87 - 5.67 (m, 1H), 4.22 - 4.05 (m, 1H), 3.80 (br.s., 2H), 2.93 (t, *J* = 11.3 Hz, 2H), 2.83 (s, 3H), 2.26 - 2.14 (m, 2H), 1.86 - 1.54 (m, 2H).

**Example 5:**

***N*$^4$-Cyclopentyl-*N*$^2$-(1-(methylsulfonyl)piperidin-4-yl)-5-(trifluoromethyl)pyrimidine-2,4-diamine**

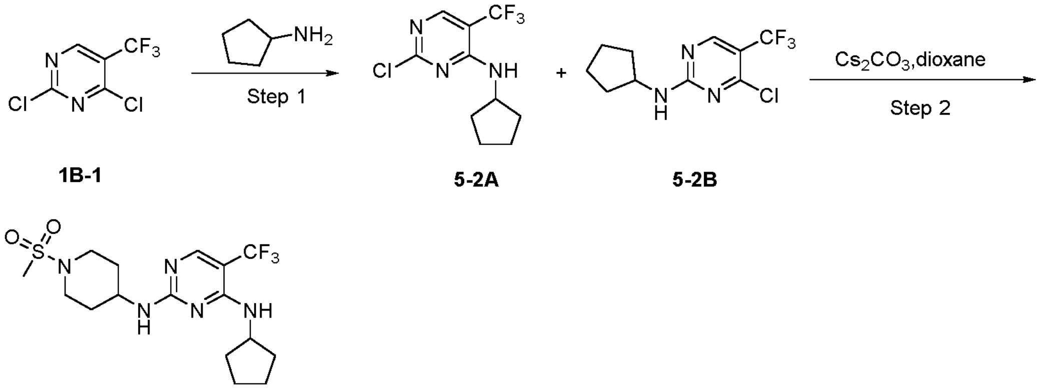


1B-1 5-2A 5-2B

Compound 5

**Step 1: 1B-1** (100 mg, 0.92 mmol) and *N,N*-diisopropylethylamine (550 mg, 1.38 mmol) were dissolved in dichloromethane (5 mL) at 0°C. Cyclopentylamine (78.2 mg, 0.92 mmol) was slowly added dropwise to the reaction mixture at this temperature. After the dropwise addition was completed, the reaction mixture was continued to stir at room temperature for 1 hour. The reaction was quenched by adding saturated sodium bicarbonate aqueous solution to the reaction mixture. The mixture was extracted with dichloromethane (100 mL × 2 times), and the organic phases were combined, washed with saturated brine first, then dried over anhydrous sodium sulfate, filtered and finally concentrated under reduced pressure to obtain 155 mg of a mixture of **5-2A** and **5-2B.** This mixture was directly used in the next reaction step without purification.

**Step 2: 5-2A** and **5-2B** (150 mg, 0.57 mmol), 1-(methylsulfonyl)piperidin-4-amine (151 mg, 0.85 mmol) and cesium carbonate (372 mg, 1.14 mmol) were dissolved in 1,4-dioxane (10 mL). The reaction mixture was heated to 120°C and reacted for 5 hours, then cooled to room temperature. The reaction mixture was concentrated under reduced pressure to remove most of the solvent, and the residue was extracted with dichloromethane. The organic phases were combined, washed with saturated brine, dried over anhydrous sodium sulfate, filtered and finally concentrated under reduced pressure. The resulting residue was purified by preparative high performance liquid chromatography to obtain 22 mg of *N*$^4$-cyclopentyl-*N*$^2$-(1-(methylsulfonyl)piperidin-4-yl)-5-(trifluoromethyl)pyrimidine-2,4-diamine (compound 5).

MS (ESI) m/z: 408.2 $[M+H]^+$.

$^1$HNMR (400 MHz, DMSO-$d_6$) δ 8.04 (s, 0.4H), 7.99 (s, 0.6H), 7.39 (d, *J* = 6.0 Hz, 0.6H), 7.21 (d, *J* = 6.8 Hz, 0.6H), 6.26 - 6.10 (m, 1H), 4.55 - 4.46 (m, 0.4H ), 4.43 - 4.29 (m, 0.6H), 3.92 - 3.73 (m, 1H), 3.62 - 3.46 (m, 2H), 2.67 - 2.72 (m, 5H), 2.03 - 1.78 (m, 4H), 1.76 - 1.39 (m, 8H).

**Example 6:**

**4-(Cyclopentyloxy)-2-((1-((1-methyl-1*H*-pyrazol-4-yl)sulfonyl)piperidin-4-yl)amino)pyrimidine-5-carbonitrile**

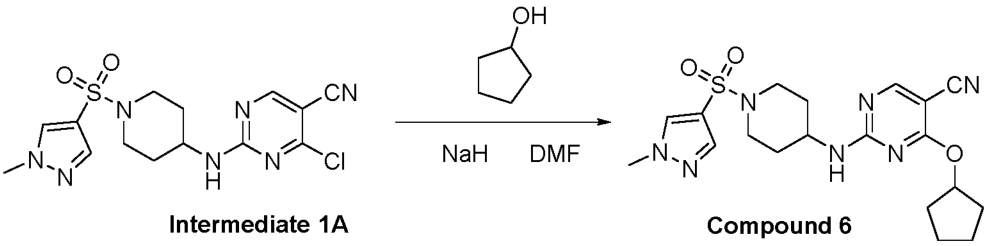


Intermediate 1A

Compound 6

Cyclopentanol (33.7 mg, 0.4 mmol) was dissolved in *N,N*-dimethylformamide (2 mL) at room temperature. Subsequently, sodium hydride (9.7 mg, 0.4 mmol) was added to the above reaction mixture under an ice bath and nitrogen atmosphere, and compound **1A** (100.0 mg, 0.3 mmol) was added after the above reaction mixture was stirred for 15 minutes under an ice bath. The reaction mixture was heated to 100°C and stirred for 1 hour. Water (10 mL) was added to the reaction mixture to quench the reaction. The mixture was extracted with ethyl acetate (30 mL × 3 times), and the organic phases were combined. The organic phases were washed with saturated brine (10 mL × 3 times) first, then dried over anhydrous sodium sulfate, filtered and finally concentrated under reduced pressure. The resulting residue was purified by preparative high performance liquid chromatography to obtain 9.2 mg of 4-(cyclopentyloxy)-2-((1-((1-methyl-1*H*-pyrazol-4-yl)sulfonyl)piperidin-4-yl)amino)pyrimidine-5-carbonitrile (compound 6).

MS (ESI) m/z: 432.2 $[M+H]^+$.

$^1H$ NMR (400 MHz, DMSO-$d_6$) δ 8.47 (s, 0.4H), 8.43 (s, 0.6H), 8.33 (s, 0.5H), 8.31 (s, 0.5H), 8.27 (d, *J* = 7.3 Hz, 0.6H), 8.11 (d, *J* = 7.8 Hz, 0.4H), 7.79 (s, 0.5H), 7.77 (s, 0.4H), 5.48 - 5.38 (m, 1H), 3.91 (s, 3H), 3.78 (br.s, 1H), 3.53 - 3.40 (m, 2H), 2.44 - 2.30 (m, 2H), 2.02 - 1.84 (m, 4H), 1.78 - 1.51 (m, 8H).

**Example 7:**

**(*S*)-*N*-(1-((1-Methyl-1*H*-pyrazol-4-yl)sulfonyl)piperidin-4-yl)-4-((tetrahydrofuran-3-yl)oxy)-5-(trifluoromethyl)pyrimidin-2-amine**

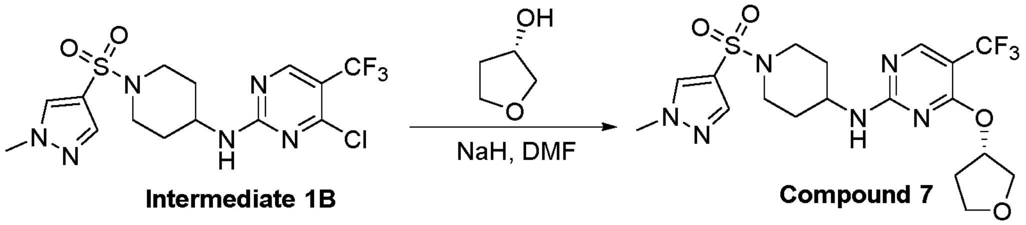


Intermediate 1B

Compound 7

(S)-3-Hydroxytetrahydrofuran (16.0 g, 0.2 mmol) was dissolved in *N,N*-dimethylformamide (1 mL) at room temperature under nitrogen atmosphere. Subsequently, sodium hydride (7.2 mg, 0.2 mmol, 60% dispersed in mineral oil) was added to the above solution under cooling in an ice-water bath. After the reaction mixture was stirred at room temperature for 10 minutes, compound **1B** (50.0 mg, 0.1 mmol) was slowly added to the reaction mixture. The reaction system was heated to 100°C and continued to stir for 1 hour. Water (20 mL) was added to the reaction mixture to quench the reaction. The mixture was extracted with ethyl acetate (5 mL × 3 times), and the organic phases were combined. The organic phases were washed with saturated brine (20 mL × 3 times) first, then dried over anhydrous sodium sulfate, filtered and finally concentrated under reduced pressure. The resulting residue was purified by preparative high performance liquid chromatography to obtain 28.0 mg of (*S*)-*N* (1-((1-methyl-1*H*-pyrazol-4-yl)sulfonyl)piperidin-4-yl)-4-((tetrahydrofuran-3-yl)oxy)-5-(trifluoromethyl)pyrimidin-2-amine (compound **7**).

MS (ESI) m/z: 477.2 $[M+H]^+$.

$^1H$ NMR (400 MHz, DMSO-$d_6$) δ 8.41 - 8.27 (m, 2H), 8.03 (d, *J* = 7.3 Hz, 0.6H), 7.88 (d, *J* = 7.2 Hz, 0.4H), 7.78 (d, *J* = 6.4 Hz, 1H), 5.65 - 5.50 (m, 1H), 4.03 - 3.85 (m, 4H), 3.85 - 3.66 (m, 4H), 3.55 - 3.42 (m, 2H), 2.48 - 2.30 (m, 2H), 2.29 - 2.14 (m, 1H), 2.07 - 1.85 (m, 3H), 1.70 - 1.50 (m, 2H).

**Example 8:**

**(*S*)-2-((1-((4-(1-Methyl-1*H*-pyrazol-4-yl)phenyl)sulfonyl)piperidin-4-yl)amino)-4-((tetrahydrofuran-3-yl)amino)pyrimidine-5-carbonitrile**

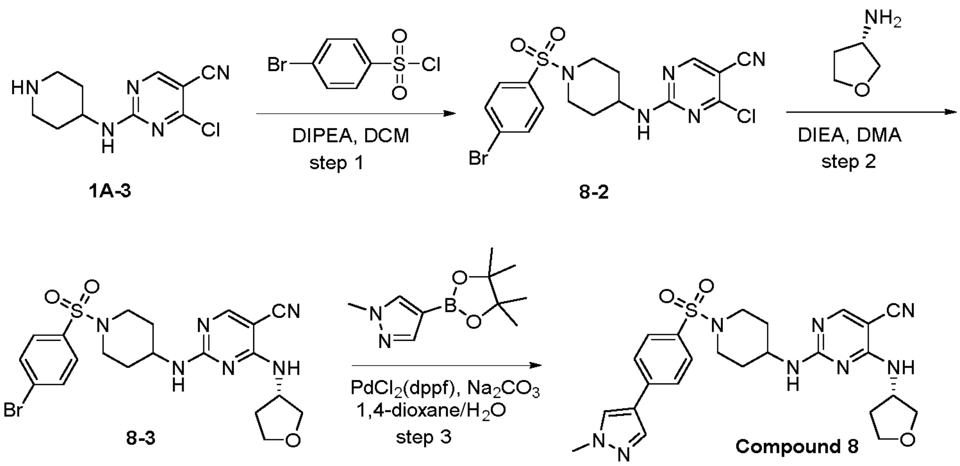


1A-3 8-2 8-3 Compound 8

**Step 1:** Compound **1A-3** (556.0 mg, 2.4 mmol) was dissolved in dichloromethane (3 mL) at room temperature. Subsequently, *N,N*-diisopropylethylamine (940.0 mg, 7.3 mmol) was added to the reaction mixture. p-Bromobenzenesulfonyl chloride (743.0 mg, 3.3 mmol) was added dropwise to the above solution at 0°C, and the reaction mixture was stirred overnight at room temperature. Water (20 mL) was added to the reaction mixture to quench the reaction. The mixture was extracted with ethyl acetate (30 mL × 3 times), and the organic phases were combined. The organic phases were washed with saturated brine (30 mL) first, then dried over anhydrous sodium sulfate, filtered and finally concentrated under reduced pressure. The resulting residue was purified by silica gel column chromatography to obtain 830.0 mg of 2-((1-((4-bromophenyl)sulfonyl)piperidin-4-yl)amino)-4-chloropyrimidine-5-carbonitrile **(8-2)**.

MS (ESI) m/z: 456.0 $[M+H]^+$.

**Step 2:** Compound **8-2** (100.0 mg, 0.2 mmol) was dissolved in *N,N*-dimethylacetamide (1 mL) at room temperature. Subsequently, *N,N*-diisopropylethylamine (85.0 mg, 0.7 mmol) and (*S*)-3-aminotetrahydrofuran(29.0 mg, 0.3 mmol) were sequentially added to the above reaction mixture. The reaction mixture was heated to 80°C and stirred for 4 hours. Water (20 mL) was added to the reaction mixture to quench the reaction. The mixture was extracted with ethyl acetate (30 mL × 3 times), and the organic phases were combined. The organic phases were washed with water (30 mL × 3 times) and saturated brine (30 mL) first, then dried over anhydrous sodium sulfate, filtered and finally concentrated under reduced pressure. The resulting residue was purified by preparative high performance liquid chromatography to obtain 95.0 mg of (*S*)-2-((1-((4-bromophenyl)sulfonyl)piperidin-4-yl)amino)-4-((tetrahydrofuran-3-yl)amino)pyrimidine-5-carbonitrile **(8-3).**

MS (ESI) m/z: 507.0 $[M+H]^+$.

**Step 3:** Compound **8-3** (95.0 mg, 0.2 mmol) was dissolved in 1,4-dioxane/water (5/1 mL) at room temperature under nitrogen atmosphere. Subsequently, 1-methyl-4-pyrazole boronic acid pinacol ester (50.0 mg, 0.2 mmol), $PdCl_2$(dppf) (15.0 mg, 0.02 mmol) and anhydrous sodium carbonate (42 mg, 0.4 mmol) were added to the above reaction mixture in portions. The reaction mixture was heated to 100°C and stirred for 1 hour. The reaction mixture was cooled to room temperature and concentrated under reduced pressure. The resulting residue was purified by preparative high performance liquid chromatography to obtain 35.6 mg of (*S*)-2-((1-((4-(1-methyl-1*H*-pyrazol-4-yl)phenyl)sulfonyl)piperidin-4-yl)amino)-4-((tetrahydrofuran-3-yl)amino)pyrimidine-5-carbonitrile (compound **8**). ee value: 98.4%.

MS (ESI) m/z: 509.2 $[M+H]^+$.

$^1H$ NMR (400 MHz, DMSO-$d_6$) δ 8.32 (s, 1H), 8.17 (s, 0.3H), 8.13 (s, 0.7H), 8.00 (s, 1H), 7.82 (s, 0.7H), 7.80 (s, 1.3H), 7.73 - 7.64 (m, 2.7H), 7.50 (d, *J* = 5.9 Hz, 1H), 7.38 (d, *J* = 6.8 Hz, 0.4H), 4.59 - 4.37 (m, 1H), 3.89 (s, 3H), 3.84 - 3.61 (m, 5H), 3.59 - 3.45 (m, 3H), 2.15 - 1.80 (m, 5H), 1.63 - 1.46 (m, 2H).

**Example 9:**

**4-(Cyclopentylidenemethyl)-2-((1-((1-methyl-1*H*-pyrazol-4-yl)sulfonyl)piperidin-4-yl)amino)pyrimidine-5-carbonitrile**

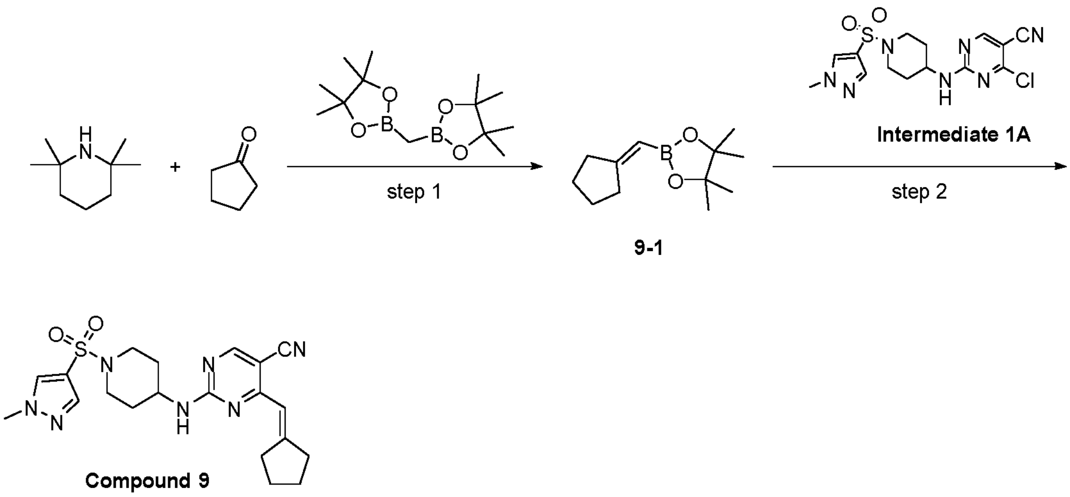

9-1
Compound 9

**Step 1:** 2,2,6,6-Tetramethylpiperidine (850.0 mg, 6.0 mmol) was dissolved in THF (10 mL) at room temperature. The mixture was cooled to -30°C, and then n-butyllithium (3.76 mL, 6.0 mmol) was slowly added dropwise to the above solution at -30°C under nitrogen atmosphere. The reaction mixture was stirred at -30°C for 30 minutes. After 30 minutes, the above solution was cooled to -78°C. A mixed solution of bis[(pinacolato)boryl]methane (1350.0 mg, 5.0 mmol) and tetrahydrofuran (5 mL), and another mixed solution of cyclopentanone (422 mg, 5.0 mmol) and tetrahydrofuran (5 mL) were slowly added dropwise in portions. The reaction mixture was stirred at room temperature for 18 hours. Ammonium chloride aqueous solution (10 mL) was added to the reaction mixture to quench the reaction. The mixture was concentrated under reduced pressure and extracted with ethyl acetate (40 mL × 3 times), and the organic phases were combined. The organic phases were washed with saturated brine (30 mL) first, then dried over anhydrous sodium sulfate, filtered and finally concentrated under reduced pressure. The resulting residue was purified by silica gel column chromatography to obtain 510 mg of 2-(cyclopentylidenemethyl)-4,4,5,5-tetramethyl-1,3,2-dioxaborolane (1A).

$^1H$ NMR (400 MHz, $CDCl_3$) δ 5.27 (t, *J* = 2.0 Hz, 1H), 2.52 (t, *J* = 7.2 Hz, 2H), 2.36 (t, *J* = 7.1 Hz, 2H), 1.77 - 1.59 (m, 4H), 1.25 (s, 12H).

**Step 2:** Compound **1A** (100.0 mg, 0.3 mmol) was dissolved in 1,4-dioxane/water (5/1 mL) at room temperature under nitrogen atmosphere. Subsequently, 2-(cyclopentylidenemethyl)-4,4,5,5-tetramethyl-1,3,2-dioxaborolane (62.0 mg, 0.3 mg mol), $PdCl_2$(dppf) (20.0 mg, 0.03 mmol) and anhydrous sodium carbonate (56 mg, 0.5 mmol) were sequentially added to the above reaction mixture. The reaction mixture was heated to 100°C and stirred for 1 hour. The reaction mixture was cooled to room temperature and concentrated under reduced pressure. The resulting residue was purified by preparative high performance liquid chromatography to obtain 44 mg of 4-(cyclopentylidenemethyl)-2-((1-((1-methyl-1*H*-pyrazol-4-yl)sulfonyl)piperidin-4-yl)amino)pyrimidine-5-carbonitrile (compound **9**).

MS (ESI) m/z: 428.2 $[M+H]^+$.

$^1H$ NMR (400 MHz, DMSO-$d_6$) δ 8.58 (s, 0.3H), 8.55 (s, 0.7H), 8.35 (s, 0.7H), 8.32 (s, 0.3H), 8.16 (d, *J* = 7.5 Hz, 0.7H), 7.95 (d, *J* = 7.5 Hz, 0.4H), 7.79 (s, 0.6H), 7.77 (s, 0.4H), 6.47 (s, 0.7H), 6.42 (s, 0.4H), 3.91 (s, 3H), 3.83 - 3.70 (m, 1H), 3.56 - 3.45 (m, 2H), 2.96 - 2.76 (m, 2H), 2.59 - 2.53 (m, 2H), 2.44 - 2.35 (m, 2H), 2.01 - 1.86 (m, 2H), 1.77 - 1.52 (m, 6H).

**Example 10:**

**4-(Cyclopentylmethyl)-2-((1-((1-methyl-1*H*-pyrazol-4-yl)sulfonyl)piperidin-4-yl)amino)pyrimidine-5-carbonitrile**

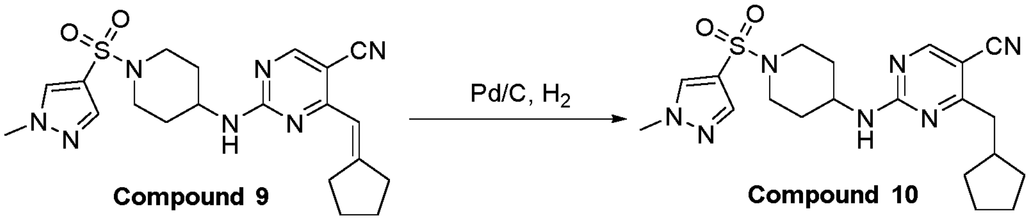


Compound 9

Compound 10

4-(Cyclopentylidenemethyl)-2-((1-((1-methyl-1*H*-pyrazol-4-yl)sulfonyl)piperidin-4-yl)amino)pyrimidine-5-carbonitrile (33 mg, 0.07 mmol) was dissolved in methanol (2 mL) at room temperature. Subsequently, 10% palladium-carbon (10 mg) was added to the above solution. After the reaction system was replaced with hydrogen for 3 times, the reaction mixture was stirred for 1 hour under hydrogen atmosphere. After filtration, the reaction mixture was concentrated under reduced pressure. The resulting residue was purified by preparative high performance liquid chromatography to obtain 3.6 mg of 4-(cyclopentylmethyl)-2-((1-((1-methyl-1H-pyrazol-4-yl)sulfonyl)piperidin-4-yl)amino)pyrimidine-5-carbonitrile (compound **10**).

MS (ESI) m/z: 430.2 $[M+H]^+$.

$^1H$ NMR (400 MHz, DMSO-$d_6$) δ 8.61 (s, 0.5H), 8.56 (s, 0.5H), 8.33 (s, 0.5H), 8.32 (s, 0.5H), 8.26 (d, *J* = 8.0 Hz, 0.5H), 8.22 (d, *J* = 7.2 Hz, 0.5H), 7.77 (d, *J* = 6.8 Hz, 1H), 3.91 (s, 3H), 3.79 (br.s., 1H), 3.51 - 3.43 (m, 2H), 2.69 - 2.64 (m, 2H), 2.46 - 2.38 (m, 2H), 2.26 - 2.19 (m, 1H), 1.98 - 1.85 (m, 2H), 1.72 - 1.42 (m, 8H), 1.26 - 1.14 (m, 2H).

**Example 11:**

**4-((1,3-*cis*)-3-Hydroxycyclopentyl)amino)-2-((1-((1-methyl-1*H*-pyrazol-4-yl)sulfonyl)piperidin-4-yl)amino)pyrimidine-5-carbonitrile**

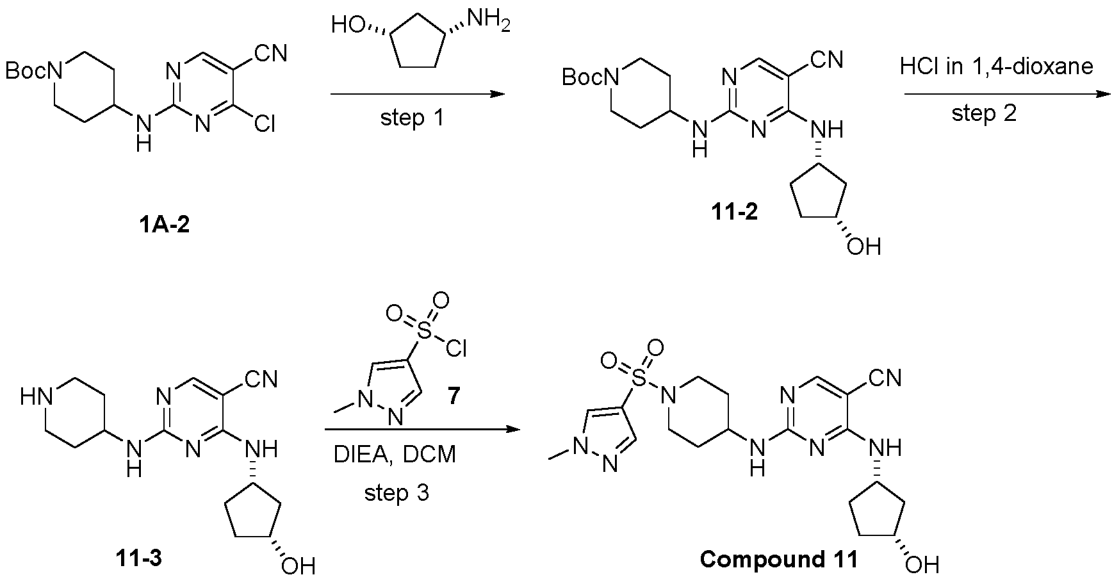


1A-2

11-2

11-3

Compound 11

**Step 1:** Compound **1A-2** (80.0 mg, 0.2 mmol) was dissolved in *N,N*-dimethylformamide (1 mL) at room temperature. Subsequently, *N,N*-diisopropylethylamine (50.0 mg, 0.39 mmol) and (1,3-*cis*)-3-aminocyclopentanol hydrochloride (49.0 mg, 0.3 mmol) were sequentially added to the above reaction mixture. The reaction mixture was heated to 120°C and stirred for 6 hours. The reaction mixture was cooled to room temperature and concentrated under reduced pressure. The resulting residue was quenched with water (10 mL). The mixture was extracted with ethyl acetate (30 mL × 3 times), and the organic phases were combined. The organic phases were washed with saturated brine (10 mL × 3 times) first, then dried over anhydrous sodium sulfate, filtered and finally concentrated under reduced pressure. The resulting residue was purified by silica gel column chromatography to obtain 53.0 mg of tert-butyl 4-((5-cyano-4-(((1,3-*cis*)-3-hydroxycyclopentyl)amino)pyrimidin-2-yl)amino)piperidine-1-carboxylate **(11-2)**.

MS (ESI) m/z: 403.2 $[M+H]^+$.

**Step 2:** Compound **11-2** (53.0 mg, 0.1 mmol) was dissolved in 1,4-dioxane (1 mL) at room temperature. Subsequently, a hydrogen chloride-dioxane solution (4 mol/L, 2 mL) was added thereto. The reaction mixture was stirred at room temperature for 2 hours. The reaction mixture was concentrated under reduced pressure to obtain 40.0 mg of 4-((1,3-*cis*)-3-hydroxycyclopentyl)amino)-2-(piperidin-4-ylamino)pyrimidine-5-carbonitrile **(11-3).**

MS (ESI) M/Z:303.2 $[M+H]^+$.

**Step 3:** Compound **11-3** (40.0 mg, 0.1 mmol) was dissolved in dichloromethane (3 mL) at room temperature. Subsequently, *N,N*-diisopropylethylamine (50.0 mg, 0.6 mmol) was added thereto. 1-Methyl-1*H*-pyrazole-4-sulfonyl chloride (28.0 mg, 0.1 mmol) was added dropwise to the above solution at 0°C, and the reaction mixture was stirred overnight at room temperature. Water (20 mL) was added to the reaction mixture to quench the reaction. The mixture was extracted with ethyl acetate (30 mL × 3 times), and the organic phases were combined. The organic phases were washed with saturated brine (30 mL) first, then dried over anhydrous sodium sulfate, filtered and finally concentrated under reduced pressure. The resulting residue was purified by preparative high performance liquid chromatography to obtain 28.4 mg of 4-((1,3-*cis*)-3-hydroxycyclopentyl)amino)-2-((1-((1-methyl-1*H*-pyrazol-4-yl)sulfonyl)piperidin-4-yl)amino)pyrimidine-5-carbonitrile (compound **11**).

MS (ESI) m/z: 447.2 $[M+H]^+$.

$^1$H NMR (400 MHz, DMSO-$d_6$) δ 8.32 (s, 0.6H), 8.3 1 (s, 0.4H), 8.17 (s, 0.4H), 8.13 (s, 0.5H), 7.78 (s, 0.6H), 7.76 (s, 0.4H), 7.65 (d, *J* = 7.2 Hz, 0.6H), 7.48 (d, *J* = 7.6 Hz, 0.4H), 7.07 (d, *J* = 7.6 Hz, 0.6H), 7.01 (d, *J* = 8.0 Hz, 0.4H), 4.81 (d, *J* = 3.6 Hz, 0.5H), 4.77 (d, *J* = 4.0 Hz, 0.7H), 4.51 - 4.29 (m, 1H), 4.17 - 4.08 (m, 1H), 3.90 (s, 3H), 3.72 (br.s., 1H), 3.51 - 3.39 (m, 3H), 2.41 - 2.31 (m, 1H), 2.05 - 1.79 (m, 4H), 1.76 - 1.46 (m, 6H).

**Example 12 and Example 13:**

**4-((1*S*,3*R*)-3-Hydroxycyclopentyl)amino)-2-((1-((1-methyl-1*H*-pyrazol-4-yl)sulfonyl)piperidin-4-yl)amino)pyrimidine-5-carbonitrile**

**4-((1*R*,3*S*)-3-Hydroxycyclopentyl)amino)-2-((1-((1-methyl-1*H*-pyrazol-4-yl)sulfonyl)piperidin-4-yl)amino)pyrimidine-5-carbonitrile**

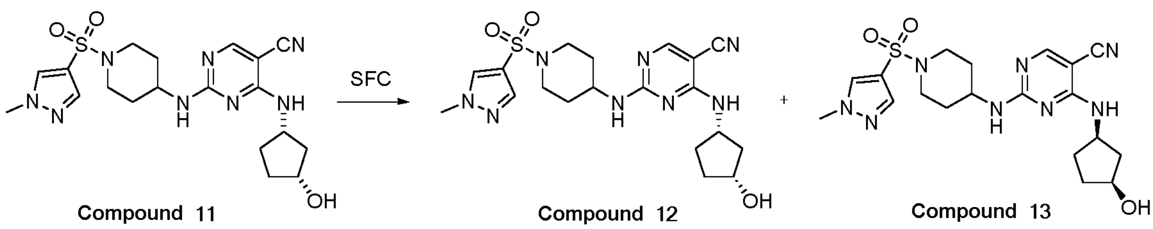

Compound 11 Compound 12 + Compound 13

Compound 12:

Compound **11** was subjected to chiral resolution, and the resolution conditions were as follows: preparative column 0.46 cm I.D. * 15 cmL; mobile phase: $CO_2$: MeOH (0.1% DEA) = 60:40; flow rate: 2.5 mL/min; detection wavelength: 254 nm. The product was collected and lyophilized under reduced pressure. A compound with a retention time of 3.77 minutes was obtained, and the ee value was 99.66%. The absolute configuration was not determined. It is an enantiomer of compound **13.**

MS (ESI) m/z: 447.2 $[M+H]^+$.

$^1$H NMR (400MHz, DMSO-$d_6$) δ 8.32 (s, 0.6H), 8.31 (s, 0.4H), 8.17 (s, 0.4H), 8.13 (s, 0.5H), 7.78 (s, 0.6H), 7.76 (s, 0.4H), 7.65 (d, *J* = 7.2 Hz, 0.6H), 7.48 (d, *J* = 7.6 Hz, 0.4H), 7.07 (d, *J* = 7.6 Hz, 0.6H), 7.01 (d, *J* = 8.0 Hz, 0.4H), 4.81 (d, *J* = 3.6 Hz, 0.5H), 4.77 (d, *J* = 4.0 Hz, 0.7H), 4.51 - 4.29 (m, 1H), 4.17 - 4.08 (m, 1H), 3.90 (s, 3H), 3.72 (br.s., 1H), 3.51 - 3.39 (m, 3H), 2.41 - 2.31 (m, 1H), 2.05 - 1.79 (m, 4H), 1.76 - 1.46 (m, 6H).

Compound 13:

Compound **11** was subjected to chiral resolution, and the resolution conditions were as follows: preparative column 0.46 cm I.D. * 15 cmL; mobile phase: $CO_2$: MeOH (0.1% DEA) = 60:40; flow rate: 2.5 mL/min; detection wavelength: 254 nm. The product was collected and lyophilized under reduced pressure. A compound with a retention time of 4.60 minutes was obtained, and the ee value was 99.88%. The absolute configuration was not determined. It was an enantiomer

of compound **12**.

MS (ESI) m/z: 447.2 $[M+H]^+$.

$^1$H NMR (400MHz, DMSO-$d_6$) δ 8.32 (s, 0.6H), 8.31 (s, 0.4H), 8.17 (s, 0.4H), 8.13 (s, 0.5H), 7.78 (s, 0.6H), 7.76 (s, 0.4H), 7.65 (d, *J* = 7.2 Hz, 0.6H), 7.48 (d, *J* = 7.6 Hz, 0.4H), 7.07 (d, *J* = 7.6 Hz, 0.6H), 7.01 (d, *J* = 8.0 Hz, 0.4H), 4.81 (d, *J* = 3.6 Hz, 0.5H), 4.77 (d, *J* = 4.0 Hz, 0.7H), 4.51 - 4.29 (m, 1H), 4.17 - 4.08 (m, 1H), 3.90 (s, 3H), 3.72 (br.s., 1H), 3.51 - 3.39 (m, 3H), 2.41 - 2.31 (m, 1H), 2.05 - 1.79 (m, 4H), 1.76 - 1.46 (m, 6H).

**Example 14:**

**4-((((1*R*,2*R*)-2-Methoxycyclopentyl)amino)-2-(((1-((1-methyl-1*H*-pyrazol-4-yl)sulfonyl)piperidin-4-yl)amino)pyrimidine-5-carbonitrile**

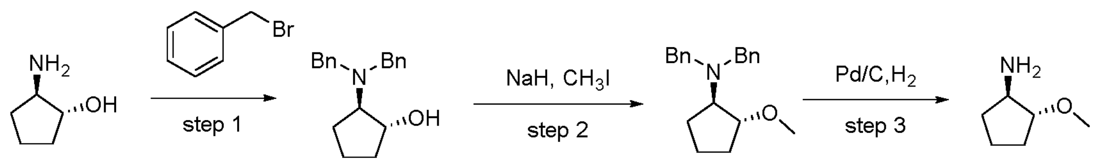


**14-1** **14-2** **14-3** **14-4**

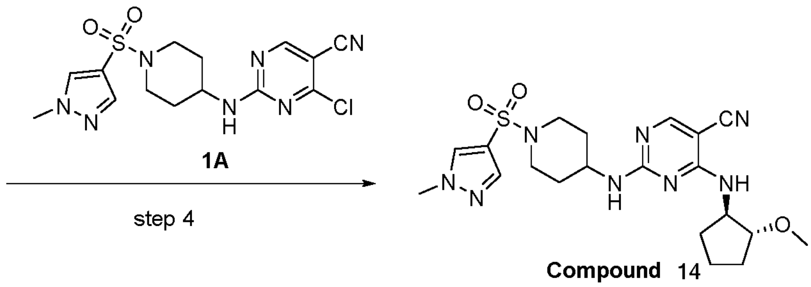


**1A**

**Compound** 14

**Step 1:** Compound (1*R*, 2*R*)-2-aminocyclopentanol hydrochloride (240.0 mg, 1.7 mmol) was dissolved in acetone/water (20 mL/1.4 mL) at room temperature. Subsequently, potassium carbonate (720.0 mg, 5.2 mmol) and benzyl bromide (0.4 mL, 3.5 mmol) were sequentially added to the above reaction mixture. The reaction mixture was heated to 56°C and stirred for 16 hours. Water (20 mL) was added to the reaction mixture to quench the reaction. The mixture was concentrated under reduced pressure and extracted with ethyl acetate (30 mL × 3 times), and the organic phases were combined. The organic phases were washed with saturated brine (30 mL) first, then dried over anhydrous sodium sulfate, filtered and finally concentrated under reduced pressure. The resulting residue was purified by silica gel column chromatography to obtain 340 mg of (1*R*,2*R*)-2-(dibenzylamino)cyclopentan-1-ol (compound **14**).

MS (ESI) m/z: 282.2 $[M+H]^+$.

**Step 2:** Compound **14-2** (100.0 mg, 0.4 mmol) was dissolved in THF (5 mL) at room temperature. Subsequently, sodium hydride (27 mg, 0.7 mmol, 60% dispersed in mineral oil) was added in portions to the above solution under cooling in an ice-water bath. After the reaction mixture was stirred at room temperature for 30 minutes, iodomethane (99 mg, 0.7 mmol) was slowly added dropwise to the above reaction mixture. The reaction mixture was continued to stir overnight at room temperature. Water (10 mL) was added to the reaction system to quench. The mixture was extracted with ethyl acetate (20 mL × 3 times), and the organic phases were combined. The organic phases were washed with saturated brine (10 mL) first, then dried over anhydrous sodium sulfate, filtered and finally concentrated under reduced pressure. The resulting residue was purified by silica gel column chromatography to obtain 80 mg of (1*R*,2*R*)-*N,N*-dibenzyl-2-methoxycyclopentan-1-amine **(14-3).**

MS (ESI) m/z: 296.2 $[M+H]^+$.

**Step 3:** Compound **14-3** (80 mg, 0.3 mmol) was dissolved in methanol (5 mL). Subsequently, 10% palladium/carbon (8 mg) was added to the above solution. After the reaction system was replaced with hydrogen three times, the reaction mixture was stirred at room temperature for 1 hour. The reaction mixture was filtered through diatomite. The filter cake was washed with methanol (10 mL × 3 times), and the resulting filtrate was concentrated under reduced pressure to obtain 46 mg of (1*R*,2*R*)-2-methoxycyclopentan-1-amine **(14-4)**.

$^1$H NMR (400 MHz, DMSO-$d_6$) δ 8.26 (br. s., 2H), 3.80 - 3.68 (m, 1H), 3.37 - 3.27 (m, 1H), 3.25 (s, 3H), 2.06 -

1.86 (m, 2H), 1.79 - 1.47 (m, 4H).

**Step 4:** Compound **14-4** (70.0 mg, 0.2 mmol) was dissolved in *N,N*-dimethylacetamide (1 mL) at room temperature. Subsequently, *N,N*-diisopropylethylamine (70.0 mg, 0.6 mmol) and compound **1A** (31.0 mg, 0.3 mmol) were sequentially added to the above reaction mixture. The reaction mixture was heated to 80°C and stirred for 4 hours. Water (20 mL) was added to the reaction mixture to quench the reaction. The mixture was extracted with ethyl acetate (30 mL × 3 times), and the organic phases were combined. The organic phases were washed with water (30 mL × 3 times) and saturated brine (30 mL) first, then dried over anhydrous sodium sulfate, filtered and finally concentrated under reduced pressure. The resulting residue was purified by preparative high performance liquid chromatography to obtain 31.5 mg of 4-((1*R*,2*R*)-2-methoxycyclopentyl)amino)-2-((1-((1-methyl-1*H*-pyrazol-4-yl)sulfonyl)piperidin-4-yl)amino)pyrimidine-5-carbonitrile (compound 14).

MS (ESI) m/z: 461.2 $[M+H]^+$.

$^1$HNMR (400 MHz, DMSO-$d_6$) δ 8.34 (s, 0.6H), 8.31 (s, 0.4H), 8.17 (s, 0.4H), 8.13 (s, 0.6H), 7.78 (s, 0.6H), 7.76 (s, 0.4H), 7.67 (d, *J* = 7.4 Hz, 0.6H), 7.46 (d, *J* = 7.8 Hz, 0.4H), 7.35 (d, *J* = 7.6 Hz, 1H), 4.47 - 4.37 (m, 0.5H), 4.28 - 4.19 (m, 0.7H), 3.91 (s, 3H), 3.79 - 3.65 (m, 2H), 3.53 - 3.41 (m, 2H), 3.20 (s, 1.3H), 3.13 (s, 1.7H), 2.44 - 2.29 (m, 2H), 2.00 - 1.72 (m, 4H), 1.72 - 1.39 (m, 6H).

**Example 15:**

**4-(Cyclopentylamino)-2-((1-((4-(1,2,3,6-tetrahydropyridin-4-yl)phenyl)sulfonyl)piperidin-4-yl)amino)pyrimidine-5-carbonitrile**

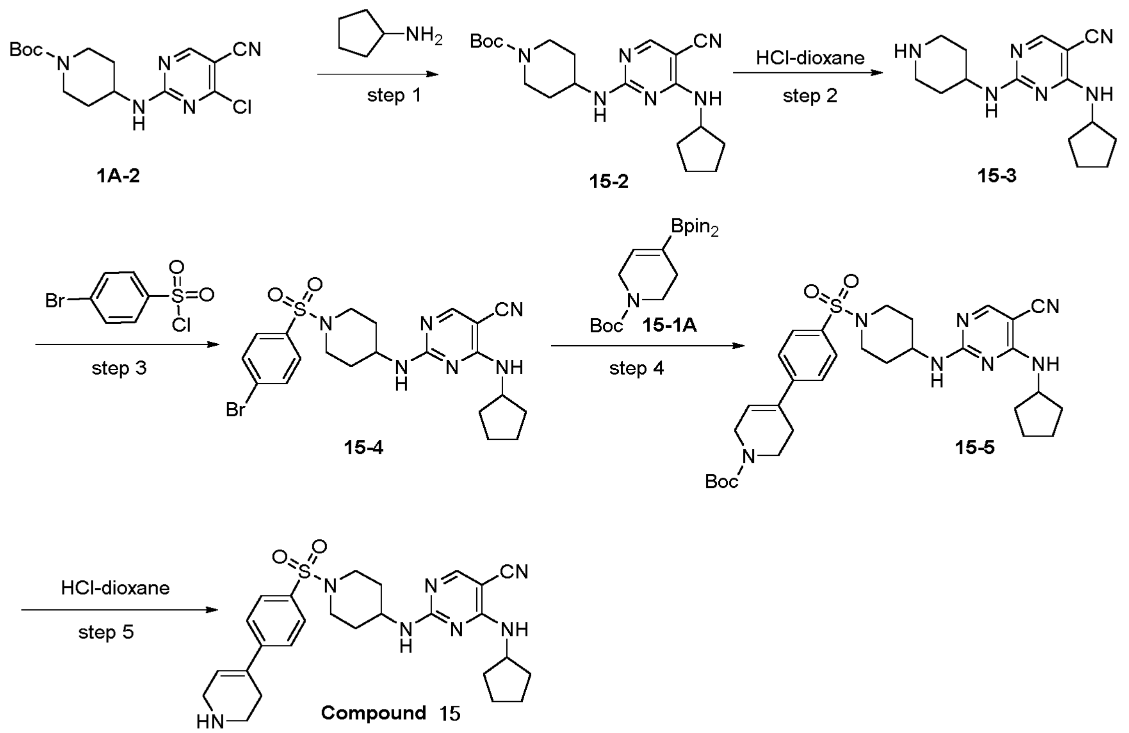


**Step 1:** Compound **1A-2** (605 mg, 1.79 mmol) was dissolved in *N,N*-dimethylacetamide (4 mL) at room temperature. Subsequently, cyclopentylamine (183.2 mg, 2.15 mmol) and N,N-diisopropylethylamine (463.9 mg, 3.59 mmol) were sequentially added to the reaction mixture. The reaction mixture was heated to 80°C and stirred overnight. The reaction mixture was cooled to room temperature and quenched by adding water (40 mL). The mixture was extracted with dichloromethane (40 mL × 3 times). The organic phases were combined, washed with saturated brine (100 mL) first, and then dried over anhydrous sodium sulfate, filtered and finally concentrated under reduced pressure. The resulting residue was purified by silica gel column chromatography to obtain 580 mg of tert-butyl 4-((5-cyano-4-(cyclopentylamino)pyrimidin-2-yl)amino)piperidine-1-carboxylate **(15-2).**

MS (ESI) m/z: 387.2 $[M+H]^+$.

**Step 2:** Compound **15-2** (580 mg, 1.50 mmol) was dissolved in 1,4-dioxane (3 mL) at room temperature.

Subsequently, a hydrogen chloride-dioxane solution (3 mL, 6 mmol) was added thereto. The reaction mixture was stirred at room temperature for 2 hours. The reaction mixture was concentrated under reduced pressure to obtain 610 mg of 4-(cyclopentylamino)-2-(piperidin-4-ylamino)pyrimidine-5-carbonitrile **(15-3)**.

MS (ESI) m/z: 287.2 $[M+H]^+$.

**Step 3:** Compound **15-3** (610.0 mg, 2.13 mmol) was dissolved in dichloromethane (9 mL) at room temperature. Subsequently, *N,N*-diisopropylethylamine (826.0 mg, 6.39 mmol) was added thereto. p-Bromobenzenesulfonyl chloride (649.0 mg, 2.56 mmol) was added dropwise to the above solution at 0°C, and the reaction mixture was stirred at room temperature for 3 hours. Water (40 mL) was added to the reaction mixture to quench the reaction. The mixture was extracted with dichloromethane (40 mL × 3 times), and the organic phases were combined. The organic phases were washed with saturated brine (100 mL) first, then dried over anhydrous sodium sulfate, filtered and finally concentrated under reduced pressure. The resulting residue was purified by silica gel column chromatography to obtain 605.0 mg of 2-((1-((4-bromophenyl)sulfonyl)piperidin-4-yl)amino)-4-(cyclopentylamino)pyrimidine-5-carbonitrile **(15-4).**

MS (ESI) m/z: 505.0 $[M+H]^+$.

**Step 4:** Compound **15-4** (300.0 mg, 0.59 mmol) was dissolved in 1,4-dioxane (4 mL) and water (0.4 mL) at room temperature. Subsequently, compound **15-1A** (130.0 mg, 0.21 mmol), sodium carbonate (126 mg, 1.19 mmol) and [1,1'-bis(diphenylphosphino)ferrocene]dichloropalladium(II) (92 mg, 0.12 mmol) were sequentially added thereto, and the reaction mixture was stirred overnight at 100°C. After cooling the reaction mixture to room temperature, water (40 mL) was added to the reaction mixture to quench. The mixture was extracted with dichloromethane (40 mL × 3 times), and the organic phases were combined. The organic phases were washed with saturated brine (100 mL) first, then dried over anhydrous sodium sulfate, filtered and finally concentrated under reduced pressure. The resulting residue was purified by silica gel column chromatography to obtain 330.0 mg of tert-butyl 4-(4-((4-((5-cyano-4-(cyclopentylamino)pyrimidin-2-yl)amino)piperidin-1-yl)sulfonyl)phenyl)-3,6-dihydropyridine-1(2*H*)-carboxylate **(15-5).**

MS (ESI) m/z: 608.2 $[M+H]^+$.

**Step 5:** Compound **15-5** (80 mg, 1.50 mmol) was dissolved in 1,4-dioxane (3 mL) at room temperature. Subsequently, a hydrogen chloride-dioxane solution (3 mL, 6 mmol) was added thereto. The reaction mixture was stirred at room temperature for 2 hours. The reaction mixture was added with dichloromethane (20 mL × 3 times) and concentrated under reduced pressure. The resulting residue was purified by preparative high performance liquid chromatography to obtain 20.2 mg of 4-(cyclopentylamino)-2-((1-((4-(1,2,3,6-tetrahydropyridin-4-yl)phenyl)sulfonyl)piperidin-4-yl)amino)pyrimidine-5-carbonitrile (compound **15**).

MS (ESI) m/z: 508.2 $[M+H]^+$.

$^1H$ NMR (400 MHz, DMSO-$d_6$) δ 8.13 (s, 0.4H), 8.10 (s, 0.6H), 7.72 - 7.64 (m, 4H), 7.60 (d, *J* = 7.2 Hz, 0.6H), 7.43 (d, *J* = 7.8 Hz, 0.4H), 7.23 (d, *J* = 6.6 Hz, 0.6H), 7.13 (d, *J* = 7.6 Hz, 0.4H), 6.43 (s, 1H), 4.44 - 4.31 (m, 0.4H), 4.27 - 4.14 (m, 0.6H), 3.68 (br.s., 1H), 3.60 - 3.46 (m, 2H), 3.40 (br.s., 2H), 2.92 (s, 2H), 2.44 - 2.31 (m, 4H), 1.95 - 1.74 (m, 4H), 1.72 - 1.36 (m, 9H).

**Example 16:**

**4-(Cyclopentylamino)-2-((1-((4-(1-methyl-1,2,3,6-tetrahydropyridin-4-yl)phenyl)sulfonyl)piperidin-4-yl)amino)pyrimidine-5-carbonitrile**

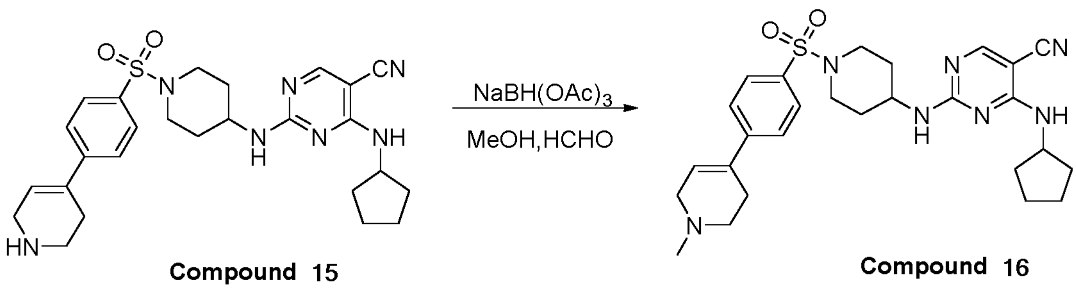


**Compound 15** **Compound 16**

Compound **15** (70.0 mg, 0.14 mmol) was dissolved in methanol (4 mL) at room temperature. Subsequently, acetic acid (16.8 mg, 0.28 mmol) and formaldehyde aqueous solution (21 mg, 0.70 mmol) were sequentially added thereto, and the mixture was stirred at room temperature for 0.5 hours; then sodium triacetoxyborohydride (152.6 mg, 0.70 mmol) was added to the system, and the reaction was continued for 0.5 hours. The reaction mixture was added with dichloromethane (20 mL × 3 times) and concentrated under reduced pressure. The resulting residue was purified

by preparative high performance liquid chromatography to obtain 9.8 mg of 4-(cyclopentylamino)-2-((1-((4-(1-methyl-1,2,3,6-tetrahydropyridin-4-yl)phenyl)sulfonyl)piperidin-4-yl)amino)pyrimidine-5-carbonitrile (compound **16**).

MS (ESI) m/z: 522.2 $[M+H]^+$.

$^1H$ NMR (400 MHz, DMSO-$d_6$) δ 8.13 (s, 0.4H), 8.10 (s, 0.6H), 7.73 - 7.64 (m, 4H), 7.60 (d, *J* = 7.2 Hz, 0.6H), 7.43 (d, *J* = 7.8 Hz, 0.4H), 7.23 (d, *J* = 6.6 Hz, 0.6H), 7.13 (d, *J* = 7.6 Hz, 0.4H), 6.41 - 6.35 (m, 1H), 4.42 - 4.30 (m, 0.4H), 4.27 - 4.14 (m, 0.6H), 3.68 (br.s., 1H), 3.60 - 3.43 (m, 2H), 3.05 (d, *J* = 3.0 Hz, 2H), 2.62 - 2.54 (m, 2H), 2.51 (s, 2H), 2.47 - 2.31 (m, 2H), 2.28 (s, 3H), 1.95 - 1.75 (m, 4H), 1.70 - 1.35 (m, 8H).

**Example 17:**

**4-(Cyclopentylamino)-2-((1-((4-(1-methylpiperidin-4-yl)phenyl)sulfonyl)piperidin-4-yl)amino)pyrimidine-5-carbonitrile**

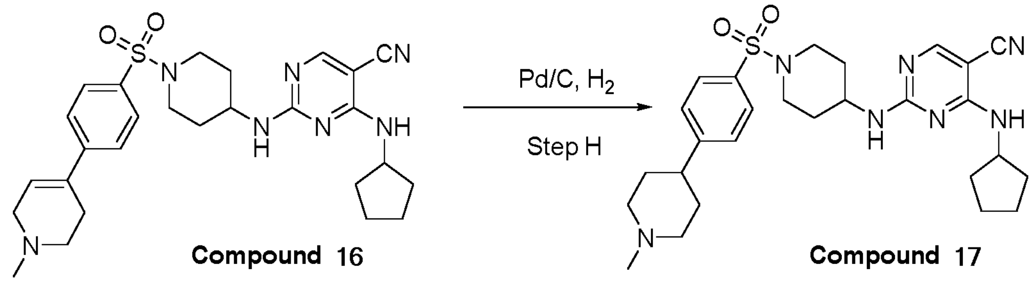


**Compound 16** **Compound 17**

Compound **16** (50.0 mg, 0.10 mmol) was dissolved in ethanol (4 mL) at room temperature. Subsequently, wet palladium on carbon (30 mg, 60%) was added thereto in portions, and stirred at room temperature for 6 hours. The reaction mixture was filtered, and the filter cake was washed with ethanol (20 mL × 3 times). The filtrate was concentrated under reduced pressure. The resulting residue was purified by preparative high performance liquid chromatography to obtain 9.3 mg of 4-(cyclopentylamino)-2-((1-((4-(1-methylpiperidin-4-yl)phenyl)sulfonyl)piperidin-4-yl)amino)pyrimidine-5-carbonitrile (compound **17**).

MS (ESI) m/z: 524.3 $[M+H]^+$.

$^1H$ NMR (400 MHz, DMSO-$d_6$) δ 8.13 (s, 0.4H), 8.10 (s, 0.6H), 7.72 - 7.58 (m, 2.5H), 7.52 (s, 1H), 7.50 (s, 1H), 7.43 (d, *J* = 7.7 Hz, 0.4H), 7.24 (d, *J* = 6.6 Hz, 0.6H), 7.13 (d, *J* = 7.6 Hz, 0.4H), 4.44 - 4.30 (m, 0.4H), 4.26 - 4.13 (m, 0.6H), 3.67 (s, 1H), 3.60 - 3.44 (m, 2H), 2.87 (d, *J* = 11.3 Hz, 2H), 2.69 - 2.55 (m, 2H), 2.44 - 2.30 (m, 2H), 2.19 (s, 3H), 2.04 - 1.33 (m, 17H).

**Example 18:**

**4-(Cyclopentylamino)-2-((1-((4-(4-methylpiperazin-1-yl)phenyl)sulfonyl)piperidin-4-yl)amino)pyrimidine-5-carbonitrile**

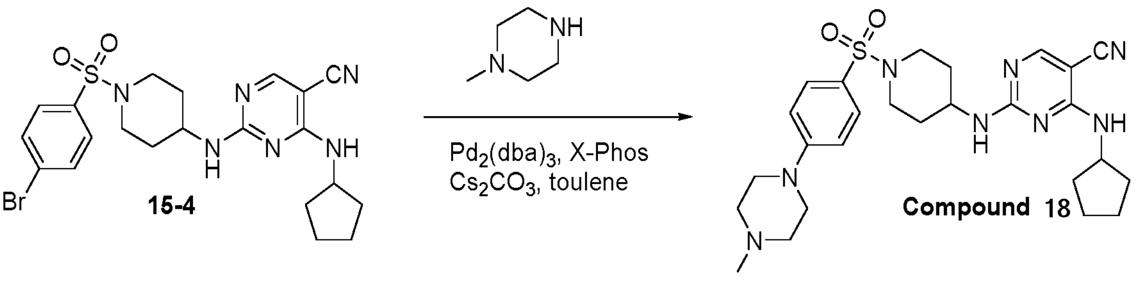


**15-4** **Compound 18**

Compound **15-4** (60.0 mg, 0.1 mmol) was dissolved in toluene (5 mL) at room temperature under nitrogen atmosphere. Subsequently, *N*-methylpiperazine (17.8 mg, 0.2 mmol), $Pd_2(dba)_3$ (11.0 mg, 0.01 mmol), 2-dicyclohexylphosphino-2',4',6'-triisopropylbiphenyl (11.4 mg, 0.02 mmol) and anhydrous cesium carbonate (78 mg, 0.2 mmol) were sequentially added to the above reaction mixture. The reaction mixture was heated to 100°C and stirred for 18 hours.

The reaction mixture was cooled to room temperature and concentrated under reduced pressure. The resulting residue was purified by preparative high performance liquid chromatography to obtain 6.3 mg of 4-(cyclopentylamino)-2-((1-((4-(4-methylpiperazin-1-yl)phenyl)sulfonyl)piperidin-4-yl)amino)pyrimidine-5-carbonitrile (compound **18**).

MS (ESI) m/z: 525.2 $[M+H]^+$.

$^1$H NMR (400 MHz, DMSO-$d_6$) δ 8.14 (s, 0.4H), 8.10 (s, 0.6H), 7.61 (d, *J* = 7.0 Hz, 0.6H), 7.51 (d, *J* = 8.8 Hz, 2H), 7.43 (d, *J* = 7.4 Hz, 0.4H), 7.24 (d, *J* = 6.8 Hz, 0.6H), 7.13 (d, *J* = 7.8 Hz, 0.4H), 7.07 (d, *J* = 9.0 Hz, 2H), 4.45 - 4.30 (m, 0.4H), 4.28 - 4.13 (m, 0.6H), 3.74 - 3.58 (m, 1H), 3.55 - 3.39 (m, 2H), 3.31 - 3.25 (m, 2H), 2.45 - 2.26 (m, 7H), 2.22 (s, 3H), 1.94 - 1.76 (m, 4H), 1.72 - 1.61 (m, 2H), 1.60 - 1.34 (m, 7H).

**Example 19:**

**4-(*trans*-2-Hydroxy-2-methylcyclopentyl)amino)-2-((1-((1-methyl-1*H*-pyrazol-4-yl)sulfonyl)piperidin-4-yl)amino)pyrimidine-5-carbonitrile**

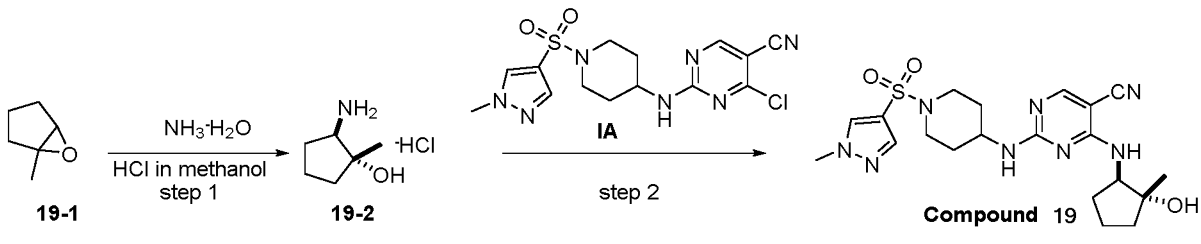

19-1 19-2 IA Compound 19

**Step 1:** Methyl-1,2-cyclopentene oxide (70.0 mg, 0.7 mmol) was dissolved in ammonia water (1 mL) at room temperature. The reaction system was heated to 90°C and reacted for 15 hours. A methanol solution of hydrochloric acid (4 mol) was added to the reaction mixture under an ice-water bath until pH = 3. The reaction mixture was concentrated under reduced pressure to obtain 200 mg of compound **19-2**, which was directly used in the next reaction step.

MS (ESI) m/z: 116.2 $[M+H]^+$.

**Step 2:** Compound **19-2** (90.0 mg, 0.2 mmol) was dissolved in *N,N*-dimethylformamide (0.5 mL) at room temperature. Subsequently, *N,N*-diisopropylethylamine (91.0 mg, 0.7 mmol) and compound **1A** (91.0 mg, 0.6 mmol) were added to the above solution. The reaction system was heated to 80°C and continued to stir for 15 hours. Water (20 mL) was added to the reaction mixture to quench the reaction. The mixture was extracted with ethyl acetate (8 mL × 3 times), and the organic phases were combined. The organic phases were washed with saturated brine (30 mL) first, then dried over anhydrous sodium sulfate, filtered and finally concentrated under reduced pressure. The resulting residue was purified by preparative high performance liquid chromatography to obtain 35.0 mg of the final product 4-(trans-2-hydroxy-2-methylcyclopentyl)amino)-2-((1-((1-methyl-1*H*-pyrazol-4-yl)sulfonyl)piperidin-4-yl)amino)pyrimidine-5-carbonitrile (compound **19**).

MS (ESI) m/z: 461.2 $[M+H]^+$.

$^1$H NMR (400 MHz, DMSO-$d_6$) δ 8.35 - 8.29 (m, 1H), 8.22 - 8.11 (m, 1H), 7.81 - 7.73 (m, 1H), 7.64 (d, *J* = 7.2 Hz, 0.6H), 7.53 (d, *J* = 7.6 Hz, 0.4H), 6.87 (d, *J* = 8.0 Hz, 0.5H), 6.77 (d, *J* = 8.4 Hz, 0.4H), 4.50 (s, 0.4H), 4.41 (s, 0.5H), 4.38 - 4.24 (m, 1H), 3.91 (s, 3H), 3.80 - 3.68 (m, 1H), 3.59 - 3.32 (m, 2H), 2.48 - 2.28 (m, 2H), 2.09 - 1.81 (m, 3H), 1.71 - 1.41 (m, 7H), 1.13 - 0.99 (m, 3H).

**Example 20 and Example 21:**

**4-((1*R*,2*R*)-2-Hydroxy-2-methylcyclopentyl)amino)-2-((1-((1-methyl-1*H*-pyrazol-4-yl)sulfonyl)piperidin-4-yl)amino)pyrimidine-5-carbonitrile**

**4-((1*S*,2*S*)-2-Hydroxy-2-methylcyclopentyl)amino)-2-((1-((1-methyl-1*H*-pyrazol-4-yl)sulfonyl)piperidin-4-yl)amino)pyrimidine-5-carbonitrile**

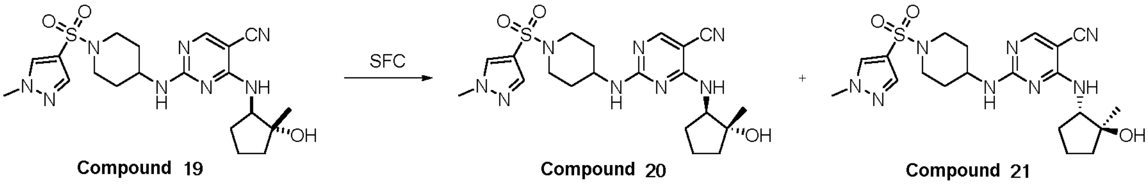


Compound 19 Compound 20 Compound 21

Compound 20:

Compound **19** was subjected to chiral resolution, and the resolution conditions were as follows: preparative column IG 25 * 250 mm, 10 μm (Daicel); mobile phase: $CO_2$: MeOH (0.2% ammonia methanol) = 60:40; flow rate: 100 g/min; detection wavelength: 214 nm. The product was collected and lyophilized under reduced pressure. The resulting product was purified by preparative high performance liquid chromatography. A compound with a retention time of 4.05 minutes was obtained, and the ee value was 100%.

The absolute configuration was not determined. It was an enantiomer of compound **21**.

MS (ESI) m/z: 461.2 $[M+H]^+$.

$^1H$ NMR (400 MHz, DMSO-$d_6$) δ 8.32 (s, 0.5H), 8.31 (s, 0.5H), 8.21 (s, 0.4H), 8.18 (s, 0.5H), 7.77 (s, 0.5H), 7.79 - 7.75 (m, 1H), 7.72 (d, *J* = 6.4 Hz, 0.6H), 7.61 (d, *J* = 6.4 Hz, 0.5H), 7.05 (d, *J* = 7.6 Hz, 0.6H), 6.84 (d, *J* = 8.0 Hz, 0.5H), 4.39 - 4.28 (m, 1H), 3.91 (s, 3H), 3.78 - 3.75 (m, 1H), 3.53 - 3.38 (m, 2H), 2.46 - 2.28 (m, 2H), 2.07 - 1.83 (m, 3H), 1.69 - 1.45 (m, 7H), 1.12 - 1.00 (m, 3H).

Compound 21:

Compound **19** was subjected to chiral resolution, and the resolution conditions were as follows: preparative column IG 25 * 250 mm, 10 μm (Daicel); mobile phase: $CO_2$: MeOH (0.2% ammonia methanol) = 60:40; flow rate: 100 g/min; detection wavelength: 214 nm. The product was collected and lyophilized under reduced pressure. The resulting product was purified by preparative high performance liquid chromatography. A compound with a retention time of 5.44 minutes was obtained, and the ee value was 100%.

The absolute configuration was not determined. It was an enantiomer of compound **20**.

MS (ESI) m/z: 461.2 $[M+H]^+$.

$^1H$ NMR (400 MHz, DMSO-$d_6$) δ 8.32 (s, 0.5H), 8.31 (s, 0.5H), 8.19 (s, 0.5H), 8.13 (s, 0.6H), 7.77 (s, 0.5H), 7.76 (s, 0.5H), 7.63 (d, *J* = 7.6 Hz, 0.7H), 7.53 (d, *J* = 7.6 Hz, 0.5H), 6.87 (d, *J* = 7.6 Hz, 0.6H), 6.76 (d, *J* = 8.0 Hz, 0.5H), 4.50 (s, 0.5H), 4.41 (s, 0.6H), 4.39 - 4.26 (m, 1H), 3.91 (s, 3H), 3.81 - 3.66 (m, 1H), 3.54 - 3.39 (m, 2H), 2.46 - 2.33 (m, 2H), 2.07 - 1.81 (m, 3H), 1.72 - 1.45 (m, 7H), 1.12 - 1.00 (m, 3H).

**Example 22:**

**4-(Cyclopentylamino)-2-((1-((4-((4-methylpiperazin-1-yl)methyl)phenyl)sulfonyl)piperidin-4-yl)amino)pyrimidine-5-carbonitrile**

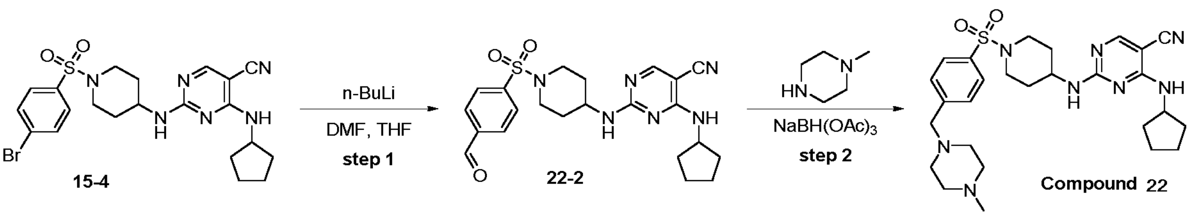


15-4 22-2 Compound 22

**Step 1:** Compound **15-4** (100.0 mg, 0.2 mmol) was dissolved in tetrahydrofuran (6 mL) at room temperature. Subsequently, a solution of n-butyllithium/n-hexane (2.5 mol/L, 0.8 mL, 2.0 mmol) was added thereto under nitrogen atmosphere at -78°C, and the reaction mixture was stirred at -78°C for 10 minutes. *N,N*-Dimethylacetamide(146.2 mg, 2.4 mmol) was further added thereto. The reaction mixture was stirred at room temperature for 2 hours. Saturated ammonium chloride aqueous solution (20 mL) was added to the reaction mixture to quench the reaction. The mixture was extracted with ethyl acetate (10 mL × 3 times), and the organic phases were combined. The organic phases were washed with saturated brine (30 mL) first, then dried over anhydrous sodium sulfate, filtered and finally concentrated under reduced pressure. The resulting residue was purified by silica gel column chromatography to obtain 80.0 mg of

4-(cyclopentylamino)-2-(((1-((4-formylphenyl)sulfonyl)piperidin-4-yl)amino)pyrimidine-5-carbonitrile (22-2).

MS (ESI) m/z: 455.2 $[M+H]^+$.

**Step 2:** Compound **22-2** (80.0 mg, 0.18 mmol) and *N*-methylpiperazine (18.0 mg, 0.18 mmol) were dissolved in 1,2-dichloroethane (1 mL) at room temperature. After the system was replaced with nitrogen three times, acetic acid (108.2 mg, 1.8 mmol) was added thereto under an ice-water bath. The reaction mixture was stirred at room temperature for 2 hours. Finally, sodium triacetoxyborohydride (114.4 mg, 0.54 mmol) was added thereto under an ice-water bath. The reaction mixture was stirred at room temperature for 2 hours. Saturated sodium bicarbonate aqueous solution (30 mL) was added to the reaction mixture to quench the reaction. The mixture was extracted with ethyl acetate (10 mL × 3 times), and the organic phases were combined. The organic phases were washed with saturated brine (30 mL) first, then dried over anhydrous sodium sulfate, filtered and finally concentrated under reduced pressure. The resulting residue was purified by preparative high performance liquid chromatography to obtain 12.2 mg of 4-(cyclopentylamino)-2-((1-((4-((4-methylpiperazin-1-yl)methyl)phenyl)sulfonyl)piperidin-4-yl)amino)pyrimidine-5-carbonitrile (compound 22).

MS (ESI) m/z: 539.2 $[M+H]^+$.

$^1$H NMR (400 MHz, DMSO-$d_6$) δ 8.17 (s, 0.4H), 8.11 (s, 0.6H), 7.87 - 7.77 (m, 2H), 7.71 - 7.62 (m, 1.6H), 7.52 - 7.43 (m, 1.4H), 7.25 (d, *J* = 6.6 Hz, 0.6H), 7.14 (d, *J* = 7.6 Hz, 0.4H), 4.50 - 4.20 (m, 1H), 3.93 - 3.70 (m, 3H), 3.61 (d, *J* = 12.4 Hz, 2H), 2.83 - 2.68 (m, 2H), 2.47 - 2.25 (m, 8H), 2.16 (s, 3H), 1.97 - 1.78 (m, 4H), 1.72 - 1.59 (m, 2H), 1.59 - 1.40 (m, 6H).

## Example 23:

**4-((1*S*,2*R*-*cis*)-2-(Dimethylamino)cyclopentyl)amino)-2-((1-((1-methyl-1*H*-pyrazol-4-yl)sulfonyl)piperidin-4-yl)amino)pyrimidine-5-carbonitrile**

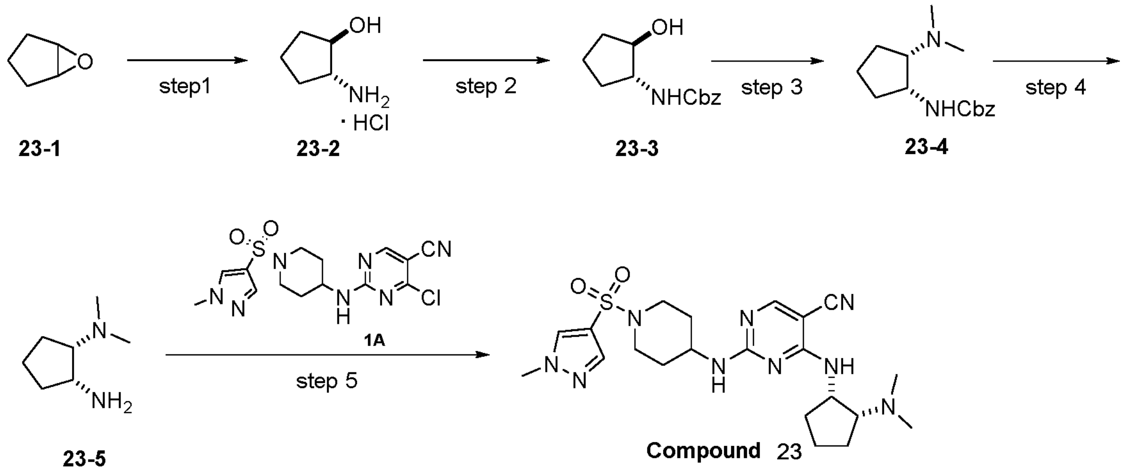


23-1 23-2 23-3 23-4 23-5 Compound 23

**Step 1:** A solution of 6-oxabicyclo[3.1.0]hexane (1000 mg, 12 mmol, 1.00 eq) and ammonia water (8 mL) was stirred at 90°C for 3 hours in a sealed tube. The reaction mixture was cooled to room temperature, and the pH was adjusted to 2 with concentrated hydrochloric acid. A large amount of white solid precipitated out of the solution, and the mixture was filtered. 1600 mg of (1*R*,2*R*-*trans*)-2-aminocyclopentan-1-ol **(23-2)** was obtained.

MS (ESI) m/z: 102.2 $[M+H]^+$.

**Step 2:** Compound **23-2** (1600 mg, 12 mmol) was dissolved in water/THF (20 mL/2 mL), and sodium hydroxide (960 mg, 24 mmol) and benzyloxycarbonyl chloride (2251 mg, 13.2 mmol) were added thereto at 0°C. The reaction mixture was warmed to room temperature and reacted for 4 hours. After quenching with water (60 mL), the reaction mixture was extracted with dichloromethane (3 × 20 mL). The organic phases were combined, washed with saturated brine (30 mL) first, then dried over anhydrous sodium sulfate and filtered. The residue was concentrated under reduced pressure and purified by silica gel column chromatography to obtain 800 mg of benzyl ((1*R*,2*R*-*trans*)-2-hydroxycyclopentyl)carbamate **(23-3).**

MS (ESI) m/z: 236.2 $[M+H^+]$.

$^1$H NMR (400 MHz, DMSO-$d_6$) δ 7.41 - 7.26 (m, 5H), 7.18 (d, *J* = 7.2 Hz, 1H), 5.00 (s, 2H), 4.67 (d, *J* = 4.3 Hz, 1H), 3.83 - 3.78 (m, 1H), 3.61 - 3.51 (m, 1H), 1.92 -1.78 (m, 1H), 1.76 - 1.72 (m, 1H), 1.64 - 1.51 (m, 2H), 1.45 - 1.30 (m, 2H).

**Step 3:** Compound **23-3** (500 mg, 2.15 mmol) was dissolved in dichloromethane (15 mL). Methanesulfonyl chloride (365 mg, 3.23 mmol) and triethylamine (0.90 mL, 6.45 mmol) were sequentially added thereto at 0°C. The

reaction mixture was warmed to room temperature and reacted for 1 hour. After quenching with water (60 mL), the reaction mixture was extracted with dichloromethane (3 × 20 mL). The organic phases were combined, washed with saturated brine (30 mL) first, then dried over anhydrous sodium sulfate, filtered and concentrated. The concentrated crude product and dimethylamine aqueous solution (10 mL) were stirred at room temperature for 4 hours. The reaction mixture was concentrated under reduced pressure to remove the solvent to obtain 360 mg of benzyl ((1R,2S-cis)-2-(dimethylamino)cyclopentyl)carbamate **(23-4).**

MS (ESI) m/z: 263.2 $[M+H]^+$.

**Step 4:** Compound **23-4** (360 mg, 1.37 mmol) was dissolved in methanol (15 mL) under hydrogen atmosphere. Palladium/carbon (10%, 730 mg) was added thereto. The reaction mixture was stirred at room temperature for 3 hours. After filtration and concentration under reduced pressure, 150 mg of (1*S*,2*R*-*cis*)-*N1, N1*-dimethylcyclopentane-1,2-diamine **(23-5)** was obtained.

MS (ESI) m/z: 129.2 $[M+H]^+$.

**Step 5:** Compound **23-5** (100 mg, 0.26 mmol) was dissolved in *N,N*-dimethylacetamide (1 mL) in a sealed tube. The mixture was added with diisopropylethylamine (67 mg, 0.52 mmol), and added with compound **1A** (37 mg, 0.29 mmol). The mixture was heated to 80°C and reacted for 2 hours. After quenching with water (60 mL), the reaction mixture was extracted with dichloromethane (3 × 20 mL). The organic phases were combined, washed with saturated brine (30 mL) first, then dried over anhydrous sodium sulfate, filtered and finally concentrated under reduced pressure. The resulting residue was purified by preparative high performance liquid chromatography to obtain 8.0 mg of 4-((1*S*,2*R*-*cis*)-2-(dimethylamino)cyclopentyl)amino)-2-((1-((1-methyl-1*H*-pyrazol-4-yl)sulfonyl)piperidin-4-yl)amino)pyrimidine-5-carbonitrile (compound **23**).

MS (ESI) m/z: 474.2 $[M+H]^+$.

$^1$H NMR (400 MHz, DMSO-$d_6$) δ 8.34 (s, 0.6H), 8.31 (s, 0.4H), 8.16 (s, 0.4H), 8.11 (s, 0.6H), 7.80 - 7.75 (m, 1H), 7.64 (d, *J*= 7.3 Hz, 0.5H), 7.46 (d, *J* = 7.5 Hz, 0.4H), 7.34 (d, *J* = 7.9 Hz, 0.5H), 7.24 (d, *J* = 8.5 Hz, 0.4H), 4.49 - 4.29 (m, 1H), 3.91 (s, 3H), 3.78 - 3.65 (m, 1H), 3.61 - 3.39 (m, 2H), 2.91 - 2.76 (m, 1H), 2.45 - 2.30 (m, 2H), 2.15 - 2.05 (m, 6H), 1.97 - 1.82 (m, 3H), 1.75 - 1.41 (m, 7H).

**Example 24:**

**4-(Cyclopentylamino)-2-((1-((4-(2-hydroxypropan-2-yl)phenyl)sulfonyl)piperidin-4-yl)amino)pyrimidine-5-carbonitrile**

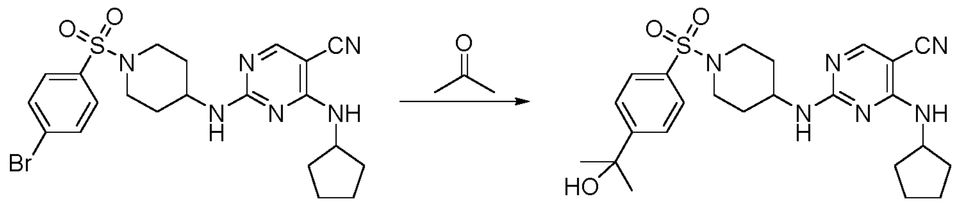

**15-4** **Compound 24**

n-BuLi (0.83 mL, 0.6 mmol, 2.5 mol/L in THF) was dissolved in THF (5 mL) at -78°C under nitrogen atmosphere. Subsequently, compound **15-4** (400.0 mg, 0.8 mmol) was slowly added to the above reaction mixture. The reaction mixture was stirred for 1 hour, and then acetone (69.0 mg, 1.2 mmol) was slowly added dropwise thereto. The reaction mixture was warmed to room temperature and stirred for 3 hours. The reaction mixture was cooled in an ice-water bath and quenched with saturated ammonium chloride aqueous solution. The mixture was extracted with ethyl acetate (40 mL × 3 times). The organic phases were combined, washed with saturated brine (100 mL) first, then dried over anhydrous sodium sulfate, filtered and finally concentrated under reduced pressure. The resulting residue was purified by preparative high performance liquid chromatography to obtain 30.2 mg of 4-(cyclopentylamino)-2-((1-((4-(2-hydroxypropan-2-yl)phenyl)sulfonyl)piperidin-4-yl)amino)pyrimidine-5-carbonitrile (compound **24**).

MS (ESI) m/z: 485.0 $[M+H]^+$.

$^1$H NMR (400 MHz, DMSO-$d_6$) δ 8.13 (s, 0.4H), 8.10 (s, 0.6H), 7.76 - 7.64 (m, 4H), 7.62 (d, *J* = 7.1 Hz, 0.6H), 7.44 (d, *J* = 7.9 Hz, 0.4H), 7.24 (d, *J* = 6.5 Hz, 0.6H), 7.14 (d, *J* = 7.4 Hz, 0.4H), 5.26 (s, 1H), 4.44 - 4.31 (m, 0.4H), 4.25 - 4.14 (m, 0.6H), 3.77 - 3.60 (m, 1H), 3.60 - 3.47 (m, 2H), 2.48 - 2.40 (m, 1H), 2.40 - 2.31 (m, 1H), 1.96 - 1.75 (m, 4H), 1.67 - 1.39 (m, 14H).

**Example 25 and Example 26:**

**(*S*)-*N*-(1-(((1*H*-Pyrazol-4-yl)sulfonyl)piperidin-4-yl)-4-((tetrahydrofuran-3-yl)oxy)-5-(trifluoromethyl)pyrimidin-2-amine**

**(*S*)-*N*-(1-((1-((1H-Pyrazol-4-yl)sulfonyl)-1*H*-pyrazol-4-yl)sulfonyl)piperidin-4-yl)-4-((tetrahydrofuran-3-yl)oxy)-5-(trifluoromethyl)pyrimidin-2-amine**

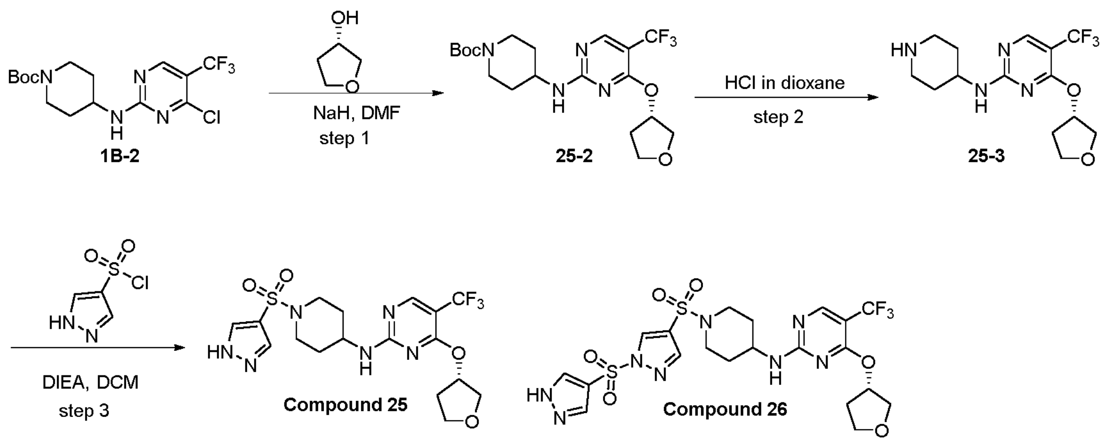

1B-2 25-2 25-3 Compound 25 Compound 26

**Step 1:**(*S*)-(-)-3-Hydroxytetrahydrofuran (696.0 mg, 7.9 mmol) was dissolved in THF (30 mL) at room temperature under nitrogen atmosphere. Subsequently, sodium hydride (316.0 mg, 7.9 mmol, 60% dispersed in mineral oil) was added to the above solution under an ice-water bath. After the reaction mixture was stirred for 10 minutes under an ice-water bath, compound **1B-2** (2.0 g, 5.2 mmol) was slowly added to the reaction mixture. The reaction system was heated to 100°C and stirred for 2 hours. The reaction mixture was cooled to room temperature. Water (50 mL) was added to the reaction mixture to quench the reaction. The mixture was extracted with ethyl acetate (15 mL × 3 times), and the organic phases were combined. The organic phases were washed with saturated brine (50 mL) first, then dried over anhydrous sodium sulfate, filtered and finally concentrated under reduced pressure. The resulting residue was purified by silica gel column chromatography to obtain 1.8 g of tert-butyl (S)-4-((4-((tetrahydrofuran-3yl)oxy)-5-(trifluoromethyl)pyrimidin-2-yl)amino)piperidine-1-carboxylate **(25-2).**

MS (ESI) m/z: 433.0 $[M+H]^+$.

$^1H$ NMR (400 MHz, DMSO-$d_6$) δ 8.36 (s, 0.4H), 8.31 (s, 0.6H), 8.01 (d, *J* = 7.2 Hz, 0.6H), 7.82 (d, *J* = 8.0 Hz, 0.4H), 5.62 - 5.55 (m, 1H), 3.99 - 3.84 (m, 4H), 3.83 - 3.73 (m, 3H), 3.00 - 2.75 (m, 2H), 2.32 - 2.18 (m, 1H), 2.06 - 1.95 (m, 1H), 1.90 - 1.77 (m, 2H), 1.40 (s, 9H), 1.39 - 1.30 (m, 2H).

**Step 2:** Compound **25-2** (1.8 g, 4.2 mmol) was dissolved in 1,4-dioxane (10 mL) at room temperature. Subsequently, a hydrogen chloride-dioxane solution (10 mL, 40 mmol) was added to the above solution. The reaction mixture was stirred at room temperature for 16 hours. The reaction mixture was concentrated under reduced pressure to obtain 1.38 g of (*S*)-*N*-(piperidin-4-yl)-4-((tetrahydrofuran-3-yl)oxy)-5-(trifluoromethyl)pyrimidin-2-amine **(25-3).**

MS (ESI) m/z: 333.0 $[M+H]^+$.

**Step 3:** Compound **25-3** (700.0 mg, 2.1 mmol) was dissolved in dichloromethane (40 mL) at room temperature. Subsequently, *N,N*-diisopropylethylamine (812.0 mg, 6.3 mmol) and pyrazole-4-sulfonyl chloride (349.0 mg, 2.1 mmol) were sequentially added to the above reaction mixture under an ice-water bath. The reaction mixture was stirred at room temperature for 3 hours. Water (30 mL) was added to the reaction mixture to quench the reaction. The mixture was extracted with dichloromethane (20 mL × 3 times), and the organic phases were combined. The organic phases were washed with saturated brine (10 mL × 3 times) first, then dried over anhydrous sodium sulfate, filtered and finally concentrated under reduced pressure. The resulting residue was purified by silica gel column chromatography to obtain 700.0 mg of (*S*)-*N*-(1-(((1*H*-pyrazol-4-yl)sulfonyl)piperidin-4-yl)-4-((tetrahydrofuran-3-yl)oxy)-5-(trifluoromethyl)pyrimidin-2-amine (compound **25**).

MS (ESI) m/z: 462.8 $[M+H]^+$.

$^1H$ NMR (400 MHz, DMSO-$d_6$) δ 13.74 (s, 1H), 8.41 - 8.33 (m, 1H), 8.32 - 8.27 (m, 1H), 8.03 (d, *J* = 7.3 Hz, 0.6H), 7.87 (d, *J* = 7.6 Hz, 0.4H), 7.83 (s, 1H), 5.63 - 5.52 (m, 1H), 3.96 - 3.84 (m, 1H), 3.83 - 3.65 (m, 4H), 3.56 - 3.42 (m, 2H), 2.47 - 2.34 (m, 2H), 2.27 - 2.14 (m, 1H), 2.02 - 1.87 (m, 3H), 1.67 - 1.52 (m, 2H).

**(*S*)-*N*-(1-((1-((1H-Pyrazol-4-yl)sulfonyl)-1*H*-pyrazol-4-yl)sulfonyl)piperidin-4-yl)-4-((tetrahydrofuran-3-yl)oxy)-5-(trifluoromethyl)pyrimidin-2-amine (compound 26)**.

MS (ESI) m/z: 592.8 $[M+H]^+$.

$^1H$ NMR (400 MHz, DMSO-$d_6$) δ 14.19 (s, 1H), 8.92 (d, J = 8.4 Hz, 1H), 8.78 - 8.35 (m, 2H), 8.31 (d, *J* = 5.6 Hz, 1H), 8.23 (s, 0.6H), 8.21 (s, 0.4H), 8.05 (d, *J* = 7.6 Hz, 0.6H), 7.88 (d, *J* = 8.0 Hz, 0.4H), 5.63 - 5.51 (m, 1H), 3.95 - 3.89 (m, 1H), 3.84 - 3.71 (m, 4H), 3.59 - 3.48 (m, 2H), 2.65 - 2.53 (m, 2H), 2.27 - 2.15 (m, 1H), 2.03 - 1.87 (m, 3H), 1.67 - 1.51 (m, 2H).

**Example 27:**

**(*S*)-2-(4-((4-((Tetrahydrofuran-3-yl)oxy)-5-(trifluoromethyl)pyrimidin-2-yl)amino)piperidin-1-yl)sulfonyl)-1*H*-pyrazol-1-yl)ethanol**

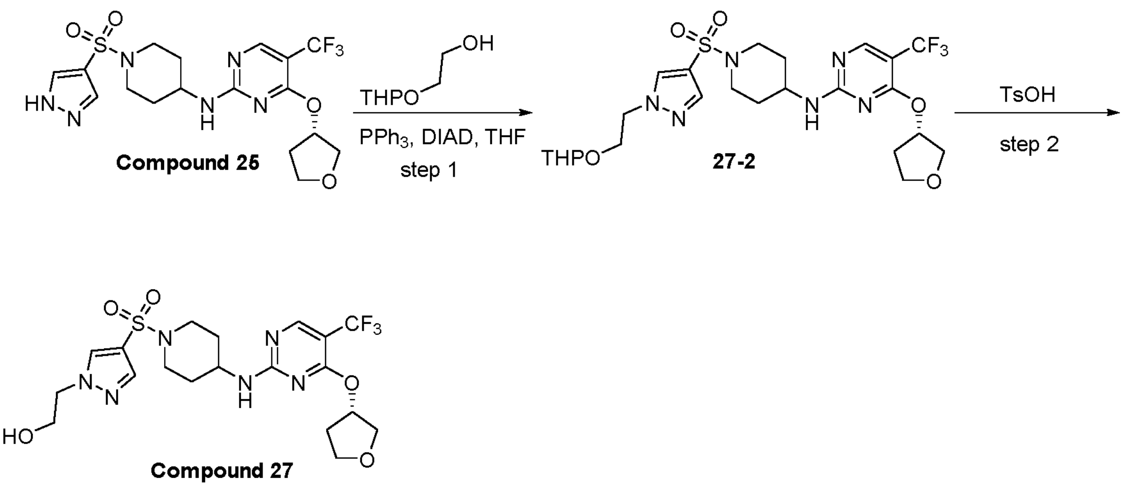


Compound 25 27-2 Compound 27

**Step 1:** Compound **25** (100.0 mg, 0.2 mmol), triphenylphosphine (275.0 mg, 1.0 mmol) and 2-(tetrahydro-2H-pyran-2-yloxy)ethanol (63.0 mg, 0.4 mmol) were dissolved in tetrahydrofuran (5 mL). Subsequently, diisopropyl azodicarboxylate (212.1 mg, 1.0 mmol) was slowly added to the reaction mixture under an ice-water bath. The reaction mixture was stirred at room temperature for 18 hours. Water (20 mL) was added to the reaction mixture to quench the reaction. The mixture was extracted with ethyl acetate (5 mL × 3 times), and the organic phases were combined. The organic phases were washed with saturated brine (20 mL) first, then dried over anhydrous sodium sulfate, filtered, and finally concentrated under reduced pressure. The resulting residue was purified by silica gel column chromatography to obtain 60.0 mg of compound **27-2**.

MS (ESI) m/z: 591.8 $[M+H]^+$.

**Step 2:** Compound **27-2** (60.0 mg, 0.1 mmol) was dissolved in methanol (5 mL) at room temperature. Subsequently, p-toluenesulfonic acid (18.0 mg, 0.1 mmol) was added to the above reaction mixture. The reaction mixture was stirred at room temperature for 16 hours. Saturated sodium bicarbonate aqueous solution (5 mL) was added to the reaction mixture to quench the reaction. Water (10 mL) was added to the mixture. The mixture was extracted with ethyl acetate (20 mL × 3 times), and the organic phases were combined. The organic phases were washed with saturated brine (10 mL × 3 times) first, then dried over anhydrous sodium sulfate, filtered and finally concentrated under reduced pressure. The resulting residue was purified by preparative high performance liquid chromatography to obtain 21.1 mg of (*S*)-2-(4-((4-((tetrahydrofuran-3-yl)oxy)-5-(trifluoromethyl)pyrimidin-2-yl)amino)piperidin-1-yl)sulfonyl)-1*H*-pyrazol-1-yl)ethanol **(compound 27).**

MS (ESI) m/z: 506.8 $[M+H]^+$.

$^1H$ NMR (400 MHz, DMSO-$d_6$) δ 8.34 - 8.26 (m, 2H), 8.03 (d, *J* = 7.4 Hz, 0.6H), 7.87 (d, *J* = 7.6 Hz, 0.4H), 7.81 (s, 0.5H), 7.80 (s, 0.4H), 5.61 - 5.51 (m, 1H), 4.95 (t, *J* = 5.3 Hz, 1H), 4.21 (t, *J* = 5.4 Hz, 2H), 3.95 - 3.85 (m, 1H), 3.83 - 3.66 (m, 6H), 3.56 - 3.44 (m, 2H), 2.46 - 2.35 (m, 2H), 2.26 - 2.13 (m, 1H), 2.04 - 1.87 (m, 3H), 1.68 - 1.52 (m, 2H).

**Example 99:**

**(*S*)-*N*-(1-(((4-Bromophenyl)sulfonyl)piperidin-4-yl)-4-((tetrahydrofuran-3-yl)oxy)-5-(trifluoromethyl)pyrimidin-2-amine**

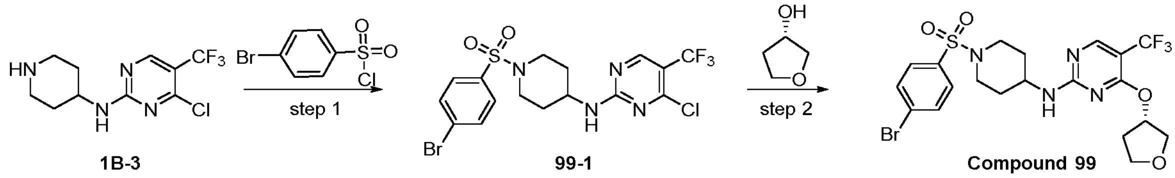


1B-3 99-1 Compound 99

**Step 1:** Compound **1B-3** (508 mg, 1.81 mmol) was dissolved in dichloromethane (30 mL) at room temperature. Subsequently, *N,N*-diisopropylethylamine (702 mg, 5.43 mmol) and 4-bromo-benzenesulfonyl chloride (508 mg, 2.00 mmol) were added thereto under an ice-water bath. After the reaction mixture was stirred at room temperature for 2 hours, the reaction was quenched by adding water (60 mL). The mixture was extracted with dichloromethane (20 mL × 3 times), and the organic phases were combined. The organic phases were washed with saturated brine (30 mL) first, then dried over anhydrous sodium sulfate, filtered and finally concentrated under reduced pressure. The resulting residue was purified by silica gel column chromatography to obtain 830 mg of *N*-(1-(1-(4-bromophenyl)sulfonyl)piperidin-4-yl)-4-chloro-5-(trifluoromethyl)pyrimidin-2-amine **(99-1).**

MS (ESI) m/z: 499.0 $[M+H]^+$.

**Step 2:** (*S*)-3-Hydroxytetrahydrofuran (42 mg, 0.48 mmol) was dissolved in THF (2 mL) in a sealed jar. Subsequently, sodium hydride (21 mg, 0.88 mmol) was added thereto under an ice-water bath. After the reaction was carried out for 15 minutes, compound **99-1** (200 mg, 0.40 mmol) was added thereto. The reaction mixture was heated to 100°C for 4 hours, and water (60 mL) was added to the reaction mixture to quench. The mixture was extracted with dichloromethane (20 mL × 3 times), and the organic phases were combined. The organic phases were washed with saturated brine (30 mL) first, then dried over anhydrous sodium sulfate, filtered and finally concentrated under reduced pressure. The resulting residue was purified by silica gel column chromatography to obtain 150 mg of (*S*)-*N*-(1-(((4-bromophenyl)sulfonyl)piperidin-4-yl)-4-((tetrahydrofuran-3-yl)oxy)-5-(trifluoromethyl)pyrimidin-2-amine **(compound 99)**.

MS (ESI) m/z: 551.0 $[M+H]^+$.

$^1H$ NMR (400 MHz, DMSO-$d_6$) δ 8.29 (s, 1H), 8.02 (d, *J* = 7.4 Hz, 0.6H), 7.92 - 7.83 (m, 2.4H), 7.73 - 7.65 (m, 2H), 5.62 - 5.51 (m, 1H), 3.96 - 3.86 (m, 1H), 3.85 - 3.68 (m, 4H), 3.63 - 3.46 (m, 2H), 2.65 - 2.55 (m, 2H), 2.28 - 2.13 (m, 1H), 2.03 - 1.84 (m, 3H), 1.64 - 1.48 (m, 2H).

**Example 100:**

**(*S*)-4-((Tetrahydrofuran-3-yl)oxy)-*N*-(1-((4-(1,2,3,6-tetrahydropyridin-4-yl)phenyl)sulfonyl)piperidin-4-yl)-5-(trifluoromethyl)pyrimidin-2-amine**

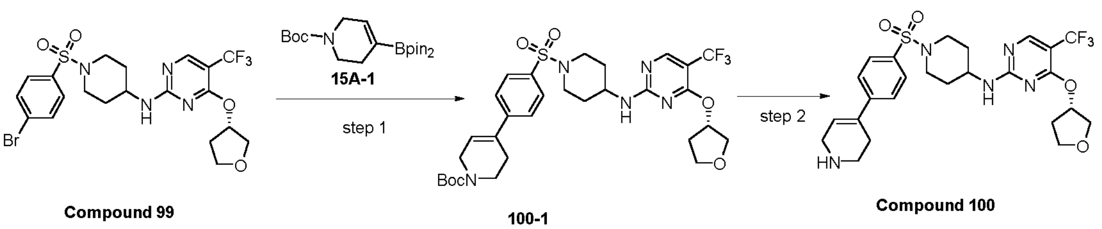


Compound 99 100-1 Compound 100

**Step 1:** Under nitrogen atmosphere, compound **99** (200 mg, 0.36 mmol), compound **15A-1** (79 mg, 0.44 mmol), [1,1'-bis(diphenylphosphino)ferrocene]dichloropalladium(II) (26 mg, 0.036 mmol) and sodium carbonate (76 mg, 0.72 mmol) were dissolved in 1,4-dioxane/water (20 mL/2 mL). The reaction mixture was heated to 110°C and stirred overnight. After the reaction mixture was cooled to room temperature, the reaction mixture was quenched by adding water (60 mL) and extracted with ethyl acetate (3 × 20 mL). The organic phases were combined, washed with saturated brine (60 mL) first, dried over anhydrous sodium sulfate, filtered and concentrated. The resulting residue was purified by silica gel

column chromatography to obtain 220 mg of tert-butyl (*S*)-4-(4-((4-((4-((tetrahydrofuran-3-yl)oxy)-5-(trifluoromethyl)pyrimidin-2-yl)amino)piperidin-1-yl)sulfonyl)phenyl)-3,6-dihydropyridine-1(2H)-carboxylate (**compound 100-1**).

MS (ESI) m/z: 653.8 $[M+H]^+$.

$^1H$ NMR (400 MHz, DMSO-$d_6$) δ 8.29 (s, 1H), 8.01 (d, *J* = 7.2 Hz, 0.6H), 7.86 (d, J = 7.4 Hz, 0.4H), 7.75 - 7.65 (m, 4H), 6.37 (s, 1H), 5.59 - 5.48 (m, 1H), 4.04 (s, 2H), 3.95 - 3.83 (m, 1H), 3.81 - 3.67 (m, 4H), 3.63 -3.49 (m, 4H), 2.59 - 2.51 (m, 2H), 2.49 - 2.39 (m, 2H), 2.25 - 2.11 (m, 1H), 2.02 - 1.85 (m, 3H), 1.64 - 1.49 (m, 2H), 1.43 (s, 9H).

**Step 2:** Compound **100-1** (120 mg, 0.18 mmol) was dissolved in 1,4-dioxane (5 mL). Dioxane hydrochloride solution (4 mL) was added thereto, and the reaction mixture was stirred overnight at room temperature. The reaction mixture was concentrated under reduced pressure. The resulting residue was purified by preparative high performance liquid chromatography to obtain 59.6 mg of (*S*)-4-((tetrahydrofuran-3-yl)oxy)-*N*-(1-((4-(1,2,3,6-tetrahydropyridin-4-yl)phenyl)sulfonyl)piperidin-4-yl)-5-(trifluoromethyl)pyrimidin-2-amine hydrochloride **(compound 100)**.

MS (ESI) m/z: 554.2 $[M+H]^+$.

$^1H$ NMR (400 MHz, DMSO-$d_6$) δ 9.65 - 9.30 (m, 2H), 8.30 (d, *J* = 7.0 Hz, 1H), 8.16 - 8.06 (m, 0.5H), 8.10 - 7.86 (s, 0.5H), 7.80 - 7.76 (m, 3H), 6.41 (br.s., 1H), 5.55 (br.s., 1H), 3.97 - 3.84 (m, 1H), 3.84 - 3.66 (m, 5H), 3.66 - 3.46 (m, 2H), 3.31 (br.s., 2H), 2.70 (s, 2H), 2.64 - 2.55 (m, 1H), 2.47 - 2.38 (m, 1H), 2.27 - 2.11 (m, 1H), 2.02 - 1.83 (m, 3H), 1.67 - 1.49 (m, 2H).

**Example 112:**

**(*S*)-4-((Tetrahydrofuran-3-yl)oxy)-*N*-(1-((4-(1,2,5,6-tetrahydropyridin-3-yl)phenyl)sulfonyl)piperidin-4-yl)-5-(trifluoromethyl)pyrimidin-2-amine**

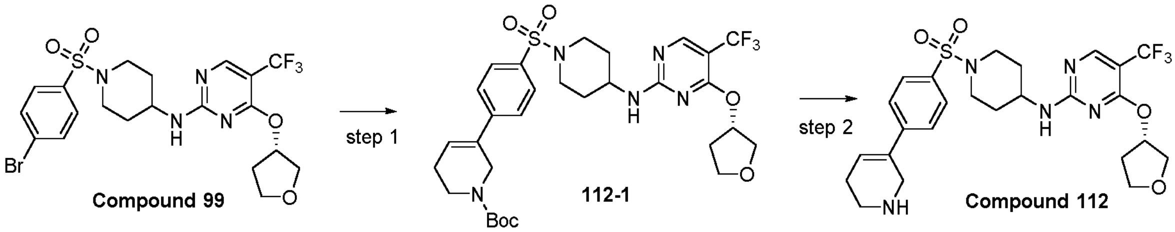

Compound 99 112-1 Compound 112

Referring to the preparation method of **Example 100,** (*S*)-4-((tetrahydrofuran-3-yl)oxy)-*N*-(1-((4-(1,2,5,6-tetrahydropyridin-3-yl)phenyl)sulfonyl)piperidin-4-yl)-5-(trifluoromethyl)pyrimidin-2-amine (**compound 112**) was obtained.

MS (ESI) m/z: 554.1 $[M+H]^+$.

$^1H$ NMR (400 MHz, DMSO-$d_6$) δ 8.28 (s, 1H), 8.01 (d, *J* = 7.3 Hz, 0.6H), 7.86 (d, *J* = 7.6 Hz, 0.4H), 7.73 - 7.57 (m, 4H), 6.45 (s, 1H), 5.62 - 5.50 (m, 1H), 3.95 - 3.84 (m, 1H), 3.82 - 3.65 (m, 4H), 3.62 - 3.47 (m, 4H), 2.81 (t, *J* = 5.6 Hz, 2H), 2.57 - 2.51 (m, 2H), 2.46 - 2.39 (m, 1H), 2.26 - 2.12 (m, 3H), 2.02 - 1.83 (m, 3H), 1.63 - 1.47 (m, 2H).

**Example 113:**

***N*-(1-((4-(Piperidin-3-yl)phenyl)sulfonyl)piperidin-4-yl)-4-(((5)-tetrahydrofuran-3-yl)oxy)-5-(trifluoromethyl)pyrimidin-2-amine**

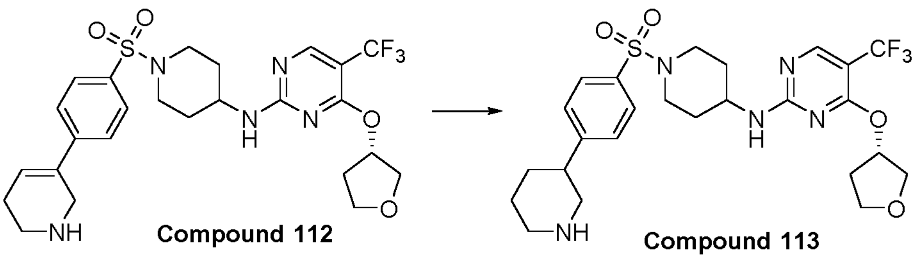
Compound 112 Compound 113

Referring to the preparation method of **Example 17**, *N*-(1-((4-(piperidin-3-yl)phenyl)sulfonyl)piperidin-4-yl)-4-(((*S*)-tetrahydrofuran-3-yl)oxy)-5-(trifluoromethyl)pyrimidin-2-amine **(compound 113)** was obtained.

MS (ESI) m/z: 556.2 $[M+H]^+$.

$^1$H NMR (400 MHz, DMSO-$d_6$) δ 8.29 (s, 1H), 8.02 (d, *J* = 7.3 Hz, 0.6H), 7.86 (d, *J* = 7.1 Hz, 0.4H), 7.69 - 7.61 (m, 2H), 7.50 (d, *J* = 8.3 Hz, 2H), 5.60 - 5.51 (m, 1H), 3.94 - 3.83 (m, 1H), 3.82 - 3.66 (m, 4H), 3.62 - 3.49 (m, 2H), 3.02 - 2.89 (m, 2H), 2.76 - 2.68 (m, 1H), 2.54 - 2.51 (m, 2H), 2.45 - 2.36 (m, 2H), 2.26 - 2.11 (m, 1H), 2.01 - 1.83 (m, 4H), 1.71 - 1.39 (m, 5H).

**Example 114 and Example 115**

***N*-(1-((4-((S)-Piperidin-3-yl)phenyl)sulfonyl)piperidin-4-yl)-4-*(*((*S*-tetrahydrofuran-3-yl)oxy)-5-(trifluoromethyl)pyrimidin-2-amine (compound 114)**

***N*-(1-((4-((*R*)-Piperidin-3-yl)phenyl)sulfonyl)piperidin-4-yl)-4-(((*S*-tetrahydrofuran-3-yl)oxy)-5-(trifluoromethyl)pyrimidin-2-amine (compound 115)**

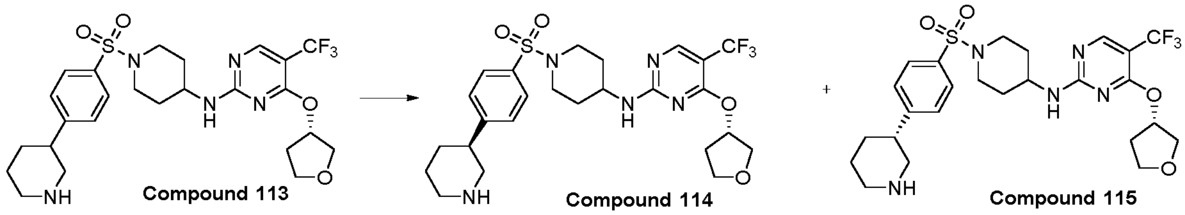

Compound 113 Compound 114 Compound 115

Compound **113** was subjected to chiral resolution, and the resolution conditions were as follows: preparative column: Daicel OZ (25 * 250 mm, 10 μm), mobile phase: $CO_2$/MeOH (0.2% ammonia methanol) = 50/50; flow rate: 100 g/min; detection wavelength: 214 nm. The product was collected and lyophilized under reduced pressure. **Compound 114** with a retention time of 2.374 minutes was obtained, and the ee value was 100%. The absolute configuration was not determined. It was an enantiomer of compound **115**.

MS (ESI) m/z: 556.2 $[M+H]^+$.

$^1$H NMR (400 MHz, DMSO) δ 8.29 (s, 1H), 8.02 (d, *J* = 7.2 Hz, 0.6H), 7.86 (d, *J* = 7.7 Hz, 0.4H), 7.74 - 7.61 (m, 2H), 7.50 (d, *J* = 8.3 Hz, 2H), 5.64 - 5.48 (m, 1H), 3.97 - 3.83 (m, 1H), 3.83 - 3.67 (m, 4H), 3.67 - 3.47 (m, 2H), 3.08 - 2.88 (m, 2H), 2.81- 2.66 (m,1H), 2.66 - 2.54 (m, 2H), 2.47 - 2.24 (m, 2H), 2.25 - 2.11 (m, 1H), 2.10 - 1.82 (m, 4H), 1.70 - 1.42 (m, 5H).

**Compound 115:**

Compound **113** was subjected to chiral resolution, and the resolution conditions were as follows: preparative column: Daicel OZ (25 * 250 mm, 10 μm), mobile phase: $CO_2$/MeOH (0.2% ammonia methanol) = 50/50; flow rate: 100 g/min; detection wavelength: 214 nm. The product was collected and lyophilized under reduced pressure. **Compound 115** with a retention time of 3.247 minutes was obtained, and the ee value was 99.34%. The absolute configuration was not determined. It was an enantiomer of compound **114.**

MS (ESI) m/z: 556.2 $[M+H]^+$.

$^1$H NMR (400 MHz, DMSO-$d_6$) δ 8.29 (s, 1H), 8.02 (d, *J* = 7.3 Hz, 0.6H), 7.86 (d, *J* = 7.5 Hz,0.4H), 7.66 (t, *J* = 6.7 Hz, 2H), 7.52 (d, *J* = 12.0 Hz, 2H), 5.55 (d, *J* = 4.3 Hz, 1H), 3.96 - 3.83 (m, 1H), 3.83 - 3.65 (m, 4H), 3.65 - 3.48 (m, 2H), 3.07 - 2.89 (m, 2H), 2.82 - 2.66 (m, 1H), 2.64 - 2.53 (m, 2H), 2.49 - 2.35 (m, 2H), 2.34 - 2.11 (m, 1H), 2.04 - 1.82 (m, 4H), 1.73 - 1.40 (m, 5H).

**Example 116:**

**(*S*)-*N*-(1-(((4-(Piperazin-1-yl)phenyl)sulfonyl)piperidin-4-yl)-4-((tetrahydrofuran-3-yl)oxy)-5-(trifluoromethyl)pyrimidin-2-amine**

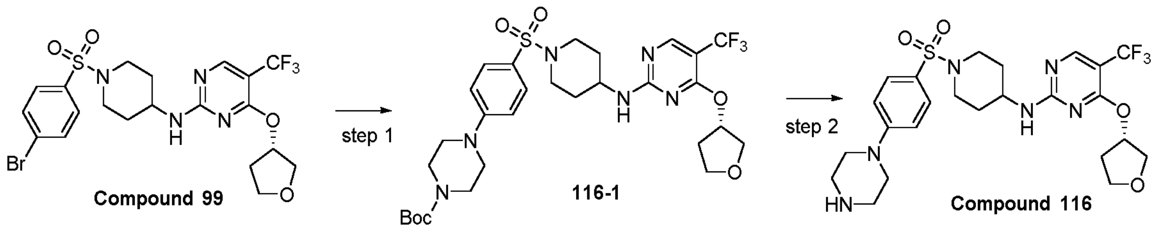


Compound 99 116-1 Compound 116

**Step 1:** Under nitrogen atmosphere, compound **99** (2.0 g, 3.7 mmol), compound tert-butyl piperazine-1-carboxylate (1.02 g, 5.5 mmol), tris(dibenzylideneacetone)dipalladium (338.8 mg, 0.37 mmol) and cesium carbonate (2.4 g, 7.4 mmol) were dissolved in 1,4-dioxane (100 mL). The reaction mixture was heated to 100°C and stirred overnight. The reaction mixture was cooled to room temperature, concentrated under reduced pressure and extracted with ethyl acetate (3 × 20 mL). The organic phases were combined, washed with saturated brine (60 mL) first, dried over anhydrous sodium sulfate, filtered and concentrated. The resulting residue was purified by silica gel column chromatography to obtain 1.35 g of compound tert-butyl (S)-4-(4-((4-(4-((tetrahydrofuran-3-yl)oxy)-5-(trifluoromethyl)pyrimidin-2-yl)amino)piperidin-1-yl)sulfonyl)phenyl)piperazine-1-carboxylate (compound **116-1**).

MS (ESI) m/z: 657.2 $[M+H]^+$.

**Step 2:** Compound **116-1** (1.35 g, 2.05 mmol) was dissolved in 1,4-dioxane (10 mL). Subsequently, a 1,4-dioxane hydrochloride solution (15 mL) was added thereto. After the reaction mixture was stirred overnight at room temperature, the reaction mixture was concentrated under reduced pressure. The reaction mixture was extracted with dichloromethane (3 × 100 mL), washed with saturated sodium bicarbonate aqueous solution (100 mL), and the organic phases were combined. The organic phase was washed with saturated brine (100 mL), dried over anhydrous sodium sulfate, filtered and concentrated. The residue was slurried with ethanol. 818 mg of ((*S*)-*N*-(1-(((4-(piperazin-1-yl)phenyl)sulfonyl)piperidin-4-yl)-4-((tetrahydrofuran-3-yl)oxy)-5-(trifluoromethyl)pyrimidin-2-amine **(compound 116)** was obtained.

MS (ESI) m/z: 557.2 $[M+H]^+$.

$^1$H NMR (400 MHz, DMSO-$d_6$) δ 8.29 (s, 1H), 8.01 (d, *J* = 7.3 Hz, 0.6H), 7.86 (d, J = 7.2 Hz, 0.4H), 7.51 (d, *J* = 8.8 Hz, 2H), 7.05 (d, *J* = 9.0 Hz, 2H), 5.62 - 5.49 (m, 1H), 3.95 - 3.83 (m, 1H), 3.83 - 3.65 (m, 4H), 3.57 - 3.43 (m, 2H), 3.27 - 3.17 (m, 4H), 2.89 - 2.76 (m, 4H), 2.46 - 2.33 (m, 3H), 2.26 - 2.12 (m, 1H), 2.02 - 1.83 (m, 3H), 1.64 - 1.46 (m, 2H).

**Example 117:**

***N*-(1-((6-((3*S*,5*R*)-3,5-Dimethylpiperazin-1-yl)pyridin-3-yl)sulfonyl)piperidin-4-yl)-4-((((*S*)-tetrahydrofuran-3-yl)oxy)-5-(trifluoromethyl)pyrimidin-2-amine**

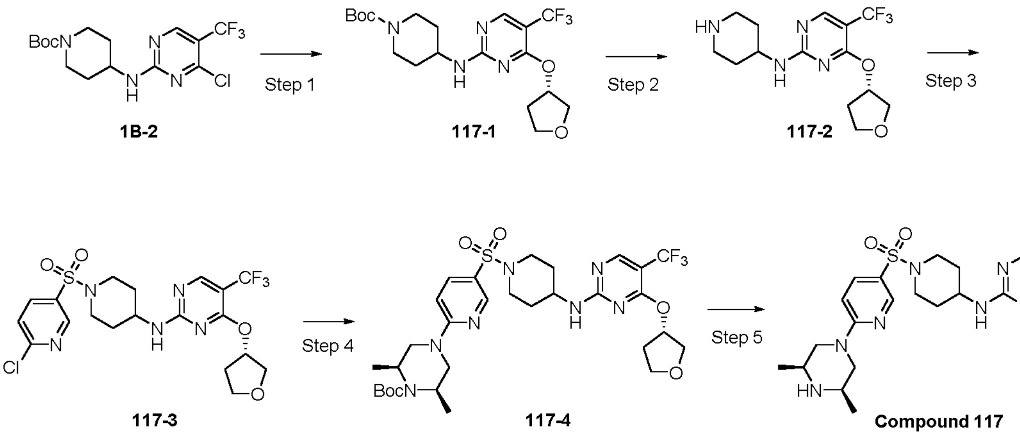


1B-2 117-1 117-2 117-3 117-4 Compound 117

**Step 1:** (*S*)-(-)-3-Hydroxytetrahydrofuran (365.0 mg, 0.83 mmol) was dissolved in THF (20 mL) at room temperature under nitrogen atmosphere. Subsequently, sodium hydride (335.0 mg, 1.1 mmol, 60% dispersed in mineral oil) was added to the above solution under an ice-water bath. After the reaction mixture was stirred for 10 minutes under an ice-water bath, compound **1B-2** (1.0 g, 0.55 mmol) was slowly added to the reaction mixture. The reaction system was heated to 100°C and stirred for 2 hours. After the reaction mixture was cooled to room temperature, water (20 mL)

was added to quench. The mixture was extracted with ethyl acetate (15 mL × 3 times), and the organic phases were combined. The organic phase was washed with saturated brine (30 mL) first, dried over anhydrous sodium sulfate, filtered and finally concentrated under reduced pressure. The resulting residue was purified by silica gel column chromatography to obtain 0.85 g of tert-butyl (*S*)-4-((4-((tetrahydrofuran-3-yl)oxy)-5-(trifluoromethyl)pyrimidin-2-yl)amino)piperidine-1-carboxylate **(compound 117-1**).

MS (ESI) m/z: 433.0 $[M+H]^+$.

$^1H$ NMR (400 MHz, DMSO-$d_6$) δ 8.36 (s, 0.4H), 8.31 (s, 0.5 H), 8.01 (d, *J* = 7.5 Hz, 0.5 H), 7.82 (d, *J* = 7.9 Hz, 0.4H), 5.61 - 5.55 (m, 1H), 3.99 - 3.85 (m, 3H), 3.82 - 3.68 (m, 3H), 2.89 (br.s., 2H), 2.31 - 2.16 (m, 1H), 2.06 - 1.95 (m, 2H), 1.91 - 1.76 (m, 2H), 1.43 - 1.30 (m, 11H).

**Step 2:** Compound **117-1** (0.85 g, 1.9 mmol) was dissolved in 1,4-dioxane (10 mL) at room temperature. Subsequently, a hydrogen chloride-dioxane solution (10 mL) was added to the above solution. The reaction mixture was stirred at room temperature for 16 hours. The reaction mixture was concentrated under reduced pressure to obtain 0.7 g of compound (*S*)-*N*-(piperidin-4-yl)-4-((tetrahydrofuran-3-yl)oxy)-5-(trifluoromethyl)pyrimidin-2-amine (compound **117-2**).

MS (ESI) m/z: 333.4 $[M+H]^+$.

**Step 3:** Compound **117-2** (150.0 mg, 2.0 mmol) was dissolved in dichloromethane (8 mL) at room temperature. Subsequently, *N*,*N*-diisopropylethylamine (774.0 mg, 6.0 mmol) and 6-chloropyridine-3-sulfonyl chloride (508 mg, 2.4 mmol) were sequentially added to the above reaction mixture under an ice-water bath. The reaction mixture was stirred at room temperature for 16 hours. Water (20 mL) was added to the reaction mixture to quench the reaction. The mixture was extracted with dichloromethane (10 mL × 3 times), and the organic phases were combined. The organic phases were washed with saturated brine (20 mL) first, then dried over anhydrous sodium sulfate, filtered and finally concentrated under reduced pressure. The resulting residue was purified by silica gel column chromatography to obtain 105 mg of (*S*)-*N*-(1-(((6-chloropyridin-3-yl)sulfonyl)piperidin-4-yl)-4-((tetrahydrofuran-3-yl)oxy)-5-(trifluoromethyl)pyrimidin-2-amine (**compound 117-3**).

MS (ESI) m/z: 508.0 $[M+H]^+$.

$^1H$ NMR (400 MHz, DMSO-$d_6$) δ 8.81 - 8.75 (m, 1H), 8.30 (s, 1H), 8.24 - 8.18 (m, 1H), 8.02 (d, *J* = 7.4 Hz, 0.5H), 7.87 - 7.79 (m, 1.5H), 5.62 - 5.50 (m, 1H), 3.95 - 3.68 (m, 5H), 3.66 - 3.49 (m, 2H), 2.78 - 2.59 (m, **2H), 2.26** - **2.12 (m, 1H), 1.98** - **1.84 (m, 2H), 1.64 - 1.45 (m, 2H).**

**Step 4:** (*S*)-*N*-(1-((6-chloropyridin-3-yl)sulfonyl)piperidin-4-yl)-4-((tetrahydrofuran-3-yl)oxy)-5-(trifluoromethyl)pyrimidin-2-amine (100 mg, 0.20 mmol) was dissolved in n-butanol (1 mL) at room temperature. Subsequently, *N*,*N*-diisopropylethylamine (0.10 mL, 0.59 mmol) and tert-butyl (2*S*,6*R*)-2,6-dimethylpiperazine-1-carboxylate (63 mg, 0.30 mmol) were sequentially added to the above reaction mixture. The reaction mixture was stirred at 120°C for 16 hours. The reaction mixture was cooled to room temperature and then concentrated under reduced pressure. The resulting residue was purified by silica gel column chromatography to obtain 120 mg of compound tert-butyl (2*S*,6*R*)-2,6-dimethyl-4-(5-((4-(((*S*)-tetrahydrofuran-3-yl)oxy)-5-(trifluoromethyl)pyrimidin-2-yl)amino)piperidin-1-ylsulfonyl)pyridin-2-ylpiperazine-1-carboxylate (**compound 117-4**).

MS (ESI) m/z: 686.2 $[M+H]^+$.

**Step 5:** Compound **117-4** (120.0 mg, 0.18 mmol) was dissolved in 1,4-dioxane (0.5 mL) at room temperature. Subsequently, a hydrogen chloride-dioxane solution (1 mL) was added thereto. The reaction mixture was stirred at room temperature for 2 hours. The reaction mixture was concentrated under reduced pressure, and saturated sodium bicarbonate aqueous solution (3 mL) was added to the resulting residue to quench. The mixture was extracted with ethyl acetate (10 mL × 3 times). The organic phases were combined, then dried over anhydrous sodium sulfate, filtered and finally concentrated under reduced pressure. The resulting residue was purified by preparative high performance liquid chromatography to obtain 69.3 mg of *N*-(1-((6-((3*S*,5*R*)-3,5-dimethylpiperazin-1-yl)pyridin-3-yl)sulfonyl)piperidin-4-yl)-4-((*S*)-tetrahydrofuran-3-yl)oxy)-5-(trifluoromethyl)pyrimidin-2-amine (compound **117**).

MS (ESI) m/z: 586.2 $[M+H]^+$.

$^1H$ NMR (400 MHz, DMSO-$d_6$) δ 8.35 (d, *J* = 2.4 Hz, 1H), 8.29 (s, 1H), 8.02 (d, *J* = 7.4 Hz, 0.6H), 7.87 (d, *J* = 7.5 Hz, 0.4H), 7.71 (dd, *J* = 9.2, 2.5 Hz, 1H), 6.95 (d, *J* = 9.1 Hz, 1H), 5.60 - 5.52 (m, 1H), 4.31 (d, *J* = 12.1 Hz, 2H), 3.95 - 3.85 (m, 1H), 3.84 - 3.68 (m, 4H), 3.58 - 3.44 (m, 2H), 2.75 - 2.67 (m, 2H), 2.59 - 2.54 (m, 2H), 2.46 - 2.34 (m, 3H), 2.26 - 2.12 (m, 1H), 2.04 - 1.81 (m, 3H), 1.64 - 1.48 (m, 2H), 1.03 (d, *J* = 6.2 Hz, 6H).

**Example 168:**

***N*-(1-((4-((3*S*,5*S*)-3,5-Dimethylpiperazin-1-yl)phenyl)sulfonyl)piperidin-4-yl)-4-((*S*)-tetrahydrofuran-3-yl)oxy)-5-(trifluoromethyl)pyrimidin-2-amine**

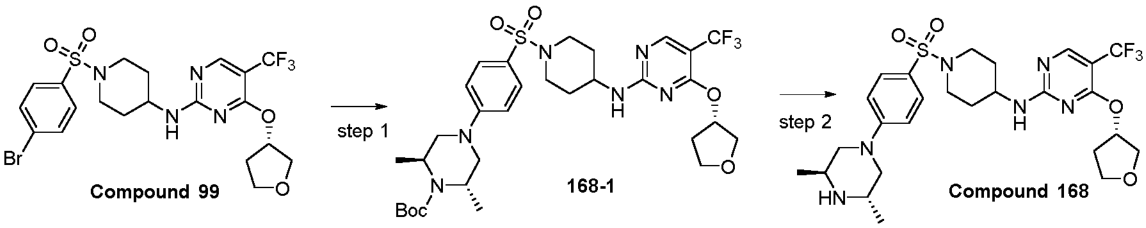


Compound 99 168-1 Compound 168

**Step 1:** Under nitrogen atmosphere, compound **99** (25 g, 45.3 mmol), tert-butyl (2*S*,6*S*)-2,6-dimethylpiperazine-1-carboxylate (11.6 g, 45.3 mmol), tris(dibenzylideneacetone)dipalladium (4.1 g, 4.53 mmol), 2-dicyclohexylphosphino-2',4',6'-triisopropylbiphenyl (1.3 g, 9.02 mmol) and cesium carbonate (29.4 g, 90.6 mmol) were dissolved in 1,4-dioxane (1.25 L). The reaction mixture was heated to 100°C and stirred overnight. The reaction mixture was concentrated under reduced pressure and extracted with ethyl acetate (3 × 100 mL). The organic phases were combined, washed with saturated brine (100 mL) first, dried over anhydrous sodium sulfate, filtered and concentrated. The resulting residue was purified by silica gel column chromatography to obtain 18.1 g of tert-butyl (2*S*,6*S*)-2,6-dimethyl-4-(4-((*S*)-tetrahydrofuran-3-yl)oxy)-5-(trifluoromethyl)pyrimidin-2-yl)amino)piperidin-1-yl)sulfonyl)phenyl)piperazin-1-carboxylate (**compound 168-1**).

MS (ESI) m/z: 685.2 [M+H]$^+$.

**Step 2:** Compound **168-1** (18.1 g, 26.4 mmol) was dissolved in 1,4-dioxane (100 mL). Subsequently, a 1,4-dioxane hydrochloride solution (100 mL) was added thereto. The reaction mixture was stirred overnight at room temperature. The reaction mixture was concentrated under reduced pressure. Sodium bicarbonate aqueous solution (300 mL) was added to the resulting residue for washing. The mixture was extracted with ethyl acetate (200 mL × 3 times). The organic phases were combined. The organic phase was washed with saturated brine (100 mL), dried over anhydrous sodium sulfate, filtered and concentrated under reduced pressure. The resulting residue was purified by silica gel column chromatography to obtain 9.2 g of *N*-(1-((4-((3*S*,5*S*)-3,5-dimethylpiperazin-1-yl)phenyl)sulfonyl)piperidin-4-yl)-4-((*S*)-tetrahydrofuran-3-yl)oxy)-5-(trifluoromethyl)pyrimidin-2-amine (**compound 168**).

MS (ESI) m/z: 585.2 [M+H]$^+$.

$^1$H NMR (400 MHz, DMSO-$d_6$) δ 8.29 (s, 1H), 8.00 (d, *J* = 7.1 Hz, 0.6H), 7.85 (d, *J* = 6.9 Hz, 0.4H), 7.49 (d, *J* = 8.8 Hz, 2H), 7.02 (d, *J* = 9.0 Hz, 2H), 5.60 - 5.51 (m, 1H), 3.95 - 3.83 (m, 1H), 3.81 - 3.65 (m, 4H), 3.57 - 3.44 (m, 2H), 3.37 - 3.33 (m, 2H), 3.21 - 3.09 (m, 2H), 3.02 - 2.91 (m, 2H), 2.46 - 2.37 (m, 2H), 2.28 - 2.03 (m, 2H), 2.02 - 1.84 (m, 3H), 1.66 - 1.47 (m, 2H), 1.06 (d, *J* = 6.4 Hz, 6H).

**Example 169:**

***N*-(1-((4-*cis*-3,5-Dimethylpiperazin-1-yl)phenyl)sulfonyl)piperidin-4-yl)-4-((*S*)-tetrahydrofuran-3-yl)oxy)-5-(trifluoromethyl)pyrimidin-2-amine**

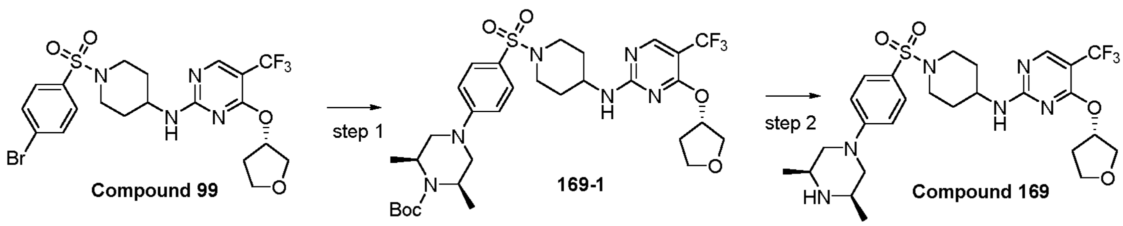


Compound 99 169-1 Compound 169

**Step 1:** Under nitrogen atmosphere, compound **99** (50 mg, 0.09 mmol), tert-butyl cis-2,6-dimethylpiperazine-1-carboxylate (23.1 mg, 0.108 mmol), tris(dibenzylideneacetone)dipalladium (8.2 mg, 0.009 mmol), 2-dicyclohexylphosphino-2',4',6'-triisopropylbiphenyl (8.6 mg, 0.018 mmol) and cesium carbonate ( 69.3 mg, 7.4 mmol) were dissolved in 1,4-dioxane (5 mL). The reaction mixture was heated to 100°C and stirred overnight. The reaction mixture was concentrated under reduced pressure and extracted with ethyl acetate (3 × 10 mL). The organic phases were combined, washed with saturated brine (30 mL) first, dried over anhydrous sodium sulfate, filtered and concentrated. The resulting residue was purified by silica gel column chromatography to obtain 40 mg of tert-butyl *cis*-2,6-dimethyl-4-(4-((4-((*S*)-tetrahydrofuran-3-yl)oxy)-5-(trifluoromethyl)pyrimidin-2-yl)amino)piperidin-1-yl)sulfonyl)phenyl)piperazine-1-carboxylate (**Compound 169-1**).

MS (ESI) m/z: 685.2 $[M+H]^+$.

**Step 2:** Compound **169-1** (40 mg, 0.06 mmol) was dissolved in 1,4-dioxane (1 mL). Subsequently, a 1,4-dioxane hydrochloride solution (1 mL) was added thereto. The reaction mixture was stirred overnight at room temperature. The reaction mixture was concentrated under reduced pressure. The reaction mixture was extracted with ethyl acetate (8 × 3 mL), washed with saturated sodium bicarbonate aqueous solution (30 mL). The organic phases were combined. The organic phase was washed with saturated brine (30 mL), dried over anhydrous sodium sulfate, filtered and concentrated under reduced pressure. The resulting residue was purified by preparative high performance liquid chromatography to obtain 6 mg of *N*-(1-((4-*cis*-3,5-dimethylpiperazin-1-yl)phenyl)sulfonyl)piperidin-4-yl)-4-((*S*)-tetrahydrofuran-3-yl)oxy)-5-(trifluoromethyl)pyrimidin-2-amine (**compound 169**).

MS (ESI) m/z: 585.2 $[M+H]^+$.

$^1H$ NMR (400 MHz, DMSO-$d_6$) δ 8.29 (s, 1H), 8.01 (d, *J* = 7.3 Hz, 0.6H), 7.86 (d, *J* = 7.0 Hz, 0.4H), 7.50 (d, *J* = 8.8 Hz, 2H), 7.06 (d, *J* = 9.1 Hz, 2H), 5.63 - 5.44 (m, 1H), 3.96 - 3.82 (m, 1H), 3.82 - 3.62 (m, 6H), 3.56 - 3.43 (m, 2H), 2.86 - 2.71 (m, 2H), 2.42 - 2.37 (m, 2H), 2.29 - 2.14 (m, 4H), 1.99 - 1.84 (m, 3H), 1.63 - 1.44 (m, 2H), 1.03 (d, *J* = 6.2 Hz, 6H).

The compounds given in Table 1 were prepared by essentially the same methods as described in the examples.

Table 1. Compound list

| Compound | Structure | $^1$HNMR | MS (ESI) |
|---|---|---|---|
| 28 | 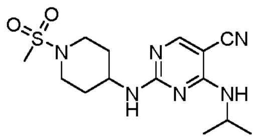 | $^1$HNMR (400 MHz, $CDCl_3$+TFA) δ 8.30 (s, 1H), 6.33 (d, *J* = 7.7 Hz, 1H), 4.48 - 4.32 (m, 1H), 4.18 - 4.07 (m, 1H), 3.69 - 3.53 (m, 2H), 3.22 - 3.11 (m, 2H), 2.92 (s, 3H), 2.15 - 2.03 (m, 2H), 1.93 - 1.80 (m, 2H), 1.37 (d, *J* = 6.6 Hz, 6H). | 338.9 |
| 29 | 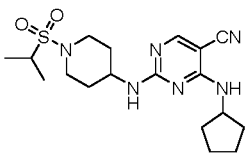 | $^1$HNMR (400 MHz, DMSO-$d_6$) δ 8.20 (s, 0.4H), 8.13 (s, 0.6H), 7.67 (d, *J* = 7.6 Hz, 0.6H), 7.49 (d, *J* = 7.6 Hz, 0.4H), 7.29 (d, *J* = 6.4 Hz, 0.6H), 7.17 (d, *J* = 8.0 Hz, 0.4H), 4.46 - 4.37 (m, 0.4H), 4.35 - 4.25 (m, 0.6H), 4.00 - 3.90 (m, 0.5H), 3.90 - 3.75 (m, 0.7H), 3.63 (d, *J* = 12.8 Hz, 2H), 3.32 - 3.26 (m, 1H), 3.05 - 2.91 (m, 2H), 1.95 - 1.78 (m, 4H), 1.71 - 1.41 (m, 8H), 1.21 (d, *J* = 6.8 Hz, 6H). | 393.2 |
| 30 | 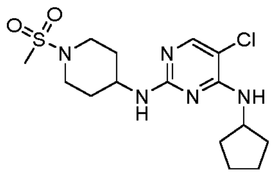 | $^1$HNMR (400 MHz, $CDCl_3$) δ 7.72 (s, 1H), 5.41 - 5.20 (m, 2H), 4.37 - 4.24 (m, 1H), 4.02 - 3.88 (m, 1H), 3.71 - 3.56 (m, 2H), 3.12 - 2.97 (m, 2H), 2.82 (s, 3H), 2.18 - 1.98 (m, 4H), 1.83 - 1.57 (m, 6H), 1.57 - 1.44 (m, 2H). | 374.1 |
| 31 | 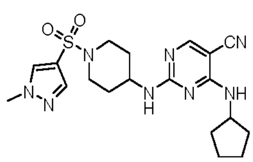 | $^1$HNMR (400 MHz, DMSO-$d_6$) δ 8.33 (s, 0.6H), 8.31 (s, 0.4H), 8.16 (s, 0.4H), 8.11 (s, 0.6H), 7.78 (s, 0.6H), 7.76 (s, 0.4H), 7.63 (d, *J* = 7.2 Hz, 0.6H), 7.45 (d, *J* = 7.7 Hz, 0.4H), 7.26 (d, *J* = 7.2 Hz, 0.6H), 7.16 (d, *J* = 7.2 Hz, 0.4H), 4.38 (q, *J* = 7.4 Hz, 0.4H), 4.25 (q, *J* = 7.0 Hz, 0.6H), 3.91 (s, 3H), 3.81 - 3.62 (m, 1H), 3.55 - 3.36 (m, 2H), 2.47 - 2.40 (m, 1H), 2.35 (t, *J* = 11.0 Hz, 1H), 2.00 - 1.77 (m, 4H), 1.73 - 1.40 (m, 8H). | 431.4 |
| 32 | 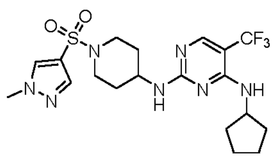 | $^1$H NMR (400 MHz, DMSO-$d_6$) δ 8.33 (s, 1H), 7.98 (s, 1H), 7.78 (s, 1H), 7.36 (d, *J* = 6.4 Hz, 0.6H), 7.18 (d, *J* = 6.8 Hz, 0.4H), 6.16 (br.s., 1H), 4.56 - 4.25 (m, 1H), 3.91 (s, 3H), 3.80 - 3.62 (m, 1H), 3.58 - 3.38 (m, 2H), 2.48 - 2.28 (m, 2H), 2.05 - 1.78 (m, 4H), 1.75 - 1.39 (m, 8H). | 474.2 |

(continued)

| Compound | Structure | $^1$HNMR | MS (ESI) |
|---|---|---|---|
| 33 | 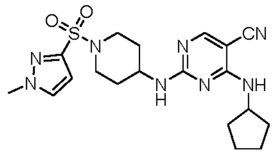 | $^1$H NMR (400 MHz, $CDCl_3$) δ 8.11 - 8.00 (m, 1H), 7.45 (br.s., 1H), 6.66 (d, *J* = 1.6 Hz, 1H), 541 - 5.00 (m, 2H), 4.37 - 4.24 (m, 1H), 4.01 (s, 3H), 3.95 - 3.75 (m, 2H), 3.65 - 3.53 (m, 1H), 3.03 - 2.88 (m, 1H), 2.79 - 2.60 (m, 1H), 2.15 - 1.97 (m, 4H), 1.83 - 1.55 (m, 6H), 1.55 - 1.35 (s, 2H). | 431.2 |
| 34 | 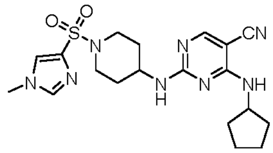 | $^1$H NMR (400 MHz, DMSO-$d_6$) δ 8.16 (s, 0.4H), 8.11 (s, 0.6H), 7.86 - 7.77 (m, 2H), 7.60 (d, *J* = 7.2 Hz, 0.6H), 7.43 (d, *J* = 7.6 Hz, 0.4H), 7.25 (d, *J* = 6.5 Hz, 0.6H), 7.14 (d, *J* = 7.4 Hz, 0.4H), 4.46 - 4.33 (m, 0.4H), 4.30 - 4.18 (m, 0.6H), 3.84 - 3.61 (m, 4H), 3.55 (d, *J* = 12.5 Hz, 2H), 2.73 - 2.54 (m, 2H), 1.96 - 1.77 (m, 4H), 1.74 - 1.60 (m, 2H), 1.60 - 1.40 (m, 6H). | 431.5 |
| 35 | 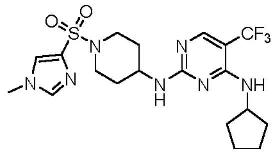 | $^1$H NMR (400 MHz, DMSO-$d_6$) δ 8.03 - 7.95 (m, 1H), 7.82 (s, 1H), 7.81 (s, 1H), 7.34 (d, *J* = 6.0 Hz, 0.6H), 7.16 (d, *J* = 6.0 Hz, 0.4H), 6.20 - 6.10 (m, 1H), 4.54 - 4.26 (m, 1H), 3.72 (s, 3H), 3.69 - 3.61 (m, 1H), 3.60 - 3.49 (m, 2H), 2.70 - 2.53 (m, 2H), 1.99 - 1.80 (m, 4H), 1.73 - 1.60 (m, 2H), 1.59 - 1.42 (m, 6H). | 474.2 |
| 36 | 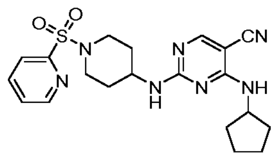 | $^1$H NMR (400 MHz, DMSO-$d_6$) δ 8.77 (d, *J* = 4.4 Hz, 1H), 8.19 - 8.08 (m, 2H), 7.96 - 7.88 (m, 1H), 7.75 - 7.66 (m, 1H), 7.62 (d, *J* = 7.2 Hz, 0.6H), 7.44 (d, *J* = 7.6 Hz, 0.4H), 7.25 (d, *J* =6.4 Hz, 0.6H), 7.14 (d, *J* = 7.6 Hz, 0.4H), 4.45 - 4.33 (m, 0.4H), 4.30 4.17 (m, 0.6H), 3.87 - 3.63 (m, 3H), 2.91 - 2.72 (m, 2H), 1.96 -1.76 (m, 4H), 1.74 - 1.60 (m, 2H), 1.59 - 1.39 (m, 6H). | 428.2 |
| 37 | 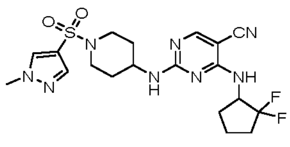 | $^1$H NMR (400 MHz, $CDCl_3$+$D_2O$) δ 8.12 (s, 1H), 7.73 (d, *J* = 9.4 Hz, 2H), 4.65 - 4.51 (m, 1H), 3.98 (s, 3H), 3.83 (br.s., 1H), 3.69 (br.s., 1H), 3.54 (br.s., 1H), 2.68 (br.s., 1H), 2.57 - 2.41 (m, 1H), 2.33 - 2.01 (m, 5H), 2.00 - 1.52 (m, 5H). | 467.2 |
| 38 | 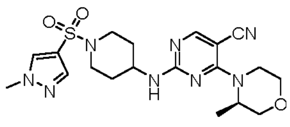 | $^1$H NMR (400 MHz, DMSO-$d_6$) δ 8.33 - 8.27 (m, 1.2H), 8.23 (s, 0.6H), 7.78 (s, 1H), 7.76 (s, 0.6H), 7.59 (d, *J* = 7.5 Hz, 0.4H), 4.70 - 4.58 (m, 1H), 4.35 - 4.24 (m, 1H), 3.91 (s, 3H), 3.89 - 3.63 (m, 3H), 3.61 - 3.54 (m, 1H), 3.51 - 3.37 (m, 3H), 3.29 - 3.18 (m, 1H), 2.48 - 2.31 (m, 2H), 1.97 - 1.82 (m, 2H), 1.65 - 1.49 (m, 2H), 1.26 (t, *J* = 6.4 Hz, 3H). | 447.2 |
| 39 | 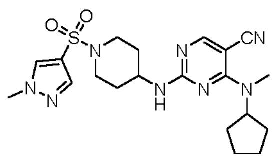 | $^1$H NMR (400 MHz, DMSO-$d_6$) δ 8.33 (s, 0.6H), 8.31 (s, 0.4H), 8.23 (s, 0.3H), 8.18 (s, 0.6H), 7.78 (s, 0.6H), 7.76 (s, 0.3H), 7.66 (d, *J* = 7.2 Hz, 0.6H), 7.46 (d, *J* = 7.4 Hz, 0.3H), 5.06 - 4.79 (m, 1H), 3.90 (s, 3H), 3.81 - 3.60 (m, 1H), 3.52 - 3.39 (m, 2H), 3.04 - 2.95 (m, 3H), 2.48 - 2.30 (m, 2H), 1.98 - 1.75 (m, 4H), 1.73 - 1.46 (m, 8H). | 445.2 |

(continued)

| Compound | Structure | $^1$HNMR | MS (ESI) |
|---|---|---|---|
| 40 | 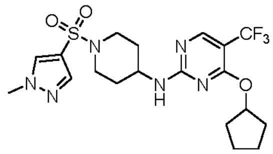 | $^1$H NMR (400 MHz, DMSO-$d_6$) δ 8.33 (d, $J$ = 4.4 Hz, 1H), 8.26 (s, 1H), 7.95 (d, $J$ = 6.9 Hz, 0.5H), 7.84 - 7.70 (m, 1.4H), 5.54 - 5.39 (m, 1H), 3.91 (s, 3H), 3.85 - 3.66 (m, 1H), 3.57 - 3.41 (m, 2H), 2.49 - 2.29 (m, 2H), 2.04 - 1.80 (m, 4H), 1.77 - 1.49 (m, 8H). | 475.2 |
| 41 | 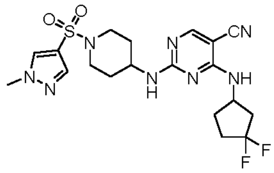 | $^1$H NMR (400 MHz, DMSO-$d_6$) δ 8.32 (s, 0.5H), 8.31 (s, 0.4H), 8.20 (s, 0.4H), 8.16 (s, 0.5H), 7.76 (s, 1H), 7.73 (d, $J$ = 7.2 Hz, 0.6H), 7.60 (d, $J$ = 6.7 Hz, 0.5H), 7.56 (d, $J$ = 7.6 Hz, 0.4H), 7.49 (d, $J$ = 7.6 Hz, 0.4H), 4.66 - 4.37 (m, 1H), 3.90 (s, 3H), 3.81 - 3.63 (m, 1H), 3.53 - 3.39 (m, 2H), 2.47 - 2.31 (m, 3H), 2.31 - 2.13 (m, 2H), 2.11 - 1.76 (m, 5H), 1.64 - 1.49 (m, 2H). | 467.2 |
| 42 | 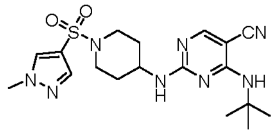 | $^1$H NMR (400 M, DMSO-$d_6$) δ 8.33 (s, 0.7H), 8.31 (s, 0.3H), 8.17 (s, 0.3H), 8.14 (s, 0.7H), 7.79 (s, 0.7H), 7.77 (s, 0.3H), 7.70 (d, $J$ = 7.6 Hz, 0.7H), 7.47 (d, $J$ = 7.6 Hz, 0.3H), 6.12 (s, 0.7H), 5.98 (s, 0.3H), 3.91 (s, 3H), 3.77 - 3.59 (m, 1H), 3.59 - 3.40 (m, 2H), 2.47 - 2.26 (m, 2H), 2.00 - 1.81 (m, 2H), 1.68 - 1.50 (m, 2H), 1.42 (s, 3H), 1.39 (s, 6H). | 419.2 |
| 43 | 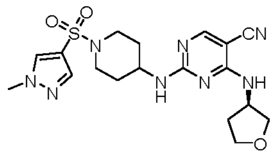 | $^1$H NMR (400 MHz, DMSO-$d_6$) δ 8.33 (s, 0.6H), 8.31 (s, 0.4H), 8.20 (s, 0.3H), 8.15 (s, 0.6H), 7.78 (s, 0.6H), 7.76 (s, 0.3H), 7.69 (d, $J$ = 7.4 Hz, 0.6H), 7.56 - 7.48 (m, 1H), 7.41 (d, $J$ = 6.5 Hz, 0.3H), 4.60 - 4.41 (m, 1H), 3.91 (s, 3H), 3.85 - 3.62 (m, 4H), 3.57 - 3.41 (m, 3H), 2.47 - 2.31 (m, 2H), 2.16 - 1.82 (m, 4H), 1.65 - 1.48 (m, 2H). | 433.2 |
| 44 | 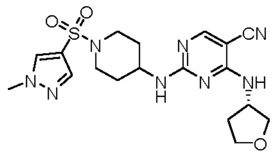 | $^1$H NMR (400 MHz, DMSO-$d_6$) δ 8.33 (s, 0.6H), 8.31 (s, 0.4H), 8.20 (s, 0.3H), 8.15 (s, 0.6H), 7.78 (s, 0.6H), 7.76 (s, 0.3H), 7.69 (d, $J$ = 7.4 Hz, 0.6H), 7.56 - 7.48 (m, 1H), 7.41 (d, $J$ = 6.5 Hz, 0.3H), 4.60 - 4.41 (m, 1H), 3.91 (s, 3H), 3.85 - 3.62 (m, 4H), 3.57 - 3.41 (m, 3H), 2.47 - 2.31 (m, 2H), 2.16 - 1.82 (m, 4H), 1.65 - 1.48 (m, 2H). | 433.2 |
| 45 | 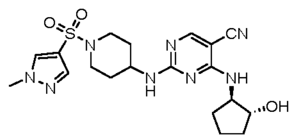 | $^1$HNMR (400 MHz, DMSO-$d_6$+TFA) δ 8.54 - 8.41 (m, 2H), 8.34 (s, 1H), 7.79 (s, 1H), 4.26 - 4.00 (m, 2H), 3.90 (s, 3H), 3.87 - 3.76 (m, 1H), 3.67 - 3.39 (m, 2H), 2.36 - 2.23 (m, 1H), 2.06 - 1.76 (m, 4H), 1.71 - 1.37 (m, 6H). | 447.2 |

(continued)

| Compound | Structure | 1HNMR | MS (ESI) |
|---|---|---|---|
| 46 | 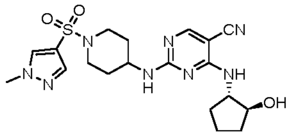 | $^1$H NMR (400 MHz, DMSO-$d_6$) δ 8.33 (s, 0.6H), 8.31 (s, 0.4H), 8.17 (s, 0.4H), 8.12 (s, 0.6H), 7.77 (s, 0.6H), 7.76 (s, 0.4H), 7.62 (d, *J* = 7.3 Hz, 0.6H), 7.47 (d, *J* = 7.6 Hz, 0.4H), 7.20 (d, *J* = 6.5 Hz, 0.6H), 7.12 (d, *J* = 7.4 Hz, 0.4H), 4.71 (d, *J* = 4.3 Hz, 0.4H), 4.65 (d, *J* = 4.2 Hz, 0.6H), 4.19 - 3.94 (m, 2H), 3.91 (s, 3H), 3.81 - 3.65 (m, 1H), 3.56 - 3.35 (m, 2H), 2.48 - 2.31 (m, 2H), 2.03 - 1.75 (m, 4H), 1.64 - 1.49 (m, 4H), 1.49 - 1.35 (m, 2H). | 447.2 |
| 47 | 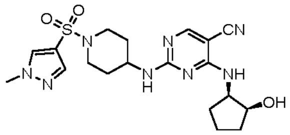 | $^1$H NMR (400 MHz, DMSO-$d_6$) δ 8.33 (s, 0.6H), 8.31 (s, 0.4H), 8.21 (s, 0.4H), 8.16 (s, 0.6H), 7.78 (s, 0.6H), 7.76 (s, 0.4H), 7.71 (d, *J* = 7.2 Hz, 0.6H), 7.54 (d, *J* = 8.0 Hz, 0.4H), 6.41 (d, *J* = 5.6 Hz, 0.6H), 6.23 (d, *J* = 7.3 Hz, 0.4H), 5.09 (d, *J* = 4.1 Hz, 1H), 4.19 - 3.98 (m, 2H), 3.90 (s, 3H), 3.83 - 3.63 (m, 1H), 3.58 - 3.38 (m, 2H), 2.47 - 2.30 (m, 2H), 2.02 - 1.82 (m, 3H), 1.82 - 1.66 (m, 2H), 1.64 - 1.35 (m, 5H). | 477.2 |
| 48 | 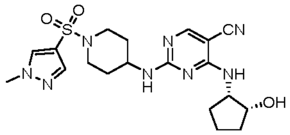 | $^1$H NMR (400 MHz, DMSO-$d_6$) δ 8.33 (s, 0.6H), 8.31 (s, 0.4H), 8.21 (s, 0.4H), 8.16 (s, 0.6H), 7.78 (s, 0.6H), 7.76 (s, 0.4H), 7.71 (d, *J* = 7.2 Hz, 0.6H), 7.54 (d, *J* = 8.0 Hz, 0.4H), 6.41 (d, *J* = 6.1 Hz, 0.6H), 6.23 (d, *J* = 7.3 Hz, 0.4H), 5.09 (d, *J* = 4.2 Hz, 1H), 4.18 - 3.97 (m, 2H), 3.90 (s, 3H), 3.83 - 3.63 (m, 1H), 3.58 - 3.38 (m, 2H), 2.47 - 2.30 (m, 2H), 2.02 - 1.82 (m, 3H), 1.82 - 1.66 (m, 2H), 1.64 - 1.35 (m, 5H). | 447.2 |
| 49 | 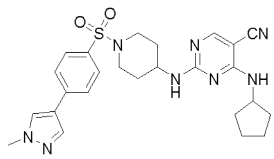 | $^1$HNMR (400 MHz, DMSO-$d_6$+TFA) δ 8.71 - 8.56 (m, 1H), 8.46 (s, 1H), 8.31 (s, 1H), 8.00 (s, 1H), 7.81 (d, *J* = 8.4 Hz, 2H), 7.71 (d, *J* = 8.5 Hz, 2H), 4.47 - 4.24 (m, 1H), 3.88 (s, 3H), 3.81 (br.s., 1H), 3.64 (d, *J* = 11.1 Hz, 1H), 3.53 (d, *J* = 12.0 Hz, 2H), 2.63 - 2.54 (m, 1H), 2.37 - 2.25 (m, 1H), 2.00 - 1.76 (m, 4H), 1.75 - 1.35 (m, 8H). | 507.1 |
| 50 | 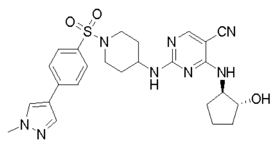 | $^1$H NMR (400 MHz, DMSO-$d_6$) δ 8.31 (s, 1H), 8.14 (s, 0.4H), 8.10 (s, 0.6H), 8.00 (s, 1H), 7.82 (s, 0.6H), 7.80 (s, 1.4H), 7.73 - 7.65 (m, 2H), 7.61 (d, *J* = 7.3 Hz, 0.6H), 7.45 (d, *J* = 7.9 Hz, 0.5H), 7.17 (d, *J* = 6.4 Hz, 0.6H), 7.10 (d, *J* = 7.4 Hz, 0.4H), 4.70 (d, *J* = 4.3 Hz, 0.4H), 4.61 (d, *J* = 4.2 Hz, 0.6H), 4.20 - 3.92 (m, 2H), 3.89 (s, 3H), 3.71 (br.s., 1H), 3.63 - 3.47 (m, 2H), 2.45 - 2.37 (m, 1H), 1.99 - 1.68 (m, 4H), 1.63 - 1.32 (m, 6H). | 523.2 |

(continued)

| Compound | Structure | $^1$HNMR | MS (ESI) |
|---|---|---|---|
| 51 | 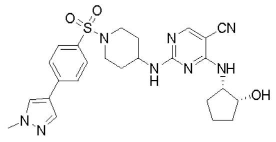 | $^1$HNMR (400 MHz, DMSO-$d_6$) δ 8.32 (s, 1H), 8.19 (s, 0.4H), 8.14 (s, 0.6H), 8.00 (s, 1H), 7.82 (s, 1H), 7.80 (s, 1H), 7.71 - 7.68 (m, 2.6H), 7.53 (d, *J* = 7.4 Hz, 0.4H), 6.39 (d, *J* = 6.3 Hz, 0.6H), 6.21 (d, *J* = 7.2 Hz, 0.4H), 5.07 (br.s., 1H), 4.10 - 3.98 (m, 2H), 3.89 (s, 3H), 3.81 - 3.62 (m, 1H), 3.60 - 3.44 (m, 2H), 2.47 - 2.41 (m, 2H), 1.99 - 1.79 (m, 3H), 1.79 - 1.64 (m, 2H), 1.63 - 1.31 (m, 5H). | 523.2 |
| 52 | 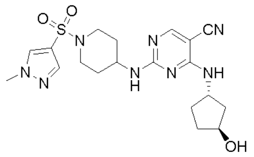<br>Absolute configuration not determined, enantiomer of compound **53** | $^1$H NMR (400 MHz, DMSO-$d_6$) δ 8.31 (s, 1H), 8.15 (s, 0.4H), 8.11 (s, 0.6H), 7.76 (s, 1H), 7.62 (d, *J* = 7.0 Hz, 0.6H), 7.47 (d, *J* = 7.7 Hz, 0.4H), 7.27 (d, *J* = 6.9 Hz, 0.6H), 7.16 (d, *J* = 8.0 Hz, 0.4H), 4.75 - 4.62 (m, 0.4H), 4.53 - 4.40 (m, 1.6H), 4.17 (br.s., 1H), 3.91 (s, 3H), 3.81 - 3.62 (m, 1H), 3.54 - 3.38 (m, 2H), 2.49 - 2.29 (m, 2H), 2.08 - 1.67 (m, 6H), 1.62 - 1.35 (m, 4H). | 447.2 |
| 53 | 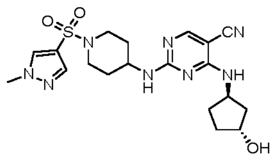<br>Absolute configuration not determined, enantiomer of compound **52** | $^1$H NMR (400 MHz, DMSO-$d_6$) δ 8.31 (s, 1H), 8.15 (s, 0.4H), 8.11 (s, 0.6H), 7.77 (s, 1H), 7.62 (d, *J* = 7.1 Hz, 0.6H), 7.47 (d, *J* = 7.8 Hz, 0.5H), 7.27 (d, *J* = 6.9 Hz, 0.6H), 7.16 (d, *J* = 7.9 Hz, 0.5H), 4.50 - 4.40 (m, 2H), 4.18 (s, 1H), 3.91 (s, 3H), 3.71 (br.s., 1H), 3.51 - 3.41 (m, 2H), 2.45 - 2.33 (m, 2H), 2.01 - 1.35 (m, 10H). | 447.2 |
| 54 | 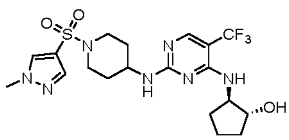 | $^1$H NMR (400 MHz, DMSO-$d_6$) δ 8.32 (s, 1H), 7.99 (s, 1H), 7.77 (s, 1H), 7.36 (d, *J* = 6.4 Hz, 0.6H), 7.21 (d, *J* = 6.8 Hz, 0.5H), 6.21 - 6.08 (m, 1H), 4.74 - 4.60 (m, 1H), 4.25 - 3.95 (m, 2H), 3.91 (s, 3H), 3.80 - 3.65 (m, 1H), 3. 65 - 3.57 (m, 3H), 2.42 - 2.28 (m, 1H), 2.08 - 1.73 (m, 4H), 1.72 - 1.50 (m, 4H), 1.50 - 1.35 (m, 2H). | 490.2 |
| 55 | 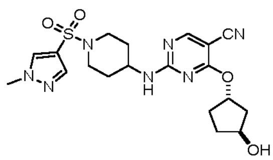<br>Relative configuration (trans) | $^1$H NMR (400 MHz, DMSO-$d_6$) δ 8.46 (s, 0.4H), 8.43 (s, 0.6H), 8.33 (s, 0.6H), 8.31 (s, 0.4H), 8.28 (d, *J* = 7.3 Hz, 0.6H), 8.13 (d, *J* = 7.6 Hz, 0.4H), 7.79 (s, 0.6H), 7.77 (s, 0.5H), 5.56 - 5.42 (m, 1H), 4.64 (dd, *J* = 5.5, 3.7 Hz, 1H), 4.32 - 4.20 (m, 1H), 3.91 (s, 3H), 3.77 (br.s., 1H), 3.48 (t, *J* = 12.2 Hz, 2H), 2.43 - 2.30 (m, 2H), 2.26 - 2.07 (m, 1H), 2.03 - 1.79 (m, 5H), 1.74 - 1.45 (m, 4H). | 448.2 |
| 56 | 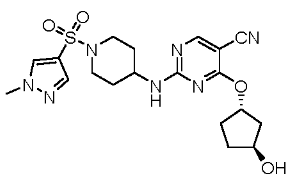<br>Absolute configuration not determined, enantiomer of compound **57** | $^1$H NMR (400 MHz, DMSO-$d_6$) δ 8.47 (s, 0.4H), 8.43 (s, 0.7H), 8.33 (s, 0.6H), 8.31 (s, 0.4H), 8.28 (d, *J* = 7.9 Hz, 0.6H), 8.13 (d, *J* = 8.4 Hz, 0.4H), 7.78 (s, 0.6H), 7.77 (s, 0.4H), 5.57 -5.42 (m, 1H), 4.69 - 4.59 (m, 1H), 4.26 (br.s., 1H), 3.91 (s, 3H), 3.76 (br.s., 1H), 3.54 - 3.41 (m, 2H), 2.48 - 2.33 (m, 2H), 2.00 - 1.81 (m, 6H), 1.75 - 1.40 (m, 4H). | 448.2 |

(continued)

| Compound | Structure | 1HNMR | MS (ESI) |
|---|---|---|---|
| 57 | 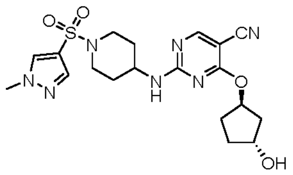 Absolute configuration not determined, enantiomer of compound **56** | $^1$H NMR (400 MHz, DMSO-$d_6$) δ 8.47 (s, 0.4H), 8.43 (s, 0.5H), 8.33 (s, 0.6H), 8.31 (s, 0.5H), 8.28 (d, *J*= 7.8 Hz, 0.6H), 8.13 (d, *J* = 7.9 Hz, 0.5H), 7.78 (s, 0.6H), 7.77 (s, 0.5H), 5.57 -5.42 (m, 1H), 4.67 - 4.59 (m, 1H), 4.26 (br.s., 1H), 3.91 (s, 3H), 3.76 (br.s., 1H), 3.54 - 3.41 (m, 2H), 2.48 - 2.33 (m, 2H), 2.06 - 1.79 (m, 6H), 1.75 - 1.44 (m, 4H). | 448.2 |
| 58 | 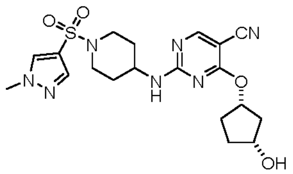 Relative configuration (cis) | $^1$H NMR (400 MHz, DMSO-$d_6$) δ 8.47 (s, 0.4H), 8.43 (s, 0.5H), 8.33 (s, 0.6H), 8.31 (s, 0.4H), 8.26 (d, *J* = 7.4 Hz, 0.6H), 8.09 (d, *J* = 7.7 Hz, 0.4H), 7.79 (s, 0.5H), 7.77 (s, 0.4H), 5.35 - 5.23 (m, 1H), 4.69 (d, *J* = 4.0 Hz, 0.5H), 4.66 (d, *J* = 4.0 Hz, 0.5H), 4.14 - 4.03 (m, 1H), 3.90 (s, 3H), 3.76 (br.s., 1H), 3.53 - 3.42 (m, 2H), 2.44 - 2.22 (m, 2H), 2.07 - 1.78 (m, 5H), 1.77 - 1.51 (m, 5H). | 448.2 |
| 59 | 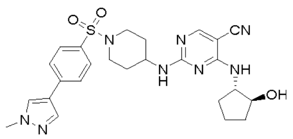 | $^1$H NMR (400 MHz, DMSO-$d_6$) δ 8.32 (s, 1H), 8.14 (s, 0.4H), 8.10 (s, 0.6H), 8.00 (s, 1H), 7.82 (s, 0.8H), 7.80 (s, 1.1H), 7.72 - 7.65 (m, 2H), 7.61 (d, *J* = 7.2 Hz, 0.7H), 7.46 (d, *J* = 7.7 Hz, 0.5H), 7.17 (d, *J* = 6.3 Hz, 0.6H), 7.10 (d, *J* = 7.0 Hz, 0.8H), 4.69 (d, *J* = 4.1 Hz, 0.5H), 4.60 (d, *J* = 4.3 Hz, 0.6H), 4.18 - 4.07 (m, 0.5H), 4.05 - 3.93 (m, 1.5H), 3.89 (s, 3H), 3.78 -3.63 (m, 1H), 3.62 - 3.51 (m, 2H), 2.46 - 2.37 (m, 2H), 2.05 - 1.68 (m, 4H), 1.63 - 1.34 (m, 6H). | 523.2 |
| 60 | 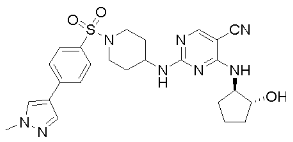 | $^1$H NMR (400 MHz, DMSO-$d_6$) δ 8.32 (s, 1H), 8.14 (s, 0.4H), 8.10 (s, 0.6H), 8.00 (s, 1H), 7.82 (s, 0.8H), 7.80 (s, 1.2H), 7.72 - 7.65 (m, 2H), 7.61 (d, *J* = 7.3 Hz, 0.6H), 7.46 (d, *J* = 7.9 Hz, 0.4H), 7.17 (d, *J* = 6.4 Hz, 0.6H), 7.10 (d, *J* = 7.4 Hz, 0.4H), 4.70 (d, *J* = 4.3 Hz, 0.4H), 4.61 (d, *J* = 4.2 Hz, 0.6H), 4.18 - 3.92 (m, 2H), 3.89 (s, 3H), 3.71 (br.s., 1H), 3.63 - 3.46 (m, 2H), 2.48 - 2.35 (m, 1H), 1.97 - 1.69 (m, 4H), 1.64 - 1.47 (m, 4H), 1.46 - 1.31 (m, 2H). | 523.2 |
| 61 | 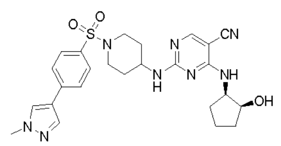 | $^1$H NMR (400 MHz, DMSO-$d_6$) δ 8.32 (s, 1H), 8.19 (s, 0.4H), 8.15 (s, 0.5H), 8.00 (s, 1H), 7.81 (d, *J* = 8.2 Hz, 2H), 7.73 - 7.65 (m, 2.6H), 7.53 (d, *J* = 7.7 Hz, 0.4H), 6.39 (d, *J* = 6.2 Hz, 0.6H), 6.21 (d, *J* = 7.3 Hz, 0.4H), 5.07 (t, *J* = 4.8 Hz, 1H), 4.15 - 3.94 (m, 2H), 3.89 (s, 3H), 3.80 - 3.61 (m 1H), 3.60 - 3.46 (m, 2H), 2.60 - 2.53 (m, 1H), 2.48 - 2.39 (m, 1H), 1.97 - 1.80 (m, 3H), 1.79 - 1.63 (m, 2H), 1.63 - 1.33 (m, 5H). | 523.2 |

(continued)

| Compound | Structure | 1HNMR | MS (ESI) |
|---|---|---|---|
| 62 | 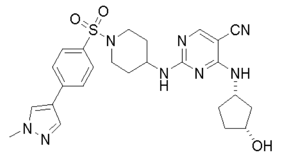 Relative configuration (cis) | 1H NMR (400 MHz, DMSO-$d_6$) δ 8.32 (s, 1H), 8.15 (s, 0.4H), 8.11 (s, 0.6H), 8.00 (s, 1H), 7.81 (d, *J* = 8.4 Hz, 2H), 7.73 - 7.66 (m, 2H), 7.64 (d, *J* = 7.2 Hz, 0.6H), 7.47 (d, *J* = 7.9 Hz, 0.4H), 7.04 (d, *J* = 7.5 Hz, 0.6H), 7.00 (d, *J* = 8.2 Hz, 0.4H), 4.80 (d, *J* = 3.6 Hz, 0.4H), 4.75 (d, *J* = 4.0 Hz, 0.6H), 4.51 - 4.27 (m, 1H), 4.09 (br.s., 1H), 3.89 (s, 3H), 3.71 (br.s., 1H), 3.62 - 3.47 (m, 2H), 2.49 - 2.37 (m, 2H), 2.03 - 1.77 (m, 4H), 1.73 - 1.48 (m, 6H). | 523.2 |
| 63 | 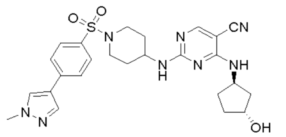 Absolute configuration not determined, enantiomer of compound **83** | 1H NMR (400 MHz, DMSO-$d_6$) δ 8.31 (s, 1H), 8.12 (s, 0.4H), 8.09 (s, 0.6H) 8.00 (s, 1H), 7.82 (s, 1H), 7.80 (s, 1H), 7.73 - 7.66 (m, 2H), 7.61 (d, *J* = 6.6 Hz, 0.6H), 7.46 (d, *J* = 8.2 Hz, 0.4H), 7.25 (d, *J* = 7.1 Hz, 0.6H), 7.15 (d, *J* = 8.2 Hz, 0.4H), 4.73 - 4.60 (m, 0.4H), 4.51 - 4.33 (m, 1.6H), 4.20 - 4.11 (m, 1H), 3.89 (s, 3H), 3.82 - 3.62 (m, 1H), 3.62 - 3.46 (m, 2H), 2.47 - 2.34 (m, 2H), 2.04 - 1.62 (m, 6H), 1.60 - 1.30 (m, 4H). | 523.2 |
| 64 | 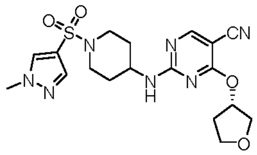 | 1H NMR (400 MHz, DMSO-$d_6$) δ 8.51 (s, 0.4H), 8.47 (s, 0.6H), 8.36 - 8.30 (m,1.5H), 8.17 (d, *J* = 7.8 Hz, 0.4H), 7.79 (s, 0.6H), 7.77 (s, 0.4H), 5.55 (br.s., 1H), 3.91 (s, 3H), 3.90 - 3.68 (m, 5H), 3.56 - 3.42 (m, 2H), 2.47 - 2.17 (m, 3H), 2.09 - 1.84 (m, 3H), 1.71 - 1.52 (m, 2H). | 434.2 |
| 65 | 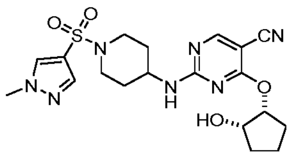 Relative configuration (cis) | 1H NMR (400 MHz, DMSO-$d_6$) δ 8.48 (s, 0.4H), 8.44 (s, 0.5H), 8.32 (d, *J* = 5.8 Hz, 1H), 8.29 (d, *J* = 7.5 Hz, 0.6H), 8.14 (d, *J* = 7.6 Hz, 0.4H), 7.78 (d, *J* = 5.2 Hz, 1H), 5.19 - 5.11 (m, 0.5H), 5.11 - 5.06 (m, 0.6H), 4.94 (s, 1H), 4.08 (s, 1H), 3.91 (s, 3H), 3.85 - 3.70 (m, 1H), 3.56 - 3.42 (m, 2H), 2.47 - 2.30 (m, 2H), 2.19 - 2.03 (m, 1H), 2.03 - 1.78 (m, 3H), 1.77 - 1.44 (m, 6H). | 448.2 |
| 66 | 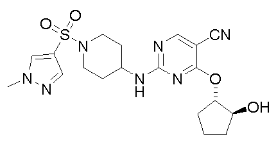 Absolute configuration not determined, enantiomer of compound **67** | 1H NMR (400 MHz, DMSO-$d_6$) δ 8.48 (s, 0.4H), 8.44 (s, 0.5H), 8.32 (d, *J* = 6.0 Hz, 1H), 8.29 (d, *J* = 7.2 Hz, 0.5H), 8.14 (d, *J* = 7.7 Hz, 0.4H), 7.78 (d, *J* = 5.5 Hz, 1H), 5.18 - 5.12 (m, 0.5H), 5.11 - 5.06 (m, 0.6H), 4.94 (dd, *J* = 4.0 Hz, 2.7 Hz, 1H), 4.12 - 4.02 (m, 1H), 3.91 (s, 3H), 3.87 - 3.72 (m, 1H), 3.54 - 3.40 (m, 2H), 2.47 - 2.31 (m, 2H), 2.16 - 2.04 (m, 1H), 2.02 - 1.76 (m, 3H), 1.75 - 1.45 (m, 6H). | 448.2 |
| 67 | 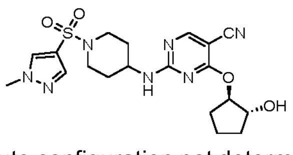 Absolute configuration not determined, enantiomer of compound **66** | 1H NMR (400 MHz, DMSO-$d_6$) δ 8.48 (s, 0.4H), 8.44 (s, 0.5H), 8.32 (d, *J* = 6.0 Hz, 1H), 8.29 (d, *J* = 7.6 Hz, 0.5H), 8.14 (d, *J* = 7.2 Hz, 0.4H), 7.77 (d, *J* = 5.6 Hz, 1H), 5.18 - 5.12 (m, 0.5H), 5.11 - 5.05 (m, 0.6H), 4.93 (dd, *J* = 4.0 Hz, 2.8 Hz, 1H), 4.13 - 4.01 (m, 1H), 3.91 (s, 3H), 3.86 - 3.70 (m, 1H), 3.55 - 3.42 (m, 2H), 2.47 - 2.31 (m, 2H), 2.17 - 2.03 (m, 1H), 2.02 - 1.78 (m, 3H), 1.77 - 1.46 (m, 6H). | 448.2 |

(continued)

| Compound | Structure | 1HNMR | MS (ESI) |
|---|---|---|---|
| 68 | 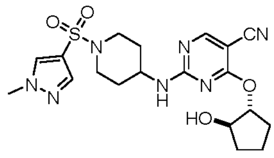 Relative configuration (trans) | $^1H$ NMR (400 MHz, DMSO-$d_6$) δ 8.47 (s, 0.5H), 8.43 (s, 0.5H), 8.33 (d, *J* = 8.5 Hz, 1H), 8.22 (d, *J* = 7.3 Hz, 0.5H), 8.06 (d, *J* = 7.7 Hz, 0.5H), 7.78 (d, *J* = 7.5 Hz, 1H), 5.22 - 5.11 (m, 1H), 4.69 (dd, *J* = 10.6,4.8 Hz, 1H), 4.20 - 4.08 (m, 1H), 3.91 (s, 3H), 3.86 - 3.66 (m, 1H), 3.54 - 3.39 (m, 2H), 2.48 - 2.35 (m, 2H), 1.99 - 1.86 (m, 3H), 1.84 -1.70 (m, 3H), 1.67 - 1.42 (m, 4H). | 448.2 |
| 69 | 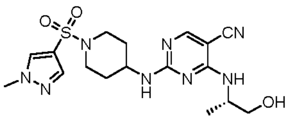 | $^1H$ NMR (400 MHz, DMSO-$d_6$) δ 8.32 (s, 0.6H), 8.31 (s, 0.4H), 8.18 (s, 0.4H), 8.13 (s, 0.6H), 7.78 (s, 0.6H), 7.76 (s, 0.4H), 7.63 (d, *J* = 7.3 Hz, 0.6H), 7.47 (d, *J* = 7.7 Hz, 0.4H), 6.85 (d, *J* = 7.8 Hz, 0.6H), 6.75 (d, *J* = 8.3 Hz, 0.4H), 4.78 - 4.68 (m, 1H), 4.29 - 4.07 (m, 1H), 3.92 (s, 3H), 3.81 - 3.62 (m, 1H), 3.53 - 3.33 (m, 4H), 2.46 - 2.30 (m, 2H), 1.90 - 1.79 (m, 2H), 1.65 - 1.49 (m, 2H), 1.09 (d, *J* = 6.6 Hz, 3H). | 421.2 |
| 70 | 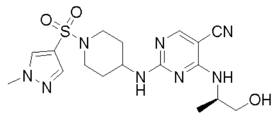 | $^1H$ NMR (400 MHz, DMSO-$d_6$) δ 8.32 (s, 0.6H), 8.31 (s, 0.4H), 8.18 (s, 0.4H), 8.13 (s, 0.6H), 7.78 (s, 0.6H), 7.76 (s, 0.4H), 7.63 (d, *J* = 7.3 Hz, 0.6H), 7.47 (d, *J* = 7.4 Hz, 0.4H), 6.85 (d, *J* = 7.9 Hz, 0.6H), 6.75 (d, *J* = 8.3 Hz, 0.4H), 4.78 - 4.68 (m, 1H), 4.29 - 4.07 (m, 1H), 3.91 (s, 3H), 3.79 - 3.62 (m, 1H), 3.53 - 3.33 (m, 4H), 2.46 - 2.30 (m, 2H), 2.04 - 1.79 (m, 2H), 1.59 - 1.56 (m, 2H), 1.09 (d, *J* = 6.6 Hz, 3H). | 421.2 |
| 71 | 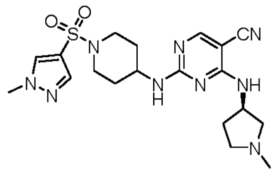 | $^1H$ NMR (400 MHz, DMSO-$d_6$) δ 8.33 (s, 0.6H), 8.31 (s, 0.4H), 8.18 (s, 0.4H), 8.15 (s, 0.6H), 7.78 (s, 0.6H), 7.76 (s, 0.4H), 7.66 (d, *J* = 7.7 Hz, 0.6H), 7.48 (d, *J* = 7.6 Hz, 0.4H), 7.32 (d, *J* = 6.1 Hz, 0.6H), 7.18 (d, *J* = 6.8 Hz, 0.4H), 4.56 - 4.33 (m, 1H), 3.91 (s, 3H), 3.81 - 3.65 (m, 1H), 3.52 - 3.39 (m, 2H), 2.79 - 2.62 (m, 1H), 2.47 - 2.34 (m, 5H), 2.22 (d, *J* = 9.1 Hz, 3H), 2.16 - 2.00 (m, 1H), 1.98 - 1.82 (m, 2H), 1.81 - 1.71 (m, 1H), 1.66 - 1.47 (m, 2H). | 446.2 |
| 72 | 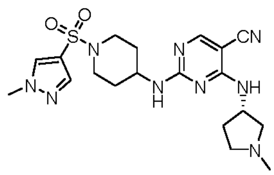 | $^1H$ NMR (400 MHz, DMSO-$d_6$) δ 8.33 (s, 0.6H), 8.31 (s, 0.4H), 8.18 (s, 0.4H), 8.13 (s, 0.6H), 7.78 (s, 0.6H), 7.76 (s, 0.4H), 7.66 (d, *J* = 7.7 Hz, 0.6H), 7.48 (d, *J* = 7.6 Hz, 0.4H), 7.32 (d, *J* = 6.1 Hz, 0.7H), 7.18 (d, *J* = 6.8 Hz, 0.4H), 4.56 - 4.33 (m, 1H), 3.91 (s, 3H), 3.80 - 3.64 (m, 1H), 3.56 - 3.41 (m, 2H), 2.80 - 2.62 (m, 2H), 2.47 - 2.28 (m, 4H), 2.26 - 2.15 (m, 3H), 2.15 - 2.00 (m, 1H), 1.98 - 1.82 (m, 2H), 1.82 - 1.71 (m, 1H), 1.65 - 1.49 (m, 2H). | 446.2 |

(continued)

| Compound | Structure | 1HNMR | MS (ESI) |
|---|---|---|---|
| 73 | 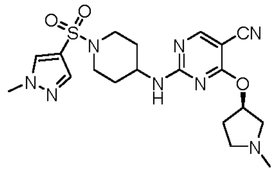 | $^1$H NMR (400 MHz, DMSO-$d_6$) δ 8.49 (s, 0.4H), 8.45 (s, 0.6H), 8.36 - 8.28 (m, 1.5H), 8.12 (d, *J* = 7.6 Hz, 0.4H), 7.79 (s, 0.6H), 7.77 (s, 0.4H), 5.44 - 5.34 (m, 1H), 3.91 (s, 3H), 3.82 - 3.70 (m, 1H), 3.56 - 3.41 (m, 2H), 2.77 - 2.59 (m, 4H), 2.47 - 2.27 (m, 3H), 2.22 (d, *J* = 4.4 Hz, 3H), 2.00 - 1.85 (m, 2H), 1.86 - 1.73 (m, 1H), 1.68 - 1.52 (m, 2H). | 447.2 |
| 74 | 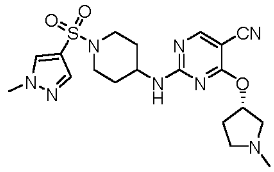 | $^1$H NMR (400 MHz, DMSO-$d_6$) δ 8.48 (s, 0.4H), 8.44 (s, 0.6H), 8.35 - 8.28 (m, 1.5H), 8.12 (d, *J* = 7.8 Hz, 0.4H), 7.79 (s, 0.6H), 7.77 (s, 0.4H), 5.44 - 5.33 (m, 1H), 3.91 (s, 3H), 3.85 - 3.69 (m, 1H), 3.57 - 3.42 (m, 2H), 2.79 - 2.60 (m, 4H), 2.44 - 2.34 (m, 1H), 2.34 - 2.20 (m, 5H), 2.01 - 1.74 (m, 3H), 1.70 - 1.51 (m, 2H). | 447.1 |
| 75 | 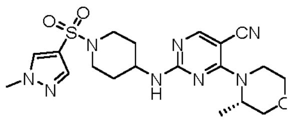 | $^1$HNMR (400 MHz, DMSO-$d_6$) δ 8.32 (s, 1H), 8.31 (s, 0.6H), 8.28 (s, 0.5H), 8.23 (s, 1H), 7.77 (s, 0.4H), 7.59 (d, *J* = 7.9 Hz, 0.4H), 4.70 - 4.58 (m, 1H), 4.35 - 4.24 (m, 1H), 3.91 (s, 3H), 3.89 - 3.85 (m, 1H), 3.78 - 3.64 (m, 2H), 3.61 - 3.54 (m, 1H), 3.51 - 3.37 (m, 3H), 3.29 - 3.18 (m, 1H), 2.48 - 2.31 (m, 2H), 1.96 - 1.80 (m, 2H), 1.64 - 1.50 (m, 2H), 1.31 - 1.21 (m, 3H). | 447.2 |
| 76 | 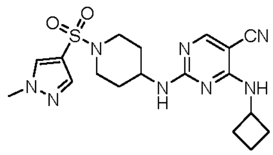 | $^1$H NMR (400 MHz, DMSO-$d_6$) δ 8.33 (s, 0.6H), 8.31 (s, 0.4H), 8.17 (s, 0.4H), 8.12 (s, 0.6H), 7.79 (s, 0.6H), 7.76 (s, 0.4H), 7.67 - 7.57 (m, 1H), 7.51 (d, *J* = 8.0 Hz, 0.6H), 7.46 (d, *J* = 7.6 Hz, 0.4H), 4.60 - 4.32 (m, 1H), 3.91 (s, 3H), 3.71 (br.s., 1H), 3.52 - 3.41 (m, 2H), 2.47 - 2.30 (m, 2H), 2.20 - 2.03 (m, 4H), 1.98 - 1.80 (m, 2H), 1.71 - 1.47 (m, 4H). | 417.1 |
| 77 | 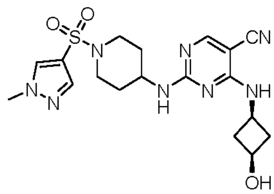 | $^1$H NMR (400 MHz, DMSO-$d_6$) δ 8.33 (s, 0.6H), 8.31 (s, 0.4H), 8.16 (s, 0.4H), 8.12 (s, 0.6H), 7.79 (s, 0.6H), 7.76 (s, 0.4H), 7.65 - 7.56 (m, 1H), 7.50 (d, *J* = 7.2 Hz, 0.5H), 7.43 (d, *J* = 7.6 Hz, 0.5H), 5.04 - 4.98 (m, 1H), 4.03 - 3.86 (m, 4H), 3.84 - 3.64 (m, 3H), 3.52 - 3.37 (m, 2H), 2.51 - 2.49 (m, 2H), 2.36 (t, *J* = 11.0 Hz, 1H), 2.00 - 1.79 (m, 4H), 1.64 - 1.48 (m, 2H). | 433.2 |
| 78 | 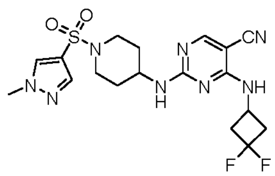 | $^1$H NMR (400 MHz, DMSO-$d_6$) δ 8.33 (s, 0.6H), 8.31 (s, 0.4H), 8.23 (s, 0.4H), 8.18 (s, 0.6H), 7.90 (d, *J* = 7.6 Hz, 0.6H), 7.82 (d, *J* = 6.8 Hz, 0.4H), 7.79 - 7.71 (m, 1.5H), 7.58 (d, *J* = 8.0 Hz, 0.4H), 4.46 - 4.35 (m, 0.4H), 4.29 - 4.15 (m, 0.6H), 3.90 (s, 3H), 3.79 - 3.66 (m, 1H), 3.55 - 3.39 (m, 2H), 2.93 - 2.70 (m, 4H), 2.48 - 2.30 (m, 2H), 1.97 - 1.81 (m, 2H), 1.67 - 1.49 (m, 2H). | 453.2 |

(continued)

| Compound | Structure | $^1$HNMR | MS (ESI) |
|---|---|---|---|
| 79 | 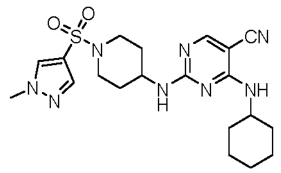 | $^1$H NMR (400 MHz, DMSO-$d_6$) δ 8.34 (s, 0.6H), 8.31 (s, 0.4H), 8.15 (s, 0.4H), 8.11 (s, 0.6H), 7.78 (s, 0.6H), 7.76 (s, 0.4H), 7.63 (dd, *J* = 6.9 Hz, 0.6H), 7.42 (d, *J* = 7.8 Hz, 0.4H), 7.15 (d, *J* = 7.2 Hz, 0.5H), 6.96 (d, *J* = 8.3 Hz, 0.5H), 4.05 - 3.94 (m, 0.5H), 3.90 (s, 3H), 3.85 - 3.69 (m, 1H), 3.68 - 3.54 (m, 0.7H), 3.53 - 3.58 (m, 2H), 2.47 - 2.29 (m, 2H), 2.00 - 1.82 (m, 2H), 1.82 - 1.65 (m, 4H), 1.65 - 1.51 (m, 3H), 1.44 - 1.27 (m, 2H), 1.27 - 1.02 (m, 3H). | 445.2 |
| 80 | 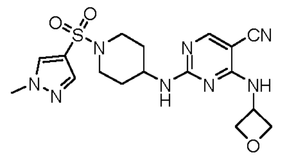 | $^1$H NMR (400 MHz, DMSO-$d_6$) δ 8.33 (s, 0.7H), 8.31 (s, 0.3H), 8.23 (s, 0.3H), 8.18 (s, 0.7H), 8.11 (d, *J* = 8.0 Hz, 0.7H), 8.03 (d, *J* = 5.2 Hz, 0.3H), 7.79 (s, 0.7H), 7.76 (s, 0.3H), 7.72 (d, *J* = 7.2 Hz, 0.7H), 7.50 (d, *J* = 7.6 Hz, 0.3H), 5.04 - 4.79 (m, 1H), 4. 72 - 4.61 (m, 2H), 4.58 (t, *J* = 6.5 Hz, 2H), 3.95 - 3.86 (m, 3H), 3.79 - 3.59 (m, 1H), 3.53 - 3.40 (m, 2H), 2.48 - 2.29 (m, 2H), 1.90 - 1.81 (m, 2H), 1.62 - 1.47 (m, 2H). | 419.2 |
| 81 | 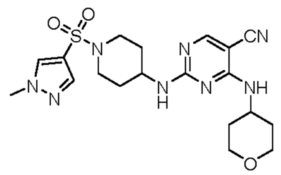 | $^1$H NMR (400 MHz, DMSO-$d_6$) δ 8.34 (s, 0.5H), 8.31 (s, 0.4H), 8.18 (s, 0.4H), 8.14 (s, 0.5H), 7.78 (s, 0.5H), 7.76 (s, 0.4H), 7.68 (d, *J* = 6.9 Hz, 0.5H), 7.48 (d, *J* = 7.9 Hz, 0.4H), 7.32 (d, *J* = 7.2 Hz, 0.5H), 7.15 (d, *J* = 8.3 Hz, 0.5H), 4.26 - 4.12 (m, 0.4H), 4.10 - 3.97 (m, 0.6H), 3.91 (s, 3H), 3.89 - 3.80 (m, 2H), 3.79 - 3.68 (m, 0.4H), 3.68 - 3.55 (m, 0.6H), 3.51 - 3.40 (m, 2H), 3.30 - 3.22 (m, 2H), 2.45 - 2.29 (m, 2H), 1.95 - 1.80 (m, 2H), 1.75 - 1.51 (m, 6H). | 447.2 |
| 82 | 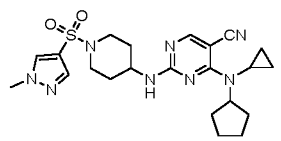 | $^1$H NMR (400 MHz, DMSO-$d_6$) δ 8.34 (s, 0.7H), 8.31 (s, 0.3H), 8.26 (s, 0.3H), 8.21 (s, 0.7H), 7.77 (br.s., 1H), 7.69 (d, *J* = 7.0 Hz, 0.7H), 7.38 (d, *J* = 7.5 Hz, 0.3H), 4.40 - 4.14 (m, 1H), 3.90 (s, 3H), 3.78 - 3.57 (m, 1H), 3.55 - 3.40 (m, 2H), 2.82 (br.s., 1H), 2.41 - 2.27 (m, 2H), 2.06 - 1.68 (m, 7H), 1.68 - 1.41 (m, 5H), 1.02 - 0.90 (m, 2H), 0.82 - 0.70 (m, 2H). | 471.2 |
| 83 | 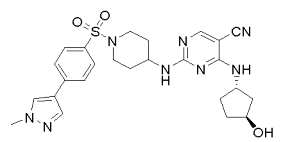 Absolute configuration not determined, enantiomer of compound **63** | $^1$H NMR (400 MHz, DMSO-$d_6$) δ 8.31 (s, 1H), 8.12 (s, 0.4H), 8.09 (s, 0.6H), 8.00 (s, 1H), 7.82 (s, 1H), 7.80 (s, 1H), 7.69 (d, *J* = 7.9 Hz, 2H), 7.61 (d, *J* = 6.9 Hz, 0.6H), 7.46 (d, *J* = 8.0 Hz, 0.4H), 7.24 (d, *J* = 6.8 Hz, 0.6H), 7.15 (d, *J* = 7.1 Hz, 0.4H), 4.74 - 4.58 (m, 0.4H), 4.52 - 4.38 (m, 1.6H), 4.15 (br.s., 1H), 3.89 (s, 3H), 3.68 (br.s., 1H), 3.62 - 3.48 (m, 2H), 2.46 - 2.37 (m, 1H), 2.05 - 1.62 (m, 7H), 1.62 - 1.33 (m, 4H). | 523.2 |

(continued)

| Compound | Structure | 1HNMR | MS (ESI) |
|---|---|---|---|
| 84 | 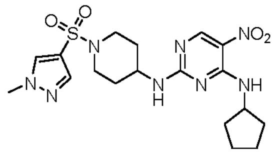 | $^1$H NMR (400 MHz, DMSO-$d_6$) δ 8.86 (s, 0.3H), 8.84 (s, 0.6H), 8.42 - 8.27 (m, 2.6H), 8.12 (d, *J* = 8.0 Hz, 0.3H), 7.79 (s, 0.7H), 7.77 (s, 0.3H), 4.52 - 4.43 (m, 0.3H), 4.43 - 4.31 (m, 0.7H), 3.91 (s, 3H), 3.84 - 3.72 (m, 1H), 3.55 - 3.41 (m, 2H), 2.47 - 2.34 (m, 1H), 2.05 - 1.83 (m, 4H), 1.78 - 1.48 (m, 9H). | 451.2 |
| 85 | 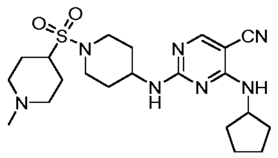 | $^1$H NMR (400 MHz, DMSO-$d_6$) δ 8.20 (s, 0.4H), 8.13 (s, 0.6H), 7.65 (d, *J* = 7.4 Hz, 0.6H), 7.47 (d, *J* = 7.7 Hz, 0.4H), 7.26 (d, *J* = 6.5 Hz, 0.6H), 7.15 (d, *J* = 8.0 Hz, 0.4H), 4.45 - 4.26 (m, 1H), 4.05 - 3.80 (m, 1H), 3.70 - 3.56 (m, 2H), 3.05 - 2.83 (m, 3H), 2.89 - 2.76 (m, 2H), 2.14 (s, 3H), 1.94 - 1.82 (m, 8H), 1.71 - 1.44 (m, 10H). | 448.2 |
| 86 | 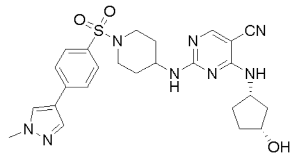 Absolute configuration not determined, enantiomer of compound **87** | $^1$H NMR (400 MHz, DMSO-$d_6$) δ 8.32 (s, 1H), 8.15 (s, 0.4H), 8.11 (s, 0.6H), 8.00 (s, 1H), 7.81 (d, *J* = 8.4 Hz, 2H), 7.73 - 7.66 (m, 2H), 7.64 (d, *J* = 7.1 Hz, 0.6H), 7.47 (d, *J* = 8.2 Hz, 0.4H), 7.04 (d, *J* = 6.9 Hz, 0.6H), 7.00 (d, *J* = 8.4 Hz, 0.4H), 4.80 (d, *J* = 3.6 Hz, 0.4H), 4.75 (d, *J* = 3.3 Hz, 0.6H), 4.51 - 4.25 (m, 1H), 4.09 (br.s., 1H), 3.89 (s, 3H), 3.71 (br.s., 1H), 3.64 - 3.47 (m, 2H), 2.49 - 2.37 (m, 2H), 2.04 - 1.77 (m, 4H), 1.74 - 1.48 (m, 6H). | 523.2 |
| 87 | 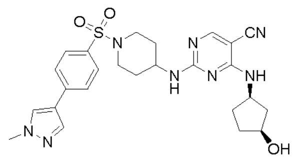 Absolute configuration not determined, enantiomer of compound **86** | $^1$H NMR (400 MHz, DMSO-$d_6$) δ 8.32 (s, 1H), 8.15 (s, 0.4H), 8.11 (s, 0.6H), 8.00 (s, 1H), 7.81 (d, *J* = 8.4 Hz, 2H), 7.73 - 7.66 (m, 2H), 7.64 (d, *J* = 7.1 Hz, 0.6H), 7.47 (d, *J* = 8.2 Hz, 0.4H), 7.04 (d, *J* = 6.9 Hz, 0.6H), 7.00 (d, *J* = 8.4 Hz, 0.4H), 4.80 (d, *J* = 3.6 Hz, 0.4H), 4.75 (d, *J* = 3.3 Hz, 0.6H), 4.51 - 4.25 (m, 1H), 4.09 (br.s., 1H), 3.89 (s, 3H), 3.71 (br.s., 1H), 3.64 - 3.47 (m, 2H), 2.49 - 2.37 (m, 2H), 2.03 - 1.75 (m, 4H), 1.74 - 1.48 (m, 6H). | 523.2 |
| 88 | 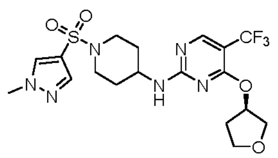 | $^1$H NMR (400 MHz, DMSO-$d_6$) δ 8.39 - 8.26 (m, 2H), 8.03 (d, *J* = 7.2 Hz, 0.6H), 7.88 (d, *J* = 7.6 Hz, 0.4H), 7.79 (s, 0.6H), 7.77 (s, 0.4H), 5.65 - 5.50 (m, 1H), 3.96 - 3.87 (m, 4H), 3.83 - 3.70 (m, 4H), 3.54 - 3.43 (m, 2H), 2.49 - 2.34 (m, 2H), 2.27 - 2.15 (m, 1H), 2.04 - 1.87 (m, 3H), 1.68 - 1.53 (m, 2H). | 477.2 |
| 89 | 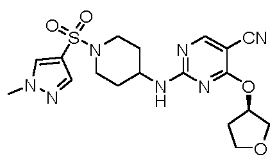 | $^1$H NMR (400 MHz, DMSO-$d_6$) δ 8.51 (s, 0.4H), 8.46 (s, 0.5H), 8.37 - 8.30 (m, 1.5H), 8.17 (d, *J* = 7.3 Hz, 0.4H), 7.79 (s, 0.6H), 7.77 (s, 0.4H), 6.06 (br.s., 1H), 5.59 - 5.52 (m, 1H), 3.91 (s, 3H), 3.89 - 3.69 (m, 5H), 3.55 - 3.43 (m, 2H), 2.46 - 2.35 (m, 1H), 2.29 - 2.16 (m, 1H), 2.06 - 1.87 (m, 3H), 1.67 - 1.52 (m, 2H). | 434.2 |

(continued)

| Compound | Structure | 1HNMR | MS (ESI) |
|---|---|---|---|
| 90 | 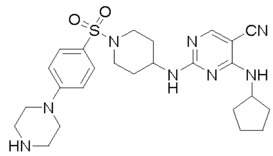 | $^1$H NMR (400 MHz, DMSO-$d_6$) δ 8.14 (s, 0.4H), 8.10 (s, 0.6H), 7.61 (d, *J* = 6.9 Hz, 0.6H), 7.50 (d, *J* = 8.9 Hz, 2H), 7.43 (d, *J* = 7.3 Hz, 0.3H), 7.24 (d, *J* = 6.6 Hz, 0.6H), 7.13 (d, *J* = 7.7 Hz, 0.4H), 7.04 (d, *J* = 9.0 Hz, 2H), 4.43 - 4.28 (m, 0.4H), 4.27 - 4.14 (m, 0.7H), 3.74 - 3.57 (m, 1H), 3.54 - 3.37 (m, 2H), 3.26 - 3.16 (m, 4H), 2.89 - 2.76 (m, 4H), 2.44 - 2.24 (m, 2H), 1.95 - 1.74 (m, 4H), 1.74 - 1.60 (m, 2H), 1.59 - 1.34 ( m, 6H). | 511.3 |
| 91 | 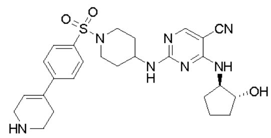 | $^1$H NMR (400 MHz, DMSO-$d_6$) δ 8.14 (s, 0.4H), 8.10 (s, 0.6H), 7.72 - 7.66 (m, 3H), 7.61 (d, *J* = 4.0 Hz, 0.6H), 7.45 (d, *J* = 4.0 Hz, 0.5H), 7.18 *(d, J* = 4.0 Hz, 0.7H), 7.11 (d, *J* = 6.4 Hz, 0.5H), 6.43 (s, 1H), 4.69 (d, *J* = 4.0 Hz, 0.4H), 4.61 (d, *J* = 4.0 Hz, 0.6H), 4.07 - 3.88 (m, 2H), 3.80 - 3.65 (m, 1H), 3.64 - 3.47 (m, 2H), 3.40 (s, 2H), 2.92 (t, *J* = 5.5 Hz, 2H), 2.43 - 2.29 (m, 4H), 2.02 - 1.69 (m, 5H), 1.68 - 1.32 (m, 7H). | 524.2 |
| 92 | 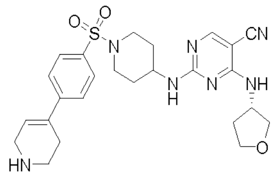 | $^1$HNMR (400 MHz, DMSO-$d_6$) δ 8.17 (s, 0.4H), 8.13 (s, 0.6H), 7.68 (s, 4.5H), 7.51 (d, *J* = 5.5 Hz, 1H), 7.39 (d, *J* = 5.2 Hz, 0.5H), 6.49 - 6.36 (m, 1H), 4.59 - 4.37 (m, 1H), 3.92 - 3.61 (m, 4H), 3.61 - 3.45 (m, 3H), 3.44 - 3.36 (m, 2H), 2.92 (t, *J* = 5.2 Hz, 2H), 2.44 - 2.23 (m, 4H), 2.07 - 1.79 (m, 4H), 1.63 - 1.46 (m, 2H). | 510.2 |
| 93 | 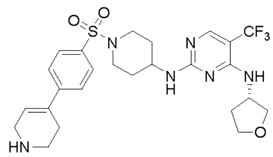 | $^1$H NMR (400 MHz, DMSO-$d_6$) δ 8.00 (s, 1H), 7.74 - 7.61 (m, 3H), 7.45 - 7.37 (m, 1H), 7.30 - 7.21 (m, 1H), 6.50 - 6.35 (m, 2H), 4.67 - 4.44 (m, 2H), 3.94 - 3.75 (m, 2H), 3.74 - 3.60 (m, 2H), 3.60 - 3.45 (m, 3H), 3.44 - 3.38 (m, 2H), 2.92 (t, *J* = 5.4 Hz, 2H), 2.42 - 2.34 (m, 2H), 2.17 - 1.83 (m, 5H), 1.61 -1.47 (m, 2H). | 553.0 |
| 94 | 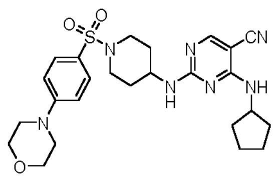 | $^1$H NMR (400 MHz, DMSO-$d_6$) δ 8.14 (s, 0.4H), 8.10 (s, 0.5H), 7.61 (d, *J* = 7.2 Hz, 0.5H), 7.54 (d, *J* = 8.7 Hz, 2H), 7.43 (d, *J* = 7.8 Hz, 0.4H), 7.24 (d, *J* = 6.6 Hz, 0.5H), 7.13 (d, *J* = 7.6 Hz, 0.3H), 7.08 (d, *J* = 8.9 Hz, 2H), 4.46 - 4.27 (m, 0.4H), 4.27 - 4.11 (m, 0.6H), 3.79 - 3.70 (m, 4H), 3.69 - 3.56 (m, 1H), 3.55 - 3.39 (m, 2H), 3.31 - 3.25 (m, 4H), 2.45 - 2.31 (m, 2H), 1.95 - 1.75 (m, 4H), 1.71 - 1.38 (m, 8H). | 512.0 |
| 95 | 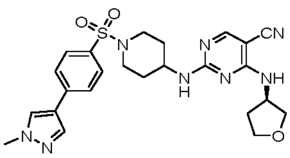 | $^1$H NMR (400 MHz, DMSO-$d_6$) δ 8.31 (s, 1H), 8.21 - 8.10 (m, 1H), 8.00 (s, 1H), 7.84 - 7.78 (m, 2H), 7.74 - 7.64 (m, 2.6H), 7.50 (d, *J* = 5.5 Hz, 1H), 7.41 - 7.35 (m, 0.5H), 4.59 - 4.37 (m, 1H), 3.89 (s, 3H), 3.84 - 3.61 (m, 4H), 3.59 - 3.45 (m, 3H), 2.48 - 2.38 (m, 2H), 2.17 - 1.78 (m, 4H), 1.63 - 1.46 (m, 2H). | 508.9 |

(continued)

| Compound | Structure | 1HNMR | MS (ESI) |
|---|---|---|---|
| 96 | 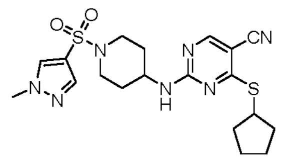 | $^1$H NMR (400 MHz, DMSO-$d_6$) δ 8.44 - 8.30 (m, 2.6H), 8.22 (d, *J* = 7.8 Hz, 0.4H), 7.78 (s, 0.6H), 7.77 (s, 0.4H), 4.11 - 3.96 (m, 1H), 3.90 (s, 3H), 3.86 - 3.71 (m, 1H), 3.60 - 3.43 (m, 2H), 2.47 - 2.34 (m, 2H), 2.25 - 2.02 (m, 2H), 2.00 - 1.87 (m, 2H), 1.78 - 1.48 (m, 8H). | 448.2 |
| 97 | 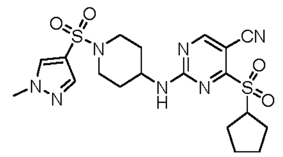 | $^1$H NMR (400 MHz, DMSO-$d_6$) δ 9.08 - 9.01 (m, 1H), 8.97 (s, 0.5H), 8.92 (s, 0.5H), 8.33 (d, *J* = 7.7 Hz, 1H), 7.78 (d, *J* = 6.2 Hz, 1H), 4.28 - 4.17 (m, 0.6H), 4.10 - 3.98 (m, 0.6H), 3.91 (s, 3H), 3.83 (br.s., 1H), 3.54 - 3.43 (m, 2H), 2.48 - 2.39 (m, 2H), 2.06 - 1.86 (m, 6H), 1.76 - 1.55 (m, 6H). | 479.8 |
| 98 | 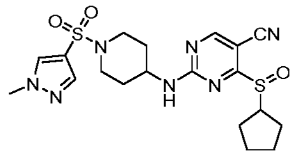 | $^1$H NMR (400 MHz, DMSO-$d_6$) δ 8.88 (d, *J* = 7.6 Hz, 0.6H), 8.82 - 8.74 (m, 1.3H), 8.32 (d, *J* = 4.0 Hz, 1H), 7.77 (d, *J* = 4.9 Hz, 1H), 3.91 (s, 3H), 3.81 (br.s., 1H), 3.58 - 3.40 (m, 3H), 2.48 - 2.39 (m, 2H), 2.13 - 1.87 (m, 4H), 1.85 - 1.73 (m, 1H), 1.72 - 1.50 (m, 7H). | 463.9 |
| 101 | 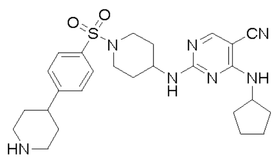 | $^1$H NMR (400 MHz, DMSO-$d_6$ + $D_2O$) δ 8.51 - 8.39 (m, 1H), 7.72 (d, *J* = 8.2 Hz, 2H), 7.51 (d, *J* = 8.2 Hz, 2H), 4.46 - 4.23 (m, 1H), 3.89 - 3.74 (m, 1H), 3.53 - 3.44 (m, 2H), 3.40 - 3.30 (m, 2H), 3.08 - 2.95 (m, 3H), 2.75 - 2.54 (m, 2H), 2.36 - 2.21 (m, 1H), 2.02 - 1.77 (m, 8H), 1.73 - 1.37 (m, 8H). | 510.0 |
| 102 | 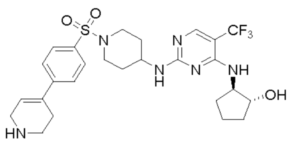 | $^1$H NMR (400 MHz, DMSO-$d_6$) δ 7.97 (s, 1H), 7.78 - 7.61 (m, 4H), 7.40 - 7.30 (m, 0.6H), 7.23 - 7.16 (m, 0.4H), 6.43 (s, 1H), 6.18 - 6.09 (m, 1H), 4.72 - 4.57 (m, 1H), 4.24 - 3.93 (m, 2H), 3.77 - 3.48 (m, 4H), 3.46 - 3.39 (m, 3H), 2.93 (t, *J* = 5.6 Hz, 2H), 2.42 - 2.33 (m, 2H), 2.06 - 1.33 (m, 10H). | 567.0 |
| 103 | 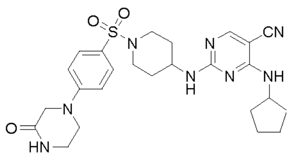 | $^1$H NMR (400 MHz, DMSO-$d_6$) δ 8.22 - 8.08 (m, 2H), 7.60 (d, *J* = 7.3 Hz, 0.6H), 7.54 (d, *J* = 8.8 Hz, 2H), 7.43 (d, *J* = 7.8 Hz, 0.4H), 7.23 (d, *J* = 6.7 Hz, 0.6H), 7.13 (d, *J* = 7.2 Hz, 0.4H), 7.02 (d, *J* = 9.0 Hz, 2H), 4.44 - 4.14 (m, 1H), 3.87 (s, 2H), 3.73 - 3.60 (m, 1H), 3.59 - 3.53 (m, 2H), 3.52 - 3.39 (m, 2H), 2.45 - 2.24 (m, 2H), 1.96 - 1.74 (m, 5H), 1.72 - 1.37 (m, 9H). | 525.0 |
| 104 | 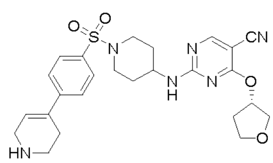 | $^1$H NMR (400 MHz, DMSO-$d_6$) δ 8.48 (s, 0.3H), 8.45 (s, 0.6H), 8.32 (d, *J* = 6.8 Hz, 0.6H), 8.15 (d, *J* = 8.4 Hz, 0.3H), 7.69 (br.s., 3H), 6.44 (br.s., 1H), 6.08 (br.s., 1H), 5.58 - 5.47 (m, 1H), 4.09 - 3.98 (m, 1H), 3.93 - 3.66 (m, 5H), 3.63 - 3.52 (m, 2H), 2.98 - 2.86 (m, 2H), 2.41 - 2.34 (m, 2H), 2.31 - 2.14 (m, 3H), 2.04 - 1.82 (m, 5H), 1.63 - 1.45 (s, 2H). | 510.9 |

(continued)

| Compound | Structure | 1HNMR | MS (ESI) |
|---|---|---|---|
| 105 | 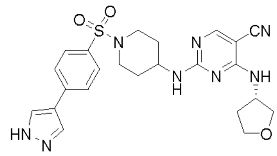 | $^1$H NMR (400 MHz, DMSO-$d_6$) δ 13.13 (s, 1H), 8.45 - 8.02 (m, 3H), 7.87 (d, *J* = 8.3 Hz, 2H), 7.76 - 7.63 (m, 2.5H), 7.50 (d, *J* = 5.6 Hz, 1H), 7.39 (d, *J* = 6.6 Hz, 0.4H), 4.58 - 4.50 (m, 0.4H), 4.47 - 4.37 (m, 0.6H), 3.96 - 3.44 (m, 7H), 2.45 - 2.37 (m, 1H), 2.20 - 1.79 (m, 5H), 1.64 - 1.45 (m, 2H). | 494.9 |
| 106 | 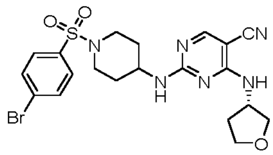 | $^1$H NMR (400 MHz, DMSO-$d_6$) δ 8.18 (s, 0.3H), 8.14 (s, 0.6H), 7.86 (d, *J* = 8.5 Hz, 2H), 7.74 - 7.63 (m, 2.5H), 7.54 - 7.47 (m, 1H), 7.40 (d, *J* = 6.5 Hz, 0.3H), 4.59 - 4.38 (m, 1H), 3.96 - 3.70 (m, 3H), 3.70 - 3.62 (m, 1H), 3.62 - 3.44 (m, 3H), 2.65 - 2.52 (m, 1H), 2.46 - 2.38 (m, 1H), 2.17 - 1.80 (m, 4H), 1.63 - 1.45 (m, 2H). | 508.8 |
| 107 | 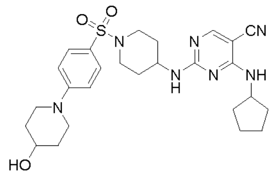 | $^1$H NMR (400 MHz, DMSO-$d_6$) δ 8.13 (s, 0.4H), 8.10 (s, 0.6H), 7.60 (d, *J* = 7.1 Hz, 0.6H), 7.48 (d, *J* = 8.8 Hz, 2H), 7.42 (d, *J* = 7.6 Hz, 0.4H), 7.24 (d, *J* = 6.3 Hz, 0.6H), 7.13 (d, *J* = 7.5 Hz, 0.4H), 7.05 (d, *J* = 9.1 Hz, 2H), 4.73 (d, *J* = 4.1 Hz, 1H), 4.43 - 4.31 (m, 0.4H), 4.26 - 4.14 (m, 0.6H), 3.79 - 3.57 (m, 4H), 3.55 - 3.39 (m, 2H), 3.12 - 2.99 (m, 2H), 2.43 - 2.23 (m, 2H), 1.96 - 1.74 (m, 6H), 1.73 - 1.34 (m, 10H). | 526.0 |
| 108 | 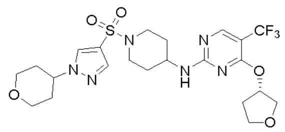 | $^1$H NMR (400 MHz, DMSO-$d_6$) δ 8.42 (s, 0.5H), 8.40 (s, 0.4H), 8.30 (s, 1H), 8.04 (d, *J* = 7.6 Hz, 0.5H), 7.87 (d, *J* = 7.6 Hz, 0.4H), 7.84 (s, 0.5H), 7.82 (s, 0.4H), 5.61 - 5.51 (m, 1H), 4.56 - 4.44 (m, 1H), 4.04 - 3.85 (m, 3H), 3.83 - 3.66 (m, 4H), 3.55 - 3.41 (m, 5H), 2.43 - 2.34 (m, 1H), 2.26 - 2.14 (m, 1H), 2.08 - 1.86 (m, 7H), 1.67 - 1.52 (m, 2H). | 546.9 |
| 109 | 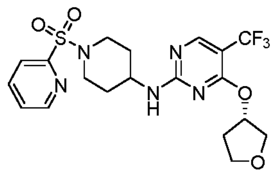 | $^1$H NMR (400 MHz, DMSO-$d_6$) δ 8.78 (d, *J* = 4.6 Hz, 1H), 8.31 (s, 0.4H), 8.30 (s, 0.6H), 8.12 (t, *J* = 7.8 Hz, 1H), 8.03 (d, *J* = 7.4 Hz, 0.6H), 7.96 - 7.90 (m, 1H), 7.87 (d, *J* = 7.8 Hz, 0.5H), 7.76 - 7.68 (m, 1H), 5.61 - 5.50 (s, 1H), 3.97 - 3.65 (m, 7H), 2.93 - 2.77 (m, 2H), 2.27 - 2.13 (m, 1H), 2.04 - 1.82 (m, 3H), 1.62 - 1.44 (m, 2H). | 473.8 |
| 110 | 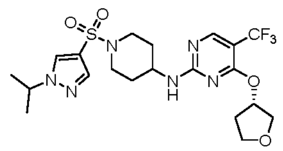 | $^1$H NMR (400 MHz, DMSO-$d_6$) δ 8.38 (d, *J* = 6.8 Hz, 1H), 8.30 (d, *J* = 2.0 Hz, 1H), 8.04 (d, *J* = 7.2 Hz, 0.6H), 7.87 (d, *J* = 7.2 Hz, 0.4H), 7.80 (d, *J* = 7.2 Hz, 1H), 5.62 - 5.52 (m, 1H), 4.66 - 4.53 (m, 1H), 3.97 - 3.66 (m, 5H), 3.58 - 3.43 (m, 2H), 2.42 - 2.35 (m, 2H), 2.26 - 2.15 (m, 1H), 2.02 - 1.89 (m, 3H), 1.66 - 1.54 (m, 2H), 1.44 (d, *J* = 6.4 Hz, 6H). | 505.0 |

(continued)

| Compound | Structure | 1HNMR | MS (ESI) |
|---|---|---|---|
| 111 | 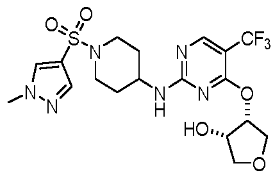 Relative configuration (cis) | $^1$H NMR (400 MHz, DMSO-$d_6$) δ 8.33 (s, 0.5H), 8.32 (s, 0.5H), 8.30 (s, 0.4H), 8.29 (s, 0.6H), 7.96 (d, *J* = 7.4 Hz, 0.5H), 7.81 (d, *J* = 7.8 Hz, 0.5H), 7.79 (s, 0.5H), 7.77 (s, 0.5H), 5.46 - 5.38 (m, 1H), 5.07 (d, *J* = 5.0 Hz, 0.4H), 5.02 (d, *J* = 5.2 Hz, 0.6H), 4.46 - 4.38 (m, 0.4H), 4.38 - 4.30 (m, 0.6H), 4.09 - 3.96 (m, 1H), 3.91 (s, 3H), 3.90 - 3.83 (m, 1H), 3.82 - 3.67 (m, 2H), 3.58 - 3.40 (m, 3H), 2.47 - 2.35 (m, 2H), 2.04 - 1.87 (m, 2H), 1.66 - 1.51 (m, 2H). | 492.8 |
| 118 | 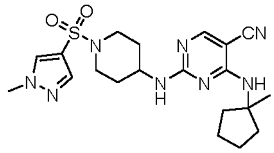 | $^1$H NMR (400 MHz, DMSO-$d_6$) δ 8.32 (s, 0.6H), 8.31 (s, 0.4H), 8.24 (s, 0.4H), 8.19 (s, 0.6H), 7.78 (s, 0.6H), 7.76 (s, 0.4H), 7.68 (d, *J* = 7.3 Hz, 0.6H), 7.49 (d, *J* = 7.8 Hz, 0.4H), 4.95 - 4.78 (m, 1H), 4.47 - 4.33 (m, 1H), 3.91 (s, 3H), 3.80 - 3.59 (m, 1H), 3.52 - 3.36 (m, 2H), 3.03 (t, *J* = 12.8 Hz, 1H), 2.48 - 2.34 (m, 2H), 2.01 - 1.80 (m, 2H), 1.76 - 1.48 (m, 7H), 1.48 - 1.32 (m, 1H), 1.21 (d, *J* = 6.8 Hz, 3H). | 445.0 |
| 119 | 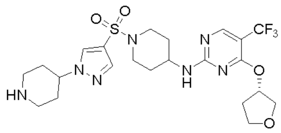 | $^1$H NMR (400 MHz, DMSO-$d_6$) δ 8.37 (s, 0.5H), 8.36 (s, 0.4H), 8.30 (s, 1H), 8.04 (d, *J* = 7.3 Hz, 0.5H), 7.87 (d, *J* = 7.6 Hz, 0.4H), 7.82 (s, 0.5H), 7.80 (s, 0.5H), 5.63 - 5.51 (m, 1H), 4.35 - 4.22 (m, 1H), 3.96 - 3.84 (m, 1H), 3.83 - 3.67 (m, 4H), 3.55 - 3.42 (m, 2H), 3.03 (d, *J* = 12.5 Hz, 2H), 2.62 - 2.52 (m, 3H), 2.47 - 2.31 (m, 2H), 2.28 - 2.13 (m, 1H), 2.03 - 1.87 (m, 5H), 1.87 - 1.73 (m, 2H), 1.66 - 1.52 (m, 2H). | 545.8 |
| 120 | 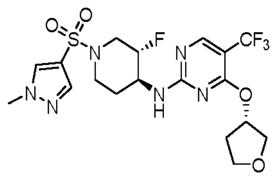 | $^1$H NMR (400 MHz, DMSO-$d_6$) δ 8.39 (s, 0.5H), 8.38 (s, 0.4H), 8.33 (s, 1H), 8.20 (d, *J* = 7.8 Hz, 0.5H), 8.05 (d, *J* = 8.2 Hz, 0.4H), 7.84 (s, 0.5H), 7.82 (s, 0.4H), 5.64 - 5.52 (m, 1H), 4.80 - 4.58 (m, 1H), 4.09 (br.s., 1H), 3.98- 3.86 (m, 4H), 3.83 - 3.70 (m, 3H), 3.70- 3.47 (m, 1H), 3.42 - 3.33 (m, 1H), 2.87 - 2.60 (m, 2H), 2.29 - 2.16 (m, 1H), 2.10 - 1.92 (m, 2H), 1.69 - 1.57 (m, 1H). | 494.8 |
| 121 | 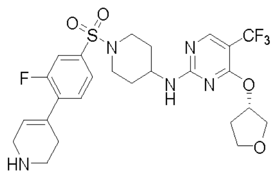 | $^1$H NMR (400 MHz, DMSO-$d_6$) δ 8.29 (s, 1H), 8.02 (d, *J* = 7.3 Hz, 0.6H), 7.86 (d, *J* = 7.5 Hz, 0.4H), 7.61 (t, *J* = 7.3 Hz, 1H), 7.58 - 7.50 (m, 2H), 6.21 - 6.13 (m, 1H), 5.60 - 5.55 (m, 1H), 3.96 - 3.86 (m, 1H), 3.85 - 3.68 (m, 4H), 3.64 - 3.51 (m, 2H), 3.44 - 3.36 ( m, 2H), 2.90 (t, *J* = 5.5 Hz, 2H), 2.69 - 2.53 (m, 3H), 2.38 - 2.30 (m, 2H), 2.26 - 2.13 (m, 1H), 2.01 - 1.85 (m, 3H), 1.64 - 1.50 (m, 2H). | 571.8 |

(continued)

| Compound | Structure | 1HNMR | MS (ESI) |
|---|---|---|---|
| 122 | 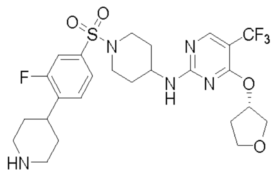 | $^1$H NMR (400 MHz, DMSO-$d_6$) δ 8.29 (s, 1H), 8.02 (d, $J$ = 7.3 Hz, 0.5H), 7.86 (d, $J$ = 7.7 Hz, 0.4H), 7.66 - 7.47 (m, 3H), 5.60 - 5.51 (m, 1H), 3.96 - 3.85 (m, 1H), 3.85 - 3.67 (m, 4H), 3.63 - 3.49 (m, 2H), 3.09 - 2.89 (m, 3H), 2.68 - 2.57 (m, 4H), 2.25 - 2.11 (m, 1H), 2.03 - 1.84 (m, 3H), 1.75 - 1.45 (m, 6H). | 573.8 |
| 123 | 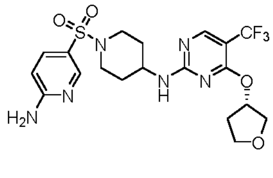 | $^1$H NMR (400 MHz, DMSO-$d_6$) δ 8.32 - 8.26 (m, 1H), 8.23 - 8.18 (m, 1H), 8.02 (d, $J$ = 7.4 Hz, 0.6H), 7.87 (d, $J$ = 7.6 Hz, 0.4H), 7.65 - 7.58 (m, 1H), 6.96 (s, 2H), 6.53 (d, $J$ = 8.8 Hz, 1H), 5.61 - 5.51 (m, 1H), 3.95 - 3.85 (m, 1H), 3.84 - 3.66 (m, 4H), 3.53 - 3.42 (m, 2H), 2.47 - 2.38 (m, 2H), 2.26 - 2.13 (m, 1H), 2.01 - 1.84 (m, 3H), 1.63 - 1.47 (m, 2H). | 488.8 |
| 124 | 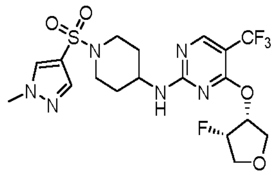 | $^1$H NMR (400 MHz, DMSO-$d_6$) δ 8.38 - 8.30 (m, 2H), 8.15 (d, $J$ = 7.4 Hz, 0.6H), 8.00 (d, $J$ = 7.7 Hz, 0.4H), 7.80 - 7.75 (m, 1H), 5.66 - 5.50 (m, 1H), 5.41 (d, $J$ = 3.0 Hz, 0.5H), 5.28 (d, $J$ = 3.0 Hz, 0.5H), 4.25 - 4.15 (m, 1H), 4.04 - 3.83 (m, 5H), 3.82 - 3.71 (m, 2H), 3.55 - 3.42 (s, 2H), 2.46 - 2.35 (m, 2H), 2.03 - 1.87 (m, 2H), 1.69 - 1.52 (m, 2H). | 494.8 |
| 125 | 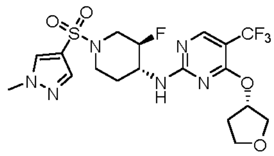 | $^1$H NMR (400 MHz, DMSO-$d_6$) δ 8.42 - 8.36 (m, 1H), 8.35 - 8.30 (m, 1H), 8.20 (d, $J$ = 7.6 Hz, 0.5H), 8.05 (d, $J$ = 7.2 Hz, 0.4H), 7.86 - 7.81 (m, 1H), 5.64 - 5.53 (m, 1H), 4.78 - 4.70 (m, 0.5H), 4.67 - 4.57 (m, 0.5H), 4.22 - 4.02 (m, 1H), 4.01 - 3.87 (m, 4H), 3.83 - 3.69 (m, 3H), 3.67 - 3.47 (m, 2H), 2.89 - 2.72 (m, 2H), 2.28 - 2.16 (m, 1H), 2.10 - 1.93 (m, 2H), 1.73 - 1.57 (m, 1H). | 494.9 |
| 126 | 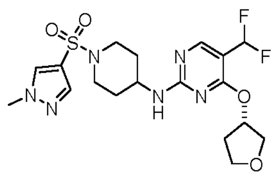 | $^1$H NMR (400 MHz, DMSO-$d_6$) δ 8.33 (s, 1H), 8.20 (s, 1H), 7.78 (s, 1H), 7.75 (d, $J$ = 6.4Hz, 0.5H), 7.58 (d, $J$ = 7.2Hz, 0.5H), 6.83 (t, $J$ = 55.1 Hz, 1H), 5.51 (s, 1H), 3.98 - 3.87 (m, 4H), 3.86 - 3.66 (m, 4H), 3.53 - 3.42 (m, 2H), 2.44 - 2.34 (m, 2H), 2.28 - 2.13 (m, 1H), 2.04 - 1.87 (m, 3H), 1.66 - 1.50 (m, 2H). | 458.9 |
| 127 | 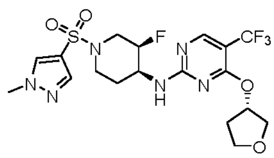 | $^1$H NMR (400 MHz, DMSO-$d_6$) δ 8.35 (d, $J$ = 4.4 Hz, 1H), 8.32 (d, $J$ = 3.1 Hz, 1H), 8.13 (d, $J$ = 7.2 Hz, 0.6H), 8.01 (d, $J$ = 7.4 Hz, 0.4H), 7.81 (s, 0.6H), 7.78 (s, 0.4H), 5.59 (br.s., 1H), 5.00 (d, $J$ = 18.7 Hz, 0.5H), 4.87 (d, $J$ = 19.2 Hz, 0.5H), 4.11 - 3.87 (m, 5H), 3.87 - 3.69 (m, 4H), 3.67 - 3.55 (m, 1H), 2.84 - 2.56 (m, 2H), 2.30 - 2.11 (m, 1H), 2.04 - 1.89 (m, 2H), 1.82 - 1.71 (m, 1H). | 494.8 |

(continued)

| Compound | Structure | ¹HNMR | MS (ESI) |
|---|---|---|---|
| 128 | 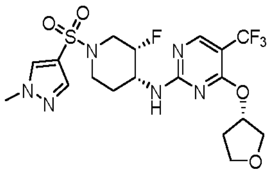 | $^1$H NMR (400 MHz, DMSO-$d_6$) δ 8.35 - 8.22 (m, 2H), 8.03 (d, *J* = 7.5 Hz, 0.6H), 7.88 (d, *J* = 7.5 Hz, 0.4H), 7.75 (s, 0.4H), 7.74 (s, 0.6H), 7.20 (br.s., 1H), 5.62 - 5.49 (m, 1H), 4.77 (s, 2H), 3.97 - 3.83 (m, 1H), 3.84 - 3.66 (m, 4H), 3.51 (d, *J* = 8.4 Hz, 2H), 2.46- 2.35 (m, 1H), 2.28 - 2.14 (m, 1H), 2.04 - 1.84 (m, 3H), 1.68 - 1.51 (m, 2H). | 494.8 |
| 129 | 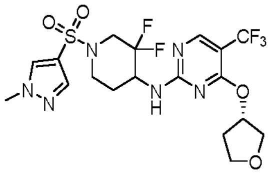 | $^1$H NMR (400 MHz, DMSO-$d_6$) δ 8.43 - 8.25 (m, 2.5H), 8.15 (d, *J* = 9.5 Hz, 0.5H), 7.88 (s, 0.6H), 7.84 (s, 0.4H), 5.62 (s, 1H), 4.76 - 4.48 (m, 1H), 4.00 - 3.85 (m, 4H), 3.84 - 3.67 (m, 4H), 3.66 - 3.44 (m, 1H), 3.15 - 2.95 (m, 1H), 2.84 - 2.69 (m, 1H), 2.30 - 2.13 (m, 1H), 2.04 - 1.78 (m, 3H). | 512.8 |
| 130 | 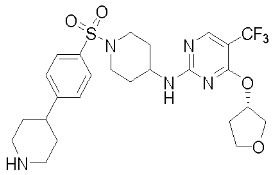 | $^1$H NMR (400 MHz, DMSO-$d_6$) δ 8.29 (s, 1H), 8.02 (d, *J* = 7.2 Hz, 0.6H), 7.86 (d, *J* = 7.1 Hz, 0.4H), 7.72 - 7.60 (m, 2H), 7.50 (d, *J* = 8.3 Hz, 2H), 5.54 (br.s., 1H), 3.96 - 3.82 (m, 1H), 3.82 - 3.67 (m, 4H), 3.62 - 3.48 (m, 2H), 3.02 (d, *J* = 12.0 Hz, 2H), 2.76 - 2.65 (m, 1H), 2.64 - 2.54 (m, 3H), 2.45 - 2.35 (m, 1H), 2.27 - 2.11 (m, 1H), 2.03 - 1.84 (m, 3H), 1.77 - 1.64 (m, 2H), 1.64 - 1.45 (m, 4H). | 556.0 |
| 131 | 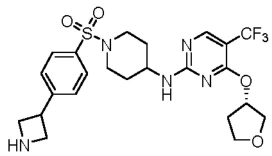 | $^1$H NMR (400 MHz, DMSO-$d_6$) δ 8.35 (br.s., 1H), 8.29 (s, 1H), 8.02 (d, *J* = 7.1 Hz, 0.6H), 7.87 (d, *J* = 7.0 Hz, 0.4H), 7.78 - 7.68 (m, 2H), 7.68 - 7.59 (m, 2H), 5.59 - 5.49 (m, 1H), 4.12 - 3.95 (m, 3H), 3.94 - 3.66 (m, 7H), 3.63 - 3.49 (m, 3H), 2.46 - 2.36 (m, 1H), 2.27 - 2.11 (m, 1H), 2.01 - 1.84 (m, 3H), 1.64 - 1.49 (m, 2H). | 527.8 |
| 132 | 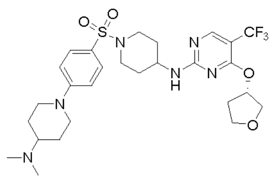 | $^1$H NMR (400 MHz, DMSO-$d_6$) δ 8.29 (s, 1H), 8.01 (d, *J* = 7.3 Hz, 0.6H), 7.86 (d, *J* = 7.4 Hz, 0.4H), 7.50 (d, *J* = 8.9 Hz, 2H), 7.06 (d, *J* = 9.1 Hz, 2H), 5.60 - 5.50 (m, 1H), 3.97 - 3.83 (m, 3H), 3.81 - 3.64 (m, 4H), 3.55 - 3.42 (m, 2H), 2.85 (t, *J* = 11.5 Hz, 2H), 2.45 - 2.39 (m, 1H), 2.39 - 2.22 (m, 2H), 2.22 - 2.09 (m, 7H), 2.03 - 1.78 (m, 5H), 1.63 - 1.48 (m, 2H), 1.48 - 1.33 (m, 2H). | 599.0 |
| 133 | 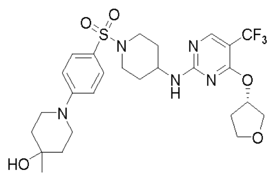 | $^1$H NMR (400 MHz, DMSO-$d_6$) δ 8.29 (s, 1H), 8.01 (d, *J* = 7.3 Hz, 0.6H), 7.86 (d, *J* = 7.7 Hz, 0.4H), 7.49 (d, *J* = 8.9 Hz, 2H), 7.05 (d, *J* = 9.1 Hz, 2H), 5.62 - 5.49 (m, 1H), 4.39 (s, 1H), 3.96 - 3.84 (m, 1H), 3.82 - 3.63 (m, 4H), 3.59 - 3.43 (m, 4H), 3.30 - 3.18 (m, 2H), 2.47 - 2.36 (m, 2H), 2.27 - 2.10 (m, 1H), 2.04 - 1.83 (m, 3H), 1.63 - 1.45 (m, 6H), 1.15 (s, 3H). | 586.0 |

(continued)

| Compound | Structure | 1HNMR | MS (ESI) |
|---|---|---|---|
| 134 | 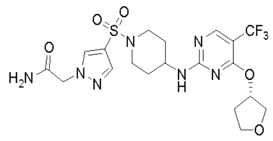 | $^1$H NMR (400 MHz, DMSO-$d_6$) δ 8.35 - 8.22 (m, 2H), 8.03 (d, *J* = 7.5 Hz, 0.6H), 7.88 (d, *J* = 7.6 Hz, 0.4H), 7.74 (d, *J* = 5.9 Hz, 1H), 7.19 (br.s., 2H), 5.57 (d, *J* = 5.1 Hz, 1H), 4.77 (s, 2H), 3.95-3.84 (m, 1H), 3.84 - 3.67 (m, 4H), 3.51 (d, *J* = 9.7 Hz, 2H), 2.48 - 2.35 (m, 2H), 2.29 - 2.13 (m, 1H), 2.03 - 1.86 (m, 3H), 1.69 - 1.49 (m, 2H). | 520.8 |
| 135 | 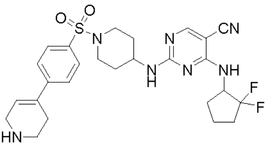 | $^1$H NMR (400 MHz, DMSO-$d_6$) δ 8.22 (s, 0.3H), 8.18 (s, 0.6H), 7.74 (d, *J* = 7.2 Hz, 0.6H), 7.67 (d, *J* = 4.0 Hz, 4H), 7.56 (d, *J* = 7.9 Hz, 0.4H), 7.42 (d, *J* = 7.8 Hz, 0.7H), 7.32 (d, *J* = 8.7 Hz, 0.4H), 6.43 (br.s., 1H), 4.95 - 4.82 (m, 0.4H), 4.71 - 4.62 (m, 0.6H), 3.80 - 3.63 (m, 1H), 3.62 - 3.45 (m, 2H), 3.44 - 3.37 (m, 2H), 2.92 (t, *J* = 5.5 Hz, 2H), 2.46 - 2.34 (m, 4H), 2.19 - 1.68 (m, 8H), 1.66 - 1.40 (m, 3H). | 544.2 |
| 136 | 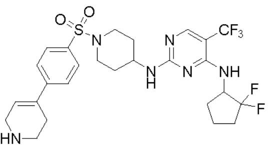 | $^1$H NMR (400 MHz, DMSO-$d_6$) δ 8.04 (br.s., 1H), 7.68 (s, 4H), 7.48 (d, *J* = 7.2 Hz, 0.6H), 7.31 (d, *J* = 7.5 Hz, 0.4H), 6.43 (s, 1H), 6.29 (d, *J* = 8.3 Hz, 0.6H), 6.16 (d, *J* = 8.6 Hz, 0.4H), 5.08 - 4.89 (m, 0.4H), 4.85 - 4.69 (m, 0.7H), 3.69 (br.s., 1H), 3.61 - 3.44 (m, 2H), 3.41 (d, *J* = 2.9 Hz, 2H), 2.93 (t, *J* = 5.6 Hz, 2H), 2.47 - 2.34 (m, 4H), 2.19 - 1.98 (m, 3H), 1.98 - 1.81 (m, 3H), 1.80 - 1.66 (m, 1H), 1.66 - 1.40 (m, 3H). | 587.0 |
| 137 | 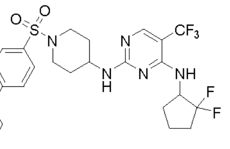 | $^1$H NMR (400 MHz, DMSO-$d_6$) δ 8.04 (s, 1H), 7.67 (d, *J* = 8.0 Hz, 2H), 7.50 (d, *J* = 8.2 Hz, 2.7H), 7.30 (s, 0.4H), 6.30 (d, *J* = 6.8 Hz, 0.6H), 6.18 (s, 0.4H), 5.05 - 4.89 (m, 0.4H), 4.84 - 4.67 (m, 0.6H), 3.70 (br.s., 1H), 3.59 - 3.43 (m, 2H), 3.02 (d, *J* = 12.1 Hz, 2H), 2.75 - 2.68 (m, 1H), 2.64 - 2.54 (m, 2H), 2.44 - 2.35 (m, 2H), 2.16 - 1.98 (m, 3H), 1.97 - 1.82 (m, 3H), 1.80 - 1.65 (m, 3H), 1.65 - 1.42 (m, 5H). | 589.1 |
| 138 | 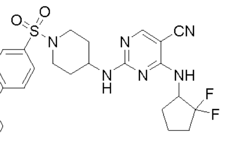 | $^1$H NMR (400 MHz, DMSO-$d_6$) δ 8.22 (s, 0.3H), 8.18 (s, 0.6H), 7.75 (d, *J* = 7.3 Hz, 0.6H), 7.66 (t, *J* = 6.7 Hz, 2H), 7.56 (d, *J* = 7.7 Hz, 0.4H), 7.50 (d, *J* = 8.2 Hz, 2H), 7.42 (d, *J* = 7.9 Hz, 0.7H), 7.32 (d, *J* = 8.8 Hz, 0.4H), 4.93 - 4.82 (m, 0.4H), 4.74 - 4.57 (m, 0.6H), 3.70 (br.s., 1H), 3.62 - 3.43 (m, 2H), 3.02 (d, *J* = 12.4 Hz, 2H), 2.75 - 2.68 (m, 1H), 2.64 - 2.54 (m, 2H), 2.45 - 2.35 (m, 2H), 2.20 - 1.81 (m, 7H), 1.79 - 1.65 (m, 3H), 1.65 - 1.40 (m, 5H). | 546.2 |

(continued)

| Compound | Structure | 1HNMR | MS (ESI) |
|---|---|---|---|
| 139 | 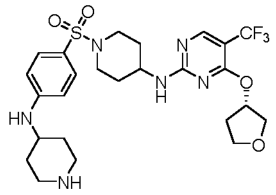 | $^1$H NMR (400 MHz, DMSO-$d_6$) δ 8.29 (s, 1H), 8.01 (d, *J* = 7.7 Hz, 0.6H), 7.86 (d, *J* = 7.5 Hz, 0.4H), 7.39 (d, *J* = 8.7 Hz, 2H), 6.68 (d, *J* = 8.9 Hz, 2H), 6.53 (d, *J* = 7.6 Hz, 1H), 5.55 (br.s., 1H), 3.94 - 3.82 (m, 1H), 3.82 - 3.61 (m, 4H), 3.47 (d, *J* = 9.4 Hz, 2H), 2.95 (d, *J* = 12.6 Hz, 2H), 2.59 - 2.52 (m, 5H), 2.45 - 2.34 (m, 2H), 2.24 - 2.12 (m, 1H), 2.02 - 1.78 (m, 5H), 1.62 - 1.42 (m, 2H), 1.31 - 1.25 (m, 1H). | 573.0 |
| 140 | 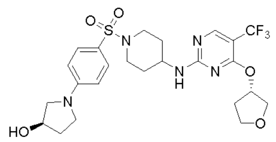 | $^1$H NMR (400 MHz, DMSO-$d_6$) δ 8.28 (s, 1H), 8.00 (d, *J* = 7.1 Hz, 0.7H), 7.85 (d, *J* = 7.2 Hz, 0.4H), 7.49 (d, *J* = 8.8 Hz, 2H), 6.63 (d, *J* = 9.0 Hz, 2H), 5.54 (br.s., 1H), 5.03 (d, *J* = 3.6 Hz, 1H), 4.42 (br.s., 1H), 3.95 - 3.83 (m, 1H), 3.80 - 3.61 (m, 4H), 3.54 - 3.41 (m, 3H), 3.41 - 3.34 (m, 2H), 3.16 (d, *J* = 10.9 Hz, 1H), 2.44 - 2.35 (m, 2H), 2.24 - 2.12 (m, 1H), 2.10 - 1.83 (m, 5H), 1.63 - 1.47 (m, 2H). | 558.0 |
| 141 | 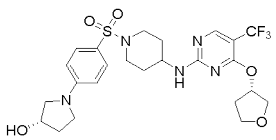 | $^1$H NMR (400 MHz, DMSO-$d_6$) δ 8.28 (s, 1H), 8.00 (d, *J* = 7.3 Hz, 0.6H), 7.85 (d, *J* = 6.9 Hz, 0.4H), 7.49 (d, *J* = 8.7 Hz, 2H), 6.63 (d, *J* = 8.9 Hz, 2H), 5.54 (br.s., 1H), 5.03 (d, *J* = 3.6 Hz, 1H), 4.42 (br.s., 1H), 3.96 - 3.81 (m, 1H), 3.80 - 3.61 (m, 4H), 3.55 - 3.42 (m, 3H), 3.42 - 3.35 (m, 2H), 3.16 (d, *J* = 10.4 Hz, 1H), 2.46 - 2.35 (m, 2H), 2.26 - 2.12 (m, 1H), 2.12 - 1.83 (m, 5H), 1.63 - 1.49 (m, 2H). | 558.0 |
| 142 | 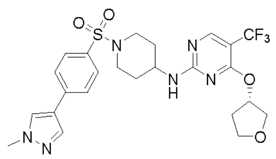 | $^1$H NMR (400 MHz, DMSO-$d_6$) δ 8.32 (s, 1H), 5.28 (s, 1H), 8.04 - 7.96 (m, 1.5H), 7.86 (d, *J* = 7.3 Hz, 0.4H), 7.82 (d, *J* = 8.4 Hz, 2H), 7.73-7.67 (m, 2H), 5.60 - 5.49 (d, *J* = 5.2 Hz, 1H), 3.96 - 3.83 (m, 4H), 3.82 - 3.65 (m, 4H), 3.58 (br.s., 2H), 2.59 - 2.52 (m, 2H), 2.24 - 2.11 (m, 1H), 2.02 - 1.85 (m, 3H), 1.64 - 1.47 (m, 2H). | 553.0 |
| 143 | 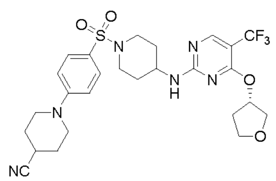 | $^1$H NMR (400 MHz, DMSO-$d_6$) δ 8.29 (s, 1H), 8.01 (d, *J* = 7.3 Hz, 0.6H), 7.85 (d, *J* = 8.0 Hz, 0.4H), 7.52 (d, *J* = 8.8 Hz, 2H), 7.09 (d, *J* = 9.1 Hz, 2H), 5.59 - 5.49 (m, 1H), 3.95 - 3.84 (m, 1H), 3.81 - 3.64 (m, 4H), 3.64 - 3.45 (m, 4H), 3.30 - 3.20 (m, 2H), 3.15 - 3.08 (m, 1H), 2.43 - 2.35 (m, 1H), 2.24 - 2.14 (m, 1H), 2.04 - 1.84 (m, 6H), 1.84 - 1.72 (m, 2H), 1.63 - 1.48 (m, 2H). | 581.0 |
| 144 | 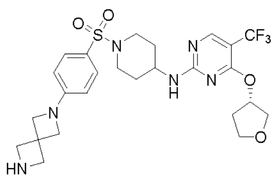 | $^1$H NMR (400 MHz, DMSO-$d_6$) δ 8.39 (s, 1H), 8.27 (s, 1H), 7.49 (d, *J* = 8.5 Hz, 2H), 6.52 (d, *J* = 8.6 Hz, 2H), 5.54 (d, *J* = 15.2 Hz, 1H), 4.07 (s, 4H), 4.00 (s, 4H), 3.93 - 3.80 (m, 1H), 3.80 -3.61 (m, 5H), 2.43 - 2.34 (m, 2H), 2.31 - 2.12 (m, 2H), 2.01 - 1.83 (m, 3H), 1.62 - 1.46 (m, 2H). | 569.1 |

(continued)

| Compound | Structure | 1HNMR | MS (ESI) |
|---|---|---|---|
| 145 | 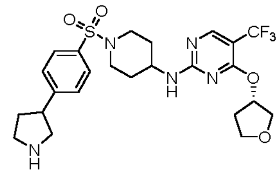 | $^1$H NMR (400 MHz, DMSO-$d_6$) δ 8.29 (s, 1H), 8.02 (d, *J* = 7.3 Hz, 0.6H), 7.86 (d, *J* = 7.6 Hz, 0.4H), 7.74 - 7.62 (m, 2H), 7.60 - 7.49 (m, 2H), 5.60 - 5.50 (m, 1H), 3.96 - 3.83 (m, 1H), 3.82 - 3.66 (m, 4H), 3.62 - 3.50 (m, 2H), 3.27 - 3.15 (m, 2H), 3.04 - 2.85 (m, 2H), 2.70 - 2.60 (m, 1H), 2.58 - 2.53 (m, 1H), 2.47 - 2.35 (m, 1H), 2.25 - 2.11 (m, 2H), 2.02 - 1.84 (m, 3H), 1.76 - 7.63 (m, 1H), 1.63 - 1.49 (m, 2H). | 542.0 |
| 146 | 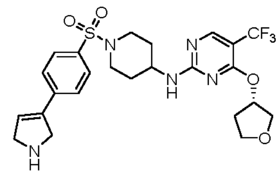 | $^1$H NMR (400 MHz, DMSO-$d_6$) δ 8.28 (s, 1H), 8.01 (d, *J* = 7.4 Hz, 0.6H), 7.86 (d, *J* = 7.4 Hz, 0.4H), 7.76 - 7.62 (m, 4H), 6.65 (s, 1H), 5.61 - 5.49 (m, 1H), 4.47 (s, 0.4H), 4.27 (s, 0.4H), 3.99 (s, 1.6H), 3.94 - 3.80 (m, 3H), 3.80- 3.66 (m, 4H), 3.64 - 3.49 (m, 2.5H), 2.53 - 2.51 (m, 2H), 2.23 - 2.12 (m, 1H), 2.00 - 1.85 (m, 3H), 1.62 - 1.49 (m, 2H). | 540.0 |
| 147 | 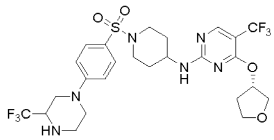 | $^1$H NMR (400 MHz, DMSO-$d_6$) δ 8.29 (s, 1H), 8.00 (d, *J* = 7.1 Hz, 0.6H), 7.85 (d, *J* = 7.6 Hz, 0.4H), 7.54 (d, *J* = 8.9 Hz, 2H), 7.12 (d, *J* = 9.1 Hz, 2H), 5.61 - 5.50 (m, 1H), 3.96 - 3.61 (m, 7H), 3.51 (br.s., 3H), 3.08 - 2.87 (m, 4H), 2.85 - 2.75 (m, 1H), 2.46 -2.34 (m, 2H), 2.29 - 2.11 (m, 1H), 2.03 - 1.83 (m, 3H), 1.64 - 1.47 (m, 2H). | 625.0 |
| 148 | 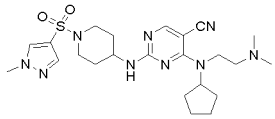 | $^1$H NMR (400 MHz, DMSO-$d_6$) δ 8.34 (s, 0.8H), 8.31 (s, 0.2H), 8.23 (s, 0.2H), 8.17 (s, 0.8H), 7.76 (s, 1H), 7.72 (d, *J* = 7.6 Hz, 0.8H), 7.41 (d, *J* = 7.9 Hz, 0.2H), 4.86 - 4.77 (m, 0.2H), 4.74 - 4.63 (m, 0.9H), 3.90 (s, 3H), 3.79 - 3.61 (m, 1H), 3.58 - 3.40 (m, 4H), 2.38 (br.s., 2H), 2.31 - 2.14 (m, 3H), 2.05 (s, 5H), 1.96 - 1.79 (m, 4H), 1.76 - 1.44 (m, 8H). | 502.2 |
| 149 | 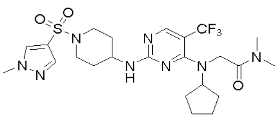 | $^1$H NMR (400 MHz, DMSO-$d_6$) δ 8.38 (s, 1H), 8.13 (s, 1H), 7.82 (s, 1H), 7.37 (d, *J* = 7.3 Hz, 1H), 4.33 - 4.23 (m, 1H), 4.15 (s, 2H), 3.92 (s, 3H), 3.42 (d, *J* = 11.9 Hz, 3H), 2.87 (s, 3H), 2.70 - 2.62 (m, 3H), 2.31 - 2.21 (m, 2H), 1.86 - 1.70 (m, 4H), 1.68 - 1.60 (m, 2H), 1.59- 1.37 (m, 6H). | 559.2 |
| 150 | 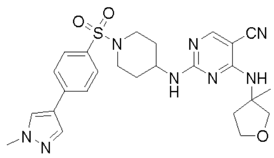 | $^1$H NMR (400 MHz, DMSO-$d_6$) δ 8.32 (s, 1H), 8.17 - 8.11 (m, 1H), 8.00 (s, 1H), 7.81 (d, *J* = 8.4 Hz, 2H), 7.75 - 7.65 (m, 3H), 7.13 (s, 0.8H), 7.02 (s, 0.3H), 3.89 (s, 3H), 3.87 - 3.73 (m, 2H), 3.72 - 3.58 (m, 4H), 3.57 - 3.50 (m, 1H), 2.41 - 2.35 (m, 2H), 1.94 - 1.82 (m, 3H), 1.63 - 1.45 (m, 3H), 1.44 (s, 1H), 1.39 (s, 2H). | 523.0 |

(continued)

| Compound | Structure | $^1$HNMR | MS (ESI) |
|---|---|---|---|
| 151 | 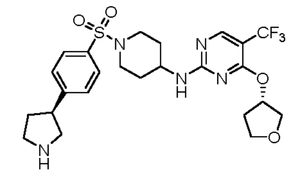 Enantiomer of 152, absolute configuration unknown | $^1$H NMR (400 MHz, DMSO-$d_6$) δ 8.29 (s, 1H), 8.02 (d, *J* = 7.2 Hz, 0.6H), 7.86 (d, *J* = 7.0 Hz, 0.4H), 7.65 (t, *J* = 6.8 Hz, 2H), 7.53 (d, *J* = 8.3 Hz, 2H), 5.61 - 5.52 (m, 1H), 3.96 - 3.83 (m, 1H), 3.83 - 3.67 (m, 4H), 3.62 - 3.47 (m, 2H), 3.26 - 3.17 (m, 2H), 3.03 - 2.96 (m, 1H), 2.95 - 2.86 (m, 1H), 2.58 - 2.52 (m, 3H), 2.44 - 2.37 (m, 1H), 2.25 - 2.11 (m, 2H), 2.03 - 1.85 (m, 3H), 1.76 - 1.65 (m, 1H), 1.64 - 1.50 (m, 2H). | 542.0 |
| 152 | 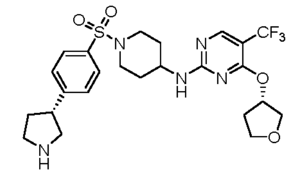 Enantiomer of 151, absolute configuration unknown | $^1$H NMR (400 MHz, DMSO-$d_6$) δ 8.29 (s, 1H), 8.02 (d, *J* = 7.3 Hz, 0.6H), 7.86 (d, *J* = 7.2 Hz, 0.4H), 7.66 (t, *J* = 6.8 Hz, 2H), 7.53 (d, *J* = 8.3 Hz, 2H), 5.60 - 5.50 (m, 1H), 3.96 - 3.83 (m, 1H), 3.82 - 3.66 (m, 4H), 3.61 - 3.49 (m, 2H), 3.26 - 3.16 (m, 2H), 3.04 - 2.96 (m, 1H), 2.95 - 2.86 (m, 1H), 2.60 - 2.52 (m, 3H), 2.45 - 2.36 (m, 1H), 2.24 - 2.11 (m, 2H), 2.04 - 1.84 (m, 3H), 1.74 - 1.65 (m, 1H), 1.65 - 1.50 (m, 2H). | 542.1 |
| 153 | 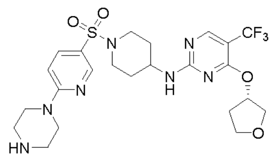 | $^1$H NMR (400 MHz, DMSO-$d_6$) δ 8.36 (d, *J* = 2.5 Hz, 1H), 8.29 (s, 1H), 8.02 (d, *J* = 7.5 Hz, 0.6H), 7.87 (d, *J* = 7.7 Hz, 0.4H), 7.72 (dd, *J* = 9.1, 2.5 Hz, 1H), 6.92 (d, *J* = 9.2 Hz, 1H), 5.60 - 5.53 (m, 1H), 3.95 - 3.86 (m, 1H), 3.83 - 3.69 (m, 4H), 3.63 - 3.54 (m, 4H), 3.54 - 3.44 (m, 2H), 2.82 - 2.72 (m, 4H), 2.58 - 2.52 (m, 3H), 2.25 - 2.14 (m, 1H), 2.02 - 1.86 (m, 3H), 1.64 - 1.50 (m, 2H). | 558.0 |
| 154 | 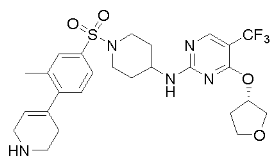 | $^1$H NMR (400 MHz, DMSO-$d_6$) δ 8.29 (s, 1H), 8.02 (d, *J* = 7.3 Hz, 0.6H), 7.87 (d, *J* = 7.3 Hz, 0.4H), 7.59 - 7.48 (m, 2H), 7.31 (d, *J* = 7.9 Hz, 1H), 5.65 (br.s., 1H), 5.60 - 5.52 (m, 1H), 3.96 - 3.85 (m, 1H), 3.83 - 3.67 (m, 4H), 3.62 - 3.49 (m, 2H), 3.40 - 3.34 (m, 2H), 2.91 (t, *J* = 5.4 Hz, 2H), 2.64 - 2.55 (m, 2H), 2.45 - 2.39 (m, 1H), 2.35 (s, 3H), 2.25 - 2.11 (m, 3H), 2.02 - 1.85 (m, 3H), 1.66 - 1.51 (m, 2H). | 568.2 |
| 155 | 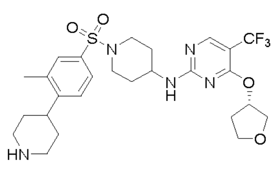 | $^1$H NMR (400 MHz, DMSO-$d_6$) δ 8.28 (s, 1H), 8.01 (d, *J* = 7.3 Hz, 0.6H), 7.86 (d, *J* = 7.6 Hz, 0.4H), 7.58 - 7.49 (m, 2H), 7.46 (d, *J* = 8.1 Hz, 1H), 5.60 - 5.50 (m, 1H), 3.95 - 3.83 (m, 1H), 3.81 - 3.66 (m, 4H), 3.61 - 3.47 (m, 2H), 3.10 - 2.96 (m, 2H), 2.92 - 2.78 (m, 1H), 2.65 - 2.52 (m, 3H), 2.45 - 2.35 (m, 4H), 2.26 - 2.11 (m, 1H), 2.02 - 1.84 (m, 3H), 1.70 - 1.44 (m, 6H). | 570.2 |
| 156 | 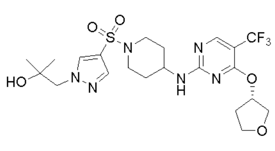 | $^1$H NMR (400 MHz, DMSO-$d_6$) δ 8.32 (s, 0.4H), 8.30 (s, 0.6H), 8.19 (s, 0.6H), 8.17 (s, 0.4H), 8.03 (d, *J* = 7.6 Hz, 0.6H), 7.86 (d, *J* = 7.8 Hz, 0.4H), 7.80 (s, 0.5H), 7.78 (s, 0.5H), 5.61 - 5.50 (m, 1H), 4.79 (s, 1H), 4.10 (s, 2H), 3.95 - 3.83 (m, 1H), 3.82 - 3.65 (m, 4H), 3.57 - 3.44 (m, 2H), 2.46 - 2.34 (m, 2H), 2.27 - 2.12 (m, 1H), 2.02 - 1.88 (m, 3H), 1.67 - 1.52 (m, 2H), 1.08 (s, 2H), 1.07 (s, 4H). | 535.1 |

(continued)

| Compound | Structure | $^1$HNMR | MS (ESI) |
|---|---|---|---|
| 157 | 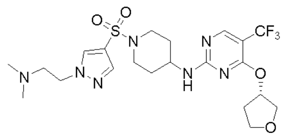 | $^1$H NMR (400 MHz, DMSO-$d_6$) δ 8.37 - 8.33 (m, 1H), 8.32 - 8.28 (s, 1H), 8.03 (d, *J* = 7.3 Hz, 0.6H), 7.86 (d, *J* = 7.5 Hz, 0.4H), 7.79 (s, 0.5H), 7.78 (s, 0.5H), 5.60 - 5.51 (m, 1H), 4.26 (t, *J* = 6.3 Hz, 2H), 3.95 - 3.84 (m, 1H), 3.93 - 3.65 (m, 4H), 3.55 - 3.43 (m, 2H), 2.71 - 2.62 (m, 2H), 2.45 - 2.35 (m, 2H), 2.27 - 2.10 (m, 7H), 2.03 - 1.86 (m, 3H), 1.67 - 1.51 (m, 2H). | 534.1 |
| 158 | 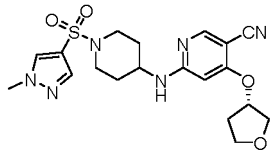 | $^1$H NMR (400 MHz, DMSO-$d_6$) δ 8.33 (s, 1H), 8.19 (s, 1H), 7.78 (s, 1H), 7.42 (d, *J* = 7.4 Hz, 1H), 6.02 (s, 1H), 5.05 (s, 1H), 3.91 (s, 3H), 3.90 - 3.67 (m, 5H), 3.44 (d, *J* = 11.4 Hz, 2H), 2.47 - 2.40 (m, 2H), 2.29 - 2.19 (m, 1H), 2.03 - 1.87 (m, 3H), 1.59 - 1.44 (m, 2H). | 433.0 |
| 159 | 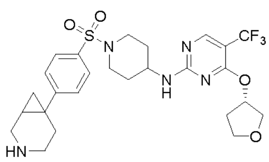 | $^1$H NMR (400 MHz, DMSO-$d_6$) δ 8.31 (s, 0.6H), 8.29 (s, 0.8H), 8.01 (d, *J* = 7.1 Hz, 0.4H), 7.86 (d, *J* = 7.6 Hz, 0.4H), 7.69 - 7.62 (m, 1.6H), 7.51 (s, 0.6H), 7.49 (s, 0.7H), 5.54 (s, 1H), 3.95 - 3.84 (m, 1H), 3.82 - 3.67 (m, 4H), 3.62 - 3.48 (m, 2H), 3.31 - .25 (m, 2H), 2.96 (d, *J* = 12.4 Hz, 1H), 2.72 - 2.68 (m, 0.5H), 2.65 - 2.55 (m, 1.4H), 2.46 - 2.35 (m, 2H), 2.26 - 2.12 (m, 1H), 2.11 - 1.85 (m, 5H), 1.66 - 1.50 (m, 2H), 1.44 - 1.33 (m, 1H), 1.10 - 0.99 (m, 2H). | 568.2 |
| 160 | 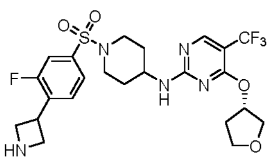 | $^1$H NMR (400 MHz, DMSO-$d_6$) δ 8.29 (s, 1H), 8.02 (d, *J* = 7.6 Hz, 0.5H), 7.86 (d, *J* = 7.5 Hz, 0.4H), 7.76 (t, *J* = 7.6 Hz, 1H), 7.64 - 7.57 (m, 1H), 7.56 - 7.48 (m, 1H), 5.60 - 5.50 (m, 1H), 4.29 - 3.87 (m, 3H), 3.84 - 3.64 (m, 7H), 3.63 - 3.51 (m, 2H), 2.65 - 2.58 (m, 1H), 2.27 - 2.13 (m, 1H), 2.02 - 1.84 (m, 3H), 1.63 - 1.49 (m, 2H), 1.34 - 1.17 (m, 1H). | 546.0 |
| 161 | 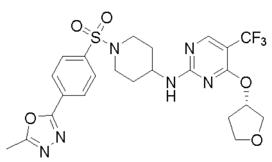 | $^1$H NMR (400 MHz, DMSO-$d_6$) δ 8.28 (s, 1H), 8.23 (s, 1H), 8.21 (s, 1H), 8.01 (d, *J* = 7.1 Hz, 0.5H), 7.99 - 7.93 (m, 2H), 7.85 (d, *J* = 7.1 Hz, 0.4H), 5.59 - 5.49 (m, 1H), 3.95 - 3.83 (m, 1H), 3.83 - 3.68 (m, 4H), 3.68 - 3.56 (m, 2H), 2.65 - 2.56 (m, 4H), 2.53 - 2.51 (m, 1H), 2.24 - 2.11 (m, 1H), 2.01 - 1.86 (m, 3H), 1.63 - 1.49 (m, 2H). | 555.0 |
| 162 | 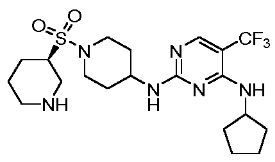 | $^1$H NMR (400 MHz, DMSO-$d_6$) δ 8.08 - 7.93 (m, 1H), 7.39 (d, *J* = 6.8 Hz, 0.5H), 7.22 (s, 0.4H), 6.26 - 6.06 (m, 1H), 4.55 - 4.30 (m, 1H), 3.99 - 3.76 (m, 1H), 3.60 (d, *J* = 10.3 Hz, 2H), 3.21 - 2.92 (m, 4H), 2.83 (d, *J* = 12.6 Hz, 1H), 2.55 - 2.52 (m, 1H), 2.39 - 2.27 (m, 1H), 2.18 (br.s., 1H), 2.05 (d, *J* = 12.5 Hz, 1H), 1.98 - 1.76 (m, 4H), 1.74 - 1.30 (m, 10H). | 477.2 |

(continued)

| Compound | Structure | $^1$HNMR | MS (ESI) |
|---|---|---|---|
| 163 | 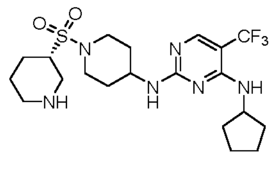 | $^1$H NMR (400 MHz, DMSO-$d_6$) δ 8.07 - 7.94 (m, 1H), 7.40 (d, *J* = 7.0 Hz, 0.5H), 7.22 (s, 0.4H), 6.22 - 6.10 (m, 1H), 4.56 - 4.30 (m, 1H), 3.98 - 3.78 (m, 1H), 3.66 - 3.55 (m, 2H), 3.21 - 3.14 (m, 1H), 3.14 - 2.92 (m, 3H), 2.83 (d, *J* = 12.2 Hz, 1H), 2.54 - 2.52 (m, 1H), 2.39 - 2.28 (m, 1.5H), 2.26 - 2.14 (m, 0.6H), 2.05 (d, *J* = 12.3 Hz, 1H), 1.98 - 1.77 (m, 4H), 1.75 - 1.30 (m, 10H). | 477.2 |
| 164 | 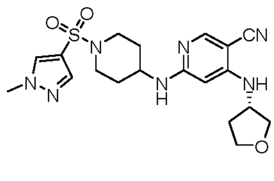 | $^1$H NMR (400 MHz, DMSO-$d_6$) δ 8.32 (s, 1H), 7.98 (s, 1H), 7.78 (s, 1H), 6.94 (d, *J* = 7.1 Hz, 1H), 6.19 (d, *J* = 5.6 Hz, 1H), 5.60 (s, 1H), 3.99 (s, 1H), 3.91 (s, 3H), 3.89 - 3.73 (m, 2H), 3.75 - 3.66 (m, 2H), 3.62 - 3.56 (m, 1H), 3.44 (d, *J* = 11.8 Hz, 2H), 2.45 - 2.40 (m, 2H), 2.21-2.10 (m, 1H), 2.03 - 1.86 (m, 3H), 1.54 - 1.43 (m, 2H). | 432.0 |
| 166 | 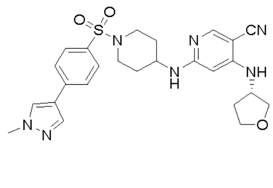 | $^1$H NMR (400 MHz, DMSO-$d_6$) δ 8.32 (s, 1H), 8.00 (s, 1H), 7.95 (s, 1H), 7.81 (d, *J* = 8.5 Hz, 2H), 7.70 (d, *J* = 8.5 Hz, 2H), 6.91 (d, *J* = 7.4 Hz, 1H), 6.17 (d, *J* = 6.0 Hz, 1H), 5.57 (s, 1H), 3.97 (br.s., 1H), 3.89 (s, 3H), 3.86 - 3.76 (m, 2H), 3.74 - 3.64 (m, 2H), 3.60 - 3.48 (m, 3H), 2.57 - 2.53 (m, 1H), 2.19 - 2.08 (m, 1H), 2.04 - 1.94 (m, 1H), 1.94 - 1.83 (m, 3H), 1.51 - 1.41 (m, 2H). | 508.2 |
| 167 | 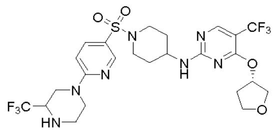 | $^1$H NMR (400 MHz, DMSO-$d_6$) δ 8.42 (s, 1H), 8.29 (s, 1H), 8.03 (d, *J* = 7.2 Hz, 0.6H), 7.87 (d, *J* = 7.4 Hz, 0.4H), 7.83 - 7.73 (m, 1H), 7.02 (d, *J* = 9.2 Hz, 1H), 5.64 - 5.50 (m, 1H), 4.47 (d, *J* = 12.3 Hz, 1H), 4.00 (d, *J* = 12.3 Hz, 1H), 3.96 - 3.86 (m, 1H), 3.84 - 3.69 (m, 4H), 3.59 - 3.44 (m, 3H), 3.25 - 3.13 (m, 2H), 3.10 - 2.97 (m, 2H), 2.81 - 2.70 (m, 1H), 2.61 - 2.53 (m, 1H), 2.48 - 2.38 (m, 1H), 2.27 - 2.14 (m, 1H), 2.05 - 1.85 (m, 3H), 1.66 - 1.50 (m, 2H). | 626.0 |
| 168 | 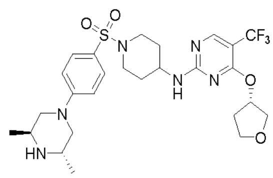 | $^1$H NMR (400 MHz, DMSO-$d_6$) δ 8.29 (s, 1H), 8.00 (d, *J* = 7.1 Hz, 0.6H), 7.85 (d, *J* = 6.9 Hz, 0.4H), 7.49 (d, *J* = 8.8 Hz, 2H), 7.02 (d, *J* = 9.0 Hz, 2H), 5.60 - 5.51 (m, 1H), 3.95 - 3.83 (m, 1H), 3.81 - 3.65 (m, 4H), 3.57 - 3.44 (m, 2H), 3.37 - 3.33 (m, 2H), 3.21 - 3.09 (m, 2H), 3.02 - 2.91 (m, 2H), 2.46 - 2.37 (m, 2H), 2.28 - 2.03 (m, 2H), 2.02 - 1.84 (m, 3H), 1.66 - 1.47 (m, 2H), 1.06 (d, *J* = 6.4 Hz, 6H). | 585.2 |
| 169 | 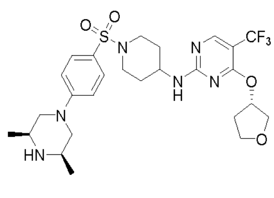 | $^1$H NMR (400 MHz, DMSO-$d_6$) δ 8.29 (s, 1H), 8.01 (d, *J* = 7.3 Hz, 0.6H), 7.86 (d, *J* = 7.0 Hz, 0.4H), 7.50 (d, *J* = 8.8 Hz, 2H), 7.06 (d, *J* = 9.1 Hz, 2H), 5.63 - 5.44 (m, 1H), 3.96 - 3.82 (m, 1H), 3.82 - 3.62 (m, 6H), 3.56 - 3.43 (m, 2H), 2.86 - 2.71 (m, 2H), 2.42 - 2.37 (m, 2H), 2.29 - 2.14 (m, 4H), 1.99 - 1.84 (m, 3H), 1.63 - 1.44 (m, 2H), 1.03 (d, *J* = 6.2 Hz, 6H). | 585.2 |

(continued)

| Compound | Structure | 1HNMR | MS (ESI) |
|---|---|---|---|
| 170 | 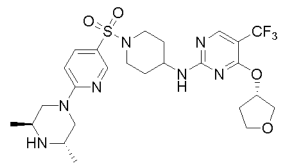 | $^1$H NMR (400 MHz, DMSO-$d_6$) δ 8.35 - 8.31 (m, 1H), 8.29 (s, 1H), 8.02 (d, *J* = 7.2 Hz, 0.6H), 7.87 (d, *J* = 8.0 Hz, 0.4H), 7.73 - 7.65 (m, 1H), 6.93 (d, *J* = 9.2 Hz, 1H), 5.61 - 5.51 (m, 1H), 3.95 - 3.85 (m, 1H), 3.83 - 3.66 (m, 6H), 3.56 - 3.45 (m, 2H), 3.30 - 3.25 (m, 2H), 3.16 - 3.07 (m, 2H), 2.62 - 2.55 (m, 2H), 2.47 - 2.39 (m, 1H), 2.26 - 2.15 (m, 1H), 2.02 - 1.85 (m, 3H), 1.66 - 1.49 (m, 2H), 1.01 (d, *J* = 6.4 Hz, 6H). | 586.2 |
| 171 | 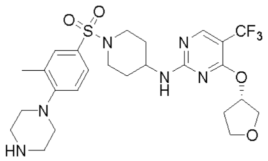 | $^1$H NMR (400 MHz, DMSO-$d_6$) δ 8.29 (s, 1H), 8.01 (d, *J* = 7.3 Hz, 0.6H), 7.86 (d, *J* = 7.5 Hz, 0.4H), 7.56 - 7.44 (m, 2H), 7.13 (d, *J* = 8.1 Hz, 1H), 5.61 - 5.49 (m, 1H), 3.95 - 3.84 (m, 1H), 3.81 - 3.67 (m, 4H), 3.59 - 3.46 (m, 2H), 2.85 (br.s., 8H), 2.57 - 2.52 (m, 2H), 2.46 - 2.34 (m, 1H), 2.32 (s, 3H), 2.27 - 2.12 (m, 1H), 2.02 - 1.84 (m, 3H), 1.64 - 1.49 (m, 2H). | 571.2 |
| 172 | 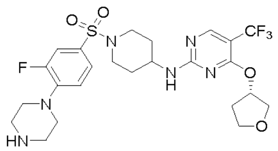 | $^1$H NMR (400 MHz, DMSO-$d_6$) δ 8.29 (s, 1H), 8.01 (d, *J* = 7.4 Hz, 0.6H), 7.86 (d, *J* = 6.9 Hz, 0.4H), 7.49 - 7.39 (m, 2H), 7.18 (t, *J* = 8.7 Hz, 1H), 5.55 (br.s., 1H), 3.96 - 3.85 (m, 1H), 3.83 - 3.68 (m, 4H), 3.60 - 3.45 (m, 2H), 3.14 - 2.99 (m, 4H), 2.89 - 2.76 (m, 4H), 2.62 - 2.52 (m, 2H), 2.46 - 2.39 (m, 1H), 2.26 - 2.13 (m, 1H), 2.02 - 1.83 (m, 3H), 1.63 - 1.47 (m, 2H). | 575.0 |
| 173 | 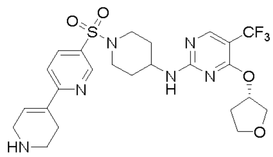 | $^1$H NMR (400 MHz, DMSO-$d_6$) δ 8.89 - 8.72 (m, 1H), 8.29 (s, 1H), 8.11 - 8.05 (m, 1H), 8.02 (d, *J* = 7.6 Hz, 0.6H), 7.86 (d, *J* = 6.4 Hz, 0.4H), 7.77 (d, *J* = 8.2 Hz, 1H), 7.04 - 6.94 (m, 1H), 5.61 - 5.50 (m, 1H), 3.95 - 3.69 (m, 5H), 3.65 - 3.53 (m, 2H), 3.49 - 3.42 (m, 2H), 2.92 (t, *J* = 5.6 Hz, 2H), 2.65 - 2.56 (m, 3H), 2.46 - 2.43 (m, 2H), 2.27 - 2.11 (m, 1H), 2.03 - 1.82 (m, 3H), 1.63 - 1.49 (m, 2H). | 555.1 |
| 174 | 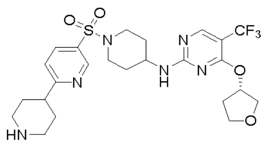 | $^1$H NMR (400 MHz, DMSO-$d_6$) δ 8.87 - 8.77 (m, 1H), 8.29 (s, 1H), 8.11 - 8.05 (m, 1H), 8.03 (d, *J* = 7.4 Hz, 0.6H), 7.87 (d, *J* = 6.9 Hz, 0.4H), 7.55 (d, *J* = 8.3 Hz, 1H), 5.61 - 5.50 (m, 1H), 3.95 - 3.85 (m, 1H), 3.83 - 3.68 (m, 4H), 3.65 - 3.52 (m, 2H), 3.10 - 3.00 (m, 2H), 2.95 - 2.81 (m, 1H), 2.72 - 2.54 (m, 4H), 2.24 - 2.13 (m, 1H), 2.02 - 1.86 (m, 3H), 1.84 - 1.74 (m, 2H), 1.70 - 1.49 (m, 4H). | 557.0 |
| 175 | 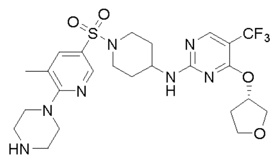 | $^1$H NMR (400 MHz, DMSO-$d_6$) δ 8.39 - 8.34 (m, 1H), 8.29 (s, 1H), 8.02 (d, *J* = 7.6 Hz, 0.6H), 7.87 (d, *J* = 7.6 Hz, 0.4H), 7.76 - 7.70 (m, 1H), 5.61 - 5.51 (m, 1H), 3.95 - 3.86 (m, 1H), 3.82 - 3.70 (m, 4H), 3.61 - 3.49 (m, 2H), 3.24 - 3.18 (m, 4H), 2.88 - 2.81 (m, 4H), 2.60 (t, *J* = 10.4 Hz, 1H), 2.53 - 2.51 (m, 1H), 2.48 - 2.43 (m, 1H), 2.30 (s, 3H), 2.25 - 2.15 (m, 1H), 2.01 - 1.87 (m, 3H), 1.64 - 1.51 (m, 2H). | 572.2 |

(continued)

| Compound | Structure | 1HNMR | MS (ESI) |
|---|---|---|---|
| 176 | 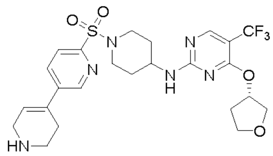 | $^1$H NMR (400 MHz, DMSO-$d_6$) δ 8.85 (s, 1H), 8.30 (d, *J* = 7.0 Hz, 1H), 8.08 (d, *J* = 8.4 Hz, 1H), 8.03 (d, *J* = 7.6 Hz, 0.6H), 7.91 - 7.82 (m, 1.4H), 6.56 (s, 1H), 5.56 (s, 1H), 3.94 - 3.86 (m, 1H), 3.80 - 3.61 (m, 6H), 3.46 - 3.38 (m, 2H), 2.93 (t, *J* = 5.5 Hz, 2H), 2.88 - 2.76 (m, 2H), 2.41 - 2.35 (m, 2H), 2.26 - 2.09 (m, 1H), 2.02 - 1.80 (m, 3H), 1.62 - 1.40 (m, 2H). | 555.1 |
| 177 | 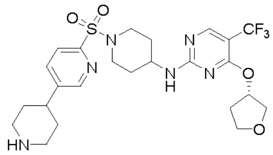 | $^1$H NMR (400 MHz, DMSO-$d_6$) δ 8.66 (s, 1H), 8.31 (d, *J* = 5.6 Hz, 1H), 8.04 (d, *J* = 7.2 Hz, 0.6H), 7.96 (d, *J* = 8.1 Hz, 1H), 7.89 - 7.86 (m, 1H), 7.86 - 7.83 (m, 0.4H), 5.61 - 5.53 (m, 1H), 3.95 - 3.86 (m, 1H), 3.84 - 3.65 (m, 6H), 3.11 (d, *J* = 12.1 Hz, 2H), 2.94 - 2.76 (m, 3H), 2.69 - 2.61 (m, 2H), 2.26 - 2.16 (m, 1H), 2.01 - 1.87 (m, 3H), 1.82 - 1.73 (m, 2H), 1.67 - 1.49 (m, 4H). | 557.0 |
| 178 | 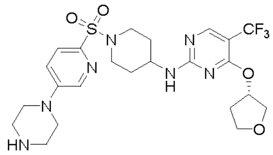 | $^1$H NMR (400 MHz, DMSO-$d_6$) δ 8.40 (d, *J* = 2.8 Hz, 1H), 8.30 (d, *J* = 6.9 Hz, 1H), 8.02 (d, *J* = 7.3 Hz, 0.6H), 7.86 (d, *J* = 7.7 Hz, 0.4H), 7.67 (dd, *J* = 8.8, 3.4 Hz, 1H), 7.40 (dd, *J* = 9.0, 2.9 Hz, 1H), 5.68 - 5.44 (m, 1H), 3.96 - 3.84 (m, 1H), 3.85 - 3.69 (m, 4H), 3.69 - 3.54 (m, 2H), 3.30 - 3.25 (m, 4H), 2.87 - 2.79 (m, 4H), 2.79 - 2.68 (m, 2H), 2.26 - 2.13 (m, 1H), 2.02 - 1.80 (m, 3H), 1.62 - 1.40 (m, 2H). | 558.0 |
| 179 | 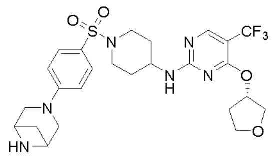 | $^1$H NMR (400 MHz, DMSO-$d_6$) δ 8.29 (s, 1H), 8.00 (d, *J* = 7.2 Hz, 0.6H), 7.86 (d, *J* = 7.6 Hz, 0.4H), 7.55 (d, *J* = 8.8 Hz, 2H), 6.84 (d, *J* = 9.2 Hz, 2H), 5.61 - 5.50 (m, 1H), 3.96 - 3.83 (m, 1H), 3.81 - 3.62 (m, 6H), 3.56 - 3.44 (m, 6H), 2.57 - 2.52 (m, 1H), 2.47 - 2.37 (m, 2H), 2.35 - 2.27 (m, 1H), 2.24 - 2.12 (m, 1H), 2.01 - 1.85 (m, 3H), 1.64 - 1.52 (m, 2H), 1.48 (d, *J* = 8.4 Hz, 1H). | 569.2 |
| 180 | 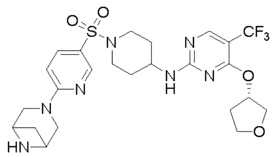 | $^1$H NMR (400 MHz, DMSO-$d_6$) δ 8.43 (s, 1H), 8.29 (s, 1H), 8.02 (d, *J* = 7.0 Hz, 0.6H), 7.87 (d, *J* = 7.6 Hz, 0.4H), 7.79 (d, *J* = 10.1 Hz, 1H), 6.75 (d, *J* = 9.1 Hz, 1H), 5.61 - 5.49 (m, 1H), 3.93 - 3.47 (m, 15H), 2.45 - 2.37 (m, 2H), 2.27 - 2.13 (m, 1H), 2.05 - 1.85 (m, 3H), 1.65 - 1.52 (m, 2H), 1.46 (d, *J* = 9.1 Hz, 1H). | 570.0 |
| 181 | 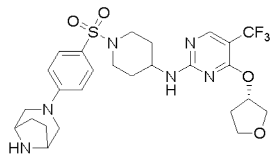 | $^1$H NMR (400 MHz, DMSO-$d_6$) δ 8.29 (s, 0.9H), 8.25 (s, 0.1H), 8.01 (d, *J* = 7.2 Hz, 0.6H), 7.86 (d, *J* = 7.2 Hz, 0.4H), 7.54 - 7.46 (m, 2H), 6.94 (d, *J* = 9.2 Hz, 2H), 5.60 - 5.52 (m, 1H), 3.95 - 3.84 (m, 1H), 3.81 - 3.67 (m, 4H), 3.64 - 3.58 (m, 2H), 3.56 - 3.52 (m, 2H), 3.51 - 3.46 (m, 2H), 2.90 (d, *J* = 11.2 Hz, 2H), 2.48 - 2.30 (m, 2H), 2.25 - 2.13 (m, 1H), 2.02 - 1.85 (m, 3H), 1.77 - 1.63 (m, 4H), 1.61 - 1.50 (m, 2H). | 583.2 |

(continued)

| Compound | Structure | $^1$HNMR | MS (ESI) |
|---|---|---|---|
| 182 | 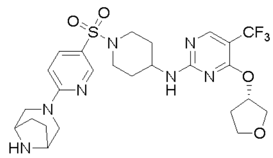 | $^1$H NMR (400 MHz, DMSO-$d_6$) δ 8.39 - 8.33 (m, 1H), 8.29 (s, 1H), 8.02 (d, *J* = 7.2 Hz, 0.6H), 7.87 (d, *J* = 7.6 Hz, 0.4H), 7.76 - 7.69 (m, 1H), 6.81 (d, *J* = 9.2 Hz, 1H), 5.61 - 5.51 (m, 1H), 4.00 - 3.87 (m, 3H), 3.82 - 3.69 (m, 4H), 3.56 - 3.46 (m, 4H), 2.97 (d, *J* = 11.6 Hz, 2H), 2.59 - 2.55 (m, 2H), 2.46 - 2.41 (m, 1H), 2.26 - 2.14 (m, 1H), 2.03 - 1.85 (m, 3H), 1.74 - 1.64 (m, 2H), 1.62 - 1.50 (m, 4H). | 584.1 |
| 183 | 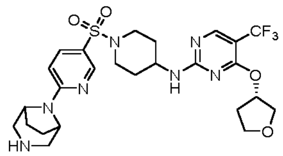 | $^1$H NMR (400 MHz, DMSO-*d6*) δ 8.34 (s, 1H), 8.29 (s, 1H), 8.03 (d, *J* = 7.0 Hz, 0.6H), 7.87 (d, *J* = 7.6 Hz, 0.4H), 7.68 (d, *J* = 9.1 Hz, 1H), 6.80 (d, *J* = 9.0 Hz, 1H), 5.51 - 5.52 (m, 1H), 4.75 - 4.38 (m, 2H), 3.97 - 3.85 (m, 1H), 3.80 - 3.70 (m, 4H), 3.56 - 3.46 (m, 2H), 2.86 - 2.75 (m, 2H), 2.61 - 2.55 (m, 4H), 2.45 - 2.41 (m, 1H), 2.25 - 2.16 (m, 1H), 1.99 - 1.84 (m, 7H), 1.61 - 1.52 (m, 2H). | 584.1 |
| 184 | 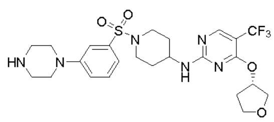 | $^1$H NMR (400 MHz, DMSO-$d_6$) δ 8.29 (s, 1H), 8.02 (d, *J* = 7.4 Hz, 0.6H), 7.86 (d, *J* = 7.6 Hz, 0.4H), 7.45 (t, *J* = 8.0 Hz, 1H), 7.24 (d, *J* = 8.1 Hz, 1H), 7.14 - 7.05 (m, 2H), 5.67 - 5.38 (m, 1H), 3.97 - 3.82 (m, 1H), 3.82 - 3.66 (m, 4H), 3.63 - 3.47 (m, 2H), 3.17 - 3.06 (m, 4H), 2.91-2.76 (m, 4H), 2.59 - 2.51 (m, 2H), 2.47 - 2.38 (m, 1H), 2.25 - 2.12 (m, 1H), 2.02 - 1.85 (m, 3H), 1.65 - 1.48 (m, 2H). | 557.2 |
| 185 | 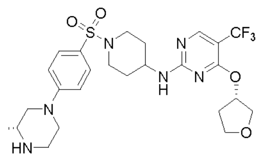 | $^1$H NMR (400 MHz, DMSO-$d_6$) δ 8.29 (s, 1H), 8.01 (d, *J* = 6.8 Hz, 0.6H), 7.86 (d, *J* = 7.7 Hz, 0.4H), 7.51 (d, *J* = 8.8 Hz, 2H), 7.05 (d, *J* = 9.1 Hz, 2H), 5.62 - 5.49 (m, 1H), 3.95 - 3.83 (m, 1H), 3.81 - 3.62 (m, 6H), 3.56 - 3.40 (m, 2H), 2.97 (d, *J* = 8.5 Hz, 1H), 2.75 - 2.69 (m, 3H), 2.46 - 2.34 (m, 3H), 2.27 - 2.09 (m, 1H), 2.02 - 1.80 (m, 3H), 1.67 - 1.47 (m, 2H), 1.03 (d, *J* = 6.3 Hz, 3H). | 571.2 |
| 186 | 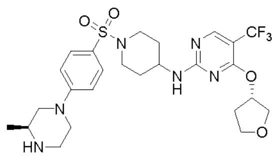 | $^1$H NMR (400 MHz, DMSO-$d_6$) δ 8.29 (s, 1H), 8.01 (d, *J* = 7.2 Hz, 0.6H), 7.86 (d, *J* = 8.0 Hz, 0.4H), 7.54 - 7.46 (m, 2H), 7.05 (d, *J* = 9.2 Hz, 2H), 5.63 - 5.50 (m, 1H), 3.93 -3.84 (m, 1H), 3.80 - 3.66 (m, 6H), 3.55 - 3.46 (m, 2H), 3.01 - 2.92 (m, 1H), 2.77 - 2.67 (m, 3H), 2.48 - 2.40 (m, 2H), 2.38 - 2.30 (m, 2H), 2.24 - 2.13 (m, 1H), 2.01 - 1.84 (m, 3H), 1.63 - 1.49 (m, 2H), 1.03 (d, *J* = 6.4 Hz, 3H). | 571.2 |

(continued)

| Compound | Structure | 1HNMR | MS (ESI) |
|---|---|---|---|
| 187 | 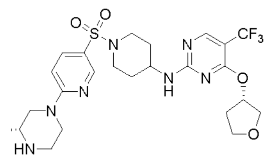 | $^1$H NMR (400 MHz, DMSO-$d_6$) δ 8.36 (d, *J* = 2.5 Hz, 1H), 8.29 (s, 1H), 8.02 (d, *J* = 7.3 Hz, 0.6H), 7.87 (d, *J* = 7.6 Hz, 0.4H), 7.76 - 7.67 (m, 1H), 6.94 (d, *J* = 9.2 Hz, 1H), 5.60 - 5.53 (m, 1H), 4.27 (t, *J* = 10.7 Hz, 2H), 3.98 - 3.85 (m, 1H), 3.85 - 3.66 (m, 4H), 3.49 (d, *J* = 10.9 Hz, 2H), 2.95 (d, *J* = 11.8 Hz, 1H), 2.84 (t, *J* = 12.1 Hz, 1H), 2.65 - 2.60 (m, 1H), 2.58 - 2.52 (m, 3H), 2.48 - 2.40 (m, 2H), 2.27 - 2.12 (m, 1H), 2.04 - 1.83 (m, 3H), 1.67 - 1.47 (m, 2H), 1.03 (d, *J* = 6.2 Hz, 3H). | 572.1 |
| 188 | 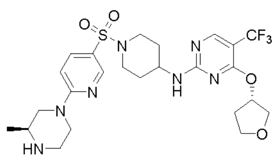 | $^1$HNMR (400 MHz, DMSO-$d_6$) δ 8.41 - 8.36 (m, 1H), 8.29 (s, 1H), 8.02 (d, *J* = 7.2 Hz, 0.6H), 7.87 (d, *J* = 7.6 Hz, 0.4H), 7.80 - 7.75 (m, 1H), 7.01 (d, *J* = 9.2 Hz, 1H), 5.61 - 5.51 (m, 1H), 4.44 - 4.32 (m, 2H), 3.95 - 3.85 (m, 1H), 3.82 - 3.67 (m, 4H), 3.58 - 3.47 (m, 2H), 3.20 - 3.14 (m, 1H), 3.07 - 2.94 (m, 2H), 2.87 - 2.75 (m, 2H), 2.58 - 2.56 (m, 1H), 2.44 - 2.38 (m, 2H), 2.25 - 2.15 (m, 1H), 2.01 - 1.87 (m, 3H), 1.63 - 1.50 (m, 2H), 1.19 - 1.11 (m, 3H). | 572.1 |
| 189 | 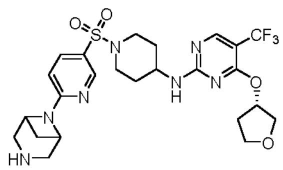 | $^1$H NMR (400 MHz, DMSO-$d_6$) δ 8.43 - 8.39 (m, 0.2H), 8.38 - 8.33 (m, 0.8H), 8.30 (d, *J* = 4.8 Hz, 1H), 8.03 (d, *J* = 6.6 Hz, 0.6H), 7.87 (d, *J* = 7.7 Hz, 0.4H), 7.81 - 7.75 (m, 0.2H), 7.75 - 7.65 (m, 0.8H), 6.74 (d, *J* = 8.5 Hz, 0.2H), 6.58 (d, *J* = 8.8 Hz, 0.8H), 5.62 - 5.52 (m, 1H), 4.53 - 4.47 (m, 0.4H), 4.38 (d, *J* = 6.0 Hz, 1.8H), 3.94 - 3.86 (m, 1H), 3.81 - 3.69 (m, 4H), 3.58 - 3.48 (m, 2H), 3.41 - 3.34 (m, 1H), 3.29 - 3.25 (m, 1H), 2.80 (d, *J* = 12.8 Hz, 2H), 2.65 - 2.55 (m, 3H), 2.47 - 2.40 (m, 1H), 2.27 - 2.12 (m, 1H), 2.03 - 1.78 (m, 4H), 1.64 - 1.44 (m, 2H). | 570.1 |
| 190 | 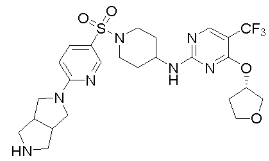 | $^1$H NMR (400 MHz, DMSO-$d_6$) δ 8.29 (s, 1H), 8.02 (d, *J* = 7.5 Hz, 0.5H), 7.87 (d, *J* = 7.1 Hz, 0.4H), 7.56 (d, *J* = 5.8 Hz, 1H), 7.53 - 7.45 (m, 1H), 7.31 (d, *J* = 7.9 Hz, 1H), 5.68 (s, 1H), 5.60 - 5.52 (m, 1H), 3.99 - 3.84 (m, 1H), 3.83 - 3.64 (m, 4H), 3.64 - 3.46 (m, 2H), 2.86 (t, *J* = 5.7 Hz, 2H), 2.64 - 2.52 (m, 3H), 2.47 - 2.38 (m, 2H), 2.36 (s, 3H), 2.24 - 2.15 (m, 1H), 2.15 - 2.07 (m, 2H), 2.01 - 1.85 (m, 3H), 1.67 - 1.49 (m, 2H). | 584.2 |
| 191 | 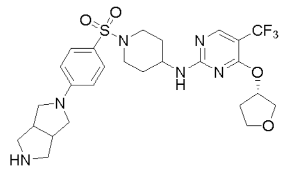 | $^1$H NMR (400 MHz, DMSO-$d_6$) δ 8.29 (s, 1H), 8.00 (d, *J* = 7.4 Hz, 0.6H), 7.85 (d, *J* = 7.6 Hz, 0.4H), 7.49 (d, *J* = 8.7 Hz, 2H), 6.73 - 6.62 (m, 2H), 5.59 - 5.50 (m, 1H), 3.96 - 3.82 (m, 1H), 3.82 - 3.60 (m, 4H), 3.58 - 3.46 (m, 4H), 3.28 - 3.06 (m, 4H), 3.06 - 2.91 (m, 2H), 2.92 - 2.76 (m, 2H), 2.71 - 2.64 (m, 1H), 2.46 - 2.34 (m, 2H), 2.24 - 2.10 (m, 1H), 2.04 - 1.80 (m, 3H), 1.64 - 1.45 (m, 2H). | 583.2 |

(continued)

| Compound | Structure | $^1$HNMR | MS (ESI) |
|---|---|---|---|
| 192 | 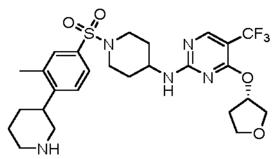 | $^1$H NMR (400 MHz, DMSO-$d_6$) δ 8.29 (s, 1H), 8.01 (d, *J* = 7.2 Hz, 0.6H), 7.86 (d, *J* = 7.6 Hz, 0.4H), 7.57 - 7.42 (m, 3H), 5.61 - 5.49 (m, 1H), 3.96 - 3.83 (m, 1H), 3.81 - 3.64 (m, 4H), 3.60 - 3.45 (m, 2H), 3.00 - 2.82 (m, 3H), 2.60 - 2.52 (m, 3H), 2.46 - 2.36 (m, 5H), 2.56 - 2.12 (m, 1H), 2.00 - 1.85 (m, 3H), 1.85 - 1.76 (m, *J* = 11.2 Hz, 1H), 1.71 - 1.43 (m, 5H). | 570.1 |
| 193 | 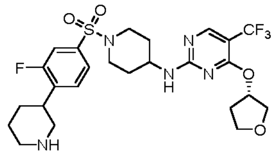 | $^1$H NMR (400 MHz, DMSO-$d_6$) δ 8.29 (s, 1H), 8.02 (d, *J* = 7.1 Hz, 0.6H), 7.86 (d, *J* = 7.6 Hz, 0.4H), 7.70 - 7.59 (m, 1H), 7.58 - 7.44 (m, 2H), 5.66 - 5.43 (m, 1H), 3.96 - 3.84 (m, 1H), 3.85 - 3.68 (m, 4H), 3.64 - 3.48 (m, 2H), 3.05 - 2.88 (m, 3H), 2.69 - 2.52 (m, 3H), 2.48 - 2.43 (m, 1H), 2.26 - 2.13 (m, 1H), 2.02 - 1.80 (m, 5H), 1.72 - 1.43 (m, 5H). | 574.1 |
| 194 | 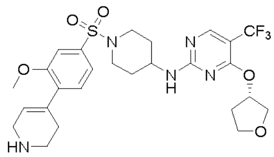 | $^1$H NMR (400 MHz, DMSO-$d_6$) δ 8.29 (s, 1H), 8.02 (d, *J* = 7.5 Hz, 0.6H), 7.87 (d, *J* = 7.7 Hz, 0.4H), 7.39 - 7.33 (m, 1H), 7.32 - 7.25 (m, 1H), 7.22 - 7.16 (m, 1H), 5.91 (s, 1H), 5.61 - 5.51 (m, 1H), 3.94 - 3.83 (m, 4H), 3.82 - 3.67 (m, 4H), 3.65 - 3.51 (m, 2H), 3.41 - 3.34 (m, 2H), 2.86 (t, *J* = 5.5 Hz, 2H), 2.68 - 2.56 (m, 2H), 2.35 - 2.26 (m, 2H), 2.24 - 2.12 (m, 1H), 2.02 - 1.85 (m, 3H), 1.65 - 1.48 (m, 2H). | 584.2 |
| 195 | 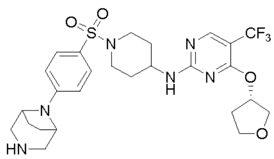 | $^1$H NMR (400 MHz, DMSO-$d_6$) δ 8.29 (s, 1H), 8.01 (d, *J* = 7.0 Hz, 0.6H), 7.86 (d, *J* = 7.1 Hz, 0.4H), 7.47 (d, *J* = 8.5 Hz, 2H), 6.89 (d, *J* = 9.0 Hz, 2H), 5.63 - 5.47 (m, 1H), 4.25 (s, 2H), 3.96 - 3.82 (m, 1H), 3.81 - 3.59 (m, 4H), 3.57 - 3.43 (m, 2H), 2.87 (d, *J* = 12.5 Hz, 2H), 2.47 - 2.34 (m, 5H), 2.25 - 2.13 (m, 1H), 2.00 - 1.83 (m, 7H), 1.64 - 1.47 (m, 2H). | 583.1 |
| 196 | 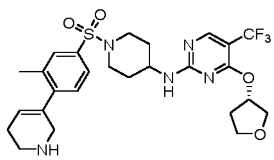 | $^1$H NMR (400 MHz, DMSO-$d_6$) δ 8.29 (s, 1H), 8.02 (d, *J* = 7.5 Hz, 0.5H), 7.87 (d, *J* = 7.1 Hz, 0.4H), 7.56 (d, *J* = 5.8 Hz, 1H), 7.53 - 7.45 (m, 1H), 7.31 (d, *J* = 7.9 Hz, 1H), 5.68 (s, 1H), 5.60 - 5.52 (m, 1H), 3.99 - 3.84 (m, 1H), 3.83 - 3.64 (m, 4H), 3.64 - 3.46 (m, 2H), 2.86 (t, *J* = 5.7 Hz, 2H), 2.64 - 2.52 (m, 3H), 2.47 - 2.38 (m, 2H), 2.36 (s, 3H), 2.24 - 2.15 (m, 1H), 2.15 - 2.07 (m, 2H), 2.01 - 1.85 (m, 3H), 1.67 - 1.49 (m, 2H). | 568.2 |
| 197 | 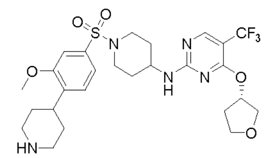 | $^1$H NMR (400 MHz, DMSO-$d_6$) δ 8.29 (s, 1H), 8.02 (d, *J* = 7.3 Hz, 0.6H), 7.86 (d, *J* = 7.4 Hz, 0.4H), 7.43 (d, *J* = 8.0 Hz, 1H), 7.34 - 7.27 (m, 1H), 7.17 (d, *J* = 7.6 Hz, 1H), 5.60 - 5.51 (m, 1H), 3.95 - 3.89 (m, 1H), 3.88 (s, 3H), 3.81 - 3.68 (m, 5H), 3.63 - 3.51 (m, 3H), 3.02 (d, *J* = 11.8 Hz, 3H), 2.68 - 2.55 (m, 4H), 2.27 - 2.12 (m, 1H), 2.02 - 1.88 (m, 3H), 1.67 - 1.43 (m, 6H). | 586.1 |

(continued)

| Compound | Structure | 1HNMR | MS (ESI) |
|---|---|---|---|
| 198 | 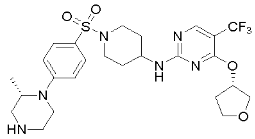 | $^1$H NMR (400 MHz, DMSO-$d_6$) δ 8.29 (s, 1H), 8.24 (s, 0.8H), 8.01 (d, *J* = 7.2 Hz, 0.6H), 7.86 (d, *J* = 7.5 Hz, 0.4H), 7.51 (d, *J* = 8.8 Hz, 2H), 7.00 (d, *J* = 9.1 Hz, 2H), 5.60 - 5.50 (m, 1H), 4.13 - 4.05 (m, 1H), 3.94 - 3.83 (m, 2H), 3.81 - 3.68 (m, 6H), 3.07 - 2.80 (m, 5H), 2.27 - 2.12 (m, 4H), 2.02 - 1.83 (m, 4H), 1.63 - 1.46 (m, 2H), 1.10 (d, *J* = 6.6 Hz, 3H). | 571.2 |
| 199 | 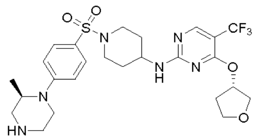 | $^1$H NMR (400 MHz, DMSO-$d_6$) δ 8.29 (s, 1H), 8.01 (d, *J* = 7.1 Hz, 0.6H), 7.86 (d, *J* = 7.7 Hz, 0.4H), 7.50 (d, *J* = 8.4 Hz, 2H), 6.99 (d, *J* = 9.0 Hz, 2H), 5.54 (s, 1H), 4.05 (s, 1H), 3.94 - 3.82 (m, 1H), 3.82 - 3.64 (m, 4H), 3.55 - 3.42 (m, 3H), 3.02 - 2.77 (m, 4H), 2.70 - 2.58 (m, 1H), 2.46 - 2.39 (m, 1H), 2.37 - 2.29 (m, 1H), 2.25 - 2.13 (m, 1H), 2.02 - 1.85 (m, 3H), 1.63 - 1.47 (m, 2H), 1.24 (s, 1H), 1.09 (d, *J* = 6.5 Hz, 3H). | 572.2 |
| 200 | 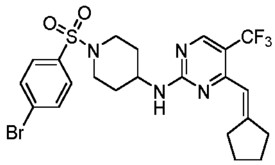 | $^1$H NMR (400 MHz, DMSO-$d_6$) δ 8.41 (s, 1H), 7.96 - 7.84 (m, 2.7H), 7.78 - 7.64 (m, 2.3H), 6.46 - 6.33 (m, 1H), 3.87 - 3.72 (m, 1H), 3.66 - 3.52 (m, 2H), 2.95 - 2.74 (m, 2H), 2.62 - 2.54 (m, 2H), 2.47 - 2.43 (m, 2H), 1.99 - 1.83 (m, 2H), 1.82 - 1.46 (m, 6H). | 547.0 |
| 201 | 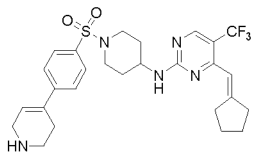 | $^1$H NMR (400 MHz, DMSO-$d_6$) δ 8.40 (s, 1H), 7.87 (d, *J* = 8.0 Hz, 0.6H), 7.73 - 7.65 (m, 4.3H), 6.45 - 6.41 (m, 1H), 6.41 - 6.34 (m, 1H), 3.80 - 3.68 (m, 1H), 3.64 - 3.51 (m, 2H), 3.41 (d, *J* = 3.0 Hz, 2H), 2.95 - 2.86 (m, 2H), 2.77 - 2.71 (m, 1H), 2.46 - 2.35 (m, 6H), 2.26 - 2.16 (m, 1H), 1.97 - 1.87 (m, 2H), 1.77 - 1.65 (m, 1H), 1.66 - 1.51 (m, 6H). | 549.1 |
| 202 | 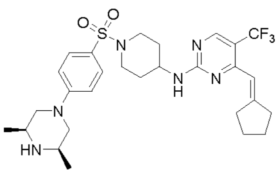 | $^1$H NMR (400 MHz, DMSO-$d_6$) δ 8.40 (s, 1H), 7.87 (d, *J* = 7.4 Hz, 0.6H), 7.72 - 7.63 (m, 0.3H), 7.50 (d, *J* = 9.0 Hz, 2H), 7.07 (d, *J* = 8.8 Hz, 2H), 6.43 - 6.33 (m, 1H), 3.82 - 3.65 (m, 3H), 3.60 - 3.47 (m, 2H), 2.95 - 2.69 (m, 4H), 2.37 - 2.24 (m, 5H), 2.23 - 1.99 (m, 1H), 1.95 - 1.86 (m, 2H), 1.78 - 1.67 (m, 0.8H), 1.63 - 1.50 (m, 5.2H), 1.04 (d, *J* = 6.2 Hz, 6H). | 580.2 |
| 203 | 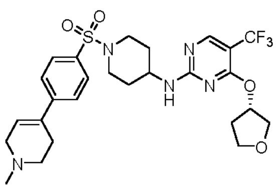 | $^1$H NMR (400 MHz, DMSO-$d_6$) δ 8.29 (s, 1H), 8.01 (d, *J* = 7.6 Hz, 0.6H), 7.86 (d, *J* = 7.6 Hz, 0.4H), 7.77 - 7.64 (m, 4H), 6.41 - 6.36 (m, 1H), 5.60 - 5.51 (m, 1H), 3.94 - 3.84 (m, 1H), 3.80 - 3.69 (m, 4H), 3.60 - 3.51 (m, 2H), 3.08 - 3.03 (m, 2H), 2.60 - 2.56 (m, 2H), 2.55 - 2.52 (m, 2H), 2.47 - 2.40 (m, 2H), 2.29 (s, 3H), 2.23 - 2.13 (m, 1H), 2.00 - 1.85 (m, 3H), 1.62 - 1.49 (m, 2H). | 568.2 |

(continued)

| Compound | Structure | 1HNMR | MS (ESI) |
|---|---|---|---|
| 204 | 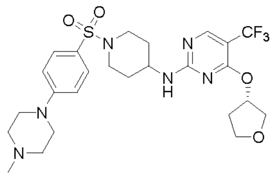 | $^1$H NMR (400 MHz, DMSO-$d_6$) δ 8.29 (s, 1H), 8.01 (d, *J* = 7.2 Hz, 0.6H), 7.86 (d, *J* = 7.2 Hz, 0.4H), 7.55 - 7.49 (m, 2H), 7.11 - 7.04 (m, 2H), 5.59 - 5.51 (m, 1H), 3.94 - 3.84 (m, 1H), 3.80 - 3.67 (m, 4H), 3.55 - 3.45 (m, 2H), 3.38 - 3.34 (m, 2H), 3.31 - 3.28 (m, 2H), 2.46 - 2.41 (m, 5H), 2.40 - 2.33 (m, 1H), 2.22 (s, 3H), 2.21 - 2.12 (m, 1H), 2.00 - 1.85 (m, 3H), 1.62 - 1.50 (m, 2H). | 571.2 |
| 205 | 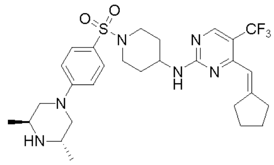 | $^1$H NMR (400 MHz, DMSO-$d_6$) δ 8.40 (s, 1H), 7.87 (d, *J* = 7.3 Hz, 0.7H), 7.62 (d, *J* = 7.2 Hz, 0.4H), 7.48 (d, *J* = 9.0 Hz, 2H), 7.03 (d, *J* = 8.9 Hz, 2H), 6.39 (s, 1H), 3.78 - 3.65 (m, 1H), 3.59 - 3.47 (m, 2H), 3.43 - 3.33 (m, 2H), 3.21 - 3.10 (m, 2H), 3.02 - 2.94 (m, 2H), 2.92 - 2.85 (m, 0.7H), 2.74 (brs, 1.4H), 2.39 - 1.99 (m, 4H), 1.96 - 1.85 (m, 2H), 1.81 - 1.67 (m, 1H), 1.66 - 1.46 (m, 5H), 1.06 (d, *J* = 6.5 Hz, 6H). | 580.2 |
| 206 | 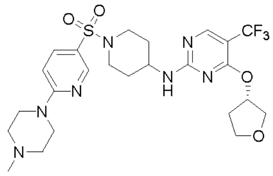 | $^1$H NMR (400 MHz, DMSO-$d_6$) δ 8.39 - 8.35 (m, 1H), 8.31 - 8.28 (m, 1H), 8.02 (d, *J* = 7.6 Hz, 0.6H), 7.87 (d, *J* = 8.0 Hz, 0.4H), 7.76 - 7.71 (m, 1H), 6.96 (d, *J* = 9.2 Hz, 1H), 5.61 - 5.51 (m, 1H), 3.94 - 3.87 (m, 1H), 3.79 - 3.71 (m, 4H), 3.67 - 3.62 (m, 4H), 3.53 - 3.47 (m, 2H), 2.58 - 2.54 (m, 1H), 2.48 - 2.42 (m, 1H), 2.41 - 2.37 (m, 4H), 2.26 - 2.18 (m, 4H), 2.01 - 1.88 (m, 3H), 1.63 - 1.49 (m, 2H). | 572.1 |
| 207 | 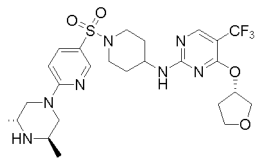 | $^1$H NMR (400 MHz, DMSO-$d_6$) δ 8.81 (brs, 1H), 8.39 (s, 1H), 8.29 (s, 1H), 8.01 (d, J = 7.2 Hz, 0.6H), 7.86 (d, J = 7.6 Hz, 0.4H), 7.82 - 7.76 (m, 1H), 7.06 (d, J = 9.2 Hz, 1H), 5.74 - 5.44 (m, 1H), 4.00 - 3.87 (m, 3H), 3.84 - 3.70 (m, 4H), 3.68 - 3.60 (m, 2H), 3.57 - 3.48 (m, 4H), 2.60 - 2.53 (m, 1H), 2.48 - 2.38 (m, 1H), 2.27 - 2.14 (m, 1H), 2.02 - 1.86 (m, 3H), 1.63 - 1.47 (m, 2H), 1.24 (d, J = 6.0 Hz, 6H). | 586.2 |
| 208 | 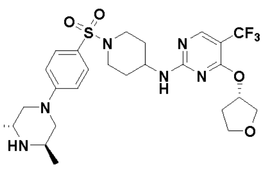 | $^1$H NMR (400 MHz, DMSO-$d_6$) δ 8.29 (s, 1H), 8.00 (d, J = 7.2 Hz, 0.6H), 7.85 (d, J = 7.7 Hz, 0.4H), 7.53 (d, J = 8.5 Hz, 2H), 7.09 (d, J = 8.9 Hz, 2H), 5.64 - 5.47 (m, 1H), 3.96 - 3.83 (m, 1H), 3.81 - 3.65 (m, 4H), 3.56 - 3.51 (m, 2H), 3.49 - 3.39 (m, 3H), 3.39 - 3.24 (m, 2H), 3.23 - 3.14 (m, 2H), 2.48 - 2.40 (m, 1H), 2.40 - 2.26 (m, 1H), 2.25 - 2.11 (m, 1H), 2.02 - 1.84 (m, 3H), 1.65 - 1.48 (m, 2H), 1.21 (d, J = 6.4 Hz, 6H). | 585.2 |
| 209 | 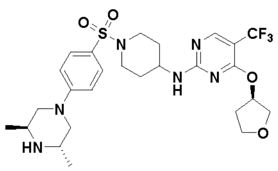 | $^1$H NMR (400 MHz, $CDCl_3$) δ 8.20 (s, 1H), 7.60 (d, *J* = 8.3 Hz, 2H), 6.89 (d, *J* = 7.7 Hz, 2H), 5.52 (br.s., 1H), 5.29 (br.s., 1H), 5.06 (br.s., 1H), 4.10 - 3.99 (m, 1H), 3.92 - 3.82 (m, 3H), 3.76 (br.s., 2H), 3.53 (br.s., 1H), 3.49 - 3.27 (m, 4H), 3.14 - 2.99 (m, 2H), 2.66 (br.s., 1H), 2.44 (br.s., 1H), 2.30 - 1.94 (m, 5H), 1.26 (d, *J* = 5.0 Hz, 6H). | 585.1 |

(continued)

| Compound | Structure | $^1$HNMR | MS (ESI) |
|---|---|---|---|
| 210 | 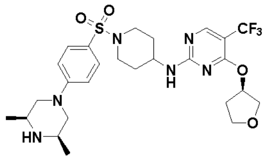 | $^1$H NMR (400 MHz, DMSO-$d_6$) δ 8.29 (s, 1H), 8.02 (d, *J* = 7.1 Hz, 0.6H), 7.87 (d, *J* = 7.1 Hz, 0.4H), 7.50 (d, *J* = 8.7 Hz, 2H), 7.06 (d, *J* = 9.0 Hz, 2H), 5.60 - 5.50 (m, 1H), 3.96 - 3.83 (m, 1.5H), 3.80 - 3.62 (m, 5.5H), 3.57 - 3.43 (m, 2H), 2.78 (br.s., 2H), 2.42 - 2.38 (m, 2H), 2.38 - 2.22 (m, 3H), 2.22 - 2.11 (m, 1H), 2.01 - 1.82 (m, 3H), 1.64 - 1.48 (m, 2H), 1.03 (d, *J* = 6.2 Hz, 6H). | 585.1 |

**Biological test evaluation:**

**CDK enzymology experiment in vitro**

In this experiment, the method of capillary migration ability change assay (MSA) was used to test the inhibitory effect of the compounds on CDK1/CDK2/CDK4/CDK6/CDK7/CDK9 kinase activity, and the half maximal inhibitory concentration ($IC_{50}$) of the compounds on CDK1/CDK2/CDK4/CDK6/CDK7/CDK9 kinase activity was obtained.

**1. Experimental materials**

CDK1/CDK2/CDK4/CDK6/CDK7/CDK9 were purchased from Carna Company; Carliper substrate CTD3/substrate 18/substrate 8 were purchased from Gil Biochemical Company; Dinaciclib/Palbociclib were purchased from Selleckchem Company; DMSO was purchased from Sigma Company; 384-well plates were purchased from Corning Company.

**2. Experimental methods**

(1) Preparation of 1×Kinase buffer.
(2) Preparation of compound concentration gradient: Test concentration of the test compound was started at 1 μM, 10 μM or 30 μM, 3-fold dilution, 10 concentrations, detected in duplicate. The test compound was diluted to a 100-fold final concentration with 100% DMSO solution in a 384 source plate. Liquid handler Echo 550 was used to transfer 250 nL of 100-fold final concentration of the compound to the target plate 384-well plate. 250 nL of DMSO was added to positive and negative control wells.
(3) 1×Kinase buffer was used to prepare a 2.5-fold final concentration of kinase solution.
(4) 10 μL of 2.5-fold final concentration kinase solution was added to compound wells and positive control wells, respectively; 10 μL of 1×Kinase buffer was added to negative control wells.
(5) The plate was centrifuged at 1000 rpm for 30 seconds, and the reaction plate was shaken to mix uniformly and incubated at room temperature for 10 minutes.
(6) 1×Kinase buffer was used to prepare a mixed solution of 25/15-fold final concentration of ATP and Kinase substrate.
(7) 15 μL of the mixed solution of 25/15-fold final concentration of ATP and substrate was added to initiate the reaction.
(8) The 384-well plate was centrifuged at 1000 rpm for 30 seconds, shaken to mix uniformly and incubated at room temperature for 10 minutes.
(9) 30 μL of stop detection solution was added to stop the kinase reaction. The 384-well plate was centrifuged at 1000 rpm for 30 seconds, and shaken to mix uniformly.
(10) The conversion rate was read with Caliper EZ Reader.

Calculation formula:

$$\%inhibition = (conversion\%_max - conversion\%_sample)/(conversion\%_max - conversion\%_min)\times 100\%$$

Where, conversion%_sample is the readings of conversion rate of the sample; conversion%_min: the average value of negative control wells, representing the readings of conversion rate of wells without enzyme activity; conversion%_max: the average value of positive control wells, representing the readings of conversion rate of wells without compound inhibition.

Fitting the dose-effect curve

Taking the log value of the concentration as the X-axis, and the percentage inhibition rate as the Y-axis, the dose-effect curve was fitted using the log(inhibitor) vs. response -Variable slope of the analysis software GraphPad Prism 5 so as to obtain the $IC_{50}$ value of each compound on the enzyme activity.

$$\text{Calculation formula: } Y = Bottom + (Top - Bottom)/(1 + 10^{\wedge}((LogIC_{50} - X) * HillSlope)).$$

3. Experimental results

Table 2 shows the inhibitory $IC_{50}$ data of the compounds of the present disclosure on CDK kinase activity. Where, compounds with $IC_{50}$<10 nM are marked with A, compounds with 10 nM≤$IC_{50}$<50 nM are marked with B, compounds with 50 nM≤$IC_{50}$<100 nM are marked with C, compounds with 100 nM≤$IC_{50}$<1000 nM are marked with D, and compounds with $IC_{50}$>1000 nM are marked with E. Where, compounds with $IC_{50}$<10 nM can be specifically divided into compounds with $IC_{50}$<0.5 nM represented by AA, compounds with 0.5 nM≤$IC_{50}$<2.5 nM represented by AB, and compounds with 2.5 nM≤$IC_{50}$<10 nM represented by AC.

**Table 2:** CDK1/CDK2/CDK4/CDK6/CDK7/CDK9 enzymology inhibition results

| Compound | CDK1 (nM) | CDK2 (nM) | CDK4 (nM) | CDK6(nM) | CDK7(nM) | CDK9(nM) |
|---|---|---|---|---|---|---|
| 1 | D | B | B | B | | |
| 2 | D | B | B | B | | |
| 3 | B | AC | AC | B | B | D |
| 4 | | B | B | | | |
| 5 | D | AC | B | B | B | D |
| 6 | | AA | AC | AB | | |
| 7 | B | AB | B | B | B | D |
| 8 | B | AB | AC | AB | C | D |
| 9 | | AB | B | | | |
| 10 | | AC | B | | | |
| 11 | B | AB | B | B | | |
| 12 | | AC | B | | | |
| 13 | | AC | B | | | |
| 14 | | B | D | | | |
| 15 | B | AB | AB | AB | B | D |

(continued)

| Compound | CDK1 (nM) | CDK2 (nM) | CDK4 (nM) | CDK6(nM) | CDK7(nM) | CDK9(nM) |
|---|---|---|---|---|---|---|
| 16 | | AB | AB | AB | | |
| 17 | | AB | AB | AB | | |
| 18 | | AB | AB | AB | | |
| 19 | | AC | B | | | |
| 20 | | B | D | | | |
| 21 | | AC | B | | | |
| 22 | | AC | B | | | |
| 23 | | AC | C | | | |
| 24 | | AB | AC | | | |
| 25 | | AA | AC | | | |
| 26 | | AA | AC | | | |
| 27 | AC | AA | AC | B | B | D |
| 28 | D | B | C | C | | |
| 29 | | B | B | | | |
| 30 | | C | D | | | |
| 31 | C | AB | AC | AB | D | D |
| 32 | B | AB | B | B | | |
| 33 | | AC | B | | | |
| 34 | B | AB | AC | B | D | D |
| 35 | B | AB | B | C | | |
| 36 | | AC | AC | | | |
| 37 | | AC | AC | | | |
| 38 | | B | C | | | |
| 39 | | AB | AC | | | |
| 40 | | AB | AC | | | |
| 41 | | AC | B | | | |
| 42 | | B | B | | | |
| 43 | | B | D | | | |
| 44 | D | AC | C | C | D | E |
| 45 | B | AC | B | D | | |
| 46 | | B | D | | | |
| 47 | | AC | B | | | |
| 48 | | AC | B | | | |
| 49 | B | AB | AC | AB | | |
| 50 | | AB | B | | | |
| 51 | | AB | AC | AB | | |
| 52 | | AC | B | | | |
| 53 | | AC | B | | | |

(continued)

| Compound | CDK1 (nM) | CDK2 (nM) | CDK4 (nM) | CDK6(nM) | CDK7(nM) | CDK9(nM) |
|---|---|---|---|---|---|---|
| 54 | | AB | B | | | |
| 55 | | AC | B | | | |
| 56 | | AC | C | | | |
| 57 | | AC | B | | | |
| 58 | | AB | B | | | |
| 59 | | AC | AC | | | |
| 60 | | AB | B | | | |
| 61 | | AB | AC | | | |
| 62 | | AB | AB | AB | | |
| 63 | | AB | AC | | | |
| 64 | | AC | B | | | |
| 65 | | AC | B | | | |
| 66 | | AC | B | | | |
| 67 | | AC | B | | | |
| 68 | | AC | B | | | |
| 69 | | B | D | | | |
| 70 | | B | C | | | |
| 71 | | AC | B | | | |
| 72 | | AC | B | | | |
| 73 | | C | D | | | |
| 74 | | B | C | | | |
| 75 | | B | B | | | |
| 76 | | AC | B | | | |
| 77 | | AC | B | | | |
| 78 | | AC | B | | | |
| 79 | | AC | B | | | |
| 80 | | AC | D | | | |
| 81 | | B | D | | | |
| 82 | | AB | AC | AB | | |
| 83 | | AB | AB | AB | | |
| 84 | | B | AC | AB | | |
| 85 | | AC | B | | | |
| 86 | | AA | AB | AB | | |
| 87 | | AB | AB | AB | | |
| 88 | | AB | B | | | |
| 89 | | B | C | | | |
| 90 | | AB | AB | AB | | |
| 91 | | AB | AC | | | |

(continued)

| Compound | CDK1 (nM) | CDK2 (nM) | CDK4 (nM) | CDK6(nM) | CDK7(nM) | CDK9(nM) |
|---|---|---|---|---|---|---|
| 92 | | AC | AC | AB | | |
| 93 | | AB | AC | AB | | |
| 94 | | AB | AC | B | | |
| 95 | | AB | B | | | |
| 96 | | AB | AC | | | |
| 97 | | AB | B | | | |
| 98 | | AA | B | | | |
| 99 | | AB | AC | | | |
| 100 | AC | AA | AB | AB | AC | D |
| 101 | | AB | AB | AB | | |
| 102 | | AB | AC | | | |
| 103 | | AB | AC | AB | | |
| 104 | | AB | AC | AB | | |
| 105 | | AB | B | | | |
| 106 | | B | D | | | |
| 107 | | AA | AB | AB | | |
| 108 | | AB | AC | | | |
| 109 | | AB | B | | | |
| 110 | | AB | B | | | |
| 111 | | AC | C | | | |
| 112 | AC | AA | AB | AB | AC | D |
| 113 | | AB | AB | AB | | |
| 114 | AC | AA | AB | AB | AC | D |
| 115 | AC | AA | AB | AB | AC | D |
| 116 | AC | AA | AB | AB | B | D |
| 117 | AC | AA | AA | AB | AC | D |
| 118 | | AB | AB | AB | | |
| 119 | | AA | AB | AB | | |
| 120 | | AC | B | | | |
| 121 | | AA | AB | AB | | |
| 122 | B | AA | AB | AB | B | D |
| 123 | | AA | AC | B | | |
| 124 | | AC | D | | | |
| 125 | | AB | B | | | |
| 126 | | B | D | | | |
| 127 | | AA | B | | | |
| 128 | | B | D | | | |
| 129 | | AC | B | | | |

(continued)

| Compound | CDK1 (nM) | CDK2 (nM) | CDK4 (nM) | CDK6(nM) | CDK7(nM) | CDK9(nM) |
|---|---|---|---|---|---|---|
| 130 | | AA | AB | AB | | |
| 131 | AC | AA | AB | AB | AC | D |
| 132 | AC | AA | AB | AB | B | D |
| 133 | AC | AA | AB | AB | B | C |
| 134 | | AA | B | | | |
| 135 | | AB | AB | AB | | |
| 136 | | AB | AC | AB | | |
| 137 | | AB | AB | AB | | |
| 138 | | AB | AB | AB | | |
| 139 | AC | AA | AB | AB | AC | D |
| 140 | | AB | AB | AB | | |
| 141 | AC | AA | AB | AB | B | D |
| 142 | AB | AA | AB | AB | AC | B |
| 143 | | AB | AC | B | | |
| 144 | AC | AA | AB | AB | AC | D |
| 145 | | AA | AB | AB | | |
| 146 | AC | AA | AB | AB | AC | D |
| 147 | AC | AB | AB | AB | B | C |
| 148 | D | B | D | D | D | E |
| 149 | | D | E | | | |
| 150 | | AB | AC | | | |
| 151 | B | AB | AB | AB | B | D |
| 152 | AC | AB | AB | AB | B | D |
| 153 | AC | AA | AB | AB | AC | D |
| 154 | B | AB | AB | AB | B | D |
| 155 | | AA | AB | AB | | |
| 156 | | AB | AC | | | |
| 157 | | AA | AC | B | | |
| 158 | | AC | B | | | |
| 159 | | AB | AB | AB | | |
| 160 | | AB | AC | AB | | |
| 161 | | AA | AB | AB | | |
| 162 | | AC | AC | B | | |
| 163 | | AC | AC | AB | | |
| 164 | | B | C | | | |
| 166 | | AB | AC | | | |
| 167 | | AB | AB | AB | | |
| 168 | AC | AA | AB | AA | AC | D |

(continued)

| Compound | CDK1 (nM) | CDK2 (nM) | CDK4 (nM) | CDK6(nM) | CDK7(nM) | CDK9(nM) |
|---|---|---|---|---|---|---|
| 169 | | AA | AB | AB | | |
| 170 | | AA | AB | AB | | |
| 171 | | AA | AB | AB | | |
| 172 | | AB | AB | AB | | |
| 173 | | AA | AB | AB | | |
| 174 | | AA | AB | AB | | |
| 175 | | AA | AB | AB | | |
| 176 | | AA | AB | AB | | |
| 177 | | AA | AB | AB | | |
| 178 | | AA | AB | AB | | |
| 179 | | AA | AB | AB | | |
| 180 | | AA | AB | AB | | |
| 181 | | AA | AB | AB | | |
| 182 | | AA | AB | AB | | |
| 183 | | AA | AB | AB | | |
| 184 | | AA | AC | B | | |
| 185 | | AA | AB | AB | | |
| 186 | | AA | AB | AB | | |
| 187 | | AA | AB | AB | | |
| 188 | | AA | AB | AB | | |
| 189 | | AA | AB | AB | | |
| 190 | | AA | AB | AB | | |
| 191 | | AA | AB | AB | | |
| 192 | | AA | AB | AB | | |
| 193 | | AA | AB | AB | | |
| 194 | | AB | AB | AB | | |
| 195 | | AA | AB | AB | | |
| 196 | | AA | AB | AB | | |
| 197 | | AA | AB | AB | | |
| 198 | | AB | AB | AB | | |
| 199 | | AA | AB | AB | | |
| 200 | | B | D | | C | |
| 201 | | AB | AC | | | |
| 202 | | AB | AC | B | | |
| 203 | | AA | AA | AB | | |
| 204 | | AA | AB | AB | | |
| 205 | | AB | AC | | | |
| 207 | | AB | AC | AB | | |

(continued)

| Compound | CDK1 (nM) | CDK2 (nM) | CDK4 (nM) | CDK6(nM) | CDK7(nM) | CDK9(nM) |
|---|---|---|---|---|---|---|
| 208 | | AB | AC | AB | | |
| 209 | | AC | B | AC | | |
| 210 | | AB | B | AC | | |

Conclusion: The compounds of the present disclosure have better CDK 2/4/6 kinase inhibitory activity, especially excellent in the inhibitory activity of CDK 2 kinase; the compounds of the present disclosure can selectively inhibit CDK 2/4/6 kinases and especially has good selectivity for CDK 2 kinase. Some compounds have inhibitory selectivity of nearly ten times, even tens of times, or 100 times or more on CDK 1/7/9 kinases compared with CDK 2 kinase.

**Cell proliferation inhibition experiment**

HCC1806/NIH:OVCAR-3 cell proliferation inhibition assay

In this experiment, the CellTiter-Glo method was used to test the inhibitory effect of the compound on the proliferation of HCC1806/NIH:OVCAR-3 cells, and the half maximal inhibitory concentration $IC_{50}$ (nM) of the compound on cells was obtained.

1. Experimental materials

HCC1806 was purchased from Tongpai (Shanghai) Biotechnology Co., Ltd.; NIH:OVCAR-3 was purchased from ATCC Cell Bank in the United States.
1640 medium, fetal bovine serum (FBS), Penicillin-Streptomycin, GlutaMAX-I Supplement were purchased from GIBCO.
PF-06873600 was purchased from Selleck Company.
CellTiter-Glo reagent, purchased from Promega Company.

2. Experimental methods

1) HCC1806/NIH:OVCAR-3 cells were seeded in a 96-well culture plate at a density of 600/1500 cells per well, 100 μL per well.
2) Day 0: Echo was used to add 100 nL of the gradient-diluted test compound to the culture plate cells. The final concentration of DMSO was 0.5%. The culture plate was placed in a cell culture incubator and incubated for 168 hours (37°C, 5% $CO_2$). For the blank control, 30 nL of DMSO was added to each well.
3) Day 7: 30 μL of Cell Titer-Glo reagent was added to each well, and kept in the dark for 30 minutes at room temperature
4) Envision microplate reader (PerkinElmer) was used to detect chemiluminescence signals.

Data analysis was performed using GraphPad Prism 6 software to obtain the $IC_{50}$ (nM) of the compounds.
Experimental results and conclusions: After testing, the compound of the present disclosure can have an $IC_{50}$ of less than 100 nM on cell proliferation of the HCC1806/NIH:OVCAR-3 cell line and has a better inhibitory effect than that of the reference compound PF-06873600.

**In vivo drug efficacy experiment of HCC1806 human breast cancer model**

**Experimental materials:**

HCC1806 was purchased from Tongpai (Shanghai) Biotechnology Co., Ltd.; NIH:OVCAR-3 was purchased from ATCC Cell Bank in the United States.
1640 medium, fetal bovine serum (FBS), Penicillin-Streptomycin were purchased from GIBCO. PF-06873600 was purchased from MCE Company.

**Experimental methods:**

Cells in the logarithmic growth phase were collected and subcutaneously inoculated into the right flank of the BALB/c nude mice to establish a tumor model. The day of inoculation was named as D0. On the fourth day after inoculation (D4), when the average tumor volume reached about 150 $mm^3$, the mice with moderate tumor volume were selected to enter the group, with 6 animals in each group. On the day of grouping, intragastric administration was started. Body weight data and tumor volume data were counted 2 to 3 times a week. Body weight-tumor growth curves was drawn. Tumor volume $V = 1/2 \times a \times b^2$, wherein a and b represent the long diameter and short diameter of the tumor, respectively.

Experimental results and conclusions: After testing, the tumors of the treatment group administered with the compound of the present disclosure are effectively inhibited. Compared with the reference compound PF-06873600, the compound of the present disclosure has a better tumor inhibitory effect, and the body weight of the mice does not decrease significantly, showing good tolerance under all treatment regimens (10 mg/kg BID, 20 mg/kg BID, 30 mg/kg QD).

**In vivo drug efficacy experiment of OVCAR-3 human ovarian cancer model**

**Experimental materials:**

OVCAR-3 was purchased from ATCC Cell Bank in the United States. 1640 medium, fetal bovine serum (FBS), Penicillin-Streptomycin were purchased from GIBCO. PF-06873600 was purchased from MCE Company.

**Experimental methods:**

Cells in the logarithmic growth phase were collected and subcutaneously inoculated into the right flank of the BALB/c nude mice to establish a tumor model. The day of inoculation was named as D0. On the 27th day after inoculation (D27), when the average tumor volume reached about 180 $mm^3$, the mice with moderate tumor volume were selected to enter the group, with 6 animals in each group. On the day of grouping, intragastric administration was started. The compounds were administered continuously to the mice for 21 days. Body weight data and tumor volume data were counted 2 to 3 times a week. Body weight-tumor growth curves was drawn. Tumor volume $V = 1/2 \times a \times b^2$, wherein a and b represent the long diameter and short diameter of the tumor, respectively.

Experimental results and conclusions: After testing, the tumors of the treatment group administered with the compound of the present disclosure are effectively inhibited. Compared with the reference compound PF-06873600, the compound of the present disclosure has a better tumor inhibitory effect, and the body weight of the mice does not decrease significantly, showing good tolerance under all treatment regimens (5 mg/kg BID, 7.5 mg/kg BID, 10 mg/kg QD, 20 mg/kg QD).

## Claims

1. A compound of formula (I-A),

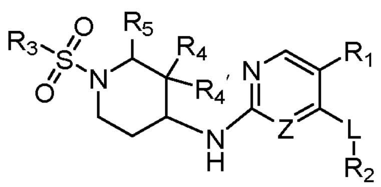

( I-A)

or a stereoisomer thereof, a tautomer thereof, a pharmaceutically acceptable salt thereof, a prodrug thereof, a hydrate thereof, a solvate thereof or an isotope-labeled derivative thereof,
wherein
$R_1$ is selected from halogen, -CN, $-NO_2$ or $C_{1-4}$ haloalkyl;
Z is selected from -CH- or N;
L is selected from a bond, $-NR_a-$, -O-, -S-, $-SO_2-$, -SO-, -CO-, $-CR_aR_b-$ or -CH=, and the $R_a$ and $R_b$ are optionally and independently selected from H, $C_{1-4}$ alkyl, $C_{1-4}$ haloalkyl, $C_{3-6}$ cycloalkyl, 3- to 6-membered heterocycloalkyl, $-SO_2R_c$, $-SOR_c$, $-COR_c$, $-(CH_2)_mNR_{aa}R_{ab}$ or - $(CH_2)_mC(O)NR_{aa}R_{ab}$, wherein the $R_c$ is selected from H, $C_{1-4}$ alkyl, and the $R_{aa}$ and $R_{ab}$ are optionally and independently selected from H, $C_{1-4}$ alkyl, $C_{1-4}$ haloalkyl, $C_{3-6}$

cycloalkyl, 3- to 6-membered heterocycloalkyl, or $R_{aa}$ and $R_{ab}$ together with the N atom to which they are attached form a 4- to 6-membered heterocycloalkyl;

$R_2$ is selected from $C_{1-4}$ alkyl, $C_{3-6}$ cycloalkyl, 3- to 6-membered heterocycloalkyl, aryl or 5- to 6-membered heteroaryl, and the $C_{1-4}$ alkyl, $C_{3-6}$ cycloalkyl, 3- to 6-membered heterocycloalkyl, aryl and 5- to 6-membered heteroaryl are optionally substituted by one or more than one $R_d$; the $R_d$ is optionally and independently selected from H, halogen, $C_{1-4}$ alkyl, $C_{1-4}$ haloalkyl, $C_{1-4}$ alkoxy, $-(CH_2)_mOH$, $-(CH_2)_mNR_eR_f$, $C_{3-6}$ cycloalkyl or 3- to 6-membered heterocycloalkyl; the $C_{3-6}$ cycloalkyl and 3- to 6-membered heterocycloalkyl in $R_d$ are optionally substituted by $C_{1-4}$ alkyl, $C_{1-4}$ haloalkyl, -OH or $-NH_2$;

$R_3$ is selected from $C_{1-4}$ alkyl, $C_{1-4}$ haloalkyl, $-(CH_2)_mNR_eR_f$, $-(CH_2)_mOH$, $-L_1$-aryl, $-L_1$-(5- to 6-membered heteroaryl), $-L_1$-($C_{3-6}$ cycloalkyl) or $-L_1$-(3- to 6-membered heterocycloalkyl), and the aryl, 5- to 6-membered heteroaryl, $C_{3-6}$ cycloalkyl and 3- to 6-membered heterocycloalkyl are optionally substituted by one or more than one $R_g$, and the $R_g$ is $R_{ga}$ or $R_{gb}$;

$R_{ga}$ is optionally and independently selected from H, halogen, -OH, $C_{1-4}$ alkyl, $C_{1-4}$ haloalkyl, $-(CH_2)_mNR_eR_f$, $R_eR_fNC(O)$-$C_{1-4}$ alkyl-, -$C_{1-4}$ alkyl-OH, $-S(O)_2$-(5- to 6-membered heteroaryl) or $C_{1-4}$ alkoxy;

$R_{gb}$ is optionally and independently selected from $-L_2$-($C_{3-6}$ cycloalkyl), $-L_2$-(3- to 6-membered heterocycloalkyl), $-L_2$-(3- to 6-membered heterocycloalkenyl), $-L_2$-aryl, $-L_2$-(5- to 6-membered heteroaryl), $-L_2$-(7- to 11-membered spiroheterocyclyl), $-L_2$-(6- to 14-membered fused heterocyclyl), wherein the $C_{3-6}$ cycloalkyl, 3- to 6-membered heterocycloalkyl, 3- to 6-membered heterocycloalkenyl, aryl, 5- to 6-membered heteroaryl, 7- to 11-membered spiroheterocyclyl, 6- to 14-membered fused heterocyclyl in $R_{gb}$ are optionally substituted by one or more than one $R_{gc}$;

$R_{gc}$ is optionally and independently selected from $C_{1-4}$ alkyl, halogen, $C_{1-4}$ haloalkyl, - $(CH_2)_mNR_eR_f$, $-(CH_2)_mOH$ or cyano;

or any two $R_{gc}$ are connected to form a $C_{1-2}$ alkylene chain;

$L_1$ is a bond or $L_1$ is optionally and independently selected from $C_{1-4}$ alkylene;

$L_2$ is a bond or $L_2$ is optionally and independently selected from $C_{1-4}$ alkylene or NH;

$R_e$ and $R_f$ are optionally and independently selected from H or $C_{1-4}$ alkyl;

m is optionally and independently selected from 0, 1, 2, 3 or 4;

$R_4$, $R_4$' and $R_5$ are optionally and independently selected from H, OH, halogen, $C_{1-4}$ alkyl, $C_{1-4}$ haloalkyl or $C_{1-4}$ alkoxy;

and when L is the bond, $R_2$ is not

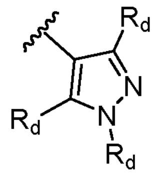

;

when L is -NH-, $R_2$ is not

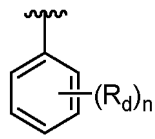

;

and when the compound of formula (I) is

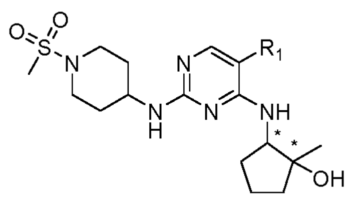

,

$R_1$ is not halogen.

2. The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to claim 1, wherein the compound is represented by formula (I-B),

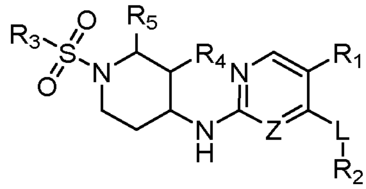

( I-B )

wherein

$R_1$ is selected from halogen, -CN, $-NO_2$ or $C_{1-4}$ haloalkyl;
Z is selected from -CH- or N;
L is selected from a bond, $-NR_a-$, -O-, -S-, $-SO_2-$, -SO-, -CO-, $-CR_aR_b-$ or -CH=, and the $R_a$ and $R_b$ are optionally and independently selected from H, $C_{1-4}$ alkyl, $C_{1-4}$ haloalkyl, $C_{3-6}$ cycloalkyl, 3- to 6-membered heterocycloalkyl, $-SO_2R_c$, $-SOR_c$, $-COR_c$, $-(CH_2)_mNR_{aa}R_{ab}$ or - $(CH_2)_mC(O)NR_{aa}R_{ab}$, wherein the $R_c$ is selected from H, $C_{1-4}$ alkyl, and the $R_{aa}$ and $R_{ab}$ are optionally and independently selected from H, $C_{1-4}$ alkyl, $C_{1-4}$ haloalkyl, $C_{3-6}$ cycloalkyl, 3- to 6-membered heterocycloalkyl, or $R_{aa}$ and $R_{ab}$ together with the N atom to which they are attached form a 4- to 6-membered heterocycloalkyl;
$R_2$ is selected from $C_{1-4}$ alkyl, $C_{3-6}$ cycloalkyl, 3- to 6-membered heterocycloalkyl, aryl or 5- to 6-membered heteroaryl, and the $C_{1-4}$ alkyl, $C_{3-6}$ cycloalkyl, 3- to 6-membered heterocycloalkyl, aryl and 5- to 6-membered heteroaryl are optionally substituted by one or more than one $R_d$; the $R_d$ is optionally and independently selected from H, halogen, $C_{1-4}$ alkyl, $C_{1-4}$ haloalkyl, $C_{1-4}$ alkoxy, $-(CH_2)_mOH$, $-(CH_2)_mNR_eR_f$, $C_{3-6}$ cycloalkyl or 3- to 6-membered heterocycloalkyl; the $C_{3-6}$ cycloalkyl and 3- to 6-membered heterocycloalkyl in $R_d$ are optionally substituted by $C_{1-4}$ alkyl, $C_{1-4}$ haloalkyl, -OH or $-NH_2$;
$R_3$ is selected from $C_{1-4}$ alkyl, $C_{1-4}$ haloalkyl, $-(CH_2)_mNR_eR_f$, $-(CH_2)_mOH$, $-L_1$-aryl, $-L_1$-(5- to 6-membered heteroaryl), $-L_1$-($C_{3-6}$ cycloalkyl) or $-L_1$-(3- to 6-membered heterocycloalkyl), and the aryl, 5- to 6-membered heteroaryl, $C_{3-6}$ cycloalkyl and 3- to 6-membered heterocycloalkyl are optionally substituted by one or more than one $R_g$, and the $R_g$ is $R_{ga}$ or $R_{gb}$;
$R_{ga}$ is optionally and independently selected from H, halogen, -OH, $C_{1-4}$ alkyl, $C_{1-4}$ haloalkyl, $-(CH_2)_mNR_eR_f$, $R_eR_fNC(O)$-$C_{1-4}$ alkyl-, $-C_{1-4}$ alkyl-OH, $-S(O)_2$-(5- to 6-membered heteroaryl) or $C_{1-4}$ alkoxy;
$R_{gb}$ is optionally and independently selected from $-L_2$-($C_{3-6}$ cycloalkyl), $-L_2$-(3- to 6-membered heterocycloalkyl), $-L_2$-(3- to 6-membered heterocycloalkenyl), $-L_2$-aryl, $-L_2$-(5- to 6-membered heteroaryl), $-L_2$-(7- to 11-membered spiroheterocyclyl), $-L_2$-(6- to 14-membered fused heterocyclyl), wherein the $C_{3-6}$ cycloalkyl, 3- to 6-membered heterocycloalkyl, 3- to 6-membered heterocycloalkenyl, aryl, 5- to 6-membered heteroaryl, 7- to 11-membered spiroheterocyclyl, 6- to 14-membered fused heterocyclyl in $R_{gb}$ are optionally substituted by one or more than one $R_{gc}$;
$R_{gc}$ is optionally and independently selected from $C_{1-4}$ alkyl, halogen, $C_{1-4}$ haloalkyl, - $(CH_2)_mNR_eR_f$, $-(CH_2)_mOH$ or cyano;
or any two $R_{gc}$ are connected to form a $C_{1-2}$ alkylene chain;
$L_1$ is a bond or $L_1$ is optionally and independently selected from $C_{1-4}$ alkylene;
$L_2$ is a bond or $L_2$ is optionally and independently selected from $C_{1-4}$ alkylene or NH;
$R_e$ and $R_f$ are optionally and independently selected from H or $C_{1-4}$ alkyl;
m is optionally and independently selected from 0, 1, 2, 3 or 4;
$R_4$ and $R_5$ are optionally and independently selected from H, OH, halogen, $C_{1-4}$ alkyl, $C_{1-4}$ haloalkyl or $C_{1-4}$ alkoxy.

3. The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to claim 1 or 2, wherein $R_1$ is selected from -CN, $-CF_3$ or $-CHF_2$.

4. The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to claim 3, wherein $R_1$ is selected from -CN or $-CF_3$.

5. The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to claim 4, wherein $R_1$ is selected from $-CF_3$.

6. The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to any one of claims 1 to 5, wherein Z is selected from N.

7. The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to any one of claims 1 to 6, wherein L is selected from the bond, -NH-, $-N(CH_3)-$, -O-, -S-, $-CH_2-$, -CH=, $-N(SO_2CH_3)-$, $-SO_2-$, - SO-, $-N(CH_2CF_3)-$,

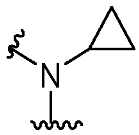 , 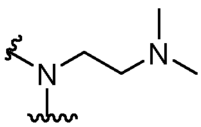 or 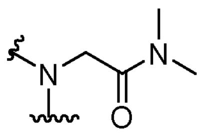 .

8. The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to claim 7, wherein L is selected from -NH- or -O-.

9. The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to any one of claims 1 to 8, wherein $R_2$ is selected from methyl, ethyl, n-propyl, isopropyl, butyl, tert-butyl,

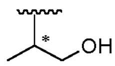 , 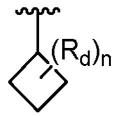 ,

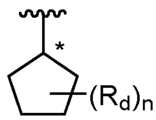 , 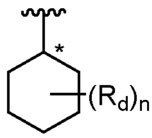 , 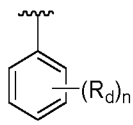 , 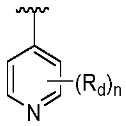 , 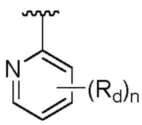 , 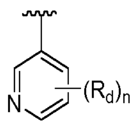 , 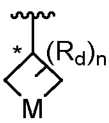 ,

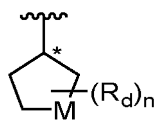 , 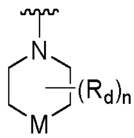 ,

preferably, $R_2$ is selected from

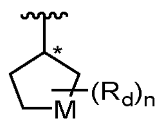 ,

wherein M is optionally and independently selected from -O- or $-NR_a-$, $R_a$ and $R_d$ are as defined in claim 1, n is independently 0, 1, 2, 3 or 4.

10. The compound of formula (I-A), the stereoisomer thereof, the tautomer thereof, the pharmaceutically acceptable salt thereof, the prodrug thereof, the hydrate thereof, the solvate thereof or the isotope-labeled derivative thereof

according to claim 9, wherein $R_2$ is selected from isopropyl, tert-butyl,

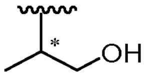, 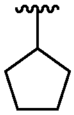, 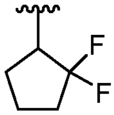, 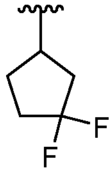, 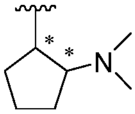, 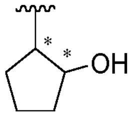,

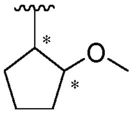, 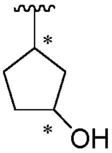, 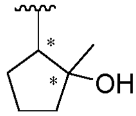, 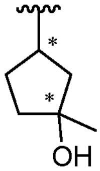, 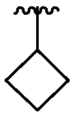, 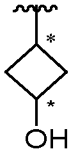, 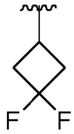, 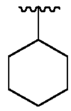, 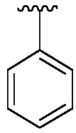, 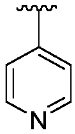,

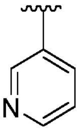, 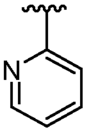, 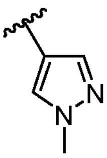, 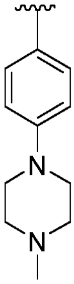, 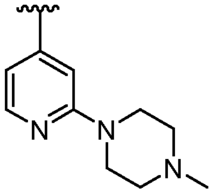, 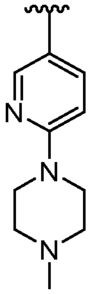, 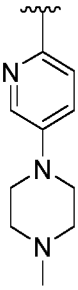, 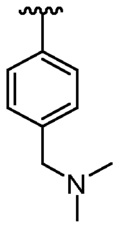,

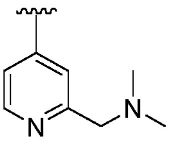, 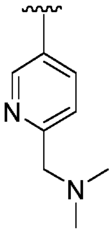, 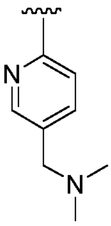, 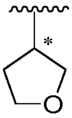, 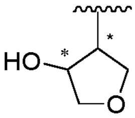, 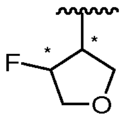, 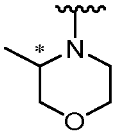, 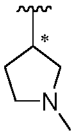, 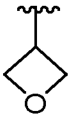,

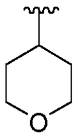, 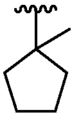, 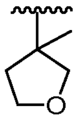,

preferably, $R_2$ is selected from

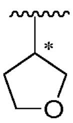.

11. The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to any one of claims 1 to 10, wherein $R_3$ is selected from methyl, isopropyl, $-(CH_2)_3N(CH_3)CH_3$,

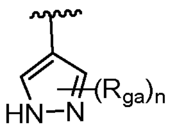, 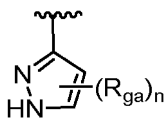,

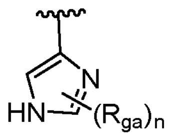 , 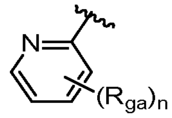 , 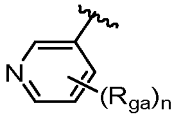 , 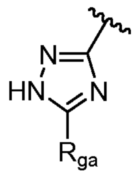 , 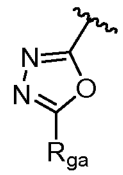 , 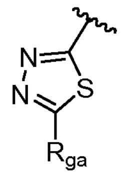 ,

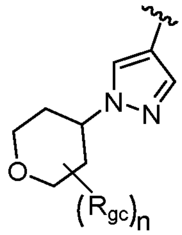 , 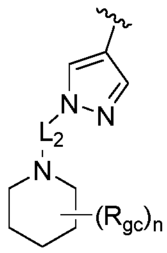 , 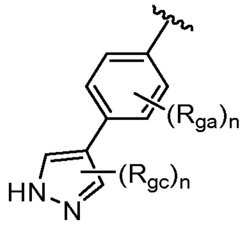 , 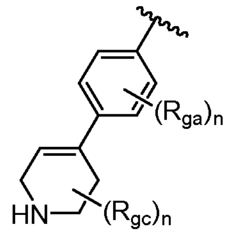 , 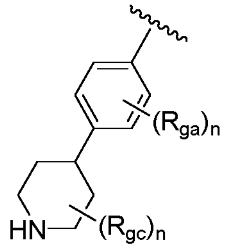 ,

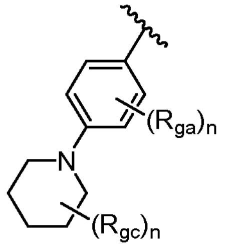 , 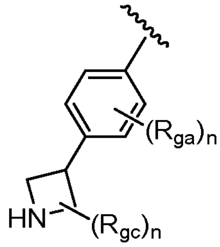 , 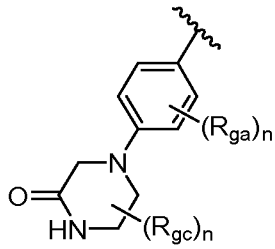 , 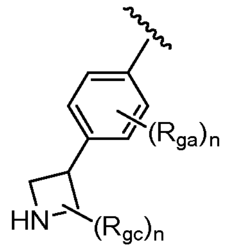 ,

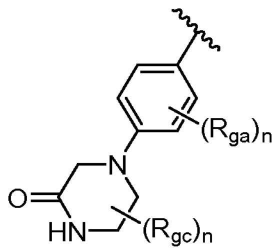 , 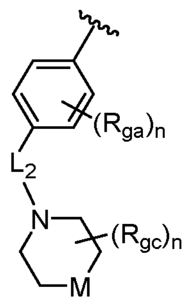 , 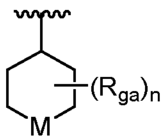 , 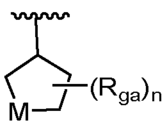 , 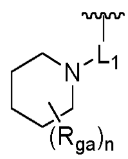 , 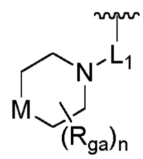 ,

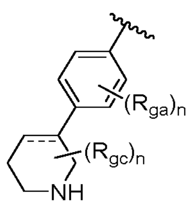 , 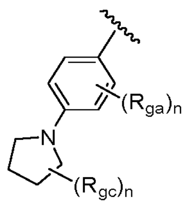 , 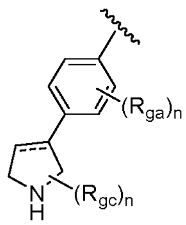 , 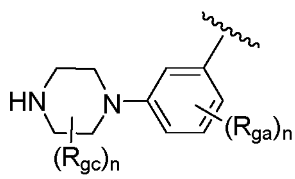 , 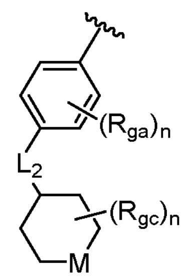 ,

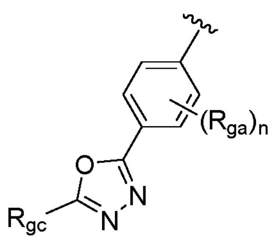 , 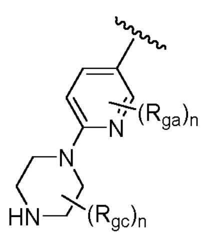 , 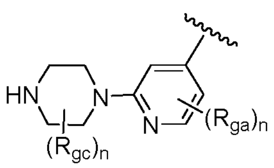 , 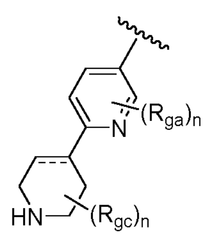 ,

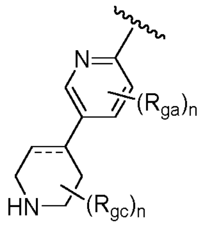, 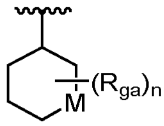, 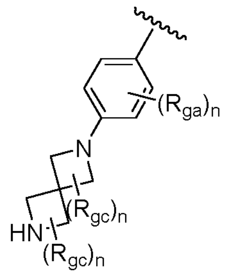, 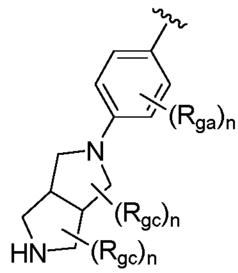, 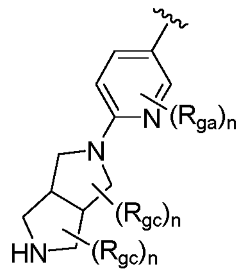,

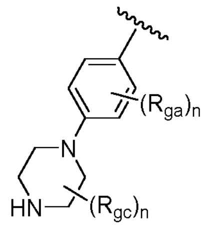,

preferably, $R_3$ is selected from

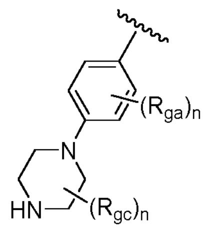,

wherein M and n are as defined in claim 9, and $R_{ga}$, $R_{gc}$, $L_1$, $L_2$ are as defined in claim 1.

**12.** The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to claim 11, wherein $R_3$ is selected from methyl, isopropyl, $-(CH_2)_3N(CH_3)CH_3$,

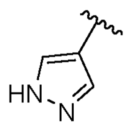, 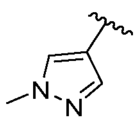, 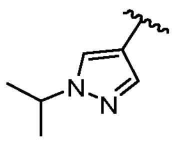,

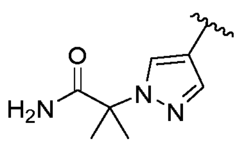, 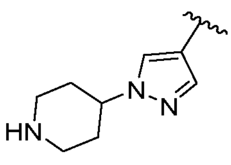, 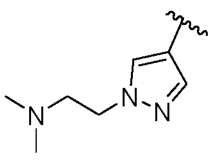, 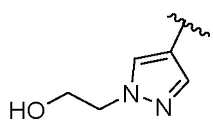, 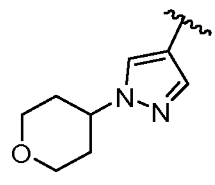,

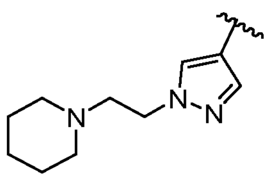, 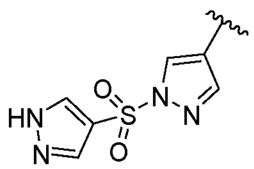, 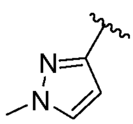, 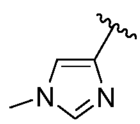, 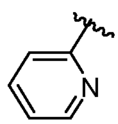, 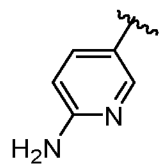,

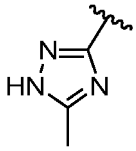 , 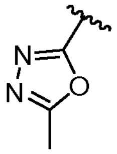 , 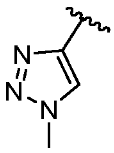 , 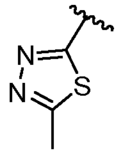 , 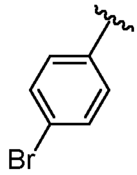 , 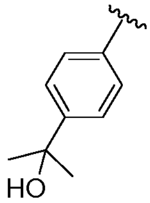 , 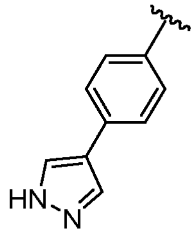 ,

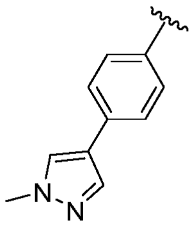 , 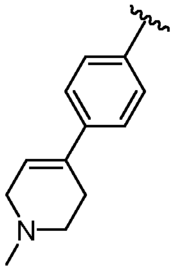 , 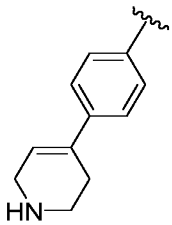 , 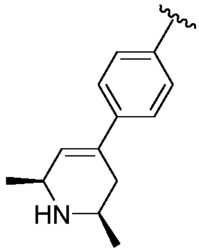 , 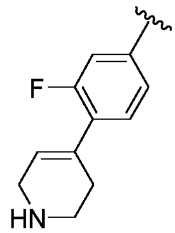 , 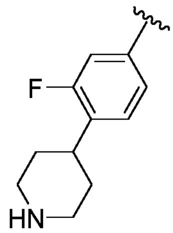 ,

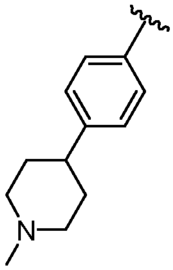 , 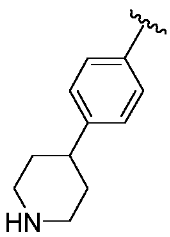 , 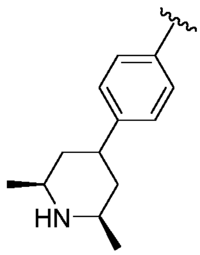 , 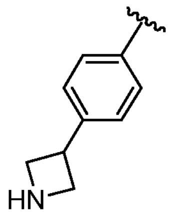 , 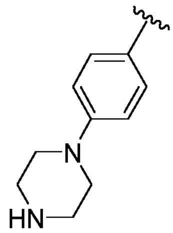 , 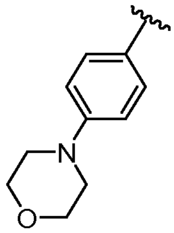 ,

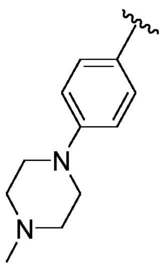 , 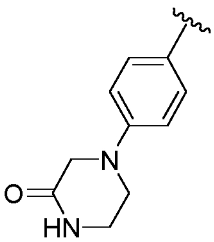 , 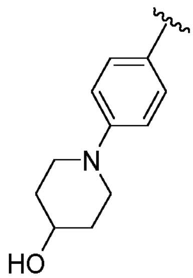 , 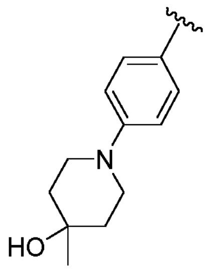 , 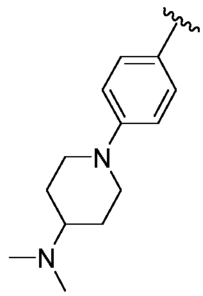 ,

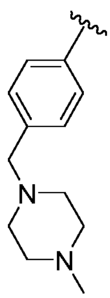 , 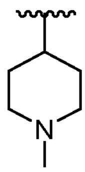 , 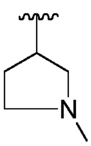 , 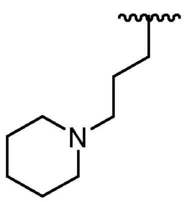 , 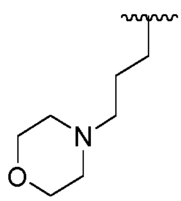 , 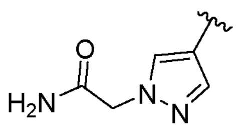 ,

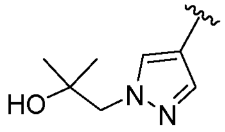 , 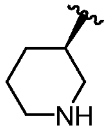 , 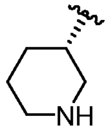 , 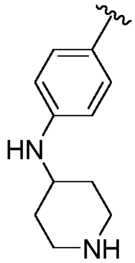 , 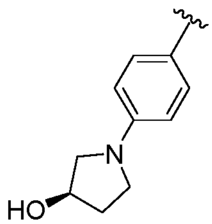 , 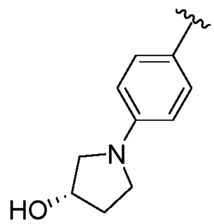 ,

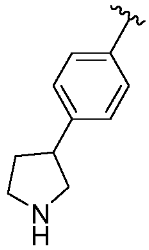 , 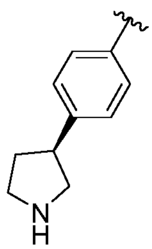 , 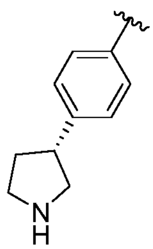 , 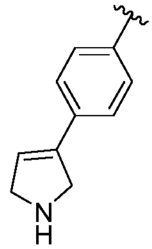 , 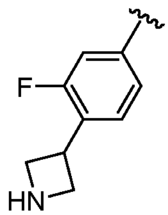 , 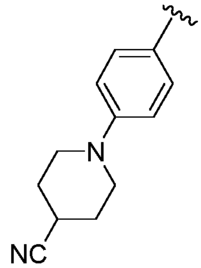 ,

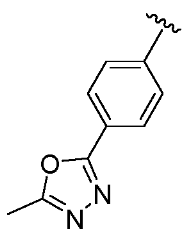 , 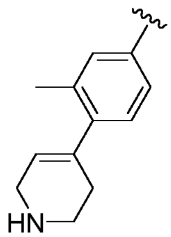 , 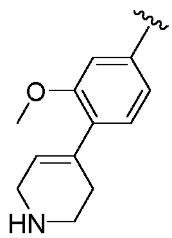 , 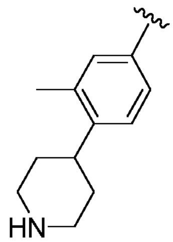 , 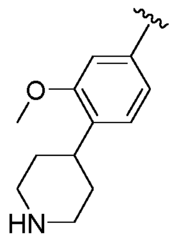 , 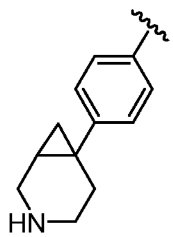 ,

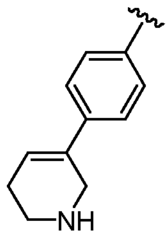 , 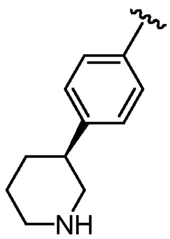 , 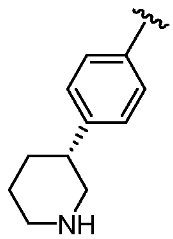 , 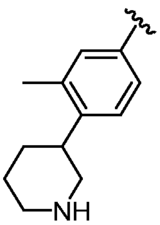 , 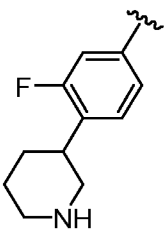 , 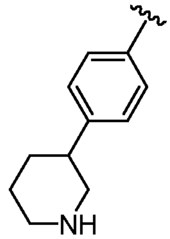 ,

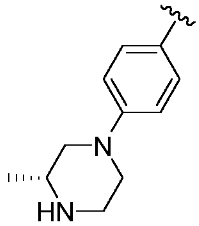 , 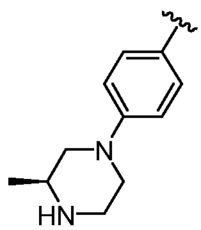 , 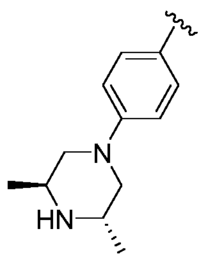 , 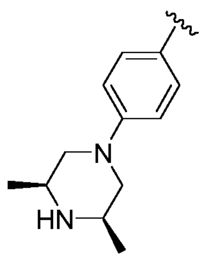 , 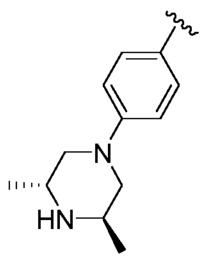 ,

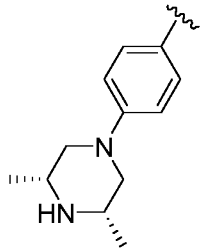 , 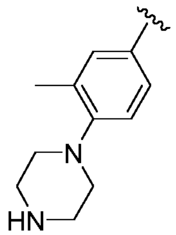 , 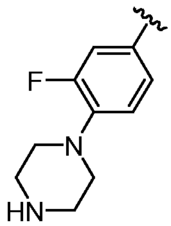 , 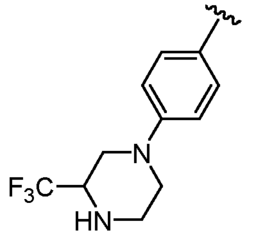 , 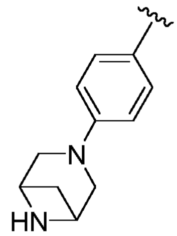

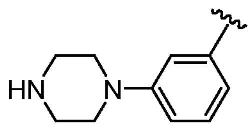 , 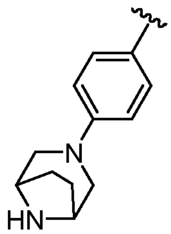 , 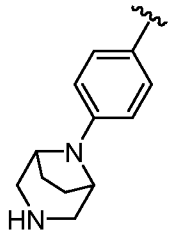 , 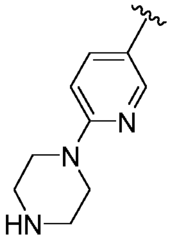 , 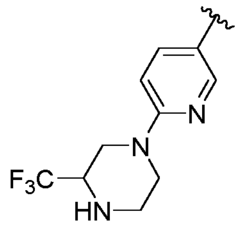

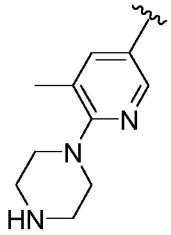 , 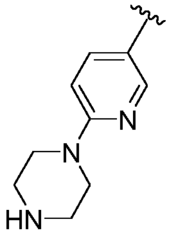 , 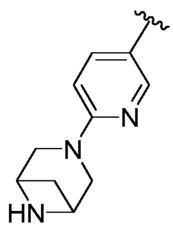 , 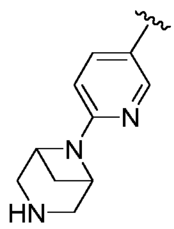 , 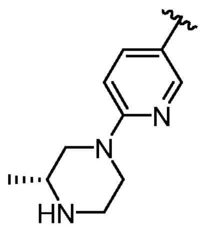 ,

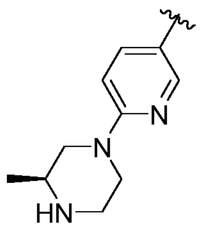 , 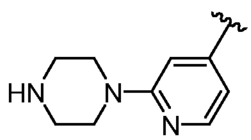 , 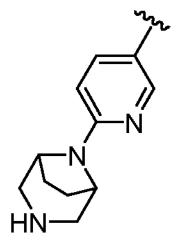 , 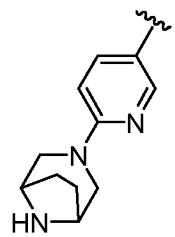 , 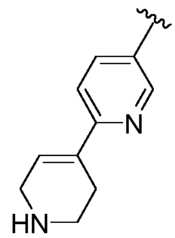 ,

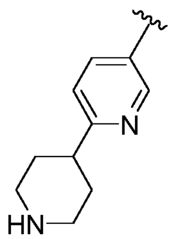 , 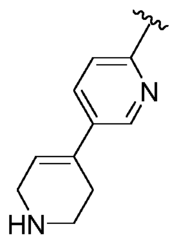 , 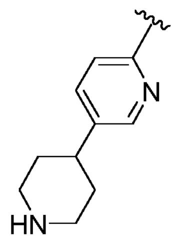 , 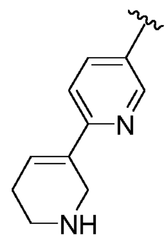 , 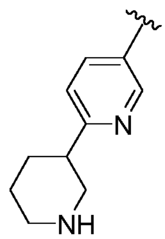 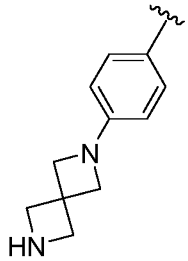 ,

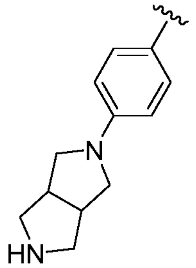 , 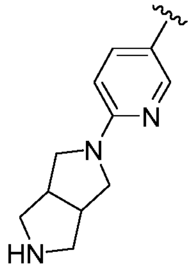 , 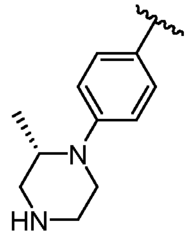 , 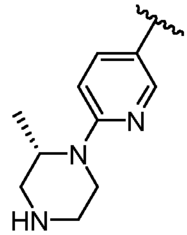 , 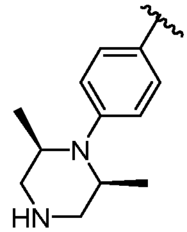 ,

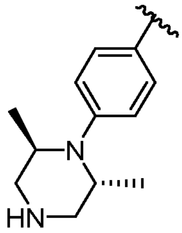, 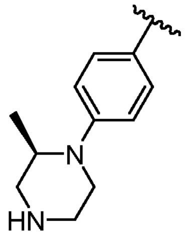, 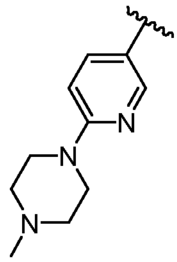, 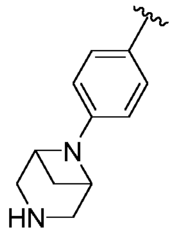,

preferably, $R_3$ is selected from

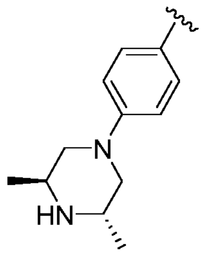.

**13.** The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to any one of claims 1 to 12, wherein $R_4$ and $R_8$ are optionally and independently selected from H, F, OH, $CH_3$, preferably, $R_4$ and $R_5$ are selected from H.

**14.** The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to any one of claims 1 to 13, wherein the compound is represented by formula (II),

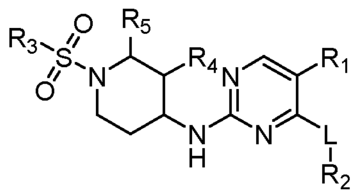

( II )

wherein $R_1$, $R_2$, $R_3$, $R_4$, $R_4$, L are as defined in any one of claims 1 to 13.

**15.** The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to claim 14, wherein the compound is represented by any one of structures in formula (II-A), formula (II-B) and formula (II-C),

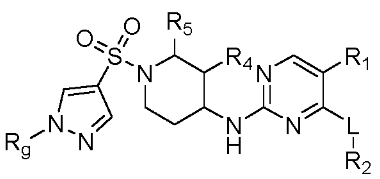

( II-A )

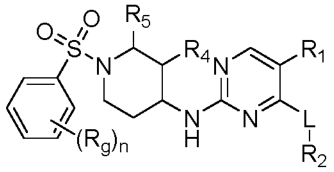

( II-B )

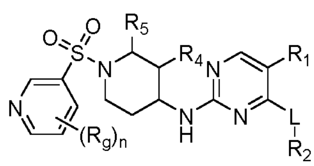

( II-C )

wherein $R_1$, $R_2$, $R_4$, $R_5$, L are as defined in claim 14, $R_g$ is as defined in any one of claims 1 to 13; n is as defined

in claim 9.

**16.** The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to claim 15, wherein the compound is represented by any one of the following structures,

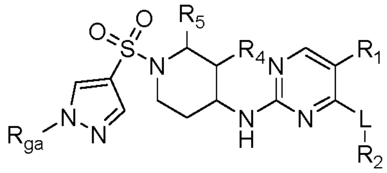

( II-A-1 )

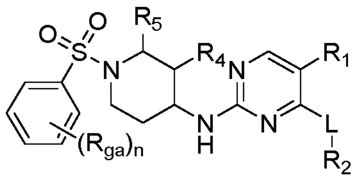

( II-B-1 )

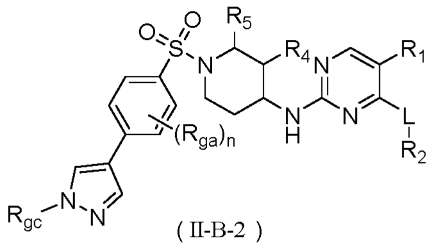

( II-B-2 )

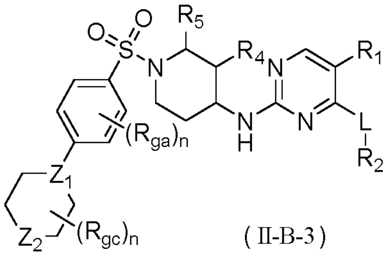

( II-B-3 )

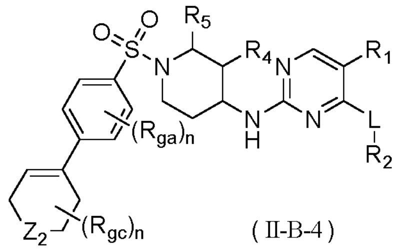

( II-B-4 )

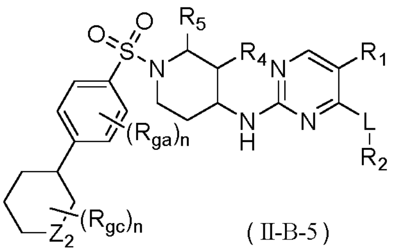

( II-B-5 )

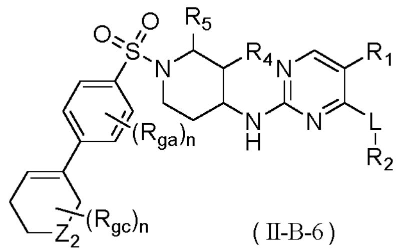

( II-B-6 )

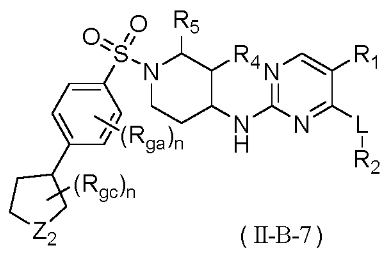

( II-B-7 )

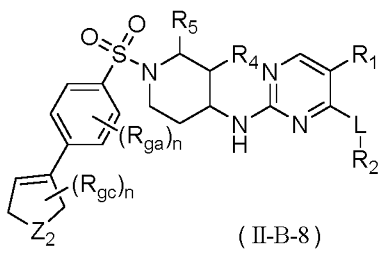

( II-B-8 )

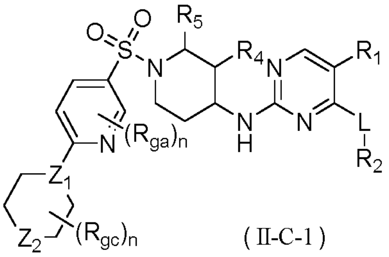
( II-C-1 )

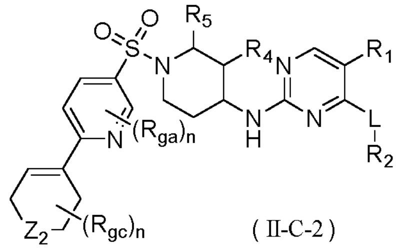
( II-C-2 )

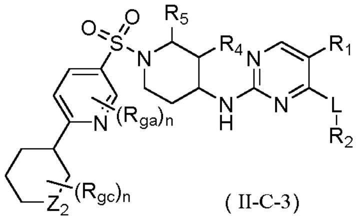
( II-C-3)

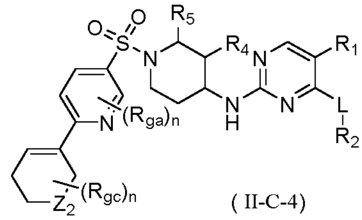
( II-C-4)

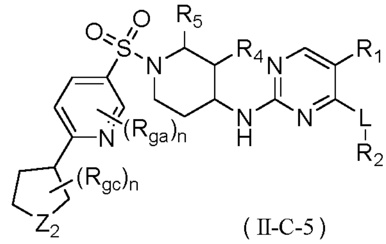
( II-C-5 )

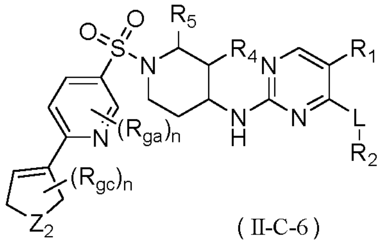
( II-C-6 )

wherein

$Z_1$ is selected from C, CH or N;
$Z_2$ is selected from $CH_2$, NH or O;
$R_1$, $R_2$, $R_4$, $R_5$, L, n are as defined in claim 15; $R_{ga}$, $R_{gc}$ are as defined in any one of claims 1 to 13.

**17.** The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to claim 16, wherein the compound is represented by any one of the following structures,

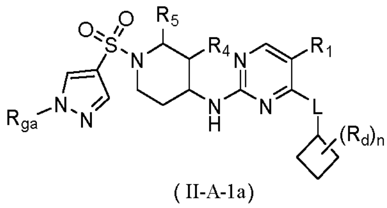
( II-A-1a)

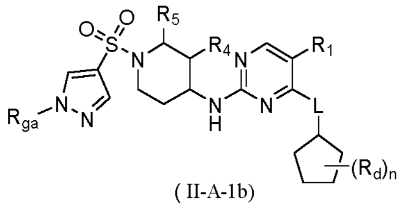
( II-A-1b)

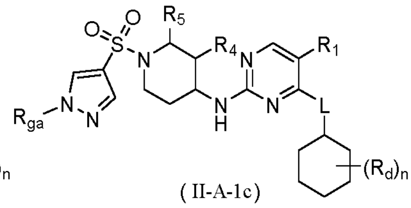
( II-A-1c)

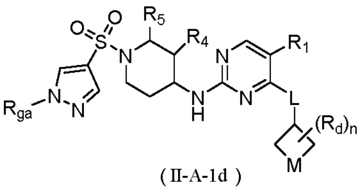
( II-A-1d )

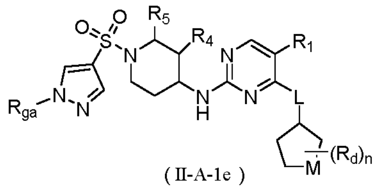
( II-A-1e )

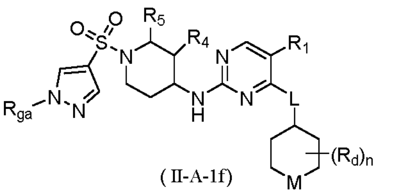
( II-A-1f)

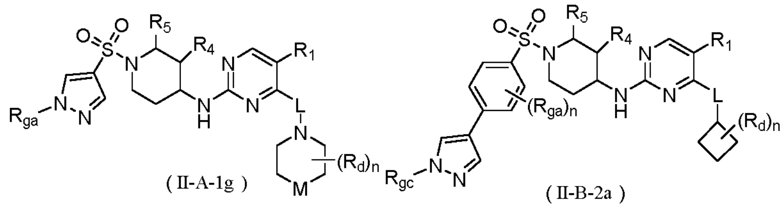

( II-A-1g )

( II-B-2a )

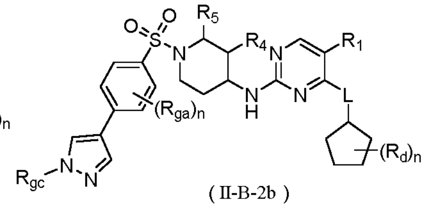

( II-B-2b )

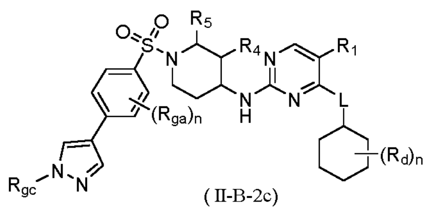

( II-B-2c)

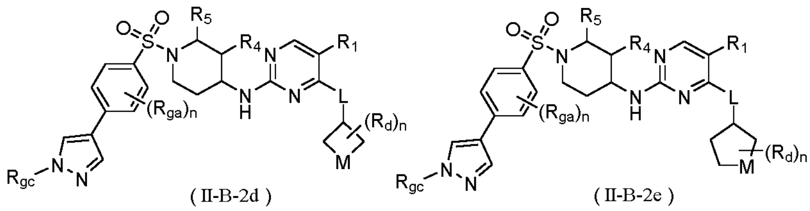

( II-B-2d )

( II-B-2e )

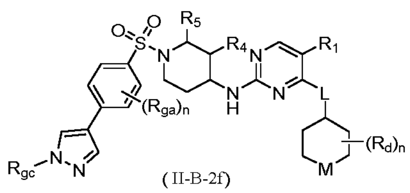

(II-B-2f)

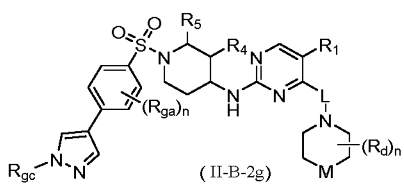

(II-B-2g)

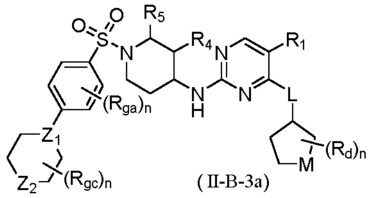

(II-B-3a)

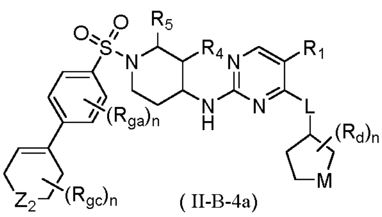

( II-B-4a)

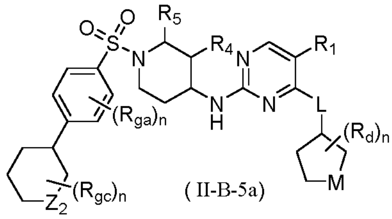

( II-B-5a)

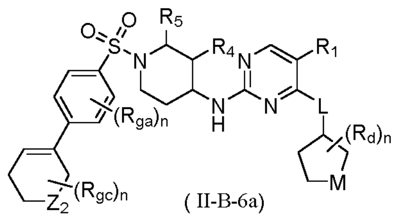

( II-B-6a)

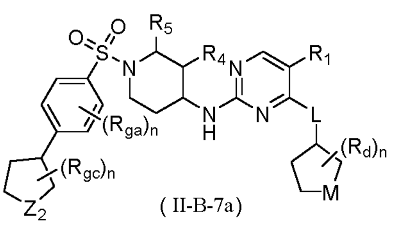

( II-B-7a)

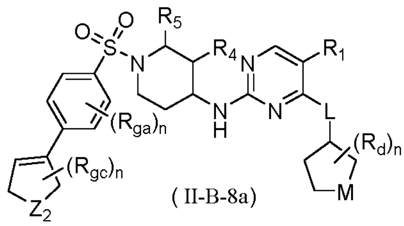

( II-B-8a)

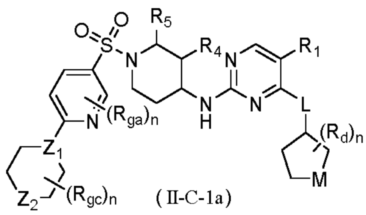

(II-C-1a)

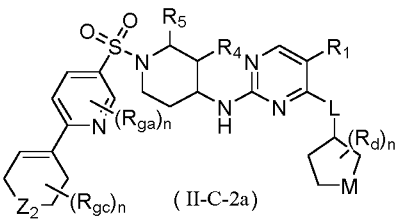

( II-C-2a)

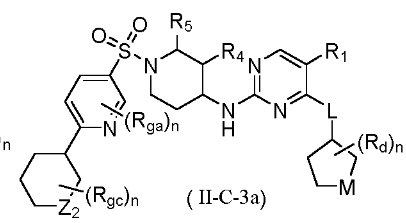

( II-C-3a)

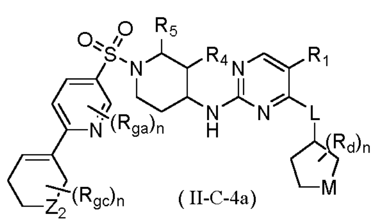

( II-C-4a)

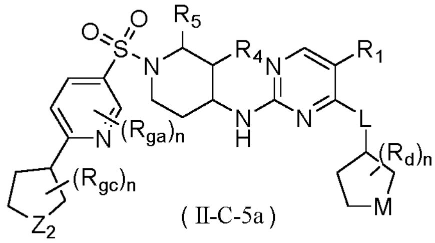
( II-C-5a )

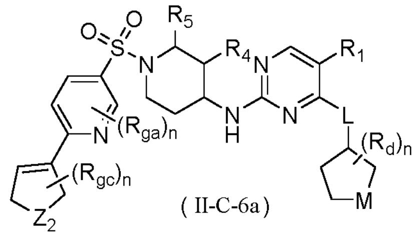
( II-C-6a )

wherein

$R_1$, L, n, $R_{ga}$, $R_{gc}$, $R_4$, $R_5$, $Z_1$, $Z_2$ are as defined in claim 16;
$R_d$ is as defined in claim 1;
M is as defined in claim 9.

**18.** The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to claim 17, wherein the compound is represented by any one of the following structures,

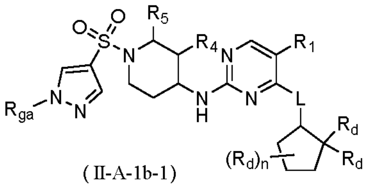
( II-A-1b-1)

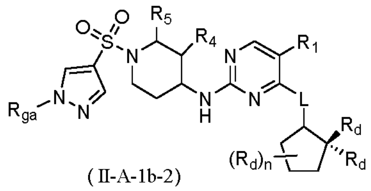
( II-A-1b-2)

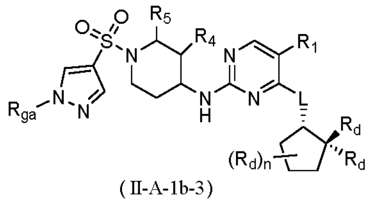
( II-A-1b-3)

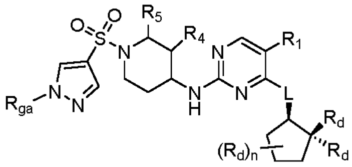
( II-A-1b-4 )

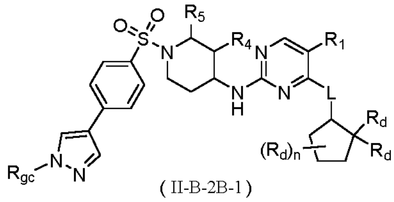
( II-B-2B-1 )

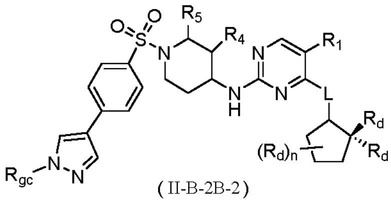
( II-B-2B-2 )

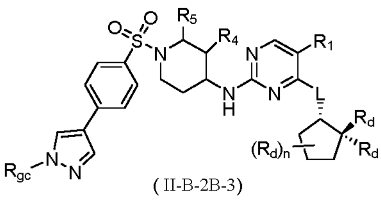
( II-B-2B-3)

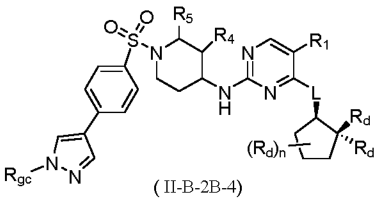
( II-B-2B-4)

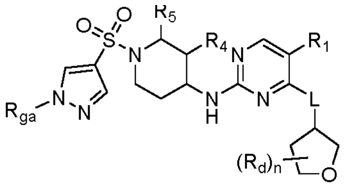
( II-A-1b-5 )

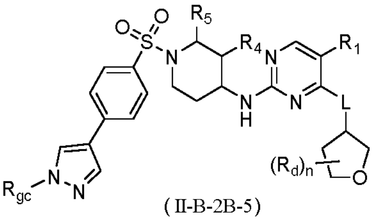
( II-B-2B-5)

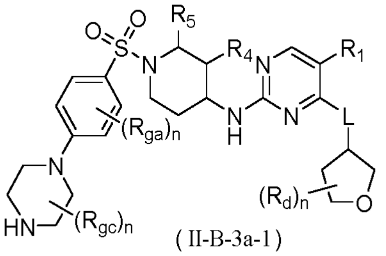
( II-B-3a-1 )

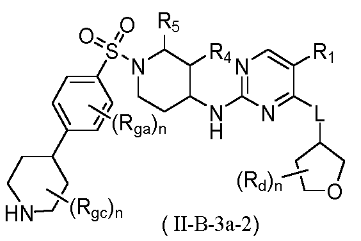
( II-B-3a-2)

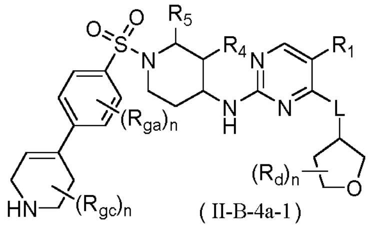
( II-B-4a-1 )

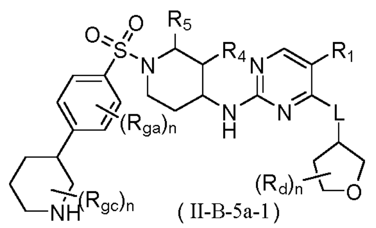
( II-B-5a-1 )

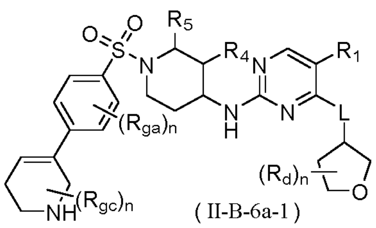
( II-B-6a-1 )

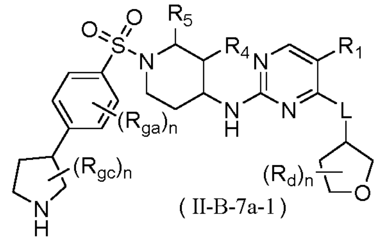
( II-B-7a-1 )

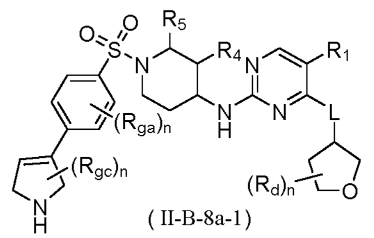
( II-B-8a-1 )

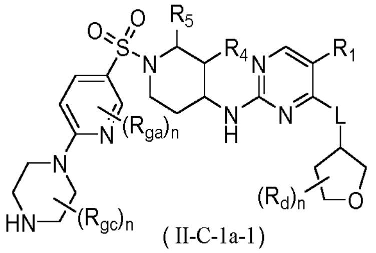
( II-C-1a-1 )

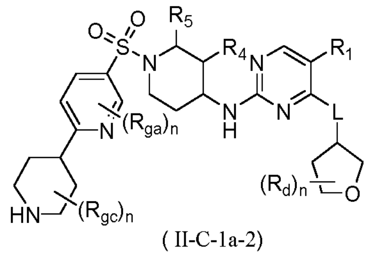
( II-C-1a-2)

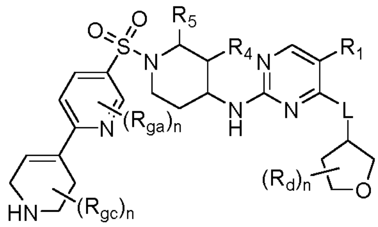
( II-C-2a-1 )

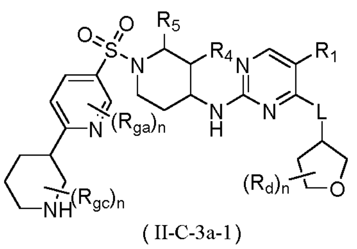
( II-C-3a-1 )

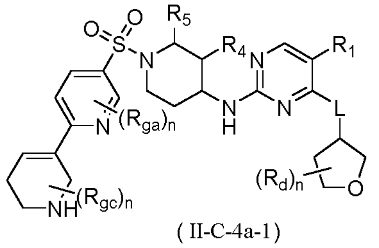
( II-C-4a-1 )

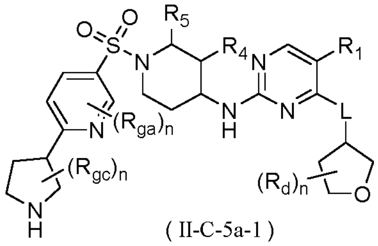
( II-C-5a-1 )

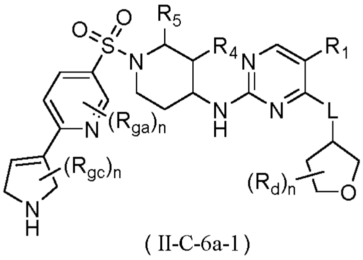
( II-C-6a-1 )

wherein
$R_1$, L, $R_d$, n, $R_{ga}$, $R_{gc}$, $R_4$, $R_5$ are as defined in claim 17.

**19.** The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to claim 14, wherein the compound is represented by any one of the following structures,

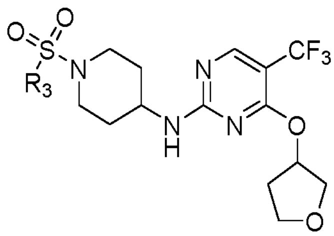
( II-D )

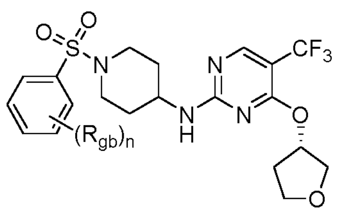
( II-D-1 )

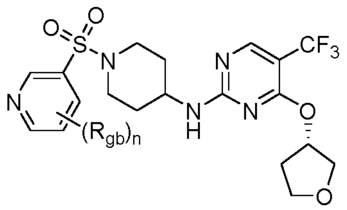

( II-D-2 )

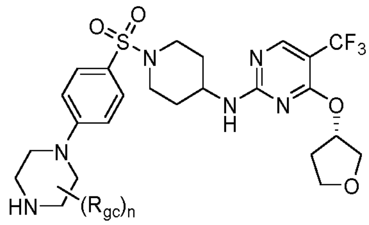

( II-D-3)

wherein $R_3$, $R_{gb}$, $R_{gc}$, n are as defined in any one of claims 1 to 13.

**20.** The compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to any one of claims 1 to 19, wherein the compound of formula (I-A) is selected from any one of the following structures,

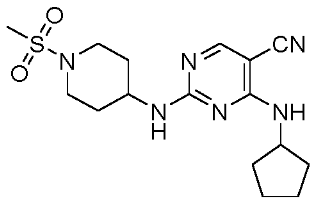

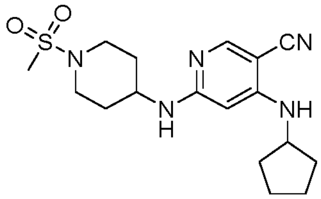

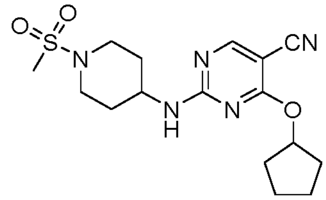

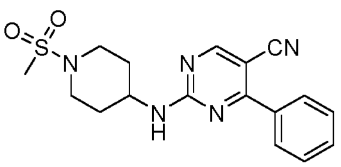

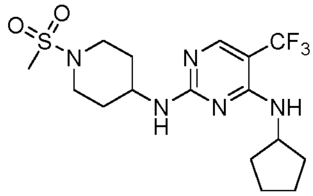

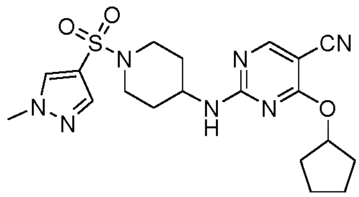

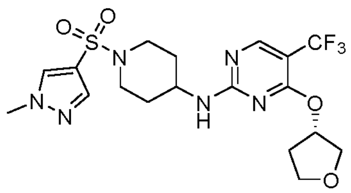

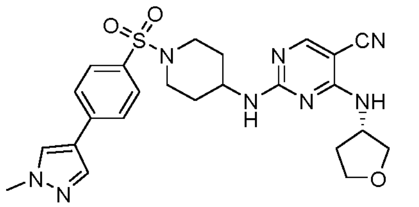

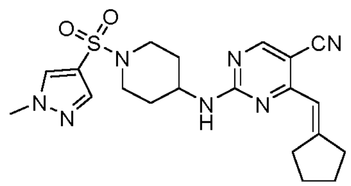

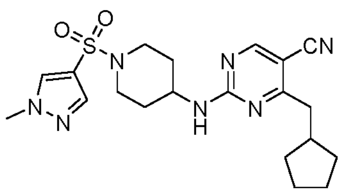

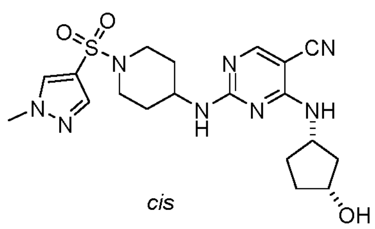

*cis*

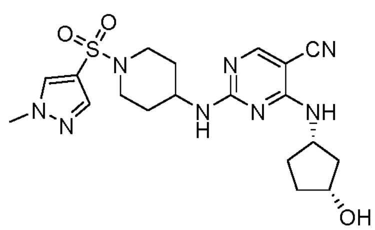

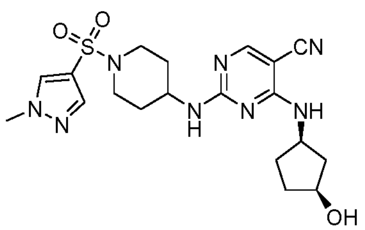

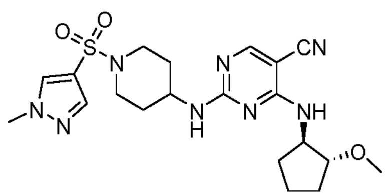

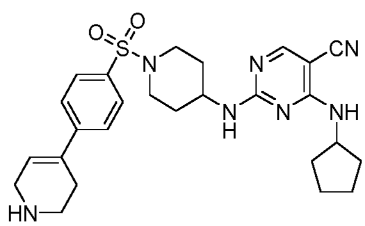

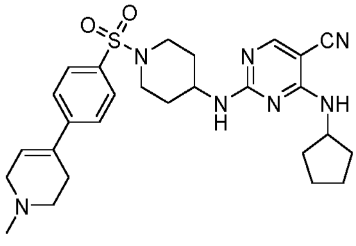
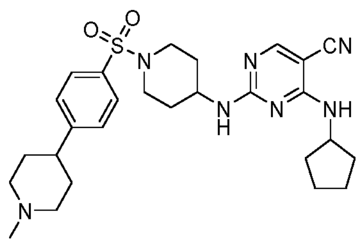
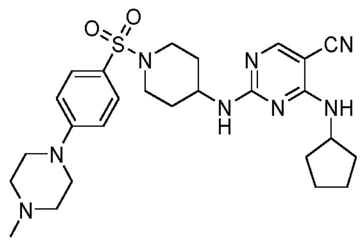

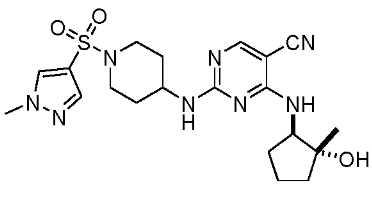
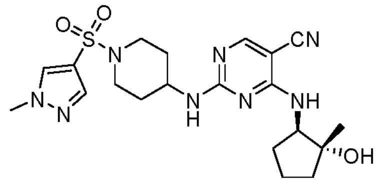
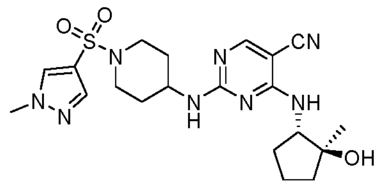

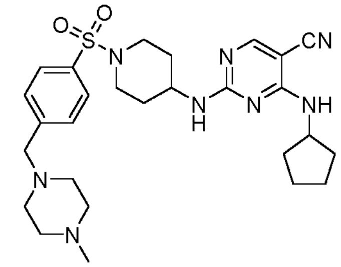
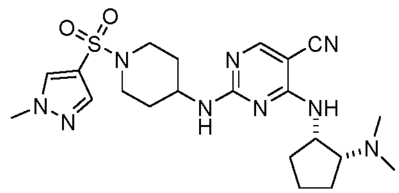

*cis*

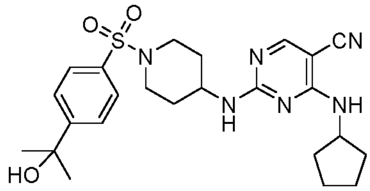

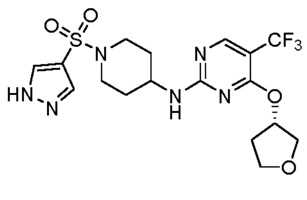
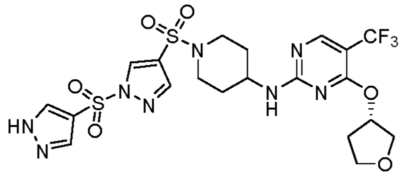
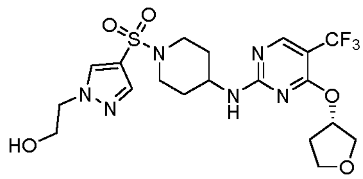

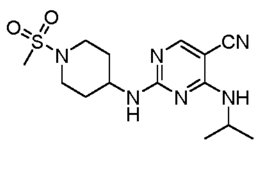
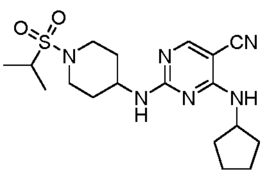
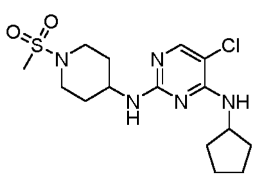
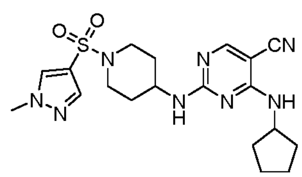

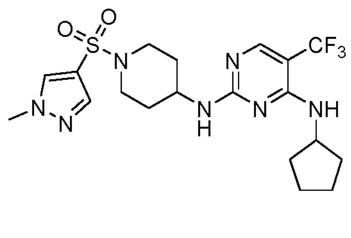
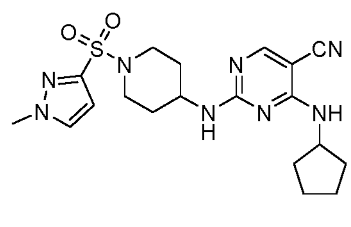
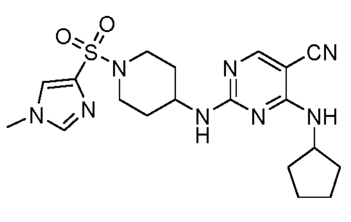

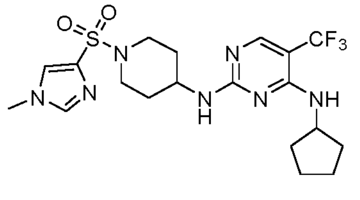
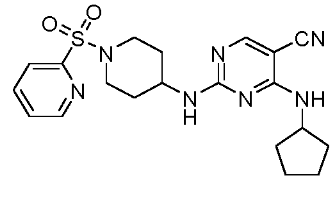
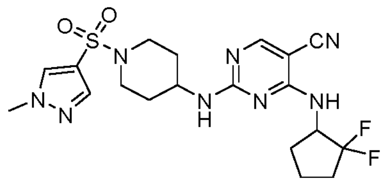

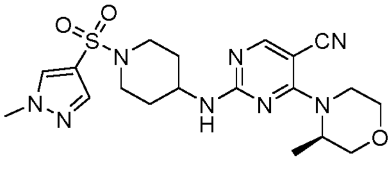
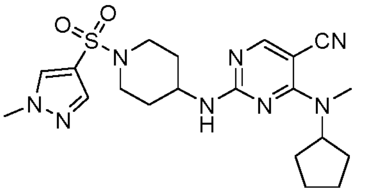
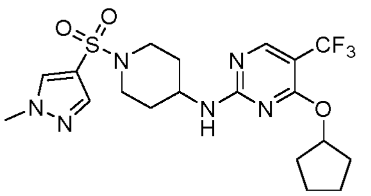

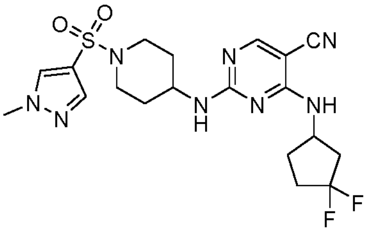
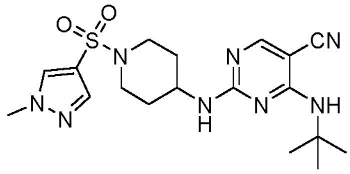
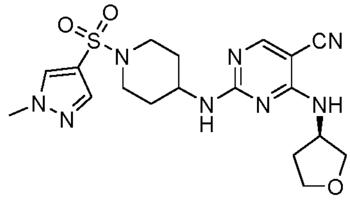

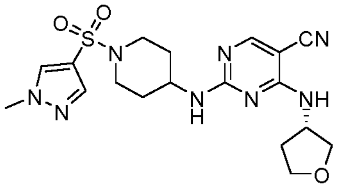
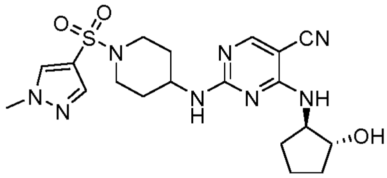
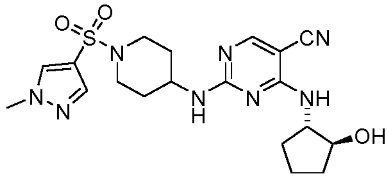

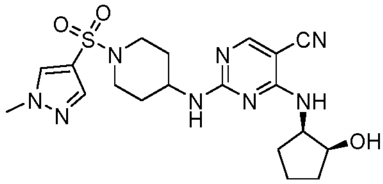
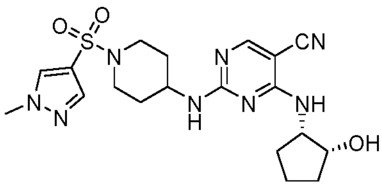
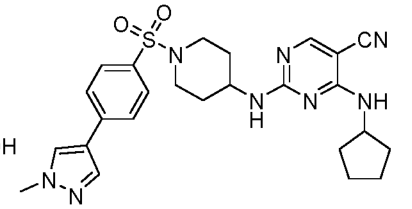

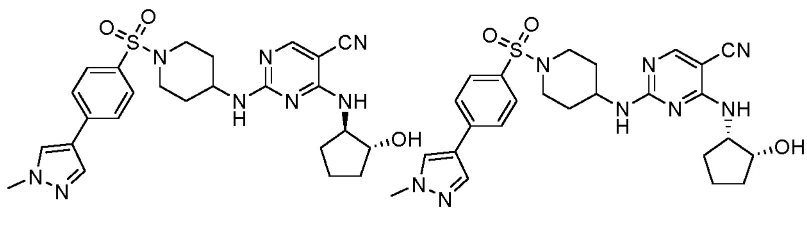
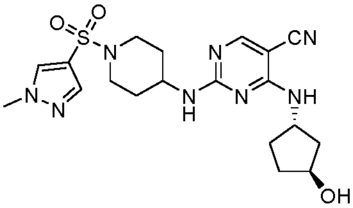

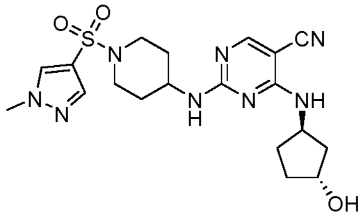
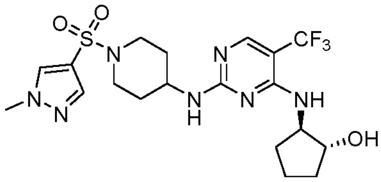
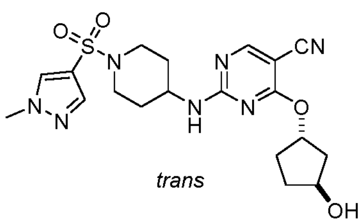

*trans*

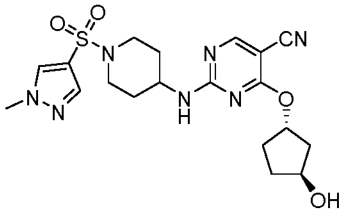

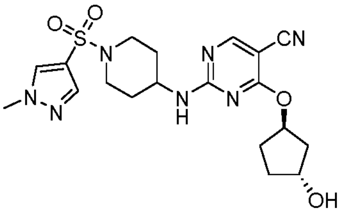

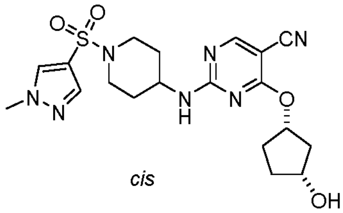

cis

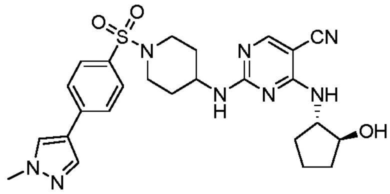

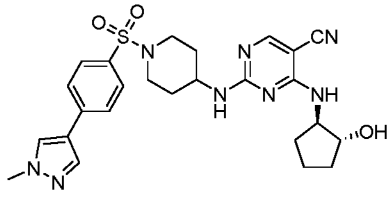

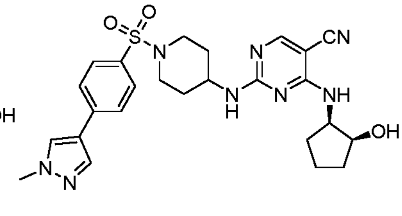

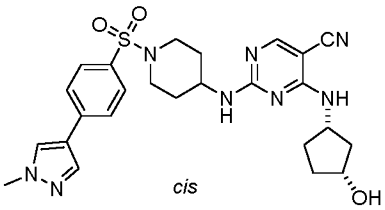

cis

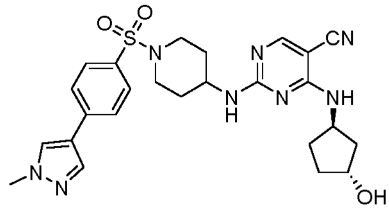

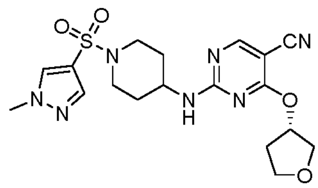

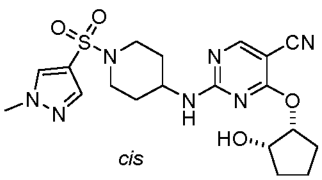

cis

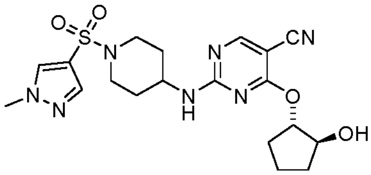

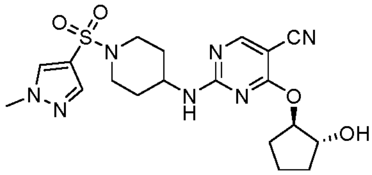

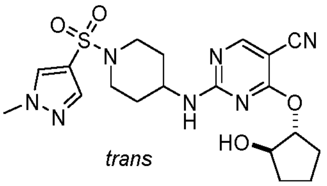

trans

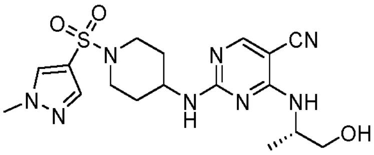

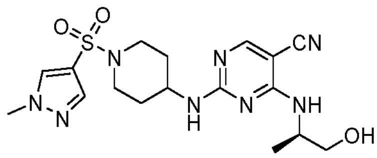

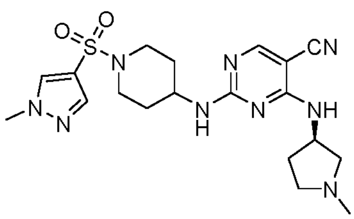

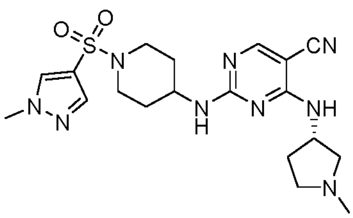

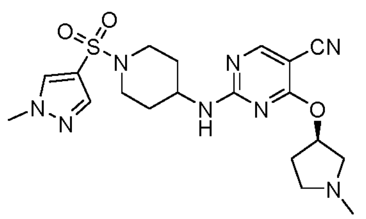

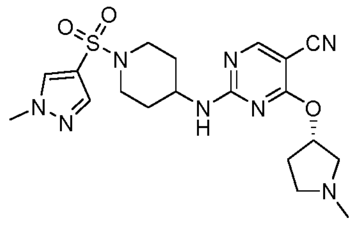
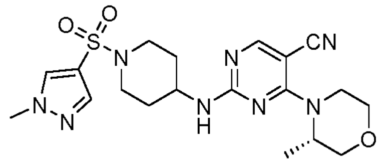
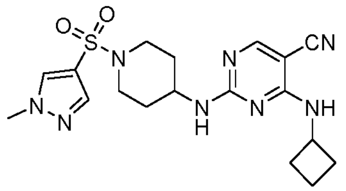

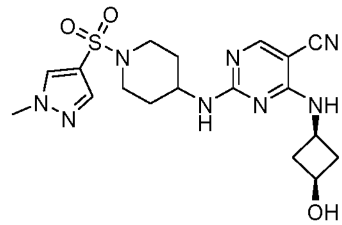
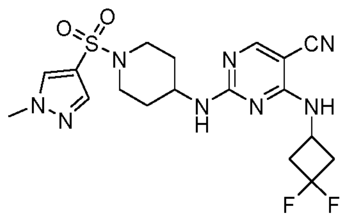
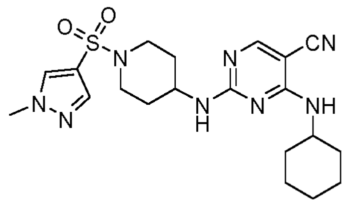

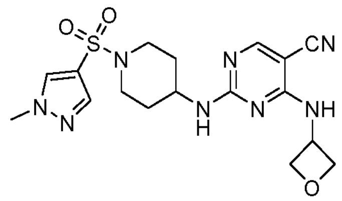
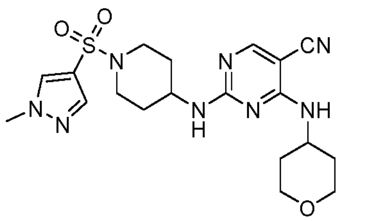
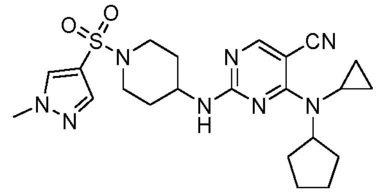

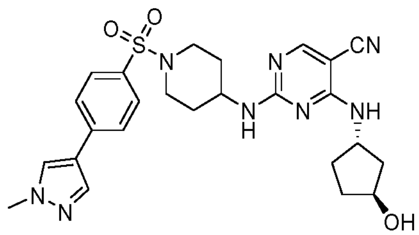
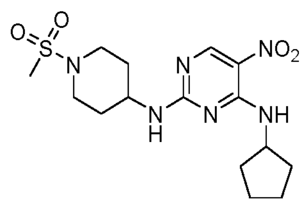
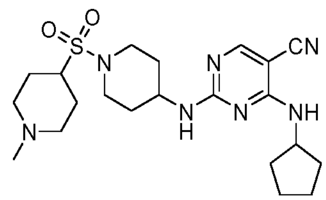

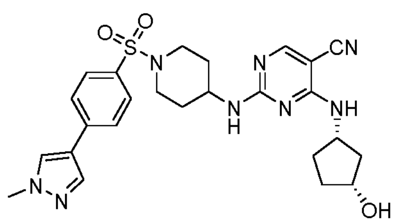
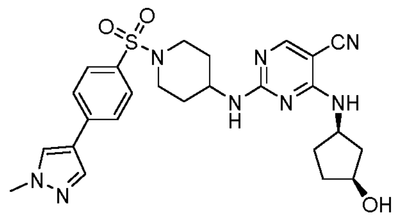
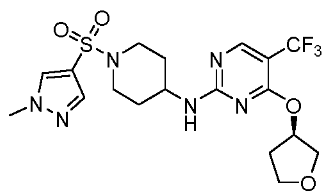

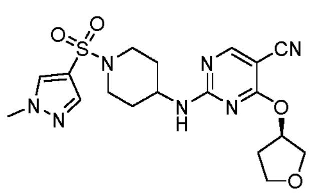
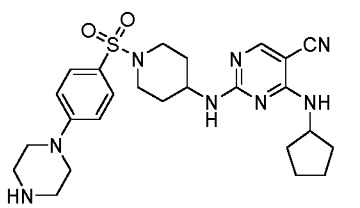
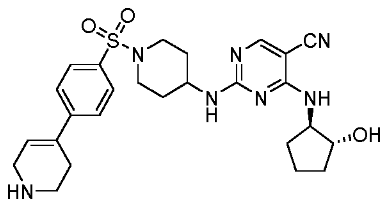

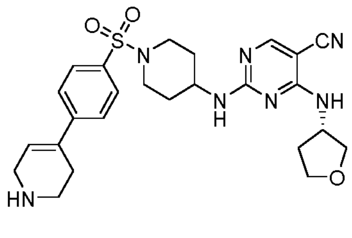
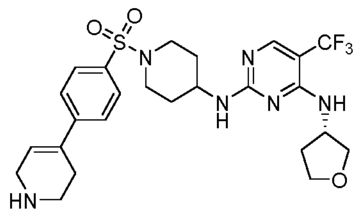
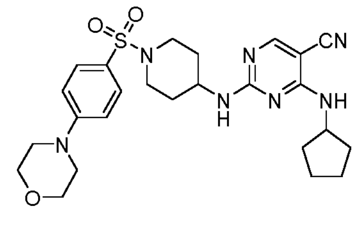

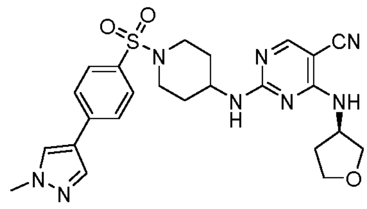
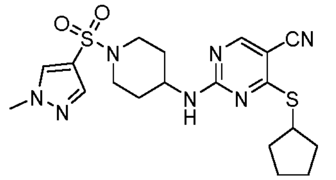
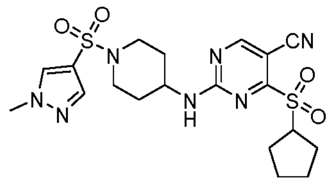

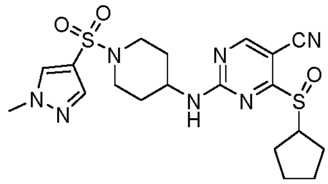
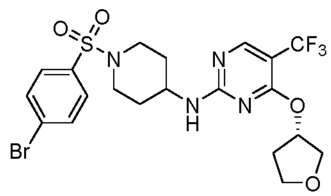
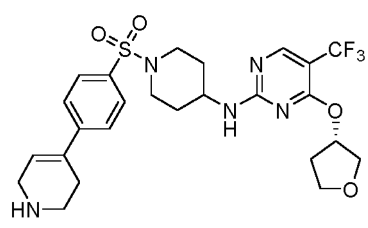

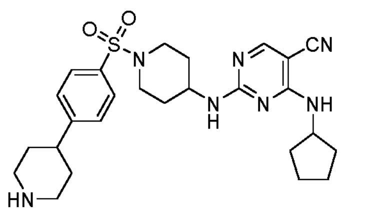
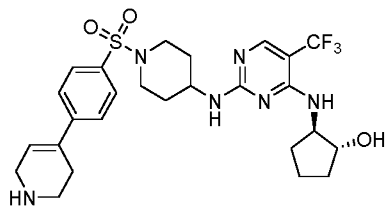
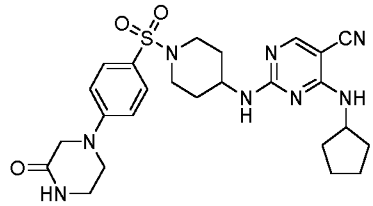

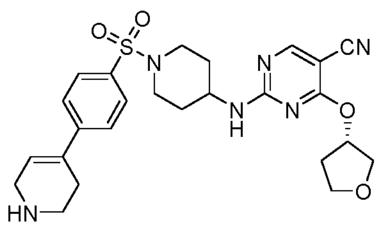
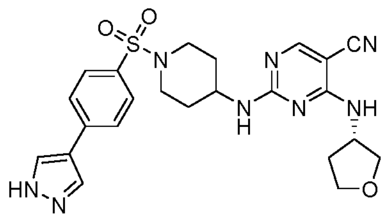
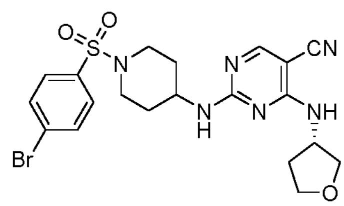

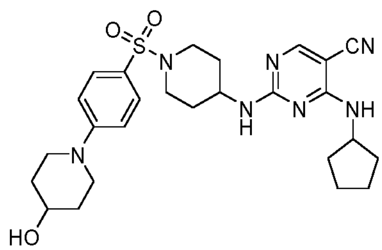
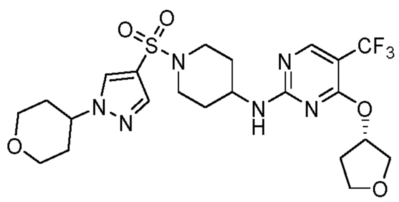
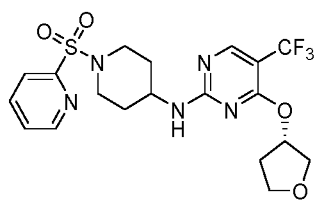

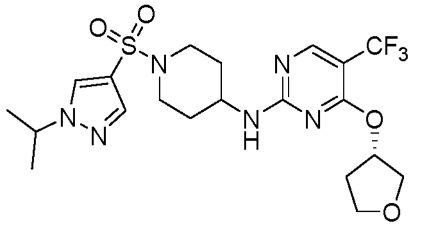
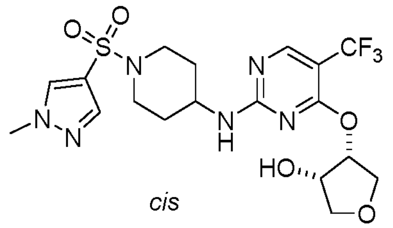

*cis*

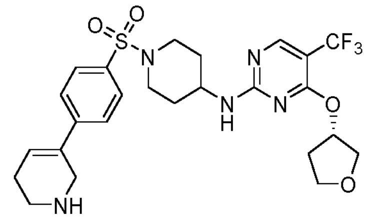
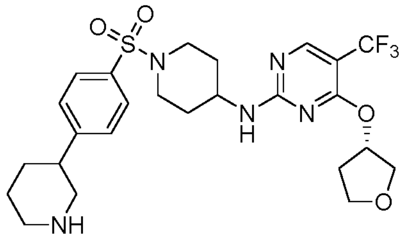
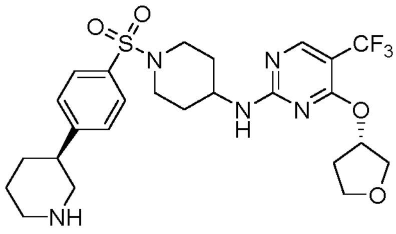

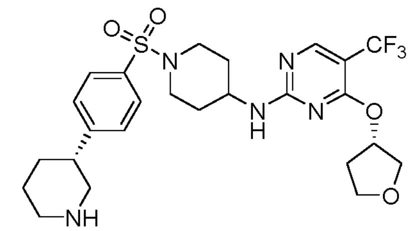
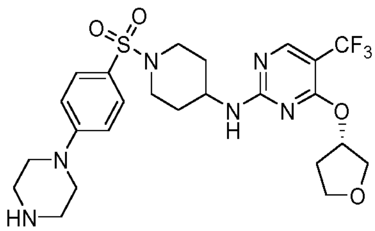
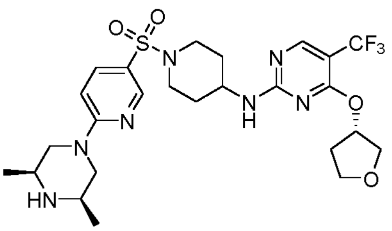

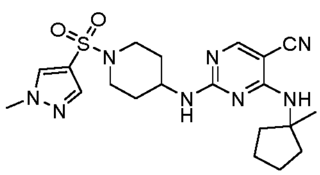
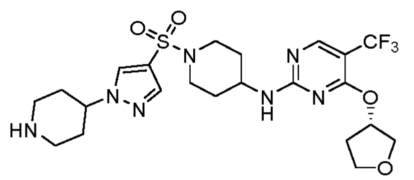
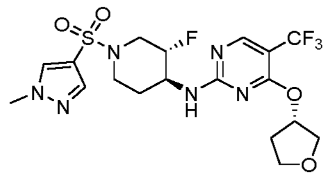

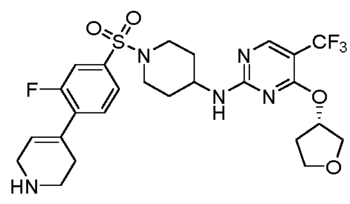
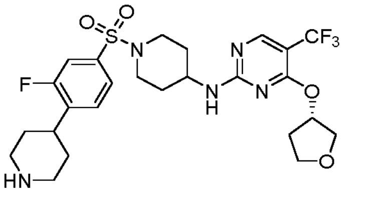
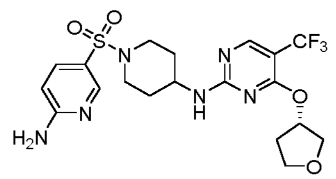

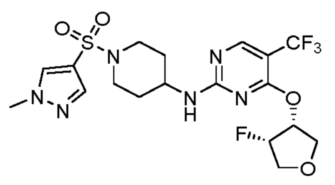
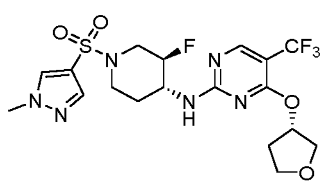
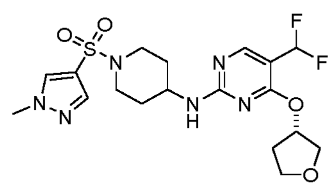

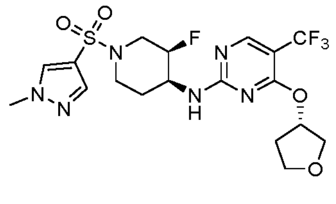
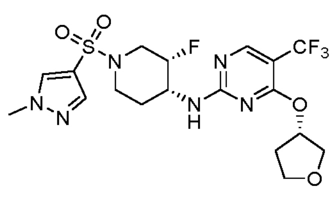

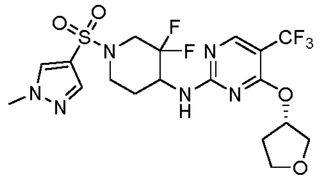
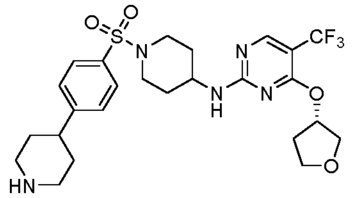
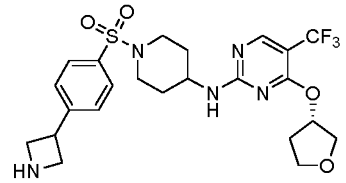
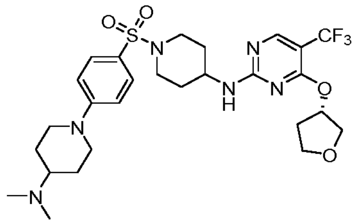
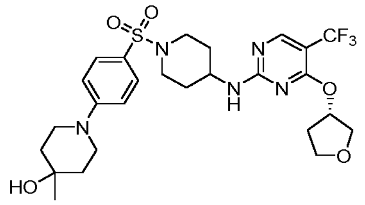
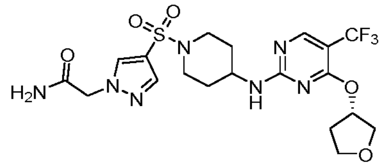
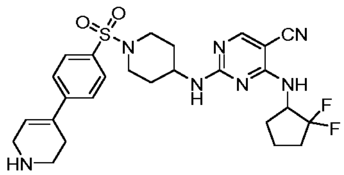
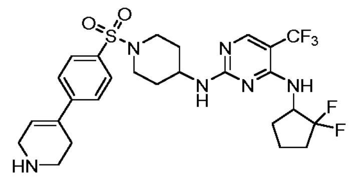
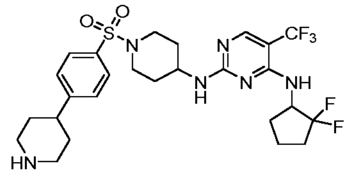
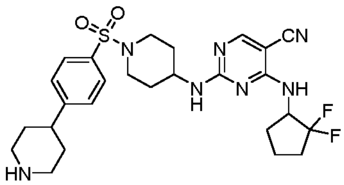
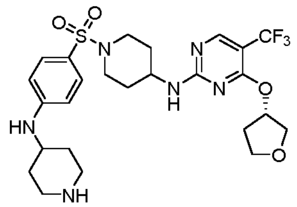
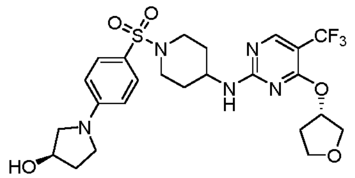
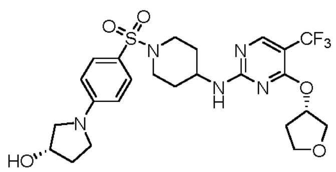
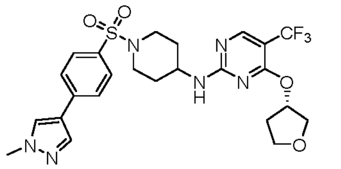
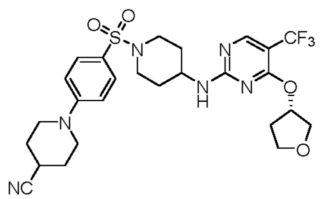
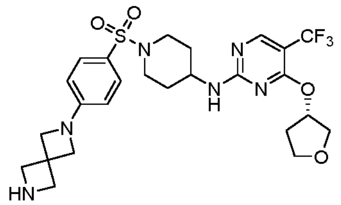
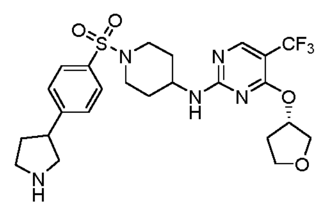
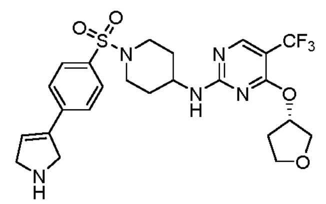
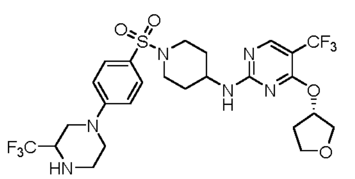
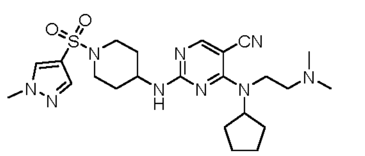
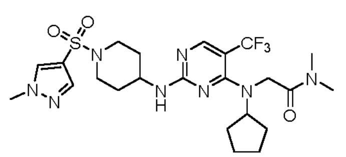

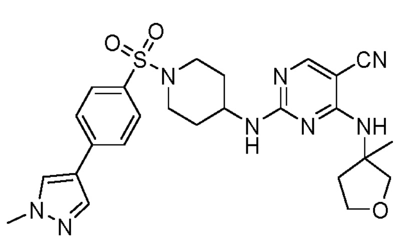
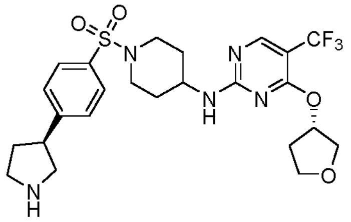
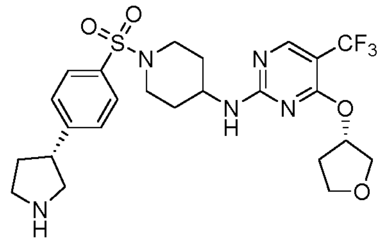
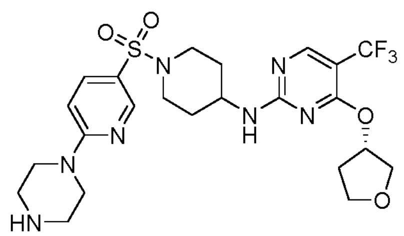
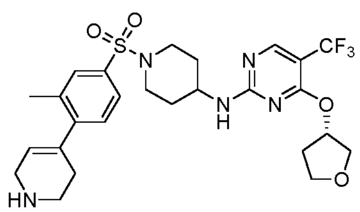
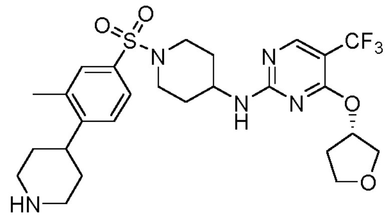
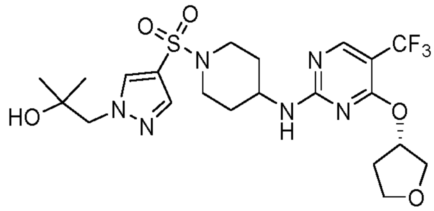
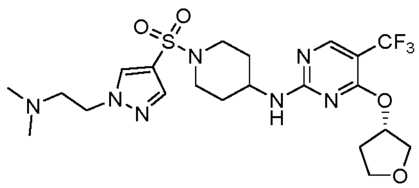
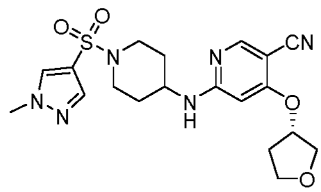
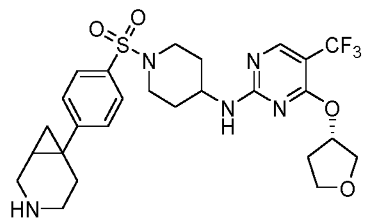
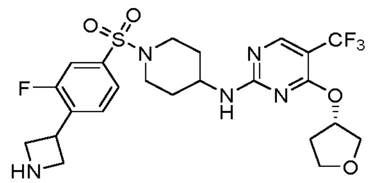
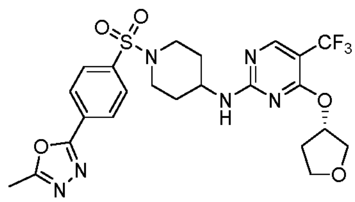
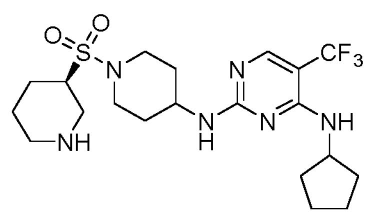
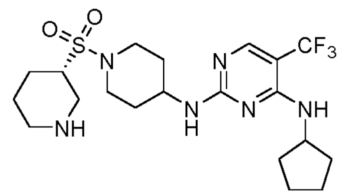
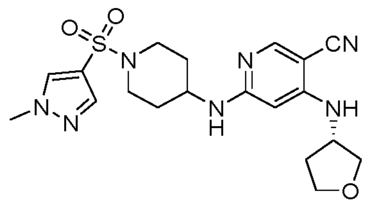
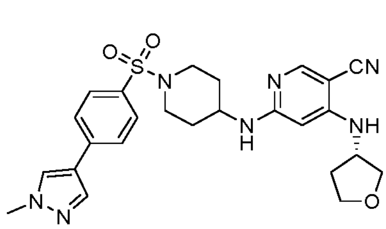
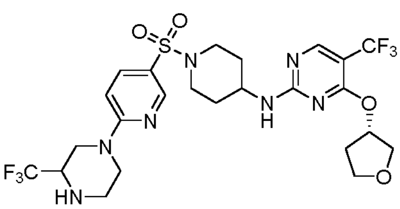
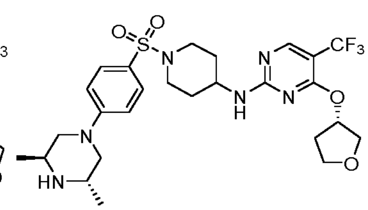
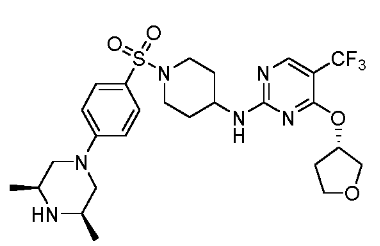
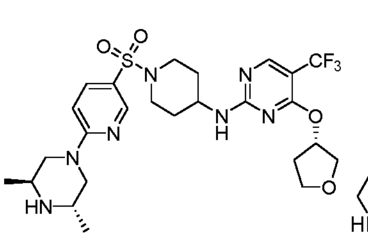
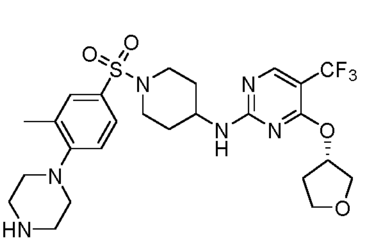

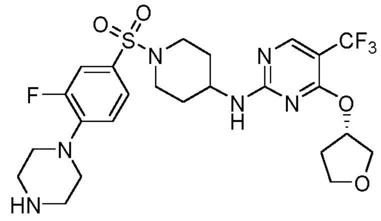
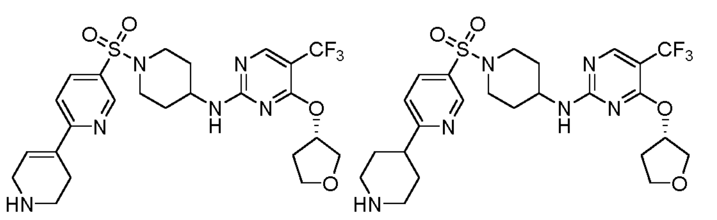

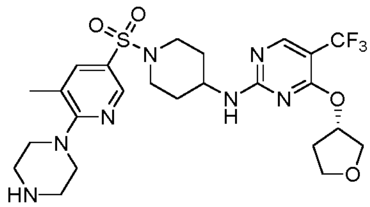
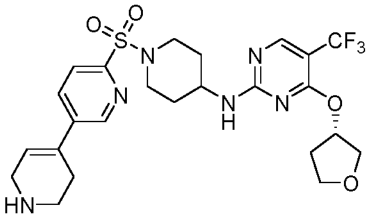
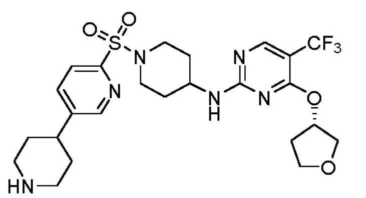

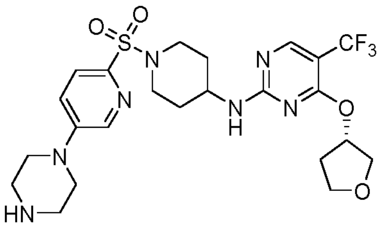
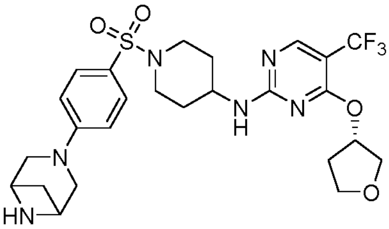
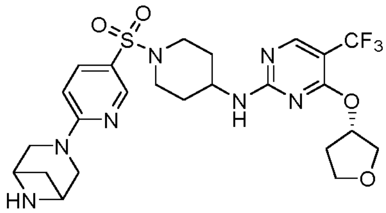

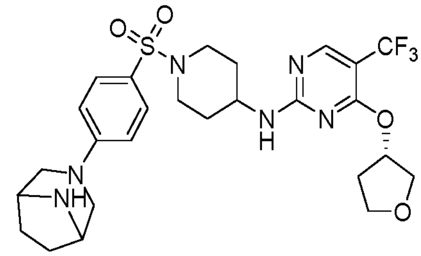
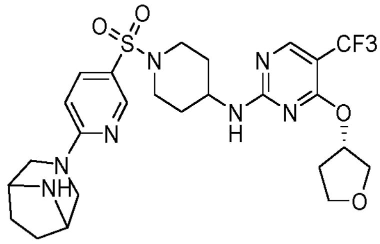
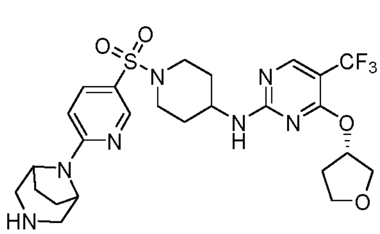

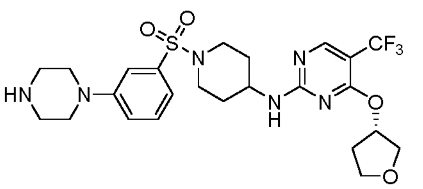
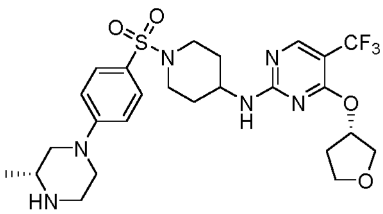
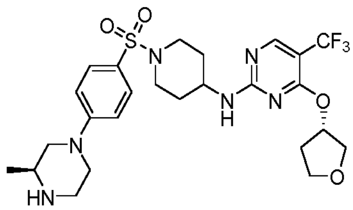

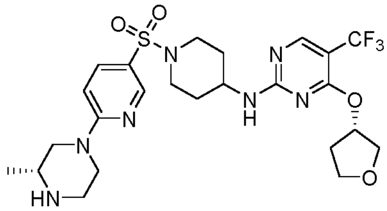
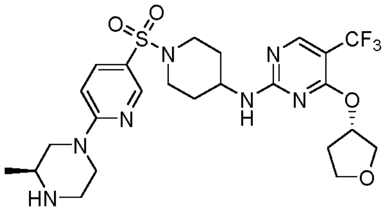
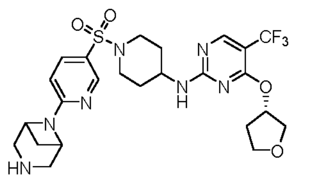

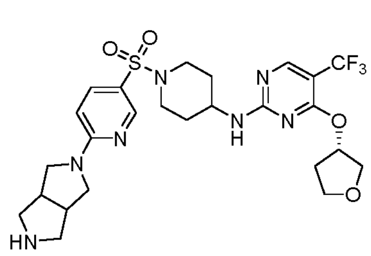
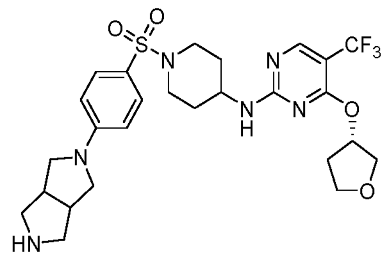
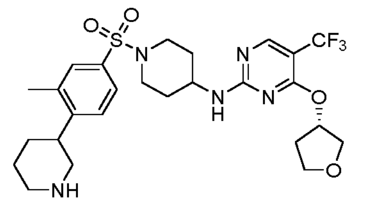

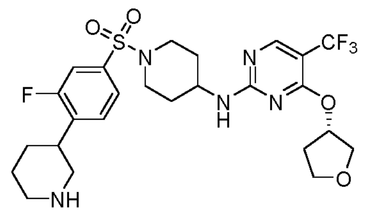
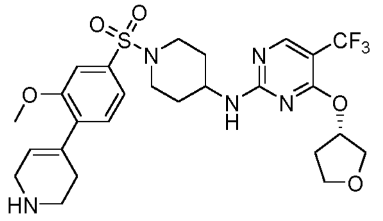
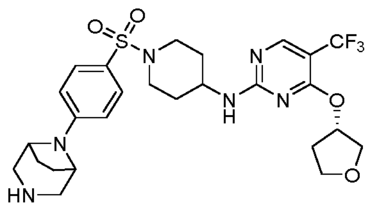

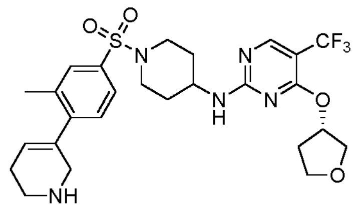
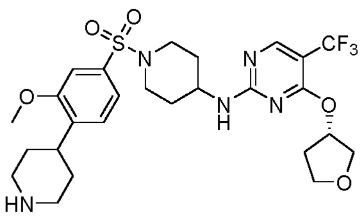
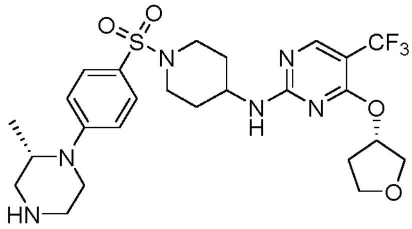

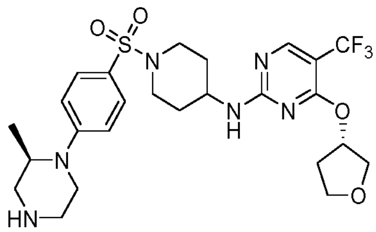,
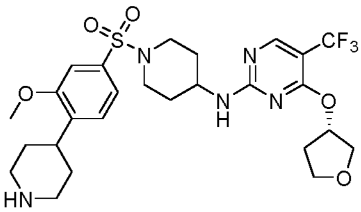,
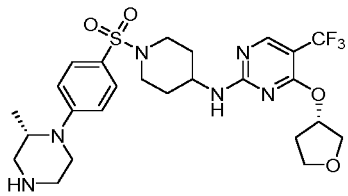,

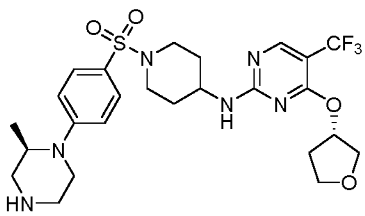,
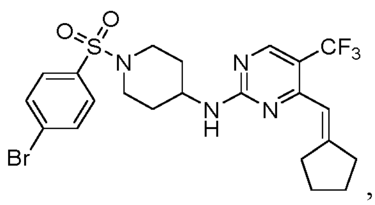,
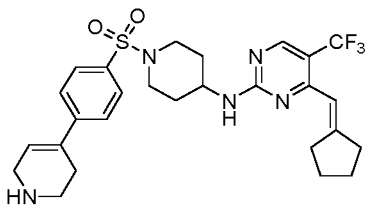,

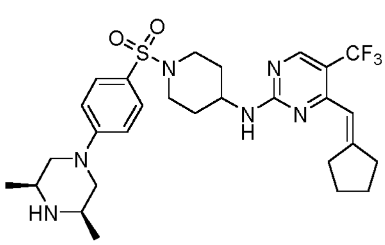,
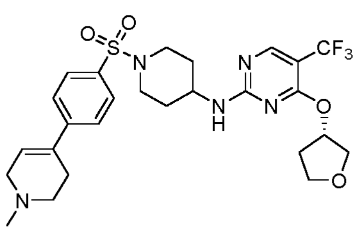,
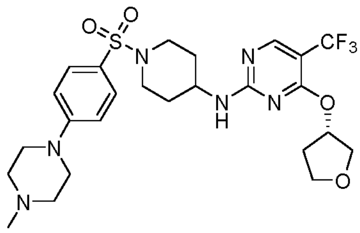,

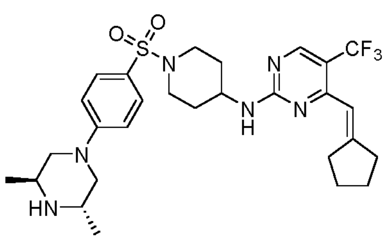, 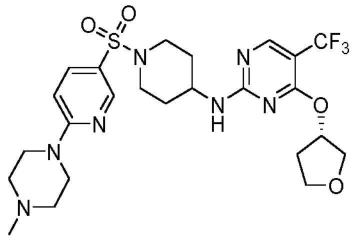, 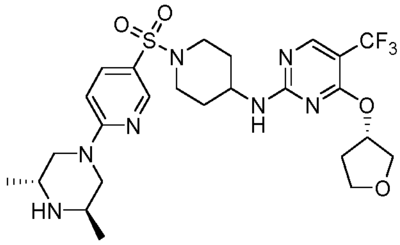,

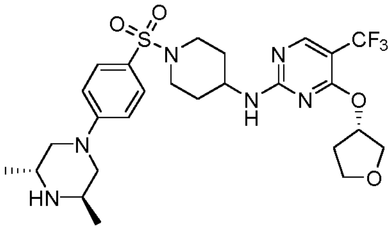, 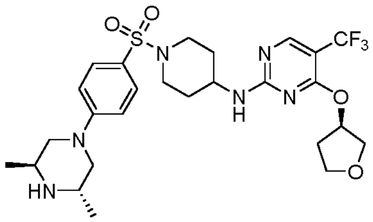, 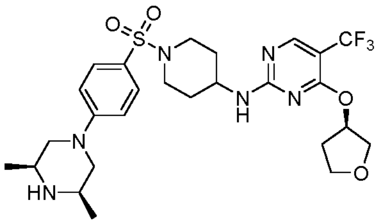.

**21.** A pharmaceutical composition comprising the compound of formula (I-A), the stereoisomer thereof, the tautomer thereof, the pharmaceutically acceptable salt thereof, the prodrug thereof, the hydrate thereof, the solvate thereof or the isotope-labeled derivative thereof according to any one of claims 1 to 20, and a pharmaceutically acceptable carrier, diluent or excipient.

**22.** A use of the compound of formula (I-A), the stereoisomer thereof, the tautomer thereof, the pharmaceutically acceptable salt thereof, the prodrug thereof, the hydrate thereof, the solvate thereof, or the isotope-labeled derivative thereof according to any one of claims 1 to 20, or the pharmaceutical composition according to claim 21 in the manufacture of a medicament, such as a medicament for the treatment of CDK-mediated cancer.

**23.** The use according to claim 22, wherein the cancer comprises ovarian cancer, breast cancer, acute myeloid leukemia (AML), chronic lymphocytic leukemia (CLL) or small lymphocytic lymphoma (SLL).

**24.** A compound of formula (III), a stereoisomer thereof or a pharmaceutically acceptable salt thereof,

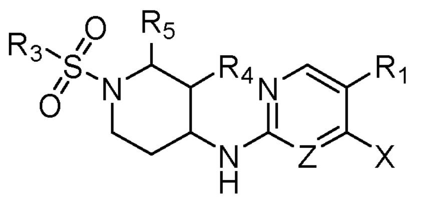

( III )

wherein

X is selected from halogen, OH, $-SO_2Me$, -OMs, OTf, OTs and H;
$R_1$, $R_3$, Z, $R_4$, $R_5$ are as defined in any one of claims 1 to 20.

**25.** The compound, the stereoisomer thereof, or the pharmaceutically acceptable salt thereof according to claim 24, wherein the compound is represented by any one of the following structures,

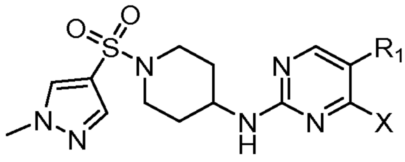

III-A

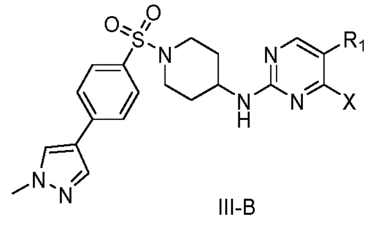

III-B

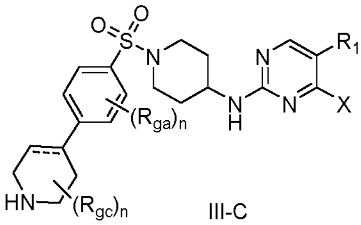

III-C

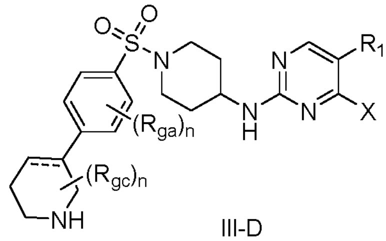

III-D

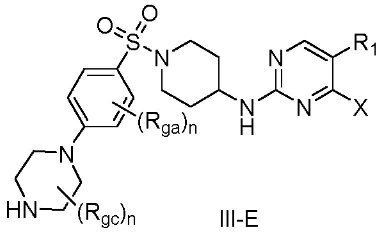

III-E

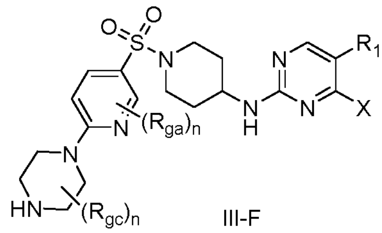

III-F

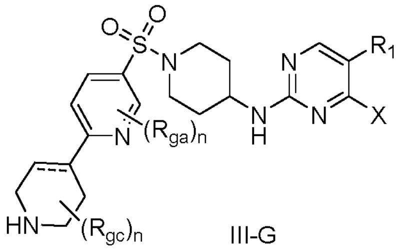

III-G

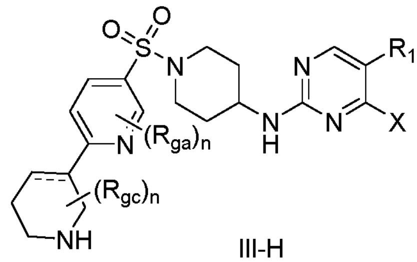

III-H

wherein $R_1$, $R_{ga}$, $R_{gc}$, n are as defined in any one of claims 1 to 20;
X is selected from halogen.

**26.** Compounds of formula (IV-1) and formula (IV-2), a stereoisomer thereof or a pharmaceutically acceptable salt thereof,

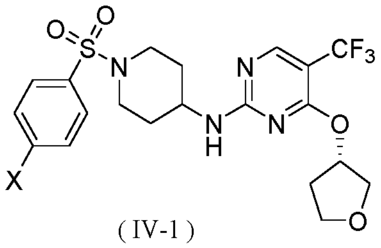

( IV-1 )

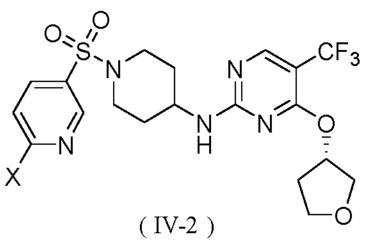

( IV-2 )

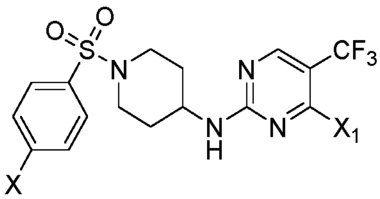

( IV-3 )

wherein X is selected from halogen, OH, $-SO_2Me$, -OMs, OTf, OTs, preferably halogen;
$X_1$ is selected from halogen, OH, $-SO_2Me$, -OMs, OTf, OTs, preferably halogen.

**27.** A use of the compound according to any one of claims 24 to 26 in the manufacture of the compound, the stereoisomer thereof, the tautomer thereof, the pharmaceutically acceptable salt thereof, the prodrug thereof, the hydrate thereof, the solvate thereof or the isotope-labeled derivative thereof according to any one of claims 1 to 20.

**INTERNATIONAL SEARCH REPORT**

International application No.
**PCT/CN2022/074491**

**A. CLASSIFICATION OF SUBJECT MATTER**

C07D 401/14(2006.01)i；C07D 403/12(2006.01)i；A61K 31/506(2006.01)i；A61P 35/00(2006.01)i

According to International Patent Classification (IPC) or to both national classification and IPC

**B. FIELDS SEARCHED**

Minimum documentation searched (classification system followed by classification symbols)

C07D，A61K，A61P

Documentation searched other than minimum documentation to the extent that such documents are included in the fields searched

Electronic data base consulted during the international search (name of data base and, where practicable, search terms used)

EPODOC; WPI; CNABS; EPTXT; USTXT; WOTXT; JPTXT; CNTXT; CNKI, STN: 齐鲁制药, 严小霞, 孙大庆, 哌啶, 磺酰, 吡啶, 嘧啶, CDK, 癌, 肿瘤, +piperidin+, +sulfon+, +pyridin+, +pyrimidin+

**C. DOCUMENTS CONSIDERED TO BE RELEVANT**

| Category* | Citation of document, with indication, where appropriate, of the relevant passages | Relevant to claim No. |
|---|---|---|
| X | "CAS RN 2463538-93-2 et al." *CAS STN REGISTRY,* 27 August 2020 (2020-08-27), L20 ANSWER 6, 9, 11, 13-14, 16, 21-23, 27-28, 31 | 1-2, 6-9, 11-16 |
| X | WO 2020180959 A1 (INCYTE CORPORATION et al.) 10 September 2020 (2020-09-10) description pages 2-4, 86-87, 138, 143, 152-153, description embodiments, claims 37-45 | 24 |
| X | CN 101490041 A (ASTRAZENECA AB) 22 July 2009 (2009-07-22) see description pages 2-6, description embodiments 119-123, 130-134, 138-140, 143-148, 154-155, 157-159, 185-187, 202-207, claims 12-23 | 1-16, 21-27 |
| X | CN 101511824 A (ASTRAZENECA AB) 19 August 2009 (2009-08-19) description embodiments 41, 43, 44, claims 40-42 | 1-2, 6-7, 11-16, 21-23 |
| X | CN 101044121 A (HOFFMANN LA ROCHE) 26 September 2007 (2007-09-26) description pages 11-16, 40-41, description embodiment | 1-27 |
| X | US 2004162303 A1 (HOFFMANN-LA ROCHE INC.) 19 August 2004 (2004-08-19) description pages 3-5, 17-18, description embodiment | 1-27 |
| X | US 2006014708 A1 (HOFFMANN-LA ROCHE INC. et al.) 19 January 2006 (2006-01-19) description pages 2-4, description embodiments, claims 22-24 | 1-27 |

☑ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 March 2022** | **28 April 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

INTERNATIONAL SEARCH REPORT

International application No.
PCT/CN2022/074491

C. DOCUMENTS CONSIDERED TO BE RELEVANT

| Category* | Citation of document, with indication, where appropriate, of the relevant passages | Relevant to claim No. |
|---|---|---|
| PX | WO 2021170076 A1 (FOCHON PHARMACEUTICALS LTD.) 02 September 2021 (2021-09-02)<br>description embodiment 14 compound 14b | 1-2, 6-14 |
| PX | US 2021047294 A1 (Incyte Corporation) 18 February 2021 (2021-02-18)<br>description pages 1-10, description embodiment | 1-16, 21-27 |
| PX | "CAS RN 2728273-28-5"<br>*CAS STN REGISTRY,* 10 November 2021 (2021-11-10),<br>L20 ANSWER 4 | 1-2, 6-9, 11-14 |

Form PCT/ISA/210 (second sheet) (January 2015)

**INTERNATIONAL SEARCH REPORT**
**Information on patent family members**

International application No.
**PCT/CN2022/074491**

| Patent document cited in search report | Publication date (day/month/year) | Patent family member(s) | Publication date (day/month/year) |
|---|---|---|---|
| WO 2020180959 A1 | 10 September 2020 | TW 202100520 A | 01 January 2021 |
| | | US 2021017156 A1 | 21 January 2021 |
| CN 101490041 A | 22 July 2009 | None | |
| CN 101511824 A | 19 August 2009 | MX 2008015721 A | 08 January 2009 |
| | | IL 195665 D0 | 01 September 2009 |
| | | RU 2008148903 A | 10 August 2010 |
| | | US 2008188503 A1 | 07 August 2008 |
| | | JP 2009542639 A | 03 December 2009 |
| | | CL 2007001882 A1 | 08 February 2008 |
| | | NO 20090328 L | 26 January 2009 |
| | | BR PI0713578 A2 | 23 October 2012 |
| | | AR 061653 A1 | 10 September 2008 |
| | | UY 30438 A1 | 31 January 2008 |
| | | CA 2655444 A1 | 03 January 2008 |
| | | EC SP088974 A | 30 January 2009 |
| | | TW 200815417 A | 01 April 2008 |
| | | EP 2046783 A2 | 15 April 2009 |
| | | AU 2007265732 A1 | 03 January 2008 |
| | | ZA 200810577 B | 26 August 2009 |
| | | KR 20090024295 A | 06 March 2009 |
| | | WO 2008002245 A2 | 03 January 2008 |
| CN 101044121 A | 26 September 2007 | ZA 200505564 A | 31 January 2007 |
| | | CO 5590920 A2 | 30 December 2005 |
| US 2004162303 A1 | 19 August 2004 | US 2006229330 A1 | 12 October 2006 |
| | | TN SN05188 A1 | 11 June 2007 |
| | | MA 27716 A1 | 02 January 2006 |
| | | EP 1628619 A2 | 01 March 2006 |
| | | NO 20053410 L | 08 September 2005 |
| | | EA 200501233 A1 | 28 April 2006 |
| | | AU 2004210408 A1 | 19 August 2004 |
| | | RS 20050610 A | 31 December 2007 |
| | | PL 382309 A1 | 20 August 2007 |
| | | HR P20050689 A2 | 31 August 2006 |
| | | BR PI0407381 A | 07 February 2006 |
| | | JP 2006523183 A | 12 October 2006 |
| | | MY 137555 A | 27 February 2009 |
| | | AR 043693 A1 | 10 August 2005 |
| | | MX PA05008333 A | 30 September 2005 |
| | | WO 2004069139 A2 | 19 August 2004 |
| | | EC SP055958 A | 16 January 2006 |
| | | CA 2512915 A1 | 19 August 2004 |
| | | CL 2004000226 A1 | 14 January 2005 |
| | | IL 169626 D0 | 04 July 2007 |
| | | TW 200423940 A | 16 November 2004 |
| | | KR 20050099544 A | 13 October 2005 |
| US 2006014708 A1 | 19 January 2006 | KR 20070028539 A | 12 March 2007 |
| | | MX 2007000574 A | 07 March 2007 |
| | | DE 602005017152 D1 | 26 November 2009 |
| | | AT 445604 T | 15 October 2009 |

Form PCT/ISA/210 (patent family annex) (January 2015)

**INTERNATIONAL SEARCH REPORT**
**Information on patent family members**

International application No.
**PCT/CN2022/074491**

| Patent document cited in search report | Publication date (day/month/year) | Patent family member(s) | Publication date (day/month/year) |
|---|---|---|---|
| | | CA 2573604 A1 | 19 January 2006 |
| | | RU 2007105558 A | 20 August 2008 |
| | | AU 2005261899 A1 | 19 January 2006 |
| | | BR PI0513366 A | 06 May 2008 |
| | | EP 1771420 A1 | 11 April 2007 |
| | | AR 050427 A1 | 25 October 2006 |
| | | CN 1980893 A | 13 June 2007 |
| | | TW 200612929 A | 01 May 2006 |
| | | WO 2006005548 A1 | 19 January 2006 |
| | | JP 2008505955 A | 28 February 2008 |
| | | ES 2330765 T3 | 15 December 2009 |
| WO 2021170076 A1 | 02 September 2021 | None | |
| US 2021047294 A1 | 18 February 2021 | WO 2021030537 A1 | 18 February 2021 |
| | | TW 202115024 A | 16 April 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110161786
- CN 202110483256
- CN 202111062178
- CN 202111398260
- CN 202210048365


### Non-patent literature cited in the description


- **HARPER, J. W. ; ADAMS, P. D.** Cyclin-Dependent Kinases. *Chem. Rev.,* 2001, vol. 101, 2511-2526
- **TADESSE S ; ANSHABO AT ; PORTMAN N ; LIM E ; TILLEY W ; CALDON CE ; WANG S.** Targeting CDK2 in cancer: challenges and opportunities for therapy. *Drug Discovery Today,* 2020, vol. 25, 406-413